# EUROPEAN PATENT APPLICATION

(11) **EP 2 873 672 A1**
(43) Date of publication of application: **20.05.2015**
(21) Application number: 13796871.5
(22) Date of filing: 15.02.2013
(51) Int. Cl.: C07F 7/08, C07D 303/12, C07D 407/12

(54) **COMPOSITION AND CURED ARTICLE COMPRISING INORGANIC PARTICLES AND EPOXY COMPOUND HAVING ALKOXYSILYL GROUP, USE FOR SAME, AND PRODUCTION METHOD FOR EPOXY COMPOUND HAVING ALKOXYSILYL GROUP**

(30) Priority: 01.06.2012 KR 20120059437; 06.07.2012 KR 20120074197; 01.02.2013 KR 20130011711
(71) Applicant: Korea Institute of Industrial Technology, Cheonan, Choongcheongnam-do 330-825 (KR)
(72) Inventor: CHUN, Hyun-Aee, Seongnam-si Gyeonggi-do 463-773 (KR); PARK, Su-Jin, Ansan-si Gyeonggi-do 426-861 (KR); PARK, Sook-Yeon, Gunpo-si Gyeonggi-do 435-756 (KR); KIM, Yun-Ju, Seoul 135-796 (KR); TAK, Sang-Yong, Busan 600-083 (KR); PARK, Sung-Hwan, Gunpo-si Gyeonggi-do 435-751 (KR); KANG, Kyung-Nam, Ansan-si Gyeonggi-do 426-896 (KR)
(74) Representative: Michalski Hüttermann & Partner Patentanwälte mbB
(86) International application number: PCT/KR2013/001211
(87) International publication number: WO 2013/180375

(57) **Abstract**

The present invention relates to: a composition and a cured article comprising inorganic particles together with an alkoxysilyl-based epoxy compound which does not require a separate silane coupling agent and exhibits outstanding heat resistance, and more specifically a low coefficient of thermal expansion (CTE) and a high glass transition temperature or no Tg in a composite material; and a use for same. The present invention provides: inorganic particles and an alkoxysilyl-based epoxy compound; and an epoxy composition comprising an epoxy compound, inorganic particles and a curing agent; and a cured article of same and a use for same. A composite article of the composition comprising the inorganic particles and the alkoxysilyl-based epoxy compound of the present invention exhibits outstanding heat resistance, which is to say a low CTE and high glass transition temperature or no Tg, as the chemical bonding efficiency is improved during epoxy composite formation, not only due to chemical bonding of a filler material and the alkoxysilyl groups in the alkoxysilyl-based epoxy compound but also due to chemical bonding between the alkoxysilyl groups of the alkoxysilyl-based epoxy compound.

## Description

### [Technical Field]

The present disclosure relates to a composition including an epoxy compound containing an alkoxysilyl group (hereinafter 'alkoxysilylated epoxy compound') exhibiting good heat resistance property and inorganic particles, a cured product formed of the composition, a use of the cured product, and a method of preparing the epoxy compound containing an alkoxysilyl group. More particularly, the present disclosure relates to a composition including an alkoxysilylated epoxy compound, a composite of which exhibits good heat resistance property, in particular, exhibiting a low coefficient of thermal expansion (CTE) and a high increasing effect of glass transition temperature(including a transition temperature-less (Tg-less) compound, not having a glass transition temperature) and not requiring a additional coupling agent, a cured product formed of the composition, a use of the cured product, and a method of preparing the epoxy compound containing an alkoxysilyl group.

### [Background Art]

The coefficient of thermal expansion (CTE) of a polymer material - specifically, a cured product formed of an epoxy compound - is about 50 to 80 ppm/°C, a significantly high level, on the level of several to ten times of the CTE of a inorganic material such as ceramic material or a metal, (for example, the CTE of silicon is 3 to 5 ppm/°C, while the CTE of copper is 17 ppm/°C). Thus, when the polymer material is used in conjunction with an inorganic material or metal in a semiconductor, a display, or the like, the properties and processability of the polymer material are significantly limited due to the different CTEs of the polymer material and the inorganic material or the metal material. In addition, during semiconductor packaging in which a silicon wafer and a polymer substrate are used side by side, or during a coating process in which a polymer film is coated with an inorganic shielding layer to impart gas barrier property, product defects such as the generation of cracks in an inorganic layer, the warpage of a substrate, the peeling of a coating layer, the failure of a substrate, and the like, may be generated due to a large CTE-mismatch between constituent elements due to changes in processing and/or applied temperature conditions.

Because of the high CTE of the polymer material and the resultant dimensional change of the polymer material, the development of technologies such as next generation semiconductor substrates, printed circuit boards (PCBs), packaging, organic thin film transistors (OTFTs), and flexible display substrates may be limited. Particularly, at the current time, in the semiconductor and PCB fields, designers are facing challenges in the design of next generation parts requiring high degrees of integration, miniaturization, flexibility, performance, and the like, in securing processability and reliability in parts due to polymer materials having significantly high CTE as compared to metal/ceramic materials. In other words, due to the high thermal expansion property of the polymer material at part processing temperatures, defects may be generated, processability may be limited, and the design of the parts and the securing of processability and reliability therein may be objects of concern. Accordingly, improved thermal expansion property or dimensional stability of the polymer material are necessary in order to secure processability and reliability in electronic parts.

In general, in order to improve thermal expansion property -i.e., to obtain a low CTE in a polymer material such as an epoxy compound, (1) a method of producing a composite of the epoxy compound with inorganic particles (an inorganic filler) and/or fibers and (2) a method of designing a novel epoxy compound containing a decreased CTE have been used.

When the composite of the epoxy compound and the inorganic particles as the filler is formed in order to improve thermal expansion property, a large amount of inorganic silica particles, having a diameter of about 2 to 30 µm is required to be used to obtain a CTE decrease effect. However, due to the presence of the large amount of inorganic particles, the processability and physical properties of the parts may be deteriorated. That is, the presence of the large amount of inorganic particles may decrease fluidity, and voids may be generated during the filling of narrow spaces. In addition, the viscosity of the material may increase exponentially due to the addition of the inorganic particles. Further, the size of the inorganic particles tends to decrease due to semiconductor structure miniaturization. When a filler having a particle size of 1 µm or less is used, the decrease in fluidity (viscosity increase) may be worsened. When inorganic particles having a large average particle diameter are used, the frequency of insufficient filling in the case of a composition including a resin and the inorganic particles may increase. While the CTE may largely decrease when a composition including an organic resin and a fiber as the filler is used, the CTE may remain high as compared to that of a silicon chip or the like.

As described above, the manufacturing of highly integrated and high performance electronic parts for next generation semiconductor substrates, PCBs, and the like, may be limited due to the limitations in the technology of the combination of epoxy compounds. Thus, the development of a polymer composite having improved heat resistance property - namely, a low CTE and a high glass transition temperature - is required to overcome the challenge of a lack of heat resistance property due to a high CTE and processability of a common thermosetting polymer composite.

### [Disclosure]

### [Technical Problem]

An aspect of the present disclosure may provide an epoxy composition, a composite of which exhibits good heat resistance property, particularly, a low CTE and high glass transition temperature properties.

An aspect of the present disclosure may also provide a cured product formed of an epoxy composition in accordance with an exemplary embodiment, a composite of which exhibits good heat resistance property, particularly, a low CTE and high glass transition resistance property.

An aspect of the present disclosure may also provide a use of an epoxy composition in accordance with an exemplary embodiment.

An aspect of the present disclosure may also provide a method of preparing an epoxy compound containing an alkoxysilyl group.

### [Technical Solution]

According to a first aspect of the present disclosure, at least one epoxy composition includes an epoxy compound containing an alkoxysilyl group selected from the group consisting of the following Formulae AI to KI and inorganic particles. 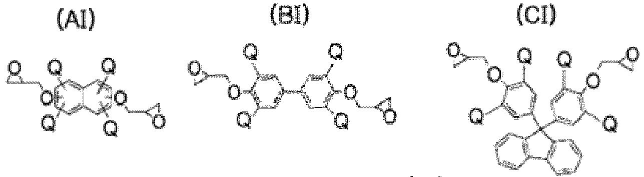 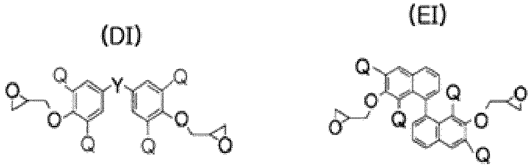 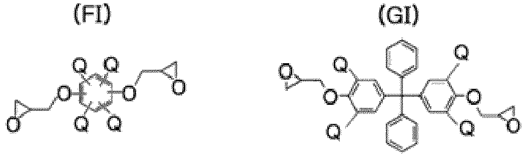 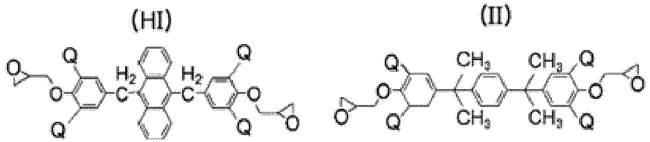 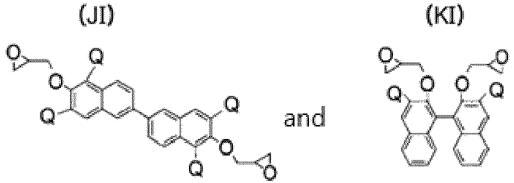 in the above Formulae AI to KI, at least one of a plurality of Q has the form of the following Formula S1, and the remainder thereof are independently selected from the group consisting of the following Formula S3, hydrogen, and -CR_{b}R_{c}-CRₐ=CH₂, where Rₐ, R_{b} and R_{c} are independently H, or an alkyl group having 1 to 6 carbon atoms, and the alkyl group may be a linear chain or a branched chain alkyl group,
in the above DI, Y is -CH₂, -C(CH₃)₂-, -C(CF₃)₂-, -S-, or -SO₂-.

[Formula S1] -CR_{b}R_{c}-CHRₐ-CH₂-SiR₁R₂R₃

in Formula S1, Rₐ, R_{b} and R_{c} are independently H, or an alkyl group having 1 to 6 carbon atoms, at least one of R₁ to R₃ is an alkoxy group having 1 to 6 carbon atoms, and the remainder thereof are alkyl groups having 1 to 10 carbon atoms, while the alkyl group and the alkoxy group may be a linear chain or a branched chain alkyl group or alkoxy group. In the case in which Formula FI includes one instance of Formula S1, a compound in which all of Rₐ, R_{b} and R_{c} in the above Formula S1 are hydrogen, and R₁ to R₃ are alkoxy groups having 1 to 6 carbon atoms is excluded. 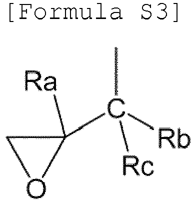 in Formula S3, Rₐ, R_{b} and R_{c} are independently H, or an alkyl group having 1 to 6 carbon atoms, and the alkyl group is a linear chain or a branched chain alkyl group.

According to a second aspect of the present disclosure, at least one epoxy composition may include an epoxy compound containing an alkoxysilyl group selected from the group consisting of the following Formulae AI to KI, inorganic particles, and a curing agent. 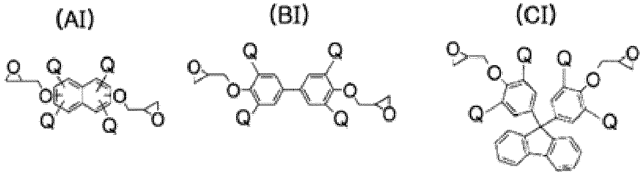 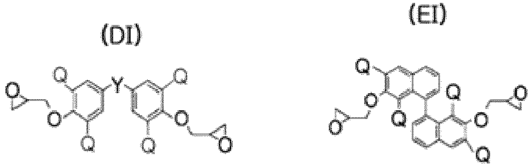 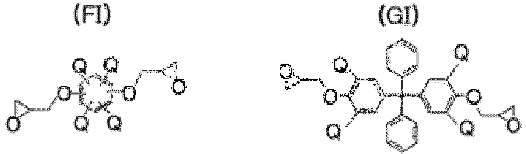 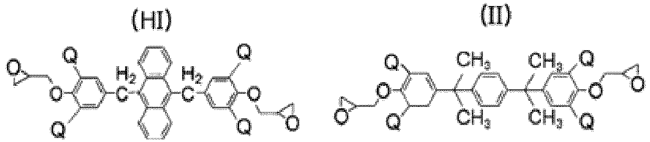 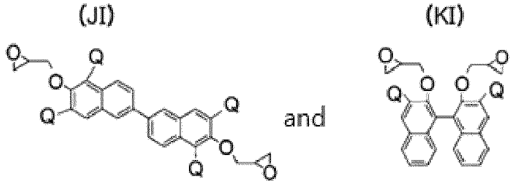 in the above Formulae AI to KI, at least one of a plurality of Q has the form of the following Formula S1, and the remainder thereof are independently selected from the group consisting of the following Formula S3, hydrogen, and -CR_{b}R_{c}-CRₐ=CH₂, where Rₐ, R_{b} and R_{c} are independently H, or an alkyl group having 1 to 6 carbon atoms, and the alkyl group may be a linear chain or a branched chain alkyl group,
in the above DI, Y is -CH₂, -C(CH₃)₂-, -C(CF₃)₂-, -S-, or -SO₂-.

[Formula S1] -CR_{b}R_{c}-CHRₐ-CH₂-SiR₁R₂R₃

in Formula S1, Rₐ, R_{b} and R_{c} are independently H, or an alkyl group having 1 to 6 carbon atoms, at least one of R₁ to R₃ is an alkoxy group having 1 to 6 carbon atoms, and the remainder thereof are alkyl groups having 1 to 10 carbon atoms, while the alkyl group and the alkoxy group may be a linear chain or a branched chain alkyl group or alkoxy group. In the case in which Formula FI includes one instance of Formula S1, a compound in which all of Rₐ, R_{b} and R_{c} in the above Formula S1 are hydrogen, and R₁ to R₃ are alkoxy groups having 1 to 6 carbon atoms is excluded. 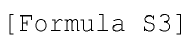 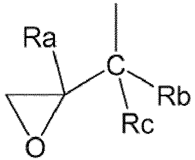 in Formula S3, Rₐ, R_{b} and R_{c} are independently H, or an alkyl group having 1 to 6 carbon atoms, and the alkyl group is a linear chain or a branched chain alkyl group.

According to a third aspect of the present disclosure, R₁ to R₃ may be an ethoxy group in the epoxy composition of the first or second aspect.

According to a fourth aspect of the present disclosure, the epoxy compound containing an alkoxysilyl group may be selected from the group consisting of the above Formulae AI to DI in the epoxy composition of the first or second aspect.

According to a fifth aspect of the present disclosure, the epoxy compound containing an alkoxysilyl group may be the above Formula DI in the epoxy composition of the fourth aspect.

According to a sixth aspect of the present disclosure, Y in the above Formula DI may be -C(CH₃)₂- in the epoxy composition of the fifth aspect.

According to a seventh aspect of the present disclosure, the epoxy compound containing an alkoxysilyl group may be one of compounds in the following Formula M in the epoxy composition of the fourth aspect.

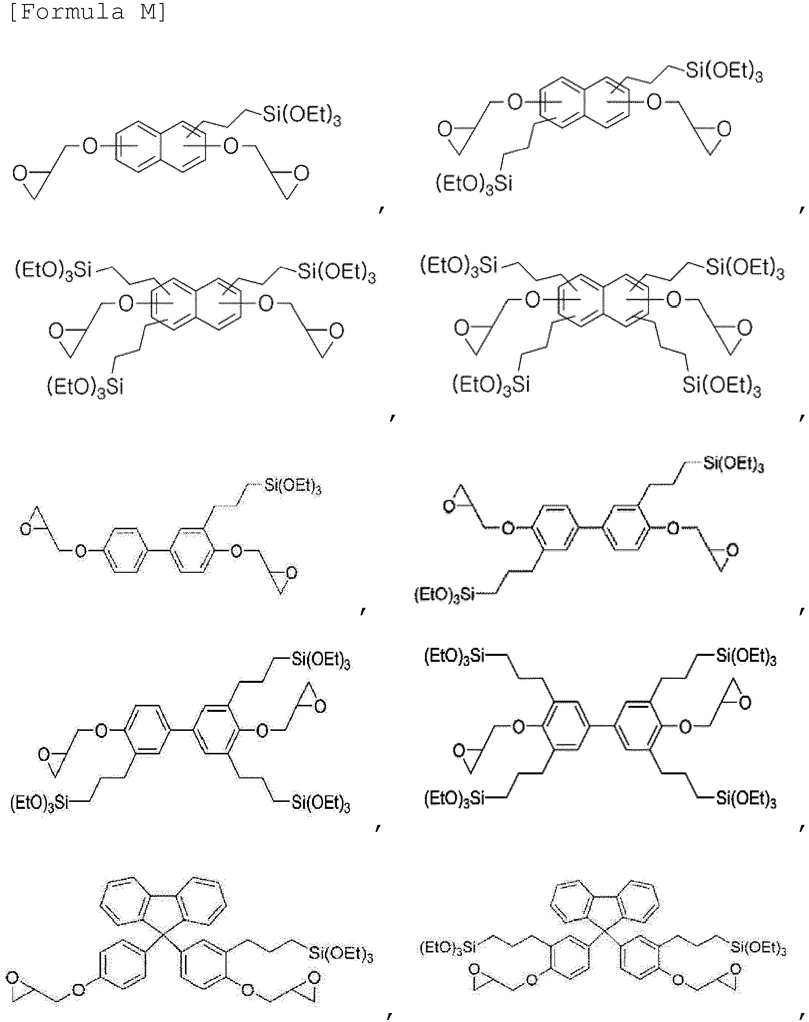

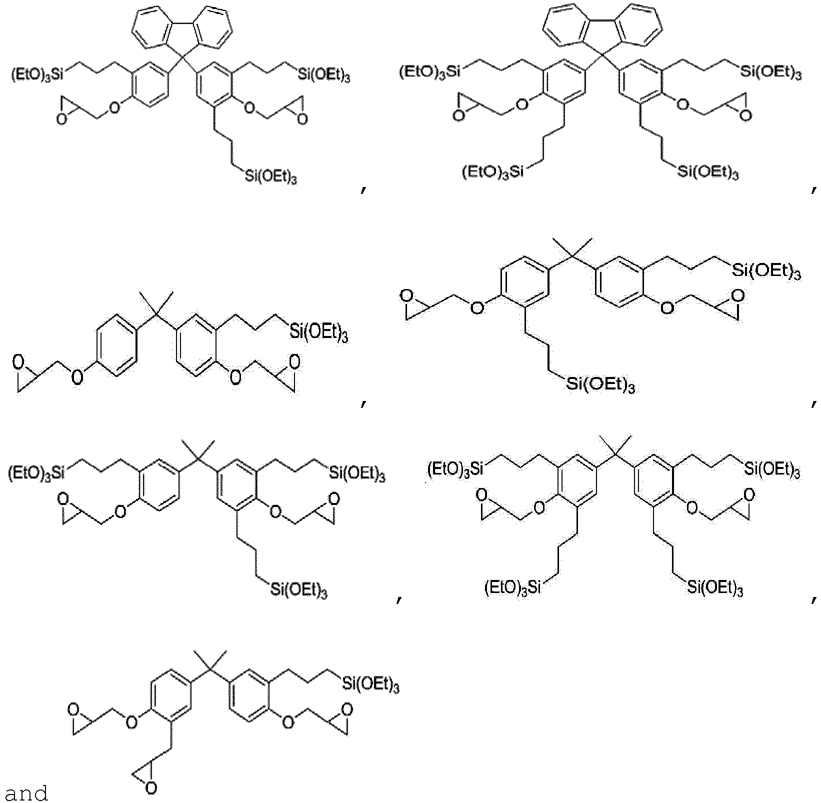

According to an eighth aspect of the present disclosure, the epoxy compound containing an alkoxysilyl group may be an epoxy polymer selected from the group consisting of the following Formulae AP to KP in the epoxy composition of the first or second aspect. 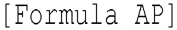 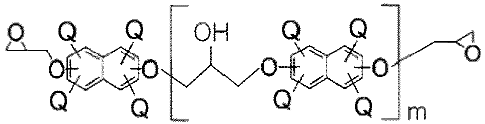 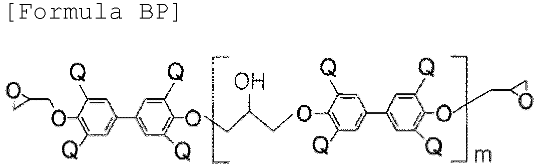 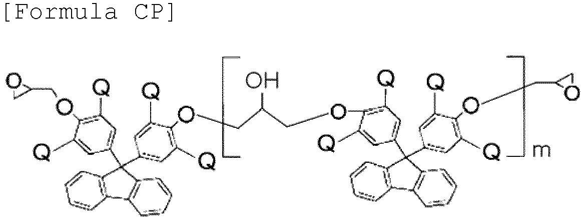 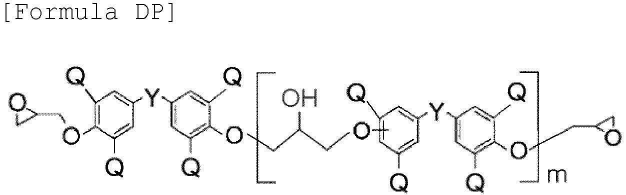 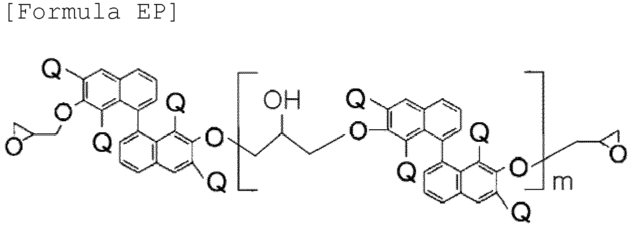 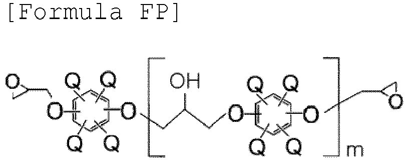 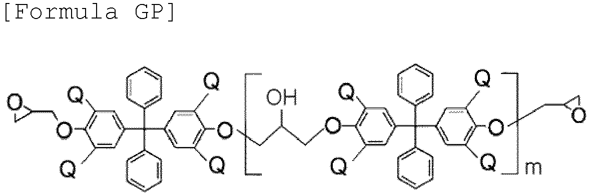 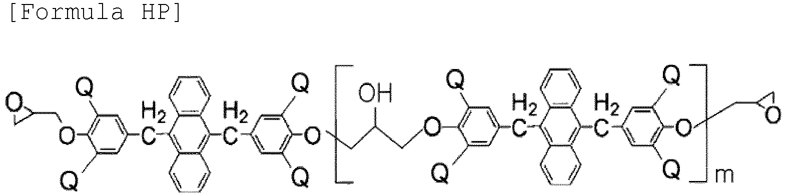 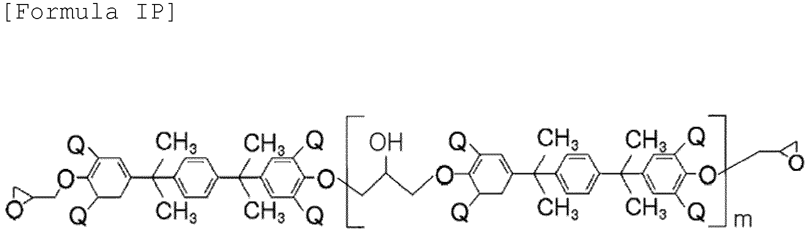 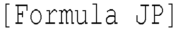 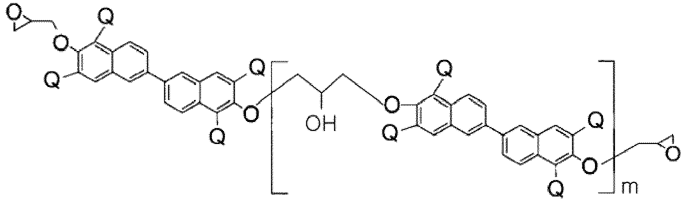 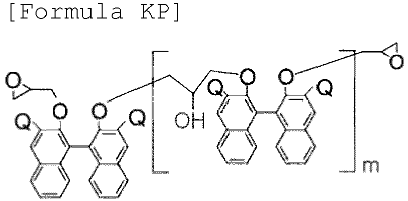 in the above Formulae AP to KP, at least one of a plurality of Q has the form of the following Formula S1, and the remainder thereof are independently selected from the group consisting of the following Formula S3, hydrogen, and -CR_{b}R_{c}-CRₐ=CH₂, where Rₐ, R_{b} and R_{c} are independently H, or an alkyl group having 1 to 6 carbon atoms, and the alkyl group may be a linear chain or a branched chain alkyl group,
m is an integer from 1 to 100,
in the above DP, Y is -CH₂, -C(CH₃)₂-, -C(CF₃)₂-, -S-, or -SO₂-.

[Formula S1] -CR_{b}R_{c}-CHRₐ-CH₂-SiR₁R₂R₃

in Formula S1, Rₐ, R_{b} and R_{c} are independently H, or an alkyl group having 1 to 6 carbon atoms, at least one of R₁ to R₃ is an alkoxy group having 1 to 6 carbon atoms, and the remainder thereof are alkyl groups having 1 to 10 carbon atoms, while the alkyl group and the alkoxy group may be a linear chain or a branched chain alkyl group or alkoxy group. 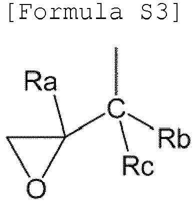 in Formula S3, Rₐ, R_{b} and R_{c} are independently H, or an alkyl group having 1 to 6 carbon atoms, and the alkyl group is a linear chain or a branched chain alkyl group.

According to a ninth aspect of the present disclosure, at least one epoxy compound selected from the group consisting of a glycidyl ether-based epoxy compound, a glycidyl-based epoxy compound, a glycidyl amine-based epoxy compound, a glycidyl ester-based epoxy compound, a rubber modified epoxy compound, an aliphatic polyglycidyl-based epoxy compound and an aliphatic glycidyl amine-based epoxy compound may be further included in the epoxy composition according to any one of the first to eighth aspects.

According to a tenth aspect of the present disclosure, the epoxy compound may include bisphenol A, bisphenol F, bisphenol S, biphenyl, naphthalene, benzene, thiodiphenol, fluorene, anthracene, isocyanurate, triphenylmethane, 1,1,2,2-tetraphenylethane, tetraphenylmethane, 4,4'-diaminodiphenylmethane, aminophenol, a cyclo aliphatic compound, or a novolak unit, as a core structure in the epoxy composition according to the ninth aspect.

According to an eleventh aspect of the present disclosure, the epoxy compound may include the bisphenol A, the biphenyl, the naphthalene, or the fluorene as the core structure in the epoxy composition according to the tenth aspect.

According to a twelfth aspect of the present disclosure, the epoxy composition may include 10 wt% to 100 wt% of the epoxy compound containing an alkoxysilyl group and 0 wt% to 90 wt% of at least one epoxy compound selected from the group consisting of the glycidyl ether-based epoxy compound, the glycidyl-based epoxy compound, the glycidyl amine-based epoxy compound, the glycidyl ester-based epoxy compound, the rubber modified epoxy compound, the aliphatic polyglycidyl-based epoxy compound and the aliphatic glycidyl amine-based epoxy compound based on the total amount of the epoxy compound contained in the epoxy composition according to the ninth aspect.

According to a thirteenth aspect of the present disclosure, the epoxy composition may include 30 wt% to 100 wt% of the epoxy compound containing an alkoxysilyl group and 0 wt% to 70 wt% of at least one epoxy compound selected from the group consisting of the glycidyl ether-based epoxy compound, the glycidyl-based epoxy compound, the glycidyl amine-based epoxy compound, the glycidyl ester-based epoxy compound, the rubber modified epoxy compound, the aliphatic polyglycidyl-based epoxy compound and the aliphatic glycidyl amine-based epoxy compound based on the total amount of the epoxy compound contained in the epoxy composition according to the twelfth aspect.

According to a fourteenth aspect of the present disclosure, the inorganic particle may be at least one selected from the group consisting of a metal oxide selected from the group consisting of silica, zirconia, titania, alumina, silicon nitride and aluminum nitride, T-10 type silsesquioxane, ladder type silsesquioxane and cage type silsesquioxane in the epoxy composition according to the first or second aspect.

According to a fifteenth aspect of the present disclosure, an content of the inorganic particles may be 5 wt% to 95 wt% based on a total amount of the epoxy composition in the epoxy composition according to the first or second aspect.

According to a sixteenth aspect of the present disclosure, an content of the inorganic particles may be 30 wt% to 95 wt% based on a total amount of the epoxy composition in the epoxy composition according to the fifteenth aspect.

According to a seventeenth aspect of the present disclosure, an content of the inorganic particles may be 5 wt% to 60 wt% based on a total amount of the epoxy composition in the epoxy composition according to the fifteenth aspect.

According to an eighteenth aspect of the present disclosure, a curing accelerator may be further included in the epoxy composition according to the first or second aspect.

According to a nineteenth aspect of the present disclosure, an electronic material includes the epoxy composition according to any one of the first to eighteenth aspects.

According to a twentieth aspect of the present disclosure, a substrate includes the epoxy composition according to any one of the first to eighteenth aspects.

According to a twenty-first aspect of the present disclosure, a film includes the epoxy composition according to any one of the first to eighteenth aspects.

According to a twenty-second aspect of the present disclosure, a laminate includes a metal layer placed on a base layer formed by using the epoxy composition according to any one of the first to eighteenth aspects.

According to a twenty-third aspect of the present disclosure, a printed circuit board includes the laminate according to the twenty-second aspect.

According to a twenty-fourth aspect of the present disclosure, a semiconductor device includes the printed circuit board according to the twenty-third aspect.

According to a twenty-fifth aspect of the present disclosure, a semiconductor packaging material includes the epoxy composition according to any one of the first to eighteenth aspects.

According to a twenty-sixth aspect of the present disclosure, a semiconductor device includes the semiconductor packaging material according to the twenty-fifth aspect.

According to a twenty-seventh aspect of the present disclosure, an adhesive includes the epoxy composition according to any one of the first to eighteenth aspects.

According to a twenty-eighth aspect of the present disclosure, a paint composition includes the epoxy composition according to any one of the first to eighteenth aspects.

According to a twenty-ninth aspect of the present disclosure, a composite material includes the epoxy composition according to any one of the first to eighteenth aspects.

According to a thirtieth aspect of the present disclosure, a cured product of the epoxy composition according to any one of the first to eighteenth aspects.

According to a thirty-first aspect of the present disclosure, the cured product may have a coefficient of thermal expansion of less than or equal to 60 ppm/°C in the cured product according to the thirtieth aspect.

According to a thirty-second aspect of the present disclosure, the cured product may have a glass transition temperature of 100°C or above, or not exhibit the glass transition temperature in the cured product according to the thirtieth aspect.

According to a thirty-third aspect of the present disclosure, a method of preparing an epoxy compound containing an alkoxysilyl group of Formulae (A14) to (K14) includes:
a first step of preparing an intermediate (11) of the following Formulae (A11) to (K11) by reacting one starting material of the following Formulae (AS) to (KS) and an allyl compound of the following Formula B1 in the presence of a base and an optional solvent;
a second step of preparing an intermediate (12) of the following Formulae (A12) to (K12) by irradiating electromagnetic waves onto one of the above intermediate (11) in the presence of an optional solvent;
a third step of preparing an intermediate (13) of the following Formulae (A13) to (K13) by reacting one of the above intermediate (12) with epichlorohydrin in the presence of a base and an optional solvent;
an optional 3-1-st step of preparing an intermediate (13') of the following Formulae (A13') to (K13') by reacting one of the above intermediate (13) with a peroxide in the presence of a base and an optional solvent; and
a fourth step of reacting one of the above intermediate (13) or one of the above intermediate (13') with an alkoxysilane of the following Formula B2 in the presence of a metal catalyst and an optional solvent. 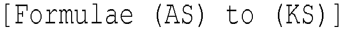 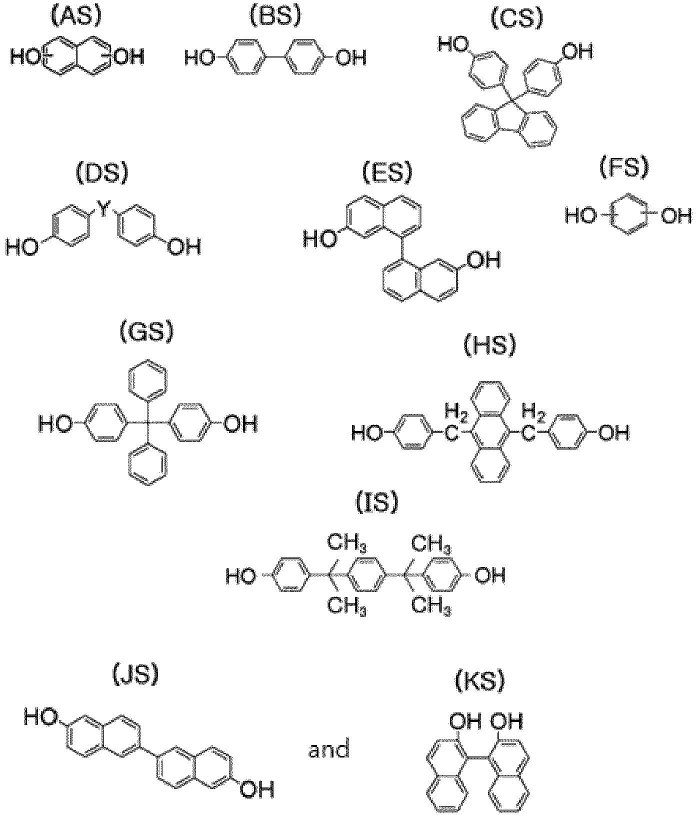 in the above Formula DS, Y is -CH₂-, -C(CH₃)₂-, -C(CF₃)₂-, -S- or -SO₂- . 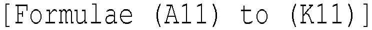 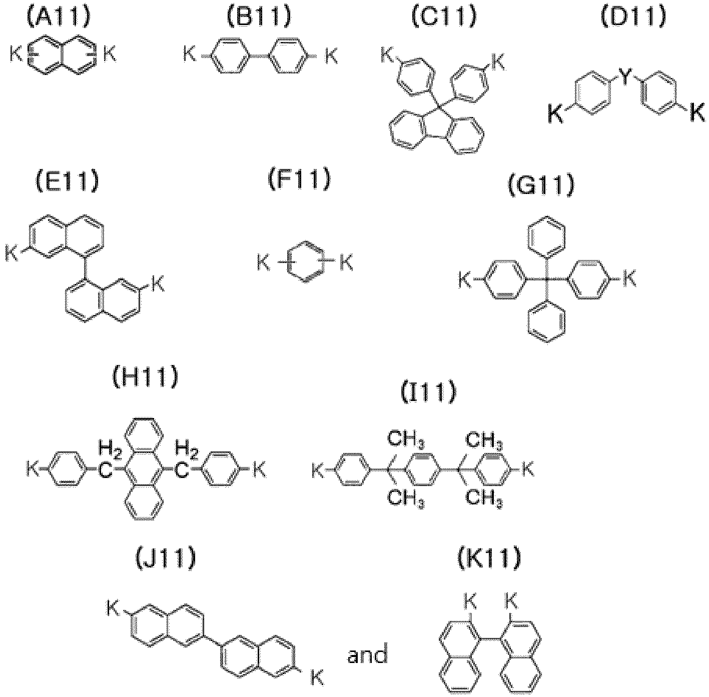 in the above Formulae A11 to K11, at least one of K is -O-CH₂-CRₐ=CR_{b}R_{c}, where Rₐ, R_{b} and R_{C} are independently H or an alkyl group having 1 to 6 carbon atoms, and the alkyl group may be a linear chain or a branched chain alkyl group, and the remainder thereof are hydroxyl groups,
   in the above Formula D11, Y is -CH₂-, -C(CH₃)₂-, -C(CF₃)₂-, -S- or -SO₂- . 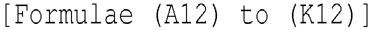 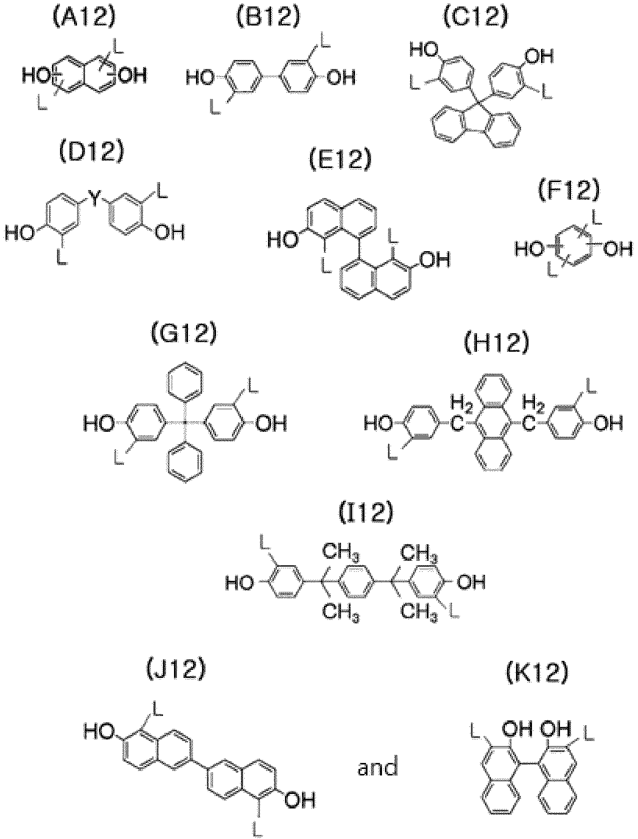 in the above Formulae A12 to K12, at least one of L is -CR_{b}R_{c}-CRₐ=CH₂, where Rₐ, R_{b} and R_{C} are independently H or an alkyl group having 1 to 6 carbon atoms, and the alkyl group may be a linear chain or a branched chain alkyl group, and the remainder thereof are hydrogen atoms,
   in the above Formula D12, Y is -CH₂-, -C(CH₃)₂-, -C(CF₃)₂-, -S- or -SO₂- . 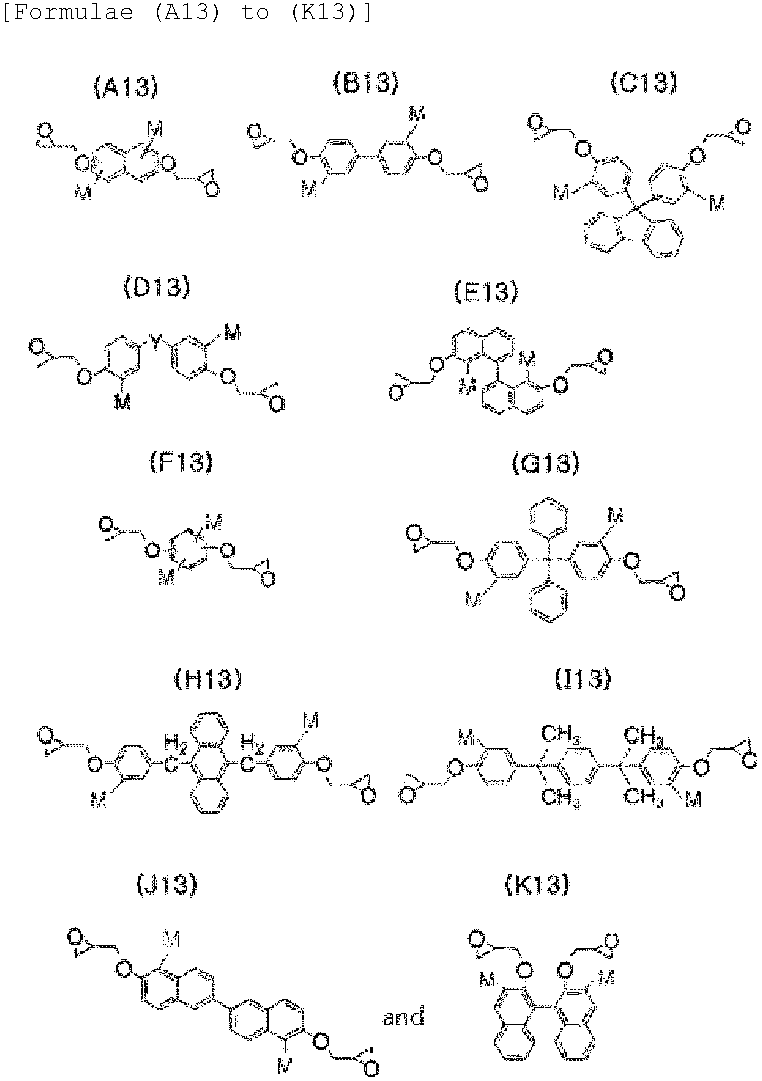 in the above Formulae A13 to K13, at least one of M is -CR_{b}R_{c}-CRₐ=CH₂, where Rₐ, R_{b} and R_{C} are independently H or an alkyl group having 1 to 6 carbon atoms, and the alkyl group may be a linear chain or a branched chain alkyl group, and the remainder thereof are hydrogen atoms,
   in the above Formula D13, Y is -CH₂-, -C(CH₃)₂-, -C(CF₃)₂-, -S- or -SO₂- . 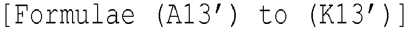 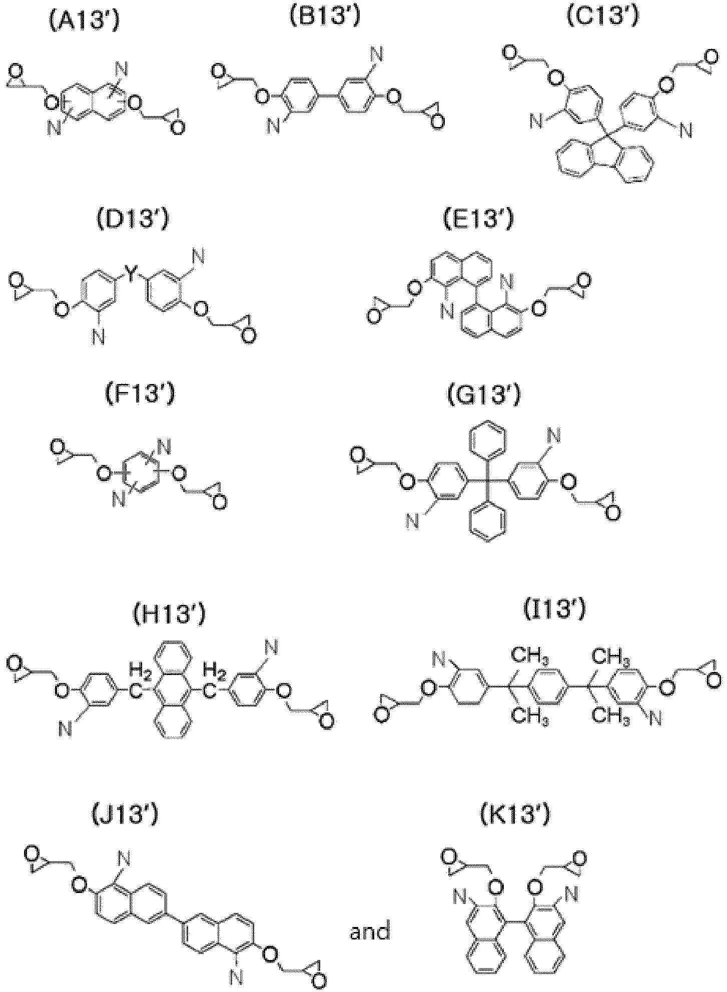 in the above Formulae A13' to K13', one of N is -CR_{b}R_{c}-CRₐ=CH₂, where Rₐ, R_{b} and R_{C} are independently H or an alkyl group having 1 to 6 carbon atoms, and the alkyl group may be a linear chain or a branched chain alkyl group, and one remainder thereof is the following Formula S3,
   in the above Formula D13' , Y is -CH₂-, -C(CH₃)₂-, -C(CF₃)₂-, -S- or -SO₂-. 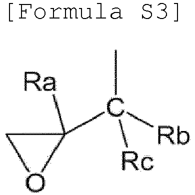 in the above Formula S3, Rₐ, R_{b} and R_{C} are independently H or an alkyl group having 1 to 6 carbon atoms, and the alkyl group may be a linear chain or a branched chain alkyl group. 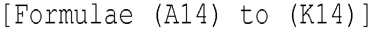 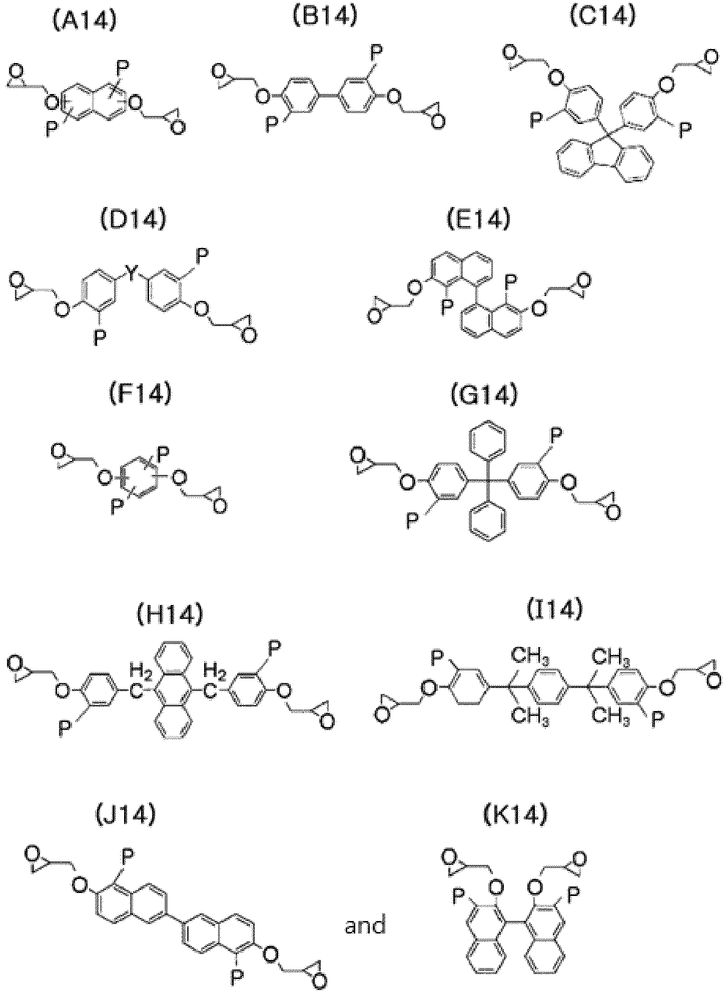 in the above Formulae A14 to K14, at least one of P has the form of the following Formula S1, and the remainder thereof are the form of the following Formula S3, hydrogen or -CR_{b}R_{c}-CRₐ=CH₂, where Rₐ, R_{b} and R_{C} are independently H or an alkyl group having 1 to 6 carbon atoms, and the alkyl group may be a linear chain or a branched chain alkyl group,
   in the above Formula D14, Y is -CH₂-, -C(CH₃)₂-, -C(CF₃)₂-, -S- or -SO₂-.

   [Formula S1] -CR_{b}R_{c}-CHRₐ-CH₂-SiR₁R₂R₃

   in Formula S1, Rₐ, R_{b} and R_{c} are independently H, or an alkyl group having 1 to 6 carbon atoms, at least one of R₁ to R₃ is an alkoxy group having 1 to 6 carbon atoms, and the remainder thereof are alkyl groups having 1 to 10 carbon atoms, while the alkyl group and the alkoxy group may be a linear chain or a branched chain alkyl group or alkoxy group. In the case in which Formula F14 includes one instance of Formula S1, a compound in which all of Rₐ, R_{b} and R_{c} in the above Formula S1 are hydrogen, and R₁ to R₃ are alkoxy groups having 1 to 6 carbon atoms is excluded. 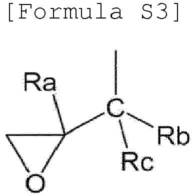 in the above Formula S3, Rₐ, R_{b} and R_{C} are independently H or an alkyl group having 1 to 6 carbon atoms, and the alkyl group may be a linear chain or a branched chain alkyl group. 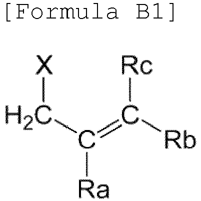 in the above Formula B1, X is Cl, Br, I, -O-SO₂-CH₃, -O-SO₂-CF₃, or -O-SO₂-C₆H₄-CH₃, Rₐ, R_{b} and R_{C} are independently H or an alkyl group having 1 to 6 carbon atoms, and the alkyl group may be a linear chain or a branched chain alkyl group.

   [Formula B2] HSiR₁R₂R₃

   in the above Formula B2, at least one of R₁ to R₃ is an alkoxy group having 1 to 6 carbon atoms, and the remainder thereof are alkyl groups having 1 to 10 carbon atoms, while the alkyl group and the alkoxy group may be a linear chain or a branched chain alkyl group or alkoxy group.

According to a thirty-fourth aspect of the present disclosure, a method of preparing an epoxy compound containing an alkoxysilyl group of the following Formulae (A26) to (J26) includes:
a first step of preparing an intermediate (11) of the following Formulae (A11) to (J11) by reacting one starting material of the following Formulae (AS) to (JS) and an allyl compound of the following Formula B1 in the presence of a base and an optional solvent;
a second step of preparing an intermediate (12) of the following Formulae (A12) to (J12) by irradiating electromagnetic waves onto one of the above intermediate (11) in the presence of an optional solvent;
a 2-1-st step of preparing an intermediate (23) of the following Formulae (A23) to (J23) by reacting one of the above intermediate (12) with an allyl compound of the following Formula B1 in the presence of a base and an optional solvent;
a 2-2-nd step of preparing an intermediate (24) of the following Formulae (A24) to (J24) by irradiating electromagnetic waves onto the above intermediate (23) in the presence of an optional solvent;
a third step of preparing an intermediate (25) of the following Formulae (A25) to (J25) by reacting one of the above intermediate (24) with epichlorohydrin in the presence of a base and an optional solvent;
an optional 3-1-st step of preparing an intermediate (25') of the following Formulae (A25') to (J25') by reacting one of the above intermediate (25) with a peroxide in the presence of a base and an optional solvent; and
a fourth step of reacting one of the above intermediate (25) or one of the above intermediate (25') with an alkoxysilane of the following Formula B2 in the presence of a metal catalyst and an optional solvent. 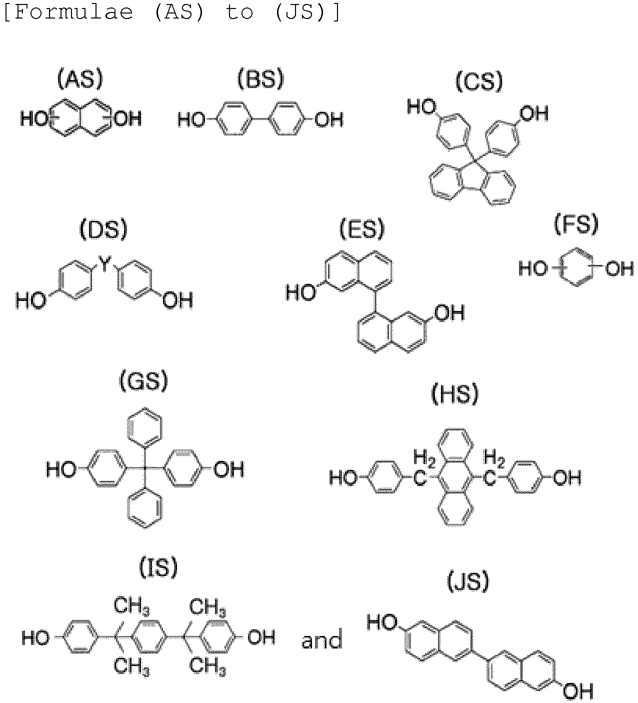 in the above Formula DS, Y is -CH₂-, -C(CH₃)₂-, -C(CF₃)₂-, -S- or -SO₂-. 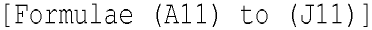 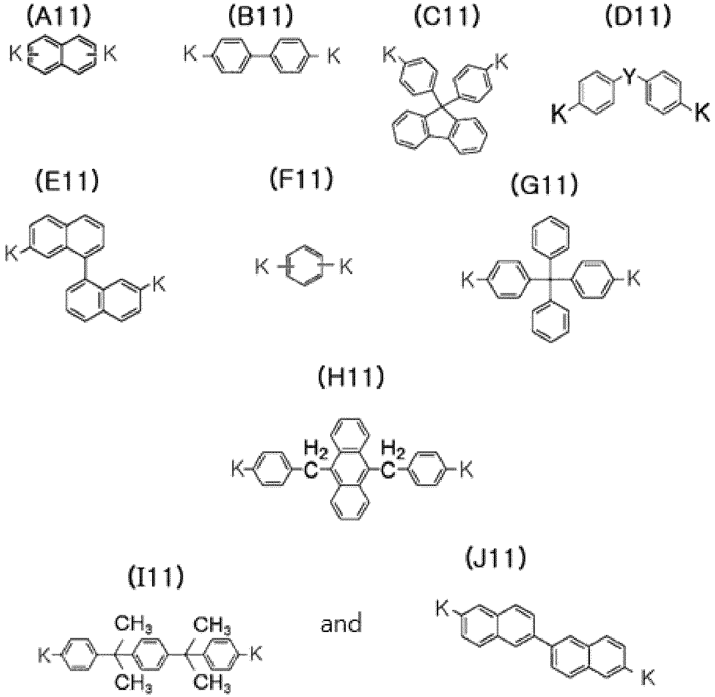 in the above Formulae A11 to J11, at least one of K is -O-CH₂-CRₐ=CR_{b})R_{c}, where Rₐ, R_{b} and R_{C} are independently H or an alkyl group having 1 to 6 carbon atoms, and the alkyl group may be a linear chain or a branched chain alkyl group, and the remainder thereof are hydroxyl groups,
   in the above Formula D11, Y is -CH₂-, -C(CH₃)₂-, -C(CF₃)₂-, -S- or -SO₂-. 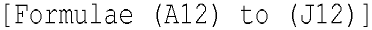 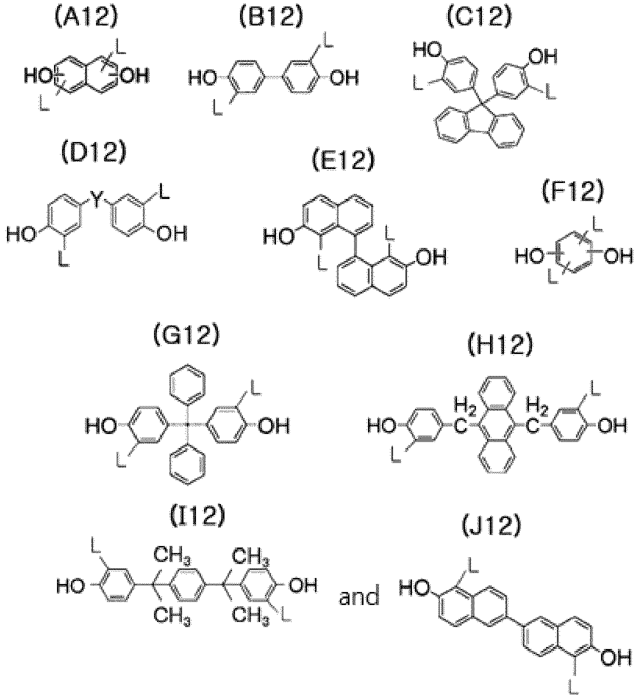 in the above Formulae A12 to J12, at least one of L is -CR_{b}R_{c}-CRₐ=CH₂, where Rₐ, R_{b} and R_{C} are independently H or an alkyl group having 1 to 6 carbon atoms, and the alkyl group may be a linear chain or a branched chain alkyl group, and the remainder thereof are hydrogen atoms,
   in the above Formula D12, Y is -CH₂-, -C(CH₃)₂-, -C(CF₃)₂-, -S- or -SO₂-. 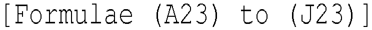 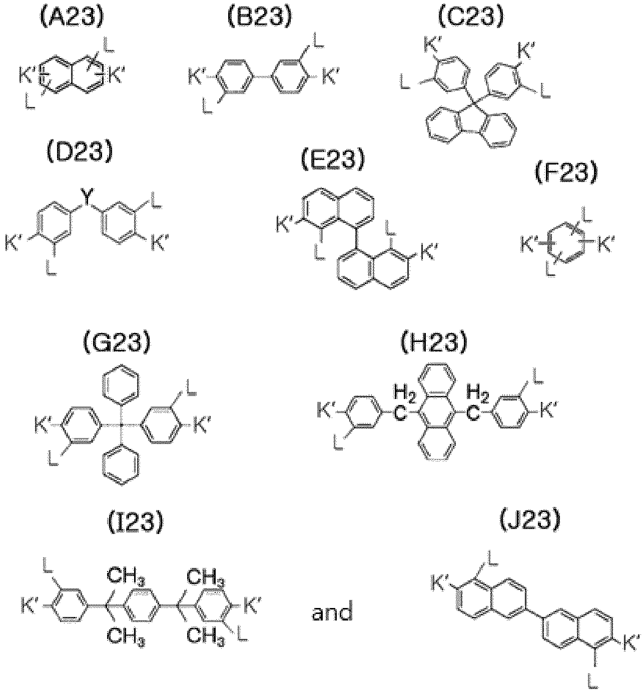 in the above Formulae A23 to J23, at least one of K' is -O-CH₂-CRₐ=CR_{b}R_{c}, where Rₐ, R_{b} and R_{C} are independently H or an alkyl group having 1 to 6 carbon atoms, and the alkyl group may be a linear chain or a branched chain alkyl group, and the remainder thereof are hydroxyl groups, and at least one of L is -CR_{b}R_{c}-CRₐ=CH₂, where Rₐ, R_{b} and R_{C} are independently H or an alkyl group having 1 to 6 carbon atoms, and the alkyl group may be a linear chain or a branched chain alkyl group, and the remainder thereof are hydrogen atoms,
   in the above Formula D23, Y is -CH₂-, -C(CH₃)₂-, -C(CF₃)₂-, -S- or -SO₂-. 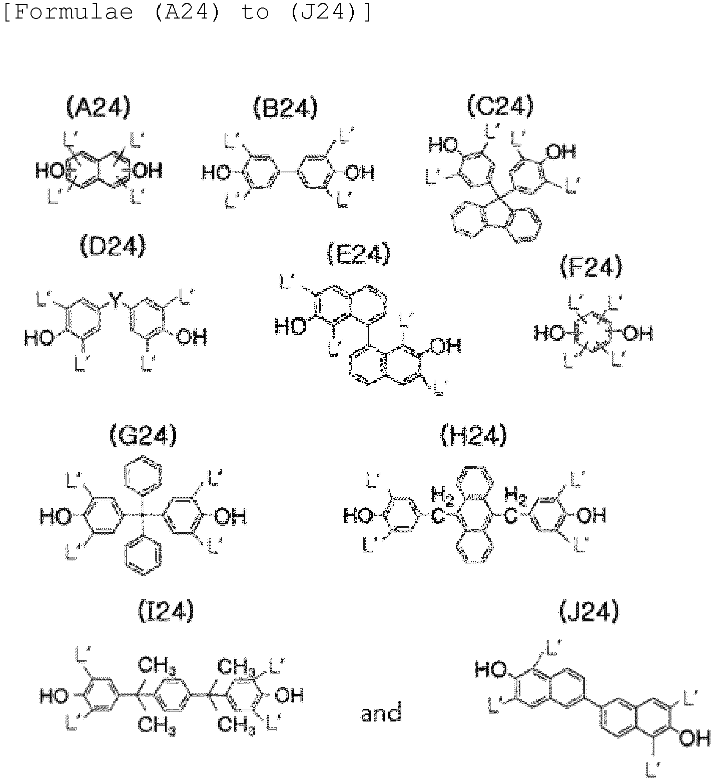 in the above Formulae A24 to J24, at least two of a plurality of L' are -CR_{b}R_{c}-CRₐ=CH₂, where Rₐ, R_{b} and R_{C} are independently H or an alkyl group having 1 to 6 carbon atoms, and the alkyl group may be a linear chain or a branched chain alkyl group, and the remainder thereof are hydrogen atoms,
   in the above Formula D24, Y is -CH₂-, -C(CH₃)₂-, -C(CF₃)₂-, -S- or -SO₂-. 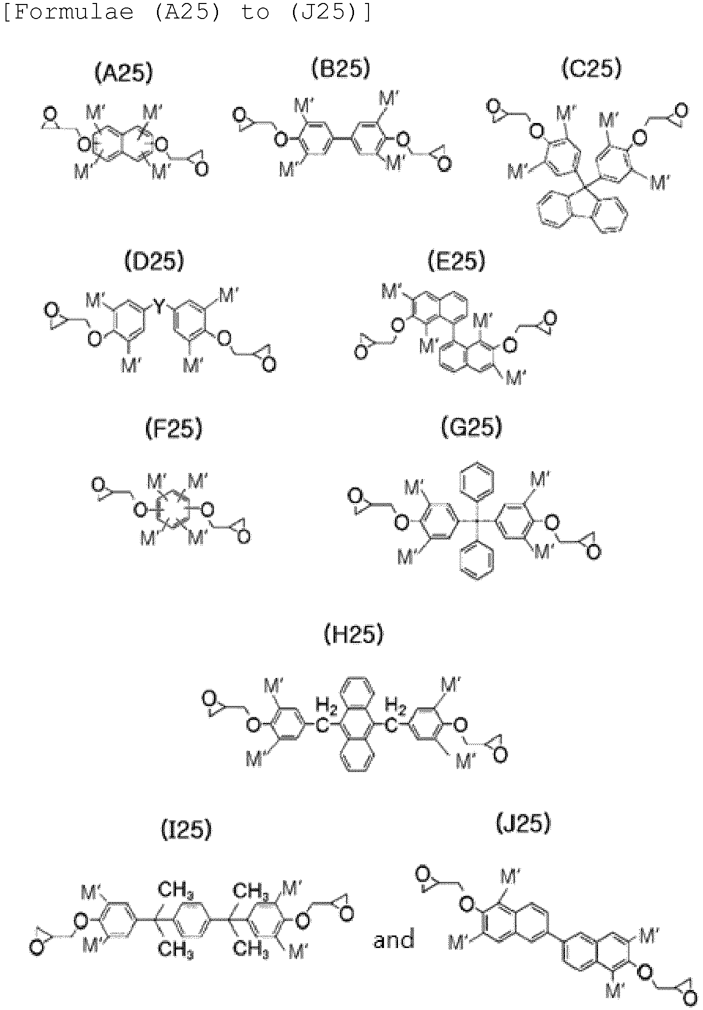 in the above Formulae A25 to J25, at least two of a plurality of M' are -CR_{b}R_{c}-CRₐ=CH₂, where Rₐ, R_{b} and R_{C} are independently H or an alkyl group having 1 to 6 carbon atoms, and the alkyl group may be a linear chain or a branched chain alkyl group, and the remainder thereof are hydrogen atoms,
   in the above Formula D25, Y is -CH₂-, -C(CH₃)₂-, -C(CF₃)₂-, -S- or -SO₂-. 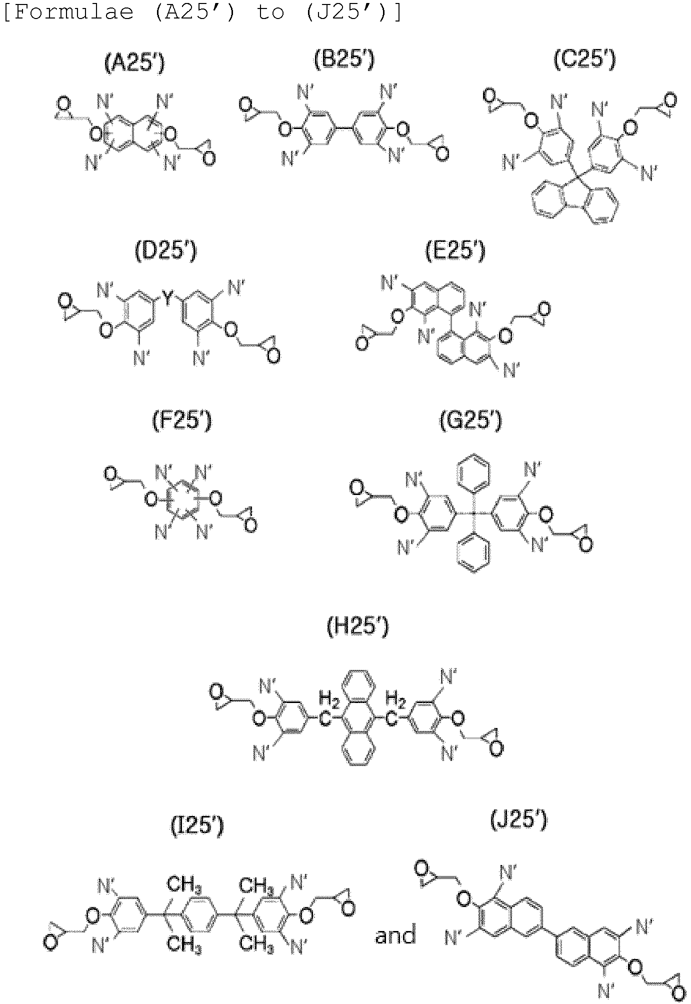 in the above Formulae A25' to J25', one to three of a plurality of N' are -CR_{b}R_{c}-CRₐ=CH₂, where Rₐ, R_{b} and R_{C} are independently H or an alkyl group having 1 to 6 carbon atoms, and the alkyl group may be a linear chain or a branched chain alkyl group, one to three thereof are the form of the following Formula S3, and the remainder thereof are hydrogen atoms,
   in the above Formula D25' , Y is -CH₂-, -C(CH₃)₂-, -C(CF₃)₂-, -S- or -SO₂-. 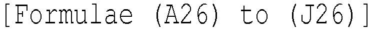 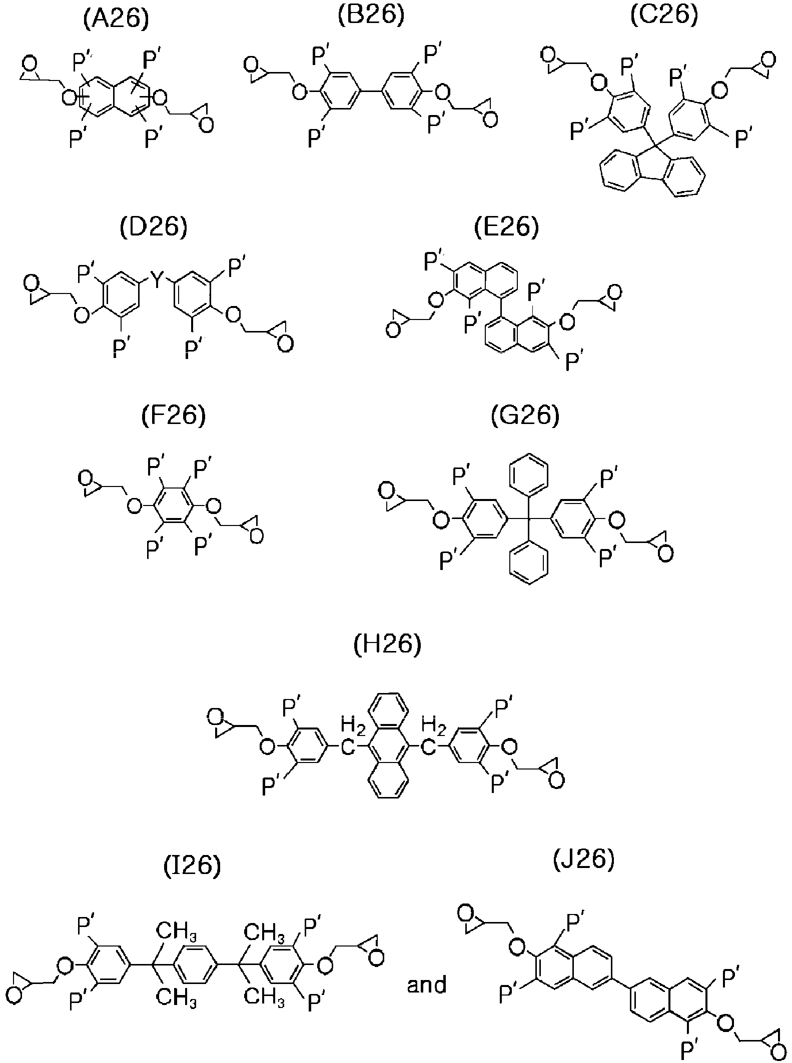 in the above Formulae A26 to J26, at least one of P' has the form of the following Formula S1, and the remainder thereof are independently selected from the group consisting of the following Formula S3, hydrogen and -CR_{b}R_{c}-CRₐ=CH₂, where Rₐ, R_{b} and R_{C} are independently H or an alkyl group having 1 to 6 carbon atoms, and the alkyl group may be a linear chain or a branched chain alkyl group,
   in the above Formula D26, Y is -CH₂-, -C(CH₃)₂-, -C(CF₃)₂-, -S- or -SO₂-.

   [Formula S1] -CR_{b}R_{c}-CHRₐ-CH₂-SiR₁R₂R₃

   in Formula S1, Rₐ, R_{b} and R_{c} are independently H, or an alkyl group having 1 to 6 carbon atoms, at least one of R₁ to R₃ is an alkoxy group having 1 to 6 carbon atoms, and the remainder are alkyl groups having 1 to 10 carbon atoms, and the alkyl group and the alkoxy group may be a linear chain or a branched chain alkyl group or alkoxy group. In the case in which Formula F26 includes one instance of Formula S1, a compound in which all of R_{a,} R_{b} and R_{c} in the above Formula S1 are hydrogen, and R₁ to R₃ are alkoxy groups having 1 to 6 carbon atoms is excluded. 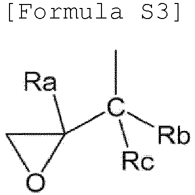 in the above Formula S3, Rₐ, R_{b} and R_{C} are independently H or an alkyl group having 1 to 6 carbon atoms, and the alkyl group may be a linear chain or a branched chain alkyl group. 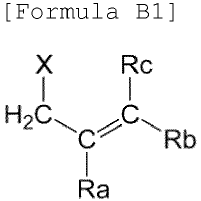 in the above Formula B1, X is Cl, Br, I, -O-SO₂-CH₃, -O-SO₂-CF₃, or -O-SO₂-C₆H₄-CH₃, Rₐ, R_{b} and R_{C} are independently H or an alkyl group having 1 to 6 carbon atoms, and the alkyl group may be a linear chain or a branched chain alkyl group.

   [Formula B2] HSiR₁R₂R₃

   in the above Formula B2, at least one of R₁ to R₃ is an alkoxy group having 1 to 6 carbon atoms, and the remainder thereof are alkyl groups having 1 to 10 carbon atoms, while the alkyl group and the alkoxy group may be a linear chain or a branched chain alkyl group or alkoxy group.

According to a thirty-fifth aspect of the present disclosure, 0.5 to 10 equivalents of an allyl group of the allyl compound of the above Formula B1 may react with respect to 1 equivalent of a hydroxyl group of the starting material in the first step in the preparation method of an epoxy compound containing an alkoxysilyl group according to the thirty-third or thirty-fourth aspect.

According to a thirty-sixth aspect of the present disclosure, the first step may be performed at a temperature of from room temperature to 100°C for 1 to 120 hours in the preparation method of an epoxy compound containing an alkoxysilyl group according to the thirty-third or thirty-fourth aspect.

According to a thirty-seventh aspect of the present disclosure, the base in the first step may be at least one selected from the group consisting of KOH, NaOH, K₂CO₃, Na₂CO₃, KHCO₃, NaHCO₃, NaH, triethylamine and diisopropylamine in the preparation method of an epoxy compound containing an alkoxysilyl group according to the thirty-third or thirty-fourth aspect.

According to a thirty-eighth aspect of the present disclosure, the solvent in the first step may be at least one selected from the group consisting of acetonitrile, tetrahydrofuran, methyl ethyl ketone, dimethylformamide, dimethyl sulfoxide and methylene chloride in the preparation method of an epoxy compound containing an alkoxysilyl group according to the thirty-third or thirty-fourth aspect.

According to a thirty-ninth aspect of the present disclosure, the second step may be performed at a temperature from 120°C to 250°C for 1 to 1,000 minutes in the preparation method of an epoxy compound containing an alkoxysilyl group according to the thirty-third or thirty-fourth aspect.

According to a fortieth aspect of the present disclosure, the solvent in the second step may be at least one selected from the group consisting of xylene, 1,2-dichlorobenzene and N,N-diethylaniline in the preparation method of an epoxy compound containing an alkoxysilyl group according to the thirty-third or thirty-fourth aspect.

According to a forty-first aspect of the present disclosure, the electromagnetic waves in the second step may be microwaves in the preparation method of an epoxy compound containing an alkoxysilyl group according to the thirty-third or thirty-fourth aspect.

According to a forty-second aspect of the present disclosure, 0.5 to 10 equivalents of an allyl group of the allyl compound of the above Formula B1 may react with respect to 1 equivalent of a hydroxyl group of the intermediate (12) in the 2-1-st step in the preparation method of an epoxy compound containing an alkoxysilyl group according to the thirty-fourth aspect.

According to a forty-third aspect of the present disclosure, the 2-1-st step may be performed at a temperature of from room temperature to 100°C for 1 to 120 hours in the preparation method of an epoxy compound containing an alkoxysilyl group according to the thirty-fourth aspect.

According to a forty-fourth aspect of the present disclosure, the base in the 2-1-st step may be at least one selected from the group consisting of KOH, NaOH, K₂CO₃, Na₂CO₃, KHCO₃, NaHCO₃, NaH, triethylamine and diisopropylamine in the preparation method of an epoxy compound containing an alkoxysilyl group according to the thirty-fourth aspect.

According to a forty-fifth aspect of the present disclosure, the solvent in the 2-1-st step may be at least one selected from the group consisting of acetonitrile, tetrahydrofuran, methyl ethyl ketone, dimethylformamide, dimethyl sulfoxide and methylene chloride in the preparation method of an epoxy compound containing an alkoxysilyl group according to the thirty-fourth aspect.

According to a forty-sixth aspect of the present disclosure, the 2-2-nd step may be performed at a temperature from 120°C to 250°C for 1 to 1,000 minutes in the preparation method of an epoxy compound containing an alkoxysilyl group according to the thirty-fourth aspect.

According to a forty-seventh aspect of the present disclosure, the solvent in the 2-2-nd step may be at least one selected from the group consisting of xylene, 1, 2-dichlorobenzene and N, N-diethylaniline in the preparation method of an epoxy compound containing an alkoxysilyl group according to the thirty-fourth aspect.

According to a forty-eighth aspect of the present disclosure, the electromagnetic waves in the 2-2-nd step may be microwaves in the preparation method of an epoxy compound containing an alkoxysilyl group according to the thirty-fourth aspect.

According to a forty-ninth aspect of the present disclosure, 1 to 10 equivalents of a glycidyl group of the epichlorohydrin may react with respect to 1 equivalent of a hydroxyl group of the above intermediate (12) or intermediate (24) in the third step in the preparation method of an epoxy compound containing an alkoxysilyl group according to the thirty-third or thirty-fourth aspect.

According to a fiftieth aspect of the present disclosure, the third step may be performed at a temperature of from room temperature to 100°C for 1 to 120 hours in the preparation method of an epoxy compound containing an alkoxysilyl group according to the thirty-third or thirty-fourth aspect.

According to a fifty-first aspect of the present disclosure, the base in the third step may be at least one selected from the group consisting of KOH, NaOH, K₂CO₃, Na₂CO₃, KHCO₃, NaHCO₃, NaH, triethylamine and diisopropylamine in the preparation method of an epoxy compound containing an alkoxysilyl group according to the thirty-third or thirty-fourth aspect.

According to a fifty-second aspect of the present disclosure, the solvent in the third step may be at least one selected from the group consisting of acetonitrile, tetrahydrofuran, methyl ethyl ketone, dimethylformamide, dimethyl sulfoxide, methylene chloride, and H₂O in the preparation method of an epoxy compound containing an alkoxysilyl group according to the thirty-third or thirty-fourth aspect.

According to a fifty-third aspect of the present disclosure, 1 to 10 equivalents of a peroxide group of the peroxide may react with respect to 1 equivalent of an allyl group of the above intermediate (13) or intermediate (25) in the 3-1-st step in the preparation method of an epoxy compound containing an alkoxysilyl group according to the thirty-third or thirty-fourth aspect.

According to a fifty-fourth aspect of the present disclosure, the peroxide in the 3-1-st step may be at least one selected from the group consisting of meta-chloroperoxybenzoic acid (m-CPBA), H₂O₂ and dimethyldioxirane (DMDO) in the preparation method of an epoxy compound containing an alkoxysilyl group according to the thirty-third or thirty-fourth aspect.

According to a fifty-fifth aspect of the present disclosure, the 3-1-st step may be performed at a temperature of from room temperature to 100°C for 1 to 120 hours in the preparation method of an epoxy compound containing an alkoxysilyl group according to the thirty-third or thirty-fourth aspect.

According to a fifty-sixth aspect of the present disclosure, the solvent in the 3-1-st step may be at least one selected from the group consisting of acetonitrile, tetrahydrofuran, methyl ethyl ketone, dimethylformamide, dimethyl sulfoxide, methylene chloride, and H₂O in the preparation method of an epoxy compound containing an alkoxysilyl group according to the thirty-third or thirty-fourth aspect.

According to a fifty-seventh aspect of the present disclosure, the base in the 3-1-st step may be at least one selected from the group consisting of KOH, NaOH, K₂CO₃, KHCO₃, triethylamine and diisopropylamine in the preparation method of an epoxy compound containing an alkoxysilyl group according to the thirty-third or thirty-fourth aspect.

According to a fifty-eighth aspect of the present disclosure, 1 to 5 equivalents of an alkoxysilane of the above Formula B2 may react with respect to 1 equivalent of an allyl group of the above intermediate (15'), intermediate (25) or intermediate (25') in the fourth step in the preparation method of an epoxy compound containing an alkoxysilyl group according to the thirty-third or thirty-fourth aspect.

According to a fifty-ninth aspect of the present disclosure, the fourth step may be performed at a temperature of from room temperature to 120°C for 1 to 72 hours in the preparation method of an epoxy compound containing an alkoxysilyl group according to the thirty-third or thirty-fourth aspect.

According to a sixtieth aspect of the present disclosure, the metal catalyst in the fourth step may include PtO₂ or H₂PtCl₆ in the preparation method of an epoxy compound containing an alkoxysilyl group according to the thirty-third or thirty-fourth aspect.

According to a sixty-first aspect of the present disclosure, the solvent in the fourth step may be at least one selected from the group consisting of toluene, acetonitrile, tetrahydrofuran, methyl ethyl ketone, dimethylformamide, dimethyl sulfoxide, and methylene chloride in the preparation method of an epoxy compound containing an alkoxysilyl group according to the thirty-third or thirty-fourth aspect.

### [Advantageous Effects]

As set forth above, according to exemplary embodiments of the present disclosure, due to chemical bonding formed between an alkoxysilyl group of an epoxy compound and inorganic particles in the composite of an epoxy composition including an epoxy compound containing an alkoxysilyl group and inorganic particles, chemical bonding efficiency between the epoxy compound and the inorganic particles may be increased. Due to the increase of the chemical bonding efficiency, heat resistance property may be improved. That is, the CTE of an epoxy composite may be decreased, and a glass transition temperature may be increased or the glass transition temperature may not be exhibited (Tg-less).

Further, when the epoxy composition is applied in a metal film of a substrate, good adhesive properties may be exhibited with respect to the metal film due to the chemical bonding between the functional group at the surface of the metal film and the alkoxysilyl group. In addition, due to the increase in chemical bonding efficiency of the composition including the alkoxysilylated epoxy compound and the inorganic particles, a silane coupling agent used in a common epoxy composition may be unnecessary in the composition including the alkoxysilylated epoxy compound. The epoxy composition including the alkoxysilylated epoxy compound and the inorganic particles may have good curing (including thermo curing and/or photo curing) efficiency, and a composite formed through the curing thereof may exhibit good thermal expansion property such as a low CTE and a high glass transition temperature or Tg-less.

In addition, an epoxy compound containing an alkoxysilyl group may be efficiently prepared by a method of preparing an epoxy compound containing an alkoxysilyl group according to the present disclosure.

### [Description of Drawings]

The above and other aspects, features and other advantages of the present disclosure will be more clearly understood from the following detailed description taken in conjunction with the accompanying drawings, in which:
FIG. 1 is a graph illustrating dimensional change with the change of the temperature of a cured product according to Comparative Example 1 and Example 2;
FIG. 2A is a graph illustrating dimensional change with the change of the temperature of a composite according to Example 16;
FIG. 2B is a graph illustrating dimensional change with the change of the temperature of a composite according to Example 19;
FIG. 2C is a graph illustrating dimensional change with the change of the temperature of a composite according to Example 22;
FIG. 2D is a graph illustrating dimensional change with the change of the temperature of a composite according to Example 28;
FIG. 3A is a graph illustrating dimensional change with the change of the temperature of a composite according to Example 25 and Comparative Example 9;
FIG. 3B is a graph illustrating dimensional change with the change of the temperature of a composite according to Example 26 and Comparative Example 10;
FIG. 3C is a graph illustrating dimensional change with the change of the temperature of a composite according to Example 27 and Comparative Example 11;
FIG. 3D is a graph illustrating dimensional change with the change of the temperature of a composite according to Example 28 and Comparative Example 12;
FIG. 4 provides graphs illustrating dimensional change of a cured product according to Comparative Example 1 (A) and dimensional change of a composite according to Comparative Example 6 (B) with the change of the temperature;
FIG. 5 provides graphs illustrating dimensional change of a cured product according to Comparative Example 2 (A) and dimensional change of a composite according to Comparative Example 16 (B) with the change of the temperature;
FIG. 6 provides a graph illustrating dimensional change with the change of the temperature according to Example 16 and Comparative Example 6;
FIG. 7 provides graphs illustrating dimensional change of a cured product according to Example 5 (A) and dimensional change of a composite according to Example 19 (B) with the change of the temperature;
FIG. 8 provides graphs illustrating dimensional change of a cured product according to Example 8 (A) and dimensional change of a composite according to Example 22 (B) with the change of the temperature; and
FIG. 9 provides graphs illustrating dimensional change of a cured product according to Example 12 (A) and dimensional change of a composite according to Example 28 (B) with the change of the temperature.

### [Best Mode for Invention]

Exemplary embodiments of the present disclosure will now be described in detail with reference to the accompanying drawings.

The disclosure may, however, be exemplified in many different forms and should not be construed as being limited to the specific embodiments set forth herein. Rather, these embodiments are provided so that this disclosure will be thorough and complete, and will fully convey the scope of the disclosure to those skilled in the art.

In the drawings, the shapes and dimensions of elements may be exaggerated for clarity, and the same reference numerals will be used throughout to designate the same or like elements.

The present disclosure provides an epoxy composition including an alkoxysilylated epoxy compound and inorganic particles, a composite obtained by curing the epoxy composition exhibiting improved heat resistance property, a particularly low CTE and a higher Tg or Tg-less, a cured product formed by using the composition, and a use of the composition. In the present disclosure, "composite" refers to a cured product formed by using a composition including an epoxy compound and inorganic particles. In the present disclosure, "cured product" refers to a cured product formed by using a composition including an epoxy compound as having general meaning, for example, a cured product formed by using a composition including an epoxy compound and a curing agent, and at least one selected from the group consisting of inorganic particles, an additional curing agent, an optional curing accelerator and other additives. In addition, the term "cured product" is also used to denote a "partially-cured product". The term "cured product" may be considered to have the same meaning as the term "composite."

When forming a composite through curing the epoxy composition including the alkoxysilylated epoxy compound and the inorganic particles in accordance with the present disclosure, an epoxy group may react with a curing agent to conduct a curing reaction, and the alkoxysilyl group may form bonding at an interface with the surface of the inorganic particles. Thus, very high chemical bonding efficiency in an epoxy composite system may be obtained, and thus, a low CTE and high glass transition temperature increasing effect or Tg-less may be achieved. Therefore, dimensional stability may be improved. In addition, additional silane coupling agents are not necessary.

Further, the epoxy composition including the alkoxysilylated epoxy compound and the inorganic particles according the present disclosure exhibits good curing property. The curing property include both thermal curing property and photo curing property.

In addition, when applying the epoxy composition of the present disclosure to a chemically treated metal film such as a copper film, a chemical bonding may be formed with a -OH group or the like on the surface of the metal produced through the metal surface treatment, thereby exhibiting good adhesion with respect to the metal film.

Hereinafter, an epoxy composition including an alkoxysilylated epoxy compound and inorganic particles, a cured product thereof, a use thereof, and a method of preparing the alkoxysilylated epoxy compound according to an embodiment of the present disclosure will be described in detail.

According to an embodiment of the present disclosure, an epoxy composition including an alkoxysilylated epoxy compound containing at least one substituent of the following Formula S1 and two epoxy groups at the core thereof, and inorganic particles is provided. According to another embodiment of the present disclosure, an epoxy composition including an alkoxysilylated epoxy compound containing at least one substituent of the following Formula S1 and two epoxy groups at the core thereof, inorganic particles, and a curing agent is provided.

[Formula S1] -CR_{b}R_{c}-CHRₐ-CH₂-SiR₁R₂R₃

in the above Formula S1, Rₐ, R_{b} and R_{c} are independently H, or an alkyl group having 1 to 6 carbon atoms, at least one of R₁ to R₃ is an alkoxy group having 1 to 6 carbon atoms, and the remainder thereof are alkyl groups having 1 to 10 carbon atoms, while the alkyl group and the alkoxy group may be a linear chain or a branched chain alkyl group or alkoxy group. Preferably, R₁ to R₃ are ethoxy groups.

In the case in which the core is benzene, and S1 is one, a compound in which all of Rₐ, R_{b} and R_{c} in the above Formula S1 are hydrogen, and R₁ to R₃ are alkoxy groups having 1 to 6 carbon atoms is excluded.

The epoxy group may be a particularly substituent of the following Formula S2.

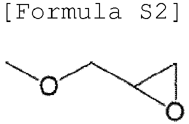

Further, the alkoxysilylated epoxy compound may further include a substituent of the following Formula S3. 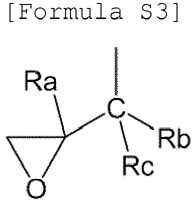 in Formula S3, Rₐ, R_{b} and R_{c} are independently H, or an alkyl group having 1 to 6 carbon atoms, and the alkyl group may be a linear chain or a branched chain alkyl group.

The term "core" refers to a linear chain or a branched chain, a cyclic or a non-cyclic, or an aromatic or an aliphatic hydrocarbon compound capable of containing at least three substituents, and may or may not include a heteroatom such as N, O, S, or P.

The term "aromatic compound" denotes an aromatic compound defined in the chemistry field and includes an aromatic compound not containing a heteroatom or a heteroaromatic compound. In the heteroaromatic compound, the heteroatom may include N, O, S, or P.

The core may be an aromatic compound. The aromatic compound may include benzene, naphthalene, biphenyl, fluorene, anthracene, phenanthrene, chrysene, pyrene, annulene, corannulene, coronene, purine, pyrimidine, benzopyrene, dibenzanthracene, or hexahelicene, without limitation, and may include a polycyclic aromatic compound obtained by combining at least one of the above compounds directly or via a covalent bond using a linker.

The linker may be -CRₑR_{f}- (where Rₑ and R_{f} are independently hydrogen, a halogen atom such as F, Cl, Br, or I, an alkyl group having 1 to 3 carbon atoms, or a cyclic compound containing 4 to 6 carbon atoms), carbonyl (-CO-), ester (-COO-), carbonate (-OCOO-), ethylene (-CH₂CH₂-), propylene (-CH₂CH₂CH₂-), ether (-O-), amine (-NH-), thioether (-S-), or sulfuryl (-SO₂-).

The core may be one selected from the group consisting of the following Formulae A' to K'. 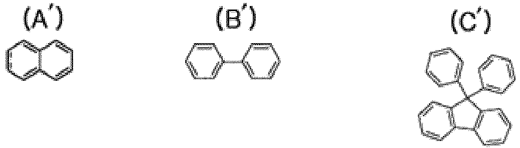 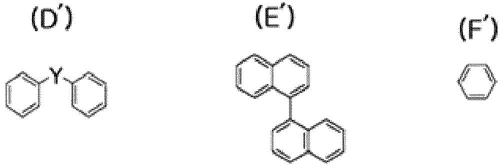 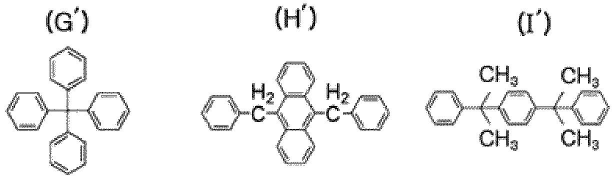 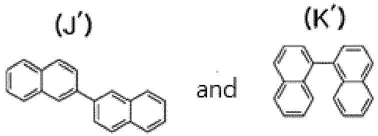 in the above D', Y is -CH₂, -C(CH₃)₂-, -C(CF₃)₂-, -S-, or -SO₂-.

In an embodiment of the present disclosure, the alkoxysilylated epoxy compound may be, for example, at least one selected from the group consisting of the following Formulae (AI) to (KI). 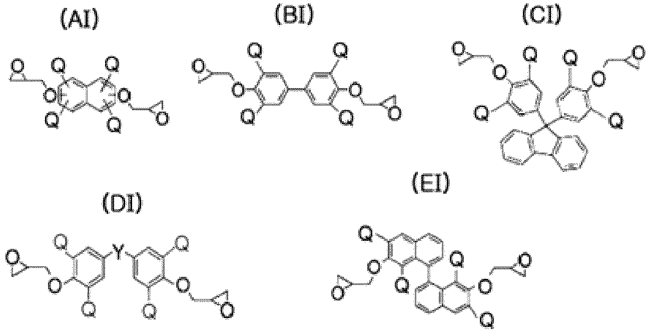 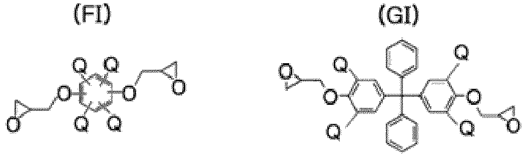 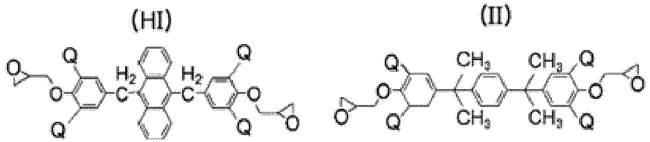 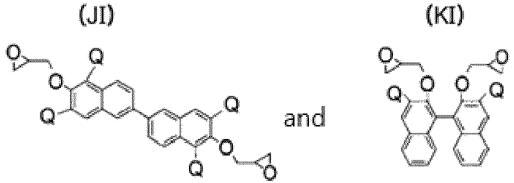 in the above Formulae (AI) to (KI), at least one, for example, one to four, of a plurality of Q is formed of the above Formula S1, and the remainder thereof are independently selected from the group consisting of the above Formula S3, hydrogen, and -CR_{b}R_{c}-CRₐ=CH₂, where Rₐ, R_{b} and R_{c} are independently H, or an alkyl group having 1 to 6 carbon atoms, and the alkyl group may be a linear chain or a branched chain alkyl group, and in the above DI, Y is -CH₂, -C(CH₃)₂-, -C(CF₃)₂-, -S-, or -SO₂-.

In the above Formula FI, in the case in which S1 is one, a compound in which all of Rₐ, R_{b} and R_{c} in Formula S1 are hydrogen, and R₁ to R₃ are alkoxy groups having 1 to 6 carbon atoms is excluded.

In addition, in an embodiment of the present disclosure, the alkoxysilylated epoxy compound may be one selected from the group consisting of the above Formulae AI to DI. In addition, in an embodiment of the present disclosure, the alkoxysilylated epoxy compound may be formed of the above Formula BI or formed of the above Formula DI. In addition, in the above Formula DI, Y may be, for example, -C(CH₃)₂-. Further, the alkoxysilylated epoxy compound of the above Formulae AI to KI may include one to three substituents of the above Formula S3. That is, by including the S3 substituent, the glass transition temperature of the composite may be further increased, or the composite may become Tg-less.

In another embodiment of the present disclosure, the alkoxysilylated epoxy compound may be one of compounds in the following Formula M.

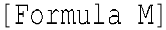

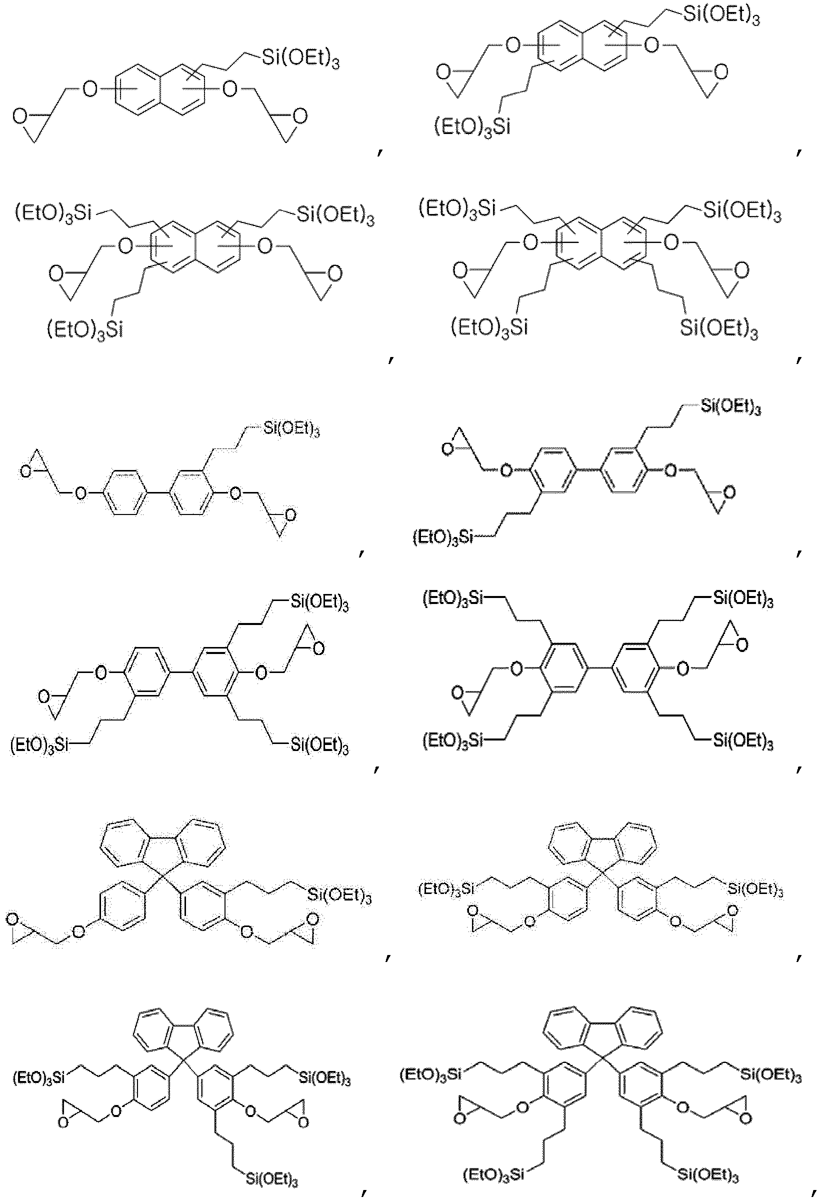

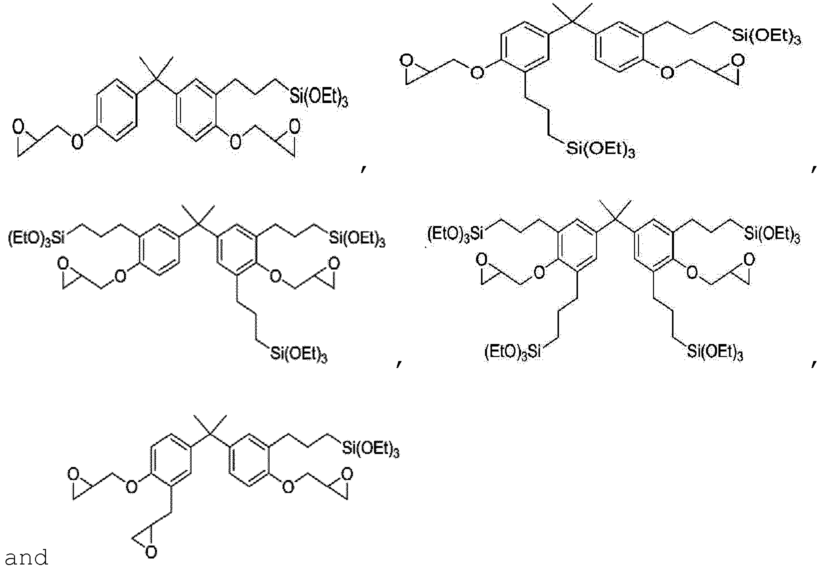

According to a further another embodiment of the present disclosure, the alkoxysilylated epoxy compound may be an epoxy polymer selected from the group consisting of the following Formulae AP to KP.

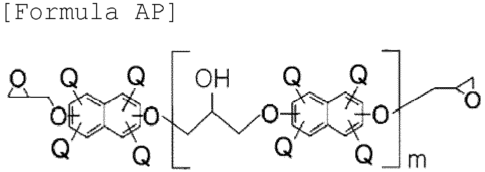

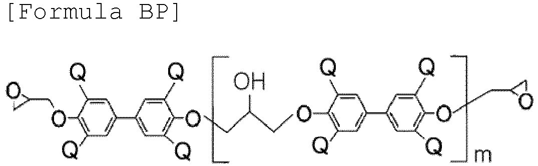

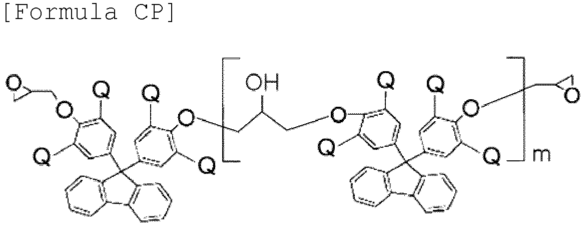

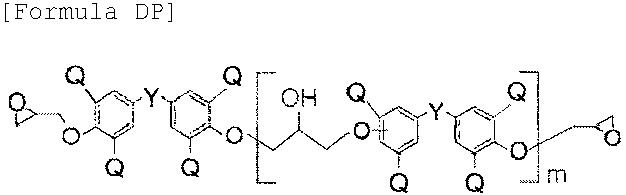

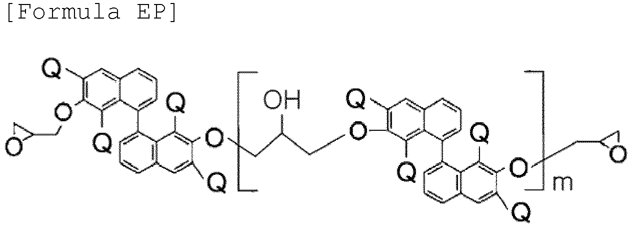

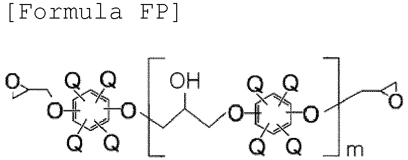

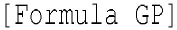

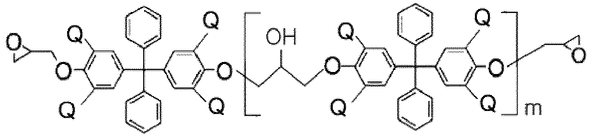

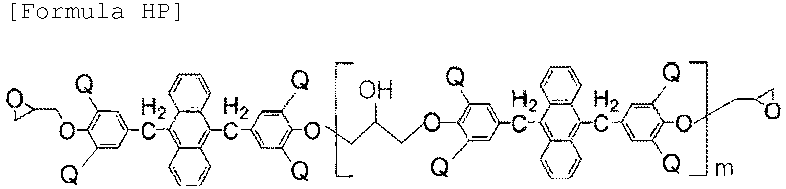

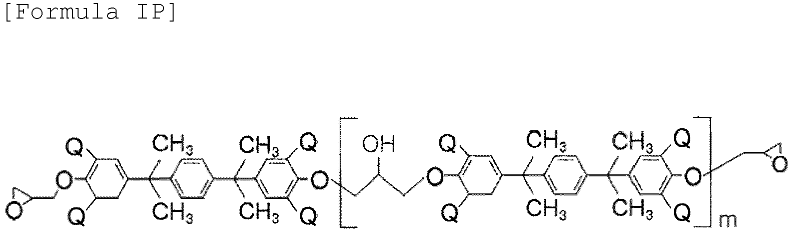

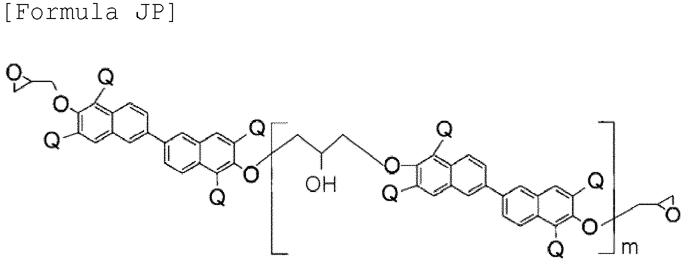

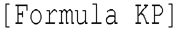

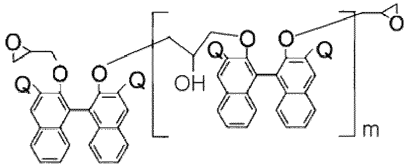

In the above Formulae AP to KP, Q and Y are the same as defined in the above Formulae (AI) to (KI), and m is an integer from 1 to 100.

An epoxy composition according to an embodiment of the present disclosure may include any kind and/or any mixing ratio known in the art only when including an alkoxysilylated epoxy compound of the above Formulae AI to KI (hereinafter an 'epoxy compound of the present disclosure') provided in any embodiments of the present disclosure and inorganic particles. In this case, the kind and the mixing ratio of the curing agent, the curing accelerator (catalyst), the inorganic material (filler) (for example, other inorganic particles and/or a fiber), other common epoxy compounds and other additives are not limited.

Further, the epoxy composition, the cured product and/or the composite may be used with various kinds of epoxy compounds in consideration of the controlling feature of physical properties according to the application and/or use thereof. Thus, in the epoxy compositions according to any embodiments of the present disclosure, the epoxy compound may include an alkoxysilylated epoxy compound of the above Formulae AI to KI according to any embodiments of the present disclosure, and any kind of epoxy compound known in this art (hereinafter a 'common epoxy compound').

The common epoxy compounds may be any epoxy compounds commonly known in this art without limitation, and may be, for example, at least one epoxy compound selected from the group consisting of a glycidyl ether-based epoxy compound, a glycidyl-based epoxy compound, a glycidyl amine-based epoxy compound, a glycidyl ester-based epoxy compound, a rubber modified epoxy compound, an aliphatic polyglycidyl-based epoxy compound and an aliphatic glycidyl amine-based epoxy compound. Further, the common epoxy compound may be at least one epoxy compound selected from the group consisting of the glycidyl ether-based epoxy compound, the glycidyl-based epoxy compound, the glycidyl amine-based epoxy compound, the glycidyl ester-based epoxy compound, the rubber modified epoxy compound, the aliphatic polyglycidyl-based epoxy compound and the aliphatic glycidyl amine-based epoxy compound including bisphenol A, bisphenol F, bisphenol S, biphenyl, naphthalene, benzene, thiodiphenol, fluorene, anthracene, isocyanurate, triphenylmethane, 1,1,2,2-tetraphenylethane, tetraphenylmethane, 4,4'-diaminodiphenylmethane, an aminophenol, a cyclo aliphatic compound, or a novolak unit, as a core structure.

For example, the common epoxy compound may be at least one epoxy compound selected from the group consisting of the glycidyl ether-based epoxy compound, the glycidyl-based epoxy compound, the glycidyl amine-based epoxy compound, the glycidyl ester-based epoxy compound, the rubber modified epoxy compound, the aliphatic polyglycidyl-based epoxy compound and the aliphatic glycidyl amine-based epoxy compound including bisphenol A, bisphenol F, bisphenol S, biphenyl, naphthalene, or fluorene as a core structure. More particularly, the common epoxy compound may include the bisphenol A, the biphenyl, the naphthalene, or the fluorene as the core structure.

Any epoxy compositions in accordance with an embodiment of the present disclosure may include without limitation, based on the total amount of an epoxy compound, from 1 wt% to 100 wt% of the alkoxysilylated epoxy compound according to any embodiments of the present disclosure and from 0 wt% to 99 wt% of the common epoxy compound; for example, from 10 wt% to 100 wt% of the alkoxysilylated epoxy compound of the present disclosure and from 0 wt% to 90 wt% of the common epoxy compound; for example, from 30 wt% to 100 wt% of the alkoxysilylated epoxy compound of the present disclosure and from 0 wt% to 70 wt% of the common epoxy compound; for example, from 50 wt% to 100 wt% of the alkoxysilylated epoxy compound of the present disclosure and from 0 wt% to 50 wt% of the common epoxy compound; for example, from 10 wt% to below 100 wt% of the alkoxysilylated epoxy compound of the present disclosure and from an excess of 0 wt% to 90 wt% of the common epoxy compound; for example, from 30 wt% to below 100 wt% of the alkoxysilylated epoxy compound of the present disclosure and from an excess of 0 wt% to 70 wt% of the common epoxy compound; for example, from 50 wt% to below 100 wt% of the alkoxysilylated epoxy compound of the present disclosure and from an excess of 0 wt% to 50 wt% of the common epoxy compound.

Any inorganic particles known to be used to decrease the thermal expansion coefficient of a common organic resin may be used. Examples of such inorganic particles may include, without limitation, at least one selected from the group consisting of at least one metal oxide selected from the group consisting of silica (including, for example, fused silica and crystalline silica), zirconia, titania, alumina, silicon nitride and aluminum nitride, T-10 type silsesquioxane, ladder type silsesquioxane, and cage type silsesquioxane. The inorganic particles may be used alone or as a mixture of two or more thereof.

In the case in which a particularly large content of the inorganic particles are mixed, the fused silica is preferably used. The fused silica may have any shape among a cataclastic shape and a spherical shape. However, the spherical shape is preferable to increase the mixing ratio of the fused silica and to restrain the increase of the fused viscosity of a forming material.

The inorganic particles having a particle size of 0.5 nm to several tens of µm (for example, from 50 µm to 100 µm) may be used in consideration of the use of a composite, particularly, the dispersibility of the inorganic particles, or the like. Since the dispersibility of the inorganic particle in the epoxy matrix may be different according to the particle size, the inorganic particles having the above-described size may preferably be used. In addition, the distribution range of the inorganic particles to be mixed is preferably increased to increase the mixing ratio of the inorganic particles.

In the epoxy composition in accordance with an embodiment of the present disclosure, the mixing content of the inorganic particles with respect to the epoxy compound may be appropriately controlled in consideration of the CTE decrease of an epoxy composite and an appropriate viscosity required during application thereof. The content of the inorganic particles may be 5 wt% to 95 wt%, for example, 5 wt% to 90 wt%, for example, 10 wt% to 90 wt%, for example, 30 wt% to 95 wt%, for example, 30 wt% to 90 wt%, for example, 5 wt% to 60 wt%, for example or 10 wt% to 50 wt%, for example, based on the total amount of the epoxy composition.

More particularly, in an exemplary embodiment, when the epoxy composition is used as a semiconductor EMC(epoxy molding compound), or the like, the content of the inorganic particles may be, for example, 30 wt% to 95 wt%, for example, 30 wt% to 90 wt%, without limitation, based on the amount of the epoxy composition in consideration of the CTE value and material processability. In other exemplary embodiments, when the epoxy composition is used in a semiconductor substrate, the content of the inorganic particles may be 5 wt% to 60 wt%, for example, 10 wt% to 50 wt% based on the total amount of the epoxy composition.

In an epoxy composition including a curing agent, any curing agent commonly known as a curing agent of an epoxy compound may be used. For example, an amine compound, a phenol compound, an anhydrous oxide-based compound may be used, without limitation.

More particularly, an aliphatic amine, an alicyclic amine, an aromatic amine, other amines and a modified amine may be used as the amine-based curing agent without limitation. In addition, an amine compound including two or more primary amine groups may be used. Particular examples of the amine curing agents may include at least one aromatic amine selected from the group consisting of 4,4'-dimethylaniline (diamino diphenyl methane, DAM or DDM), diamino diphenyl sulfone (DDS), and m-phenylene diamine, at least one aliphatic amine selected from the group consisting of diethylene triamine (DETA), diethylene tetramine, triethylene tetramine (TETA), m-xylene diamine (MXTA), methane diamine (MDA), N,N'-diethylenediamine (N,N'-DEDA), tetraethylenepentaamine (TEPA), and hexamethylenediamine, at least one alicyclic amine selected from the group consisting of isophorone diamine (IPDI), N-aminoethyl piperazine (AEP), bis(4-amino 3-methylcyclohexyl)methane, and larominc 260, other amines such as dicyanamide (DICY), or the like, and a modified amine such as a polyamide-based compound, an epoxide-based compound, or the like.

Examples of the phenol curing agent may include, without limitation, a phenol novolak resin, a cresol novolak resin, a bisphenol A novolak resin, a xylene novolak resin, a triphenyl novolak resin, a biphenyl novolak resin, a dicyclopentadiene-based resin, a phenol p-xylene resin, a naphthalene-based phenol novolak resin, a triazine compound, or the like.

Examples of the anhydrous oxide-based curing agent may include, without limitation, an aliphatic anhydrous oxide such as dodecenyl succinic anhydride (DDSA), poly azelaic poly anhydride, or the like, an alicyclic anhydrous oxide such as hexahydrophthalic anhydride (HHPA), methyl tetrahydrophthalic anhydride (MeTHPA), methylnadic anhydride (MNA), or the like, an aromatic anhydrous oxide such as trimellitic anhydride (TMA), pyromellitic acid dianhydride (PMDA), benzophenonetetracarboxylic dianhydride (BTDA), or the like, and a halogen-based anhydrous compound such as tetrabromophthalic anhydride (TBPA), chlorendic anhydride (HET), or the like.

In general, the crosslinking density of an epoxy composite may be controlled by the extent of reaction of the curing agent and the epoxy group. According to the target crosslinking density, the stoichiometric ratio of the curing agent to epoxy compound may be controlled. For example, when an amine curing agent is used, the stoichiometric equivalent ratio of the epoxy to amine may be preferably controlled to 0.5 to 2.0, for example, 0.8 to 1.5 in an reaction of the amine curing agent with the epoxy group.

Though the mixing ratio of the curing agent has been explained with respect to the amine curing agent, a phenol curing agent, an anhydrous oxide-based curing agent and any curing agents for curing epoxy compounds not separately illustrated in this application but used for curing may be used by appropriately mixing a stoichiometric amount according to the chemical reaction of the epoxy functional group and the reactive functional group of the curing agent based on the concentration of the total epoxy group in the epoxy composition according to the desired range of the crosslinking density. The above-described parts are commonly known in this field.

As a cationic photo curing agent, any photo curing agents commonly known in this field may be used, without limitation, for example, an aromatic phosphate, an aromatic iodide, an aromatic sulfonate, etc. Particularly, diphenyl iodonium tetrakis(pentafluorophenyl)borate, diphenyl iodonium hexafluorophosphate, diphenyl iodonium hexafluoroantimonate, di(4-nonylphenyl)iodonium hexafluorophosphate, triphenylsulfonium hexafluorophosphate, triphenylsulfonium hexafluoroantimonate, triphenylsulfonium tetrakis(pentafluorophenyl)borate, 4,4'-bis[diphenylsulfonio]diphenylsulfide bishexafluorophosphate, 4,4'-bis[di(β-hydroxyethoxy)phenylsulfonio]diphenylsulfide bishexafluoroantimonate, 4,4'-bis[di(β-hydroxyethoxy)phenylsulfonio]diphenylsulfide bishexafluorophosphate, etc., may be used. In general, the photo curing agent may be used in a ratio of 0.5 to 20 phr, preferably at least 1 phr, and preferably from 1 phr to 15 phr with respect to the epoxy compound.

An optional curing accelerator (catalyst) may be additionally included, as occasion demands, to promote the curing reaction of the alkoxysilylated epoxy compound and the curing agent in any epoxy compositions provided in the present disclosure. Any curing accelerators (catalysts) commonly used for curing an epoxy composition in this art may be used without limitation, for example, an imidazoles, a tertiary amines a quaternary ammonium compounds, an organic acid salt, a phosphorous compounds may be used as curing accelerators.

More particularly, for example, the imidazole-based curing accelerator such as dimethylbenzylamine, 2-methylimidazole (2MZ), 2-undecylimidazole, 2-ethyl-4-methylimidazole (2E4M), 2-phenylimidazole, 1-(2-cyanoethyl)-2-alkyl imidazole, and 2-heptadecylimidazole (2HDI); the tertiary amine-based curing accelerator such as benzyldimethylamine (BDMA), tris dimethylaminomethyl phenol (DMP-30), and triethylenediamine; the quaternary ammonium-based curing accelerator such as tetrabutylammonium bromide, or the like; diazabicycloundecene (DBU), or an organic acid of DBU; the phosphor compound-based curing accelerator such as triphenyl phosphine, phosphoric acid ester, or the like, and a Lewis acid such as BF₃-monoethylamine (BF₃-MEA), or the like, may be illustrated without limitation. Latent curing accelerators may also be used, which are provided by microcapsulating the accelerators and forming complex salts with accelerators, for example. These compounds may be used alone or as a mixture of two or more thereof according to curing conditions.

The mixing amount of the curing accelerator may be a commonly applied mixing amount in this art without limitation. For example, 0.1 to 10 parts per hundred (phr) of resin, parts per weight based on 100 parts per weight of an epoxy compound, preferably, 0.2 to 5 phr of the curing accelerator based on the epoxy compound may be used. The above-described range of the curing accelerator may be preferably used in consideration of curing reaction accelerating effect and the control of curing reaction rate. Through using the above-described range of the curing accelerator, the curing may be rapidly achieved, and the improvement of working throughput may be expected.

In the epoxy composition provided in any embodiment of the present disclosure, other additives such as an organic solvent, a releasing agent, a surface treating agent, a flame retardant, a plasticizer, bactericides, a leveling agent, a defoaming agent, a colorant, a stabilizer, a coupling agent, a viscosity controlling agent, a diluent, or the like may be mixed to control the physical properties of the epoxy composition within the range of undamaging the physical properties of the epoxy composition as occasion demands.

As described above, the term "epoxy composition" used in the present application is understood to include an epoxy compound of the present disclosure, inorganic particles, and other constituents composing the epoxy composition, for example, an optional curing agent, a curing accelerator (catalyst), other common epoxy compounds, a solvent and other additives mixed as occasion demands in this field. Meanwhile, the term "total amount of the epoxy composition" in the present disclosure is used to denote the total amount of all components other than solvents from the components composing the epoxy composition. In general, the solvent may be optionally used to control the amount of the solid content and/or the viscosity of the epoxy composition in consideration of the processability of the epoxy composition, and the like.

The epoxy composition provided in accordance with an exemplary embodiment of the present disclosure may be used as an electronic material. That is, according to a further embodiment of the present disclosure, an electronic material including or manufactured by using any epoxy compositions of an embodiment of the present disclosure may be provided. The electronic material may include, for example, a substrate, a film, a laminate obtained by placing a metal layer on a base layer obtained from the epoxy composition of the present disclosure (including a base layer including or formed by using the composition), a printed circuit board including the laminate, a packaging material (an encapsulating material), a build-up film, or the like. In addition, a semiconductor device including or formed by using the electronic material is provided. An adhesive, a paint composition or a composite material including or formed by using any epoxy compositions provided in any embodiments of the present disclosure is provided.

In accordance with other exemplary embodiments of the present disclosure, a cured product including or manufactured using the epoxy composition provided in accordance with an exemplary embodiment of the present disclosure may be provided. In the case in which the epoxy composition provided in an exemplary embodiment of the present disclosure is practically used, for example, when the epoxy composition is applied as the electronic material, or the like, a cured product may be used. In this art, the cured product of the epoxy composition including the epoxy compound and the inorganic particles may be commonly referred to as a composite.

A composite of any epoxy compositions according to exemplary embodiments of the present disclosure exhibits good heat resistance. Particularly, the composite of any epoxy compositions according to exemplary embodiments of the present disclosure may exhibit a low CTE, 60 ppm/°C or less, for example, 50 ppm/°C or less, for example, 40 ppm/°C or less, for example, 30 ppm/°C or less, for example, 25 ppm/°C or less, for example, 20 ppm/°C or less, for example, 15 ppm/°C or less, for example, 12 ppm/°C or less, for example, 10 ppm/°C or less, for example, 8 ppm/°C or less, for example, 5 ppm/°C or less, for example, 4 ppm/°C or less, for example.

A composite including 75 wt% to 85 wt% of the inorganic particles may have a low CTE of 25 ppm/°C or less, for example, 15 ppm/°C or less, for example, 12 ppm/°C or less, for example, 10 ppm/°C or less, for example, 8 ppm/°C or less, for example, 5 ppm/°C or less, for example, 4 ppm/°C or less, for example. According to another embodiment, a composite including 65 wt% to 75 wt% of the inorganic particles may have a low CTE of 40 ppm/°C or less, for example, 30 ppm/°C or less, for example, 20 ppm/°C or less, for example, 10 ppm/°C or less, for example,. According to a further another embodiment, a composite including 45 wt% to 55 wt% of the inorganic particles may have a low CTE of 60 ppm/°C or less, for example, 50 ppm/°C or less, for example, 40 ppm/°C or less, for example, 30 ppm/°C or less, for example. The physical properties of the composite are good when the CTE value is low, and the lower value of the CTE is not particularly limited.

In addition, Tg of the composite of any epoxy compositions according to the present disclosure may be higher than 100°C, for example, 130°C or above, in addition, for example, 250°C or above. Otherwise, the composite may be Tg-less. The physical properties of the composite are good when the Tg value is high, and the upper value of the Tg is not particularly limited.

In the present application, the values limited by the range include the lower limit, the upper limit, any sub ranges in the range, and all numerals included in the range, unless otherwise specifically stated. For example, C1 to C10 is understood to include all of C1, C2, C3, C4, C5, C6, C7, C8, C9 and C10. In addition, in the case when the lower limit or the upper limit of the numerical range is not defined, it would be found that the smaller or the larger value may provide the better properties. In addition, in the case when the limit is not defined, any values may be included. For example, CTE of 4 ppm/°C or less is understood to include every value in the range such as the CTE of 4, 3, 2, 1 ppm/°C, or the like.

Hereinafter, a method of preparing an alkoxysilylated epoxy compound used in an epoxy composition of the present disclosure will be explained.

The alkoxysilylated epoxy compound may be prepared by performing allylation, Claisen rearrangement, epoxidation and alkoxysilylation with respect to, for example, one of the compounds in the following Formulae (AS) to (KS). Meanwhile, in the method of preparing an alkoxysilylated epoxy compound according to an embodiment of the present disclosure, the Claisen rearrangement may be performed by irradiating electromagnetic waves. Through performing the Claisen rearrangement by irradiating the electromagnetic waves, the rearrangement may be efficiently carried out in a short period of time. As clearly understood from the method of preparing the alkoxysilylated epoxy compound in the following description, in the case in which the allylation and the Claisen rearrangement are carried out once, respectively, alkoxysilylated epoxy compounds of the following Formulae (A14) to (K14) may be obtained, and in the case in which the allylation and the Claisen rearrangement are carried out twice, respectively, alkoxysilylated epoxy compounds of the following Formulae (A26) to (J26) may be obtained. The above Formulae (AI) to (KI) includes both of the following Formulae (A14) to (K14) and Formulae (A26) to (J26).

Particularly, the alkoxysilylated epoxy compound is prepared by a method (Method 1) including allylation of one starting material of the compounds in the following Formulae (AS) to (KS) (first step), the Claisen rearrangement (second step), epoxidation (third step), optional and additional epoxidation (3-1-st step), and alkoxysilylation (fourth step) .

In the first step, a hydroxyl group of one of the starting materials of the following Formulae (AS) to (KS) is allylated to produce an intermediate (11) of the following Formulae (A11) to (K11).

In this case, one of two hydroxyl groups in the starting materials, Formulae (AS) to (KS), may be allylated, or all the two hydroxyl groups may be allylated. According to the number of the hydroxyl group allylated in the first step, the number of functional groups, i.e., alkoxysilyl groups, in the alkoxysilylated epoxy compound of target materials, Formulae (AI) to (KI), may be changed. Particularly, in the case in which only one hydroxyl group is allylated, the number of the alkoxysilyl groups of Formula S1 in the target material may be one. In the case in which two hydroxyl groups are allylated, the maximum number of the alkoxysilyl group of Formula S1 in the target material may be two, or the target material may include one alkoxysilyl group of Formula S1 and one epoxy group of Formula S3. The number of the allylated hydroxyl group may be determined by controlling the equivalent ratio of reacting materials.

In the first step, a reaction between one starting material of the above Formulae (AS) to (KS) and an allyl compound of Formula B1 is performed in the presence of a base and an optional solvent. In this case, 0.5 to 10 equivalents of the allyl group of the allyl compound of Formula B1 may react with respect to 1 equivalent of the hydroxyl group of the starting material. 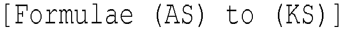 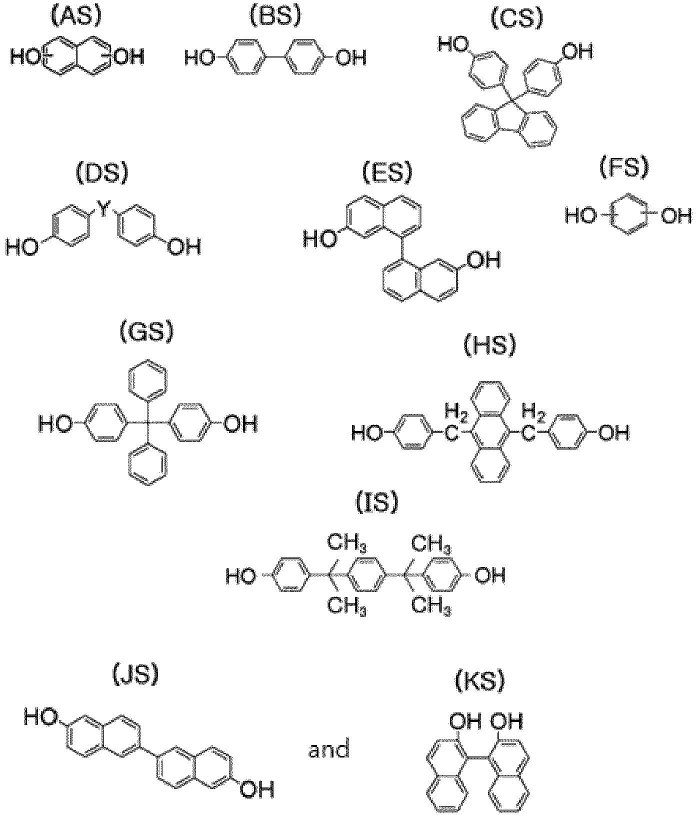 in the above Formula DS, Y is -CH₂-, -C(CH₃)₂-, -C(CF₃)₂-, -S- or -SO₂-. 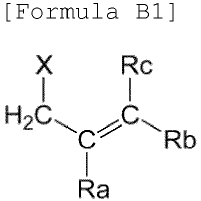 in the above Formula B1, X is Cl, Br, I, -O-SO₂-CH₃, -O-SO₂-CF₃, or -O-SO₂-C₆H₄-CH₃, Rₐ, R_{b} and R_{c} are independently H or an alkyl group having 1 to 6 carbon atoms, and the alkyl group may be a linear chain or a branched chain alkyl group.

The reaction temperature and the reaction time of the first step may change depending on the kind of reacting materials, and the reaction may be performed, for example, within a temperature range of from room temperature (for example, from 15°C to 25°C) to 100°C for 1 to 120 hours to produce one of the above intermediate (11) of the following Formulae (A11) to (K11). 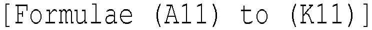 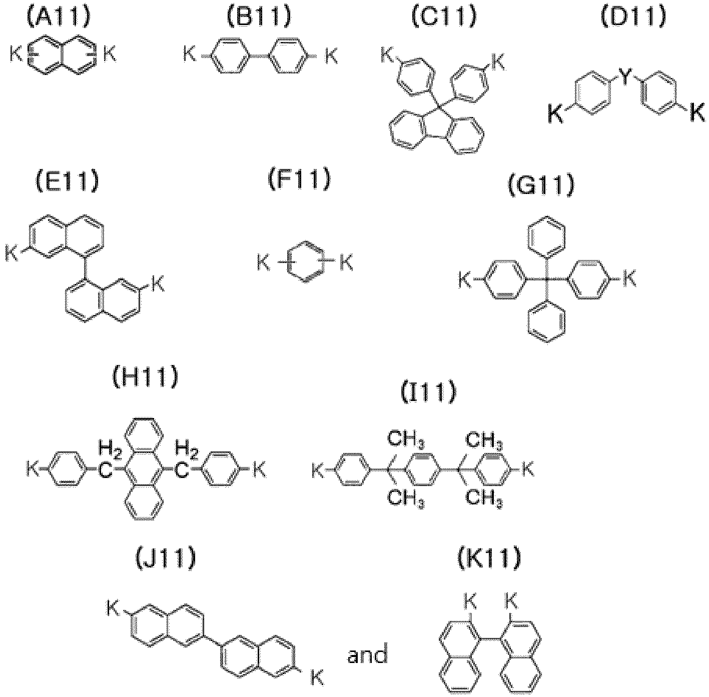 in the above Formulae A11 to K11, at least one of two K is -O-CH₂-CRₐ=CR_{b}R_{c}, where Rₐ, R_{b} and R_{c} are independently H or an alkyl group having 1 to 6 carbon atoms, and the alkyl group may be a linear chain or a branched chain alkyl group, and the remainder thereof is a hydroxyl group, and in the above Formula D11, Y is -CH₂-, -C(CH₃)₂-, -C(CF₃)₂-, -S- or -SO₂-.

The base used may include, for example, KOH, NaOH, K₂CO₃, Na₂CO₃, KHCO₃, NaHCO₃, NaH, triethylamine, and diisopropylethylamine, without limitation. These bases may be used alone or as a combination of two or more thereof. 1 to 5 equivalents of the base may be used based on 1 equivalent of the hydroxyl group of the starting material in consideration of reaction efficiency.

The solvents used during the reaction of the first step may be any solvents. For example, the solvent may not be used if the viscosity of the reacting materials at the reaction temperature is appropriate for carrying out the reaction without using an additional solvent. That is, a additional solvent is not necessary in the case in which the viscosity of the reacting materials is sufficiently low, and the mixing and stirring of the reacting materials may be easily performed without solvents. This may be easily decided upon by a person skilled in the art. In the case in which a solvent is used, any organic solvents may be used only if able to dissolve the reacting materials well, not inducing any adverse influence to the reaction, and being easily removed after the reaction. For example, acetonitrile, tetrahydrofuran (THF), methyl ethyl ketone (MEK), dimethylformamide (DMF), dimethyl sulfoxide (DMSO), methylene chloride (MC), or the like, may be used, without limitation. These solvents may be used alone or as a mixture of two or more thereof. The amount of the solvent may not be limited to being within a specific range, and an appropriate amount of the solvent may be used within a range sufficient for sufficiently dissolving the reacting materials and not adversely affecting the reaction. A person skilled in the art may select an appropriate amount of the solvent in consideration of the above-mentioned points.

In the second step, Claisen rearrangement is performed with respect to the above intermediate (11) obtained in the first step by irradiating electromagnetic waves to produce an intermediate (12) of the following Formulae (A12) to (K12).

That is, the Claisen rearrangement may be performed by irradiating the intermediate (11) with the electromagnetic waves. The reaction of the second step may be performed without an additional solvent or in the presence of a solvent as occasion demands. The solvent may include xylene, 1,2-dichlorobenzene, N,N-diethylaniline, and the like, without limitation.

The electromagnetic waves may include, for example, infrared or microwaves in consideration of reaction efficiency, without limitation, however the microwaves are preferable. The power of the microwaves is not specifically limited, however, the power of the microwaves may be from 100 to 750 W with respect to an excess of the reacting materials of from 0 to 100 g, in consideration of the reaction efficiency. Here, the reacting material denotes total reacting materials including the solvent added for performing the Claisen rearrangement reaction. Meanwhile, the power of the microwaves may be dependent on the shape of the reacting materials, the disposing shape of the reacting material in a reactor, the shape or the design of the reactor, or the like, and may be appropriately controlled by a technical expert in this field in consideration of the exemplified power range. Meanwhile, the reaction temperature and the reaction time during the irradiation of the electromagnetic waves, preferably, infrared or microwaves, and more preferably, the microwaves, may be dependent on the kind of the reacting materials, however the reaction temperature may be from 120°C to 250°C, and the reaction time may be from 1 to 1,000 minutes for performing the Claisen rearrangement.

The Claisen rearrangement reaction may be efficiently performed in a short time by irradiating the electromagnetic waves. Particularly, The Claisen rearrangement may be performed by a common heat treatment without the irradiation of the electromagnetic waves. In this case, the reaction may be performed at the temperature from 120°C to 250°C for 1 to 200 hours. However, through the irradiation of the electromagnetic waves, preferably, infrared or microwaves, and more preferably, the microwaves according to an embodiment of the present disclosure, appropriate waves may be applied, and so, reaction efficiency may be improved, and the reaction time may be decreased to 1 to 1,000 minutes. 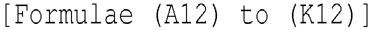 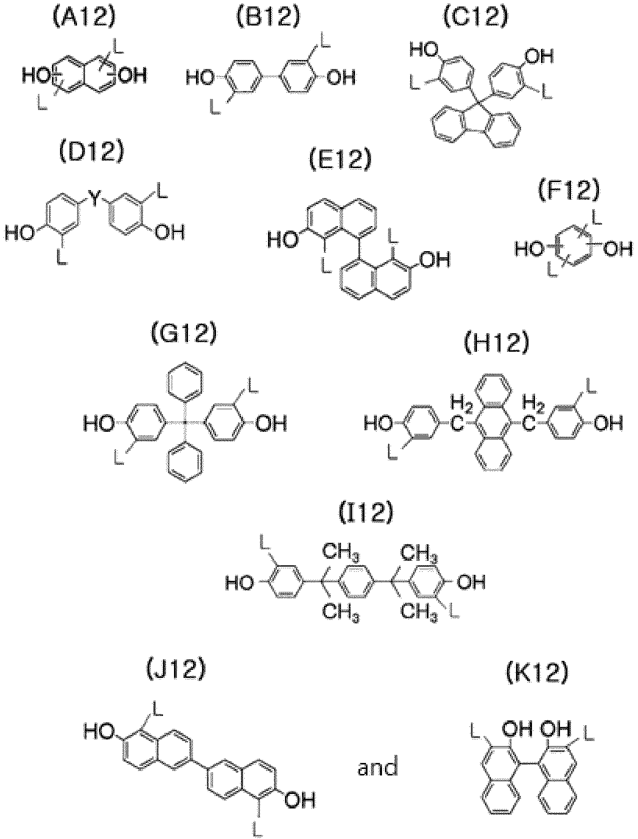 in the above Formulae A12 to K12, at least one of L is -CR_{b}R_{c}-CRₐ=CH₂, where Rₐ, R_{b} and R_{c} are independently H or an alkyl group having 1 to 6 carbon atoms, and the alkyl group may be a linear chain or a branched chain alkyl group, and the remainder thereof are hydrogen atoms, and in the above Formula D12, Y is -CH₂-, -C(CH₃)₂-, -C(CF₃)₂-, -S- or -SO₂-.

In the third step, reaction of an intermediate (12) of the compounds in the above Formulae (A12) to (K12) and epichlorohydrin is performed for the epoxidation of a hydroxyl group and producing an intermediate (13) of the following Formulae (A13) to (K13). In this case, the above intermediate (12) and the epichlorohydrin may react so that 1 to 10 equivalents of an epoxy group (glycidyl group) may react with respect to 1 equivalent of the hydroxyl group of an intermediate (12) in the presence of a base and an optional solvent to produce an intermediate (13). In addition, an excessive amount of the epichlorohydrin may be used instead of using the optional solvent. 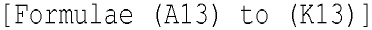 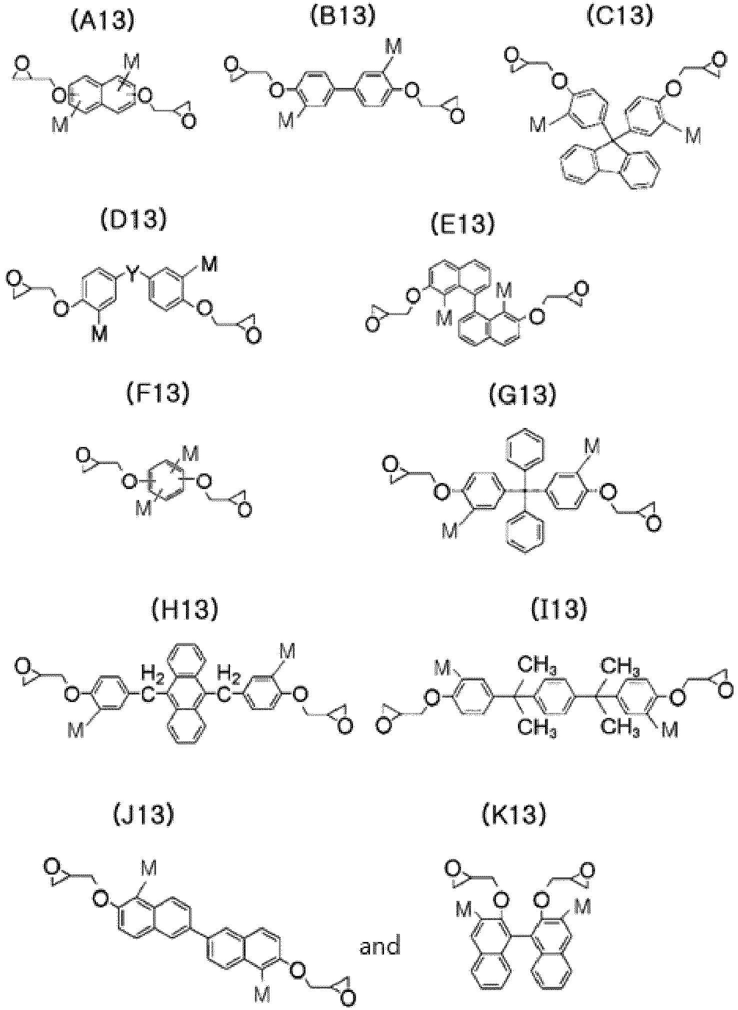 in the above Formulae A13 to K13, at least one of two M is -CR_{b}R_{c}-CRₐ=CH₂, where Rₐ, R_{b} and R_{c} are independently H or an alkyl group having 1 to 6 carbon atoms, and the alkyl group may be a linear chain or a branched chain alkyl group, and the remainder thereof is hydrogen, and in the above Formula D13, Y is -CH₂-, -C(CH₃)₂-, -C(CF₃)₂-, -S- or -SO₂-.

The reaction temperature and the reaction time of the third step may change depending on the kind of reacting materials, and the reaction may be performed, for example, within a temperature range of from room temperature (for example, from 15°C to 25°C) to 100°C for 1 to 120 hours to produce the intermediate (13).

The base used may include, for example, KOH, NaOH, K₂CO₃, Na₂CO₃, KHCO₃, NaHCO₃, NaH, triethylamine, and diisopropylethylamine, without limitation. These bases may be used alone or as a combination of two or more thereof. 1 to 5 equivalents of the base may be used based on 1 equivalent of the hydroxyl group of an intermediate (12) in consideration of reaction efficiency.

The solvents used during the reaction of the third step may be any solvents. For example, the solvent may not be used if the viscosity of the reacting materials at the reaction temperature is appropriate for carrying out the reaction without using an additional solvent. That is, a additional solvent is not necessary in the case in which the viscosity of the reacting materials is sufficiently low, and the mixing and stirring of the reacting materials may be easily performed without solvents. This may be easily decided upon by a person skilled in the art. In the case in which a solvent is used, any solvents may be used only if able to dissolve the reacting materials well, not inducing any adverse influence to the reaction, and being easily removed after the reaction. For example, acetonitrile, THF, MEK, DMF, DMSO, MC, H₂O, or the like, may be used, without limitation. These solvents may be used alone or as a mixture of two or more thereof. The amount of the solvent may not be limited to being within a specific range, and an appropriate amount of the solvent may be used within a range for sufficiently dissolving the reacting materials and not adversely affecting the reaction. A person skilled in the art may select an appropriate amount of the solvent in consideration of the above-mentioned points.

Meanwhile, in the epoxidation process in the third step, a reaction illustrated in the following Reaction 1 may be carried out to perform reaction of an epoxidized intermediate (13) with the hydroxyl group of an intermediate (12) to produce a polymer of at least a dimer as represented by the above Formulae (AP) to (KP).

In the following Reaction 1, an intermediate (B13) is produced by the epoxidation of an intermediate (B12), where all M in B13 are -CH₂-CH=CH₂. 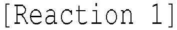 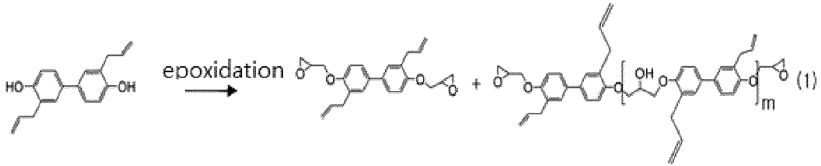 where m is an integer from 1 to 100.

After performing the third step, 3-1-st step of additional epoxidation for the epoxidation of an allyl group may be optionally performed as occasion demands. In the 3-1-st step, the allyl group of an intermediate (13) is oxidized and epoxidized to produce an intermediate (13') of the following Formulae (A13') to (K13').

In the 3-1-st step, an intermediate (13) and a peroxide react in the presence of an optional base and an optional solvent. In this case, 1 to 10 equivalents of the peroxide group of the peroxide react with respect to 1 equivalent of the allyl group of the above intermediate (13). 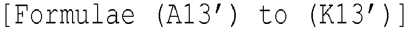 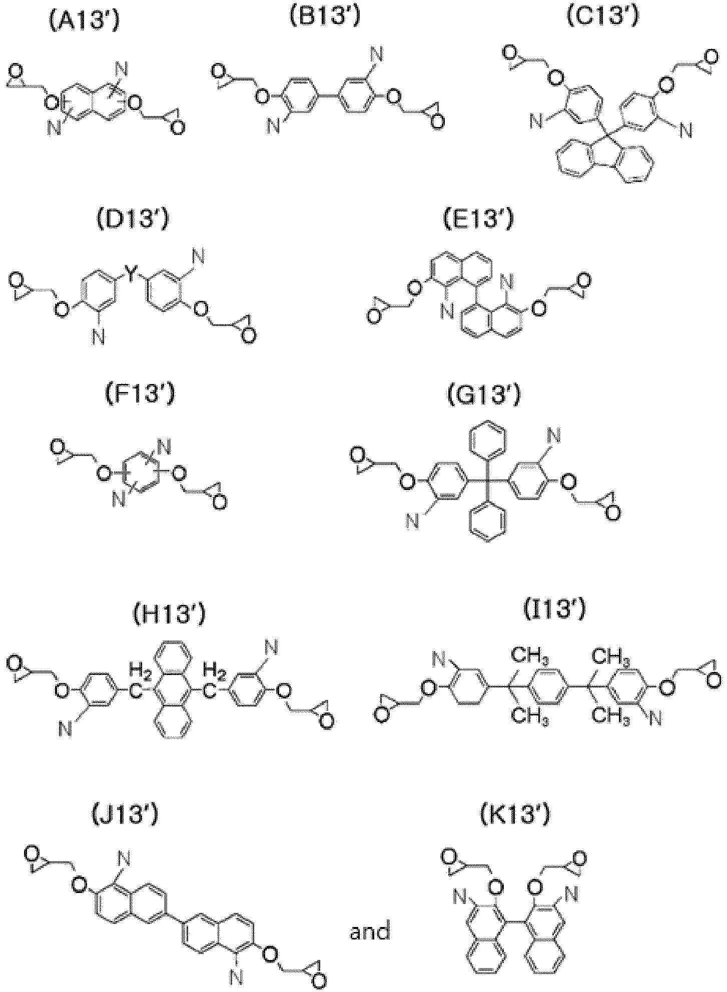 in the above Formulae A13' to K13', one of N is -CR_{b}R_{c}-CRₐ=CH₂, where Rₐ, R_{b} and R_{c} are independently H or an alkyl group having 1 to 6 carbon atoms, and the alkyl group may be a linear chain or a branched chain alkyl group, and the remainder thereof are the form of the following Formula S3, and in the above Formula D13', Y is -CH₂-, -C(CH₃)₂-, -C(CF₃)₂-, -S- or -SO₂-.

The reaction temperature and the reaction time of the 3-1-st step may change depending on the kind of reacting materials, and the reaction may be performed, for example, within a temperature range of from room temperature (for example, from 15°C to 25°C) to 100°C for 1 to 120 hours.

The peroxide may be, for example, m-CPBA, H₂O₂, and DMDO, without limitation. One of the peroxides may be used alone, or two or more thereof may be used simultaneously.

The base may be optionally used in the 3-1-st step as occasion demands. The base is used to neutralize an acid component that may remain after the reaction according to the kind of the peroxide. The base used may include, for example, KOH, NaOH, K₂CO₃, KHCO₃, triethylamine, and diisopropylethylamine. These bases may be used alone or as a combination of two or more thereof. In the case in which the base is used, 0.1 to 5 equivalents of the base may be used on the basis of 1 equivalent of the allyl group of an intermediate (13) in consideration of reaction efficiency.

The solvents used during the reaction of the 3-1-st step may be any solvents. For example, the solvent may not be used if the viscosity of the reacting materials at the reaction temperature is appropriate for carrying out the reaction of the 3-1-st step without using an additional solvent. That is, a additional solvent is not necessary in the case in which the viscosity of the reacting materials is sufficiently low, and the mixing and stirring of the reacting materials may be easily performed without solvents. This may be easily decided upon by a person skilled in the art. In the case in which a solvent is used, any solvents may be used only if able to dissolve the reacting materials well, not inducing any adverse influence to the reaction, and being easily removed after the reaction. For example, acetonitrile, THF, MEK, DMF, DMSO, MC, H₂O, or the like, may be used without limitation. These solvents may be used alone or as a mixture of two or more thereof. The amount of the solvent may not be limited to being within a specific range, and an appropriate amount of the solvent may be used within a range for sufficiently dissolving the reacting materials and not adversely affecting the reaction. A person skilled in the art may select an appropriate amount of the solvent in consideration of the above-mentioned points.

In the fourth step, one of the above intermediate (13) or one of the above intermediates (13') in the case of performing the optional 3-1-st step and an alkoxysilane of the following Formula B2 react to perform the alkoxysilylation of the above intermediate (13) or (13') to produce an epoxy compound containing an alkoxysilyl group.

In the fourth step, the allyl group of an intermediate (13) or an intermediate (13') and the alkoxysilane react according to equivalent ratio on the basis of stoichiometry. Thus, the alkoxysilane of Formula B2 may react with the allyl group of the above intermediate (13) or the above intermediate (13') so that 1 to 5 equivalents of the alkoxysilane of Formula B2 may react with respect to 1 equivalent of the allyl group of the above intermediate (13) or the above intermediate (13').

[Formula B2] HSiR₁R₂R₃

in the above Formula B2, at least one of R₁ to R₃ is an alkoxy group having 1 to 6 carbon atoms, and the remainder thereof are alkyl groups having 1 to 10 carbon atoms, while the alkyl group and the alkoxy group may be a linear chain or a branched chain alkyl group or alkoxy group. Preferably, R₁ to R₃ may be an ethoxy group.

The reaction temperature and the reaction time of the fourth step may change depending on the kind of reacting materials, and the reaction may be performed, for example, within a temperature range of from room temperature (for example, from 15°C to 25°C) to 100°C for 1 to 120 hours.

In the fourth step, the metal catalyst may include a platinum catalyst, for example, PtO₂ or chloroplatinic acid (H₂PtCl₆), without limitation. 1x10⁻⁴ to 0.05 equivalents of the platinum catalyst with respect to 1 equivalent of the allyl group of an intermediate (13) or (13') may be preferably used in consideration of reaction efficiency.

The solvents used during the reaction of the fourth step may be any solvent. For example, the solvent may not be used if the viscosity of the reacting materials at the reaction temperature is appropriate for carrying out the reaction of the fourth step without using an additional solvent. That is, an additional solvent is not necessary in the case in which the viscosity of the reacting materials is sufficiently low, and the mixing and stirring of the reacting materials may be easily performed without solvents. This may be easily decided upon by a person skilled in the art. In the case in which a solvent is used, any aprotic solvents may be used only if able to dissolve the reacting materials well, not inducing any adverse influence to the reaction, and being easily removed after the reaction. For example, toluene, acetonitrile, THF, MEK, DMF, DMSO, MC, or the like, may be used without limitation. These solvents may be used alone or as a mixture of two or more thereof. The amount of the solvent may not be limited to being within a specific range, and an appropriate amount of the solvent may be used within a range for sufficiently dissolving the reacting materials and not adversely affecting the reaction. A person skilled in the art may select an appropriate amount of the solvent in consideration of the above-mentioned points.

In the fourth step, the allyl group of the above intermediate (13) or (13') may be alkoxysilylated via the reaction of the above intermediate (13) or (13') with the alkoxysilane of the above Formula B2 to produce an alkoxysilylated epoxy compound of the following Formulae (A14) to (K14) according to an embodiment of the present disclosure. 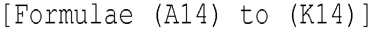 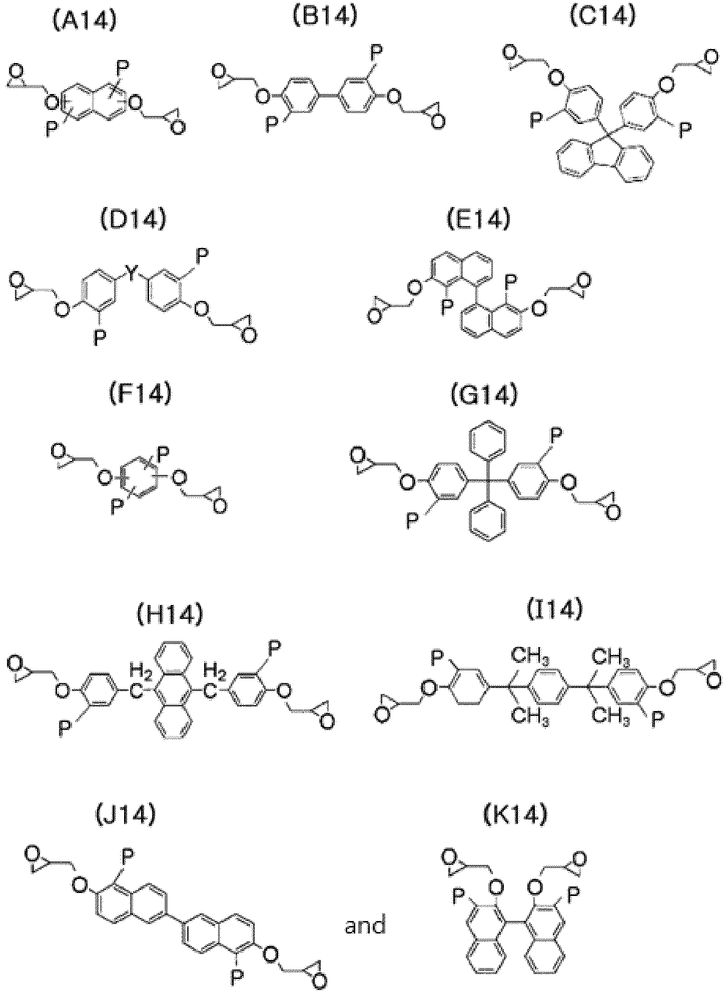 in the above Formulae A14 to K14, at least one of P is the above Formula S1, and the remainder thereof are the above Formula S3, hydrogen or -CR_{b}R_{c}-CRₐ=CH₂, where Rₐ, R_{b} and R_{c} are independently H or an alkyl group having 1 to 6 carbon atoms, and the alkyl group may be a linear chain or a branched chain alkyl group, and preferably are the above Formula S3. In the above Formula D14, Y is -CH₂-, -C(CH₃)₂-, -C(CF₃)₂-, -S- or -SO₂-. Here, in Formula FI, in the case in which S1 is one, a compound in which all of Rₐ, R_{b} and R_{c} are H, and R₁ to R₃ are alkoxy groups having 1 to 6 carbon atoms in the above S1, is excluded.

Exemplary Reaction Schemes (I) to (III) according to the above Method 1 are illustrated in the following. A bisphenol A compound of Formula D1, where Y is -C(CH₃)₂- is illustrated. Reaction Scheme (I) corresponds to a case in which only one hydroxyl group is allylated in the allylation in the first step, Reaction Scheme (II) corresponds to a case in which two hydroxyl groups are allylated in the allylation in the first step, and Reaction Scheme (III) corresponds to a case in which an additional epoxidation of the 3-1-st step is carried out.

Reaction Scheme (I) corresponds to a case in which one of P is -(CH₂)₃SiR₁R₂R₃ (R₁ to R₃ are the same as defined above), and one of P is H in Formula D14, Reaction Scheme (II) corresponds to a case in which all P are -(CH₂)₃SiR₁R₂R₃ (R₁ to R₃ are the same as defined above) in Formula D14, and Reaction Scheme (III) corresponds to a case in which one of P is -(CH₂)₃SiR₁R₂R₃ (R₁ to R₃ are the same as defined above), and one of P is a substituent of Formula S3.

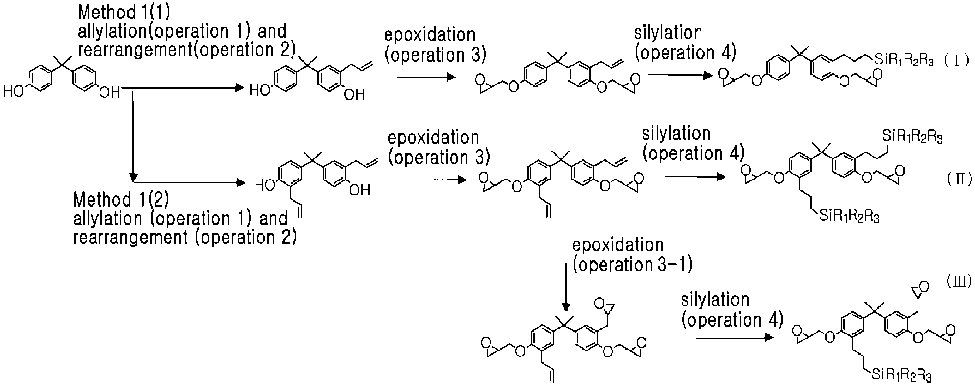

In addition, the alkoxysilylated epoxy compound may be prepared by a method (Method 2) including allylation of a starting material of the above Formulae (AS) to (JS) (first step), Claisen rearrangement (second step), allylation (2-1-st step), Claisen rearrangement (2-2-nd step), epoxidation (third step), an optional epoxidation (3-1-st step), and alkoxysilylation (fourth step).

The first step and the second step in Method 2 are the same as the first step and the second step in the above Method 1, respectively. However, the Claisen rearrangement may not be carried out twice with the starting material of Formula (KS) due to the structure thereof, the starting material of Formula (KS) may not be applied in Method 2. As described in Method 1, one or two of the hydroxyl groups of the starting material may be allylated in the first step.

In the 2-1-st step, by performing the allylation of the hydroxyl group of an intermediate (12) of the above Formulae (A12) to (J12), an intermediate (23) of the following Formulae (A23) to (J23) may be obtained.

In the 2-1-st step, an intermediate (12) and the allyl compound of the above Formula B1 react in the presence of a base and an optional solvent. In this case, 0.5 to 10 equivalents of the allyl group of the allyl compound of the above Formula B1 react with respect to 1 equivalent of the hydroxyl group of the above intermediate (12).

The 2-1-st step is the same as the first step in the above Method 1. Particularly, reaction conditions including the reaction temperature, the reaction time, the equivalent ratio of the reacting materials, and the kind of the base and the amount thereof used and the optional solvent are the same as those in the first step in the above Method 1. 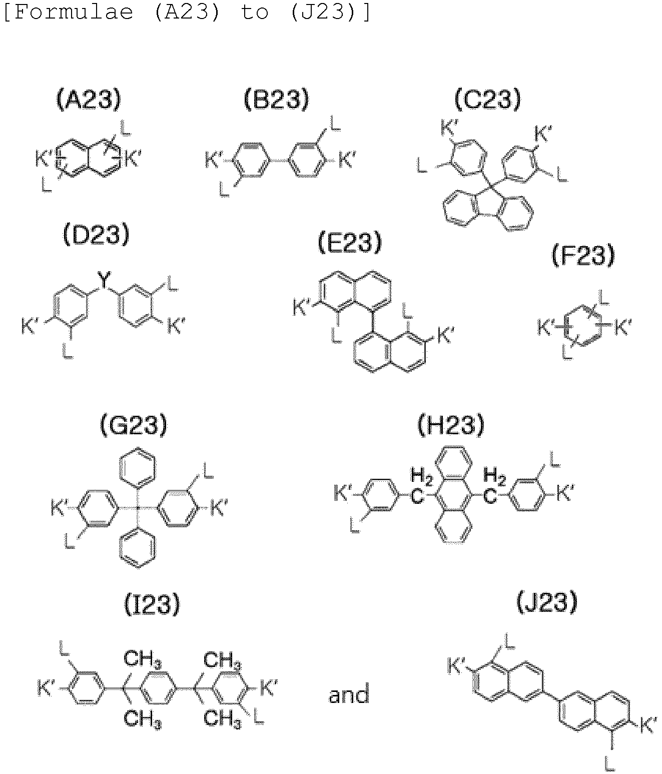 in the above Formulae A23 to J23, at least one of K' is -O-CH₂-CRₐ=CR_{b}R_{c}, where Rₐ, R_{b} and R_{c} are independently H or an alkyl group having 1 to 6 carbon atoms, and the alkyl group may be a linear chain or a branched chain alkyl group, and the remainder thereof are hydroxyl groups, and at least one of L is -CR_{b}R_{c}-CRₐ=CH₂, where Rₐ, R_{b} and R_{c} are independently H or an alkyl group having 1 to 6 carbon atoms, and the alkyl group may be a linear chain or a branched chain alkyl group, and the remainder thereof are hydrogen atoms. In the above Formula D23, Y is -CH₂-, -C(CH₃)₂-, -C(CF₃)₂-, -S- or -SO₂-.

In the above 2-1-st step, only one of the two hydroxyl groups in an intermediate (12) may be allylated, or all the two hydroxyl groups may be allylated. According to the number of the allylated hydroxyl group, the number of the alkoxysilyl functional group, i.e., S1 and/or S3 alkoxy substituents in the alkoxysilylated epoxy compound of target materials of Formulae (A26) to (J26) (or Formulae (AI) to (JI)) may be changed. In this case, the number of the allylated hydroxyl group may be determined by controlling the equivalent ratio of the reacting materials.

In the 2-2-nd step, an intermediate (23) obtained in the 2-1-st step is exposed to electromagnetic waves, preferably infrared or microwaves, and more preferably, microwaves to carry out Claisen rearrangement and to produce an intermediate (24) of the following Formulae (A24) to (J24). The 2-2-nd step is the same as the second step in the above Method 1. Particularly, all reaction conditions including the kind and the power of the electromagnetic waves, the reaction temperature, the reaction time, and the kind and the amount used of the optional solvent are the same as those in the second step of the above Method 1. 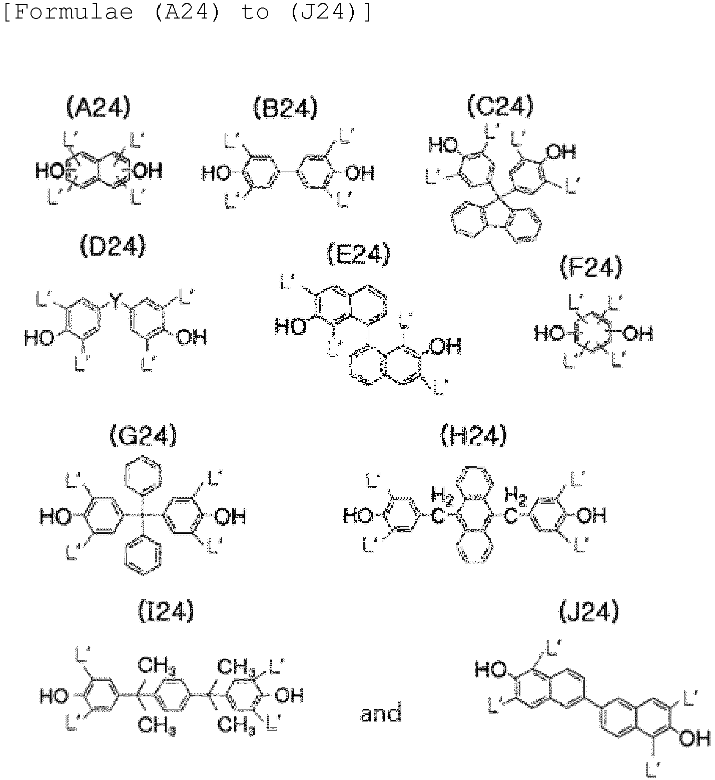 in the above Formulae A24 to J24, at least two, for example, at least three of a plurality of L' is -CR_{b}R_{c}-CRₐ=CH₂, where Rₐ, R_{b} and R_{c} are independently H or an alkyl group having 1 to 6 carbon atoms, and the alkyl group may be a linear chain or a branched chain alkyl group, and the remainder thereof are hydrogen atoms. In the above Formula D24, Y is -CH₂-, -C(CH₃)₂-, -C(CF₃)₂-, -S- or -SO₂-.

In the third step, the hydroxyl group of an intermediate (24) may be epoxidized by performing reaction of an intermediate (24) of the above Formulae (A24) to (J24) with epichlorohydrin to produce an intermediate (25) of the following Formulae (A25) to (J25). In this case, the reaction of an intermediate (24) with the epichlorohydrin is performed so that 1 to 10 equivalents of the epoxy group react with respect to 1 equivalent of the hydroxyl group of an intermediate (24) in the presence of a base and an optional solvent to produce an intermediate (25). The third step in Method 2 is the same as the third step in Method 1. Particularly, all reaction conditions including the reaction temperature, the reaction time, the equivalent ratio of reacting materials, and the kind of the base and the amount thereof used and the optional solvent are the same as those in the third step of the above Method 1. 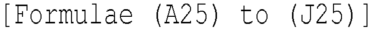 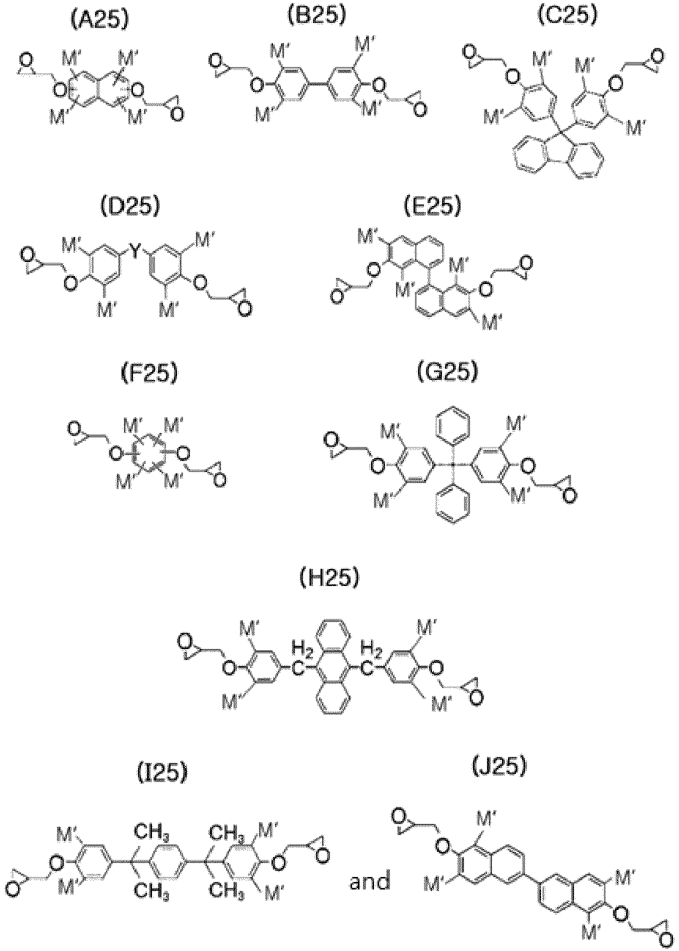 in the above Formulae A25 to J25, at least two, for example, at least three of a plurality of M' are -CR_{b}R_{c}-CRₐ=CH₂, where Rₐ, R_{b} and R_{c} are independently H or an alkyl group having 1 to 6 carbon atoms, and the alkyl group may be a linear chain or a branched chain alkyl group, and the remainder thereof are hydrogen atoms. In the above Formula D25, Y is -CH₂-, -C(CH₃)₂-, -C(CF₃)₂-, -S- or -SO₂-.

Meanwhile, as described in Method 1, the reaction of an epoxidized intermediate (25) and the hydroxyl group of an intermediate (24) is performed in the epoxidation process of the third step as illustrated in the following Reaction 2 to produce a polymer of at least a dimer represented by the above Formulae (AP) to (KP).

In the following Reaction 2, an intermediate (B24) is epoxidized to produce an intermediate (B25). In the above intermediate B25, all M' represent -CH₂-CH=CH₂. 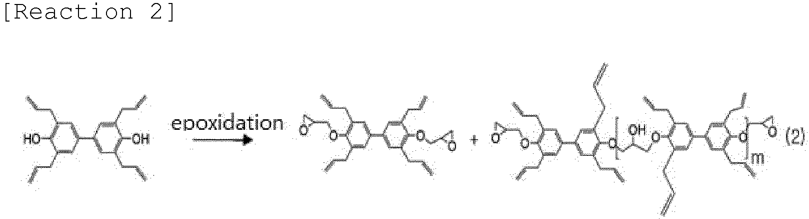 where m is an integer from 1 to 100.

After conducting the third step, additional 3-1-st step for an optional epoxidation of the allyl group may be conducted as occasion demands. In the 3-1-st step, the allyl group of an intermediate (25) is oxidized and epoxidized to produce an intermediate (25') of the following Formulae (A25') to (J25').

In the 3-1-st step, the reaction of a peroxide with the above intermediate (25) is performed in the presence of an optional base and an optional solvent. In this case, the reaction of an intermediate (25) and the peroxide is carried out so that 1 to 10 equivalents of the peroxide group of the peroxide react with 1 equivalent of the allyl group of the above intermediate (25). The 3-1-st step in Method 2 is the same as the 3-1-st step in the above Method 1. Particularly, all reaction conditions including the reaction temperature, the reaction time, the equivalent ratio of reacting materials, and the kind of the base and the amount thereof used and the optional solvent are the same as those in the 3-1-st step of the above Method 1. 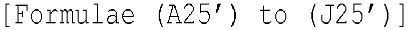 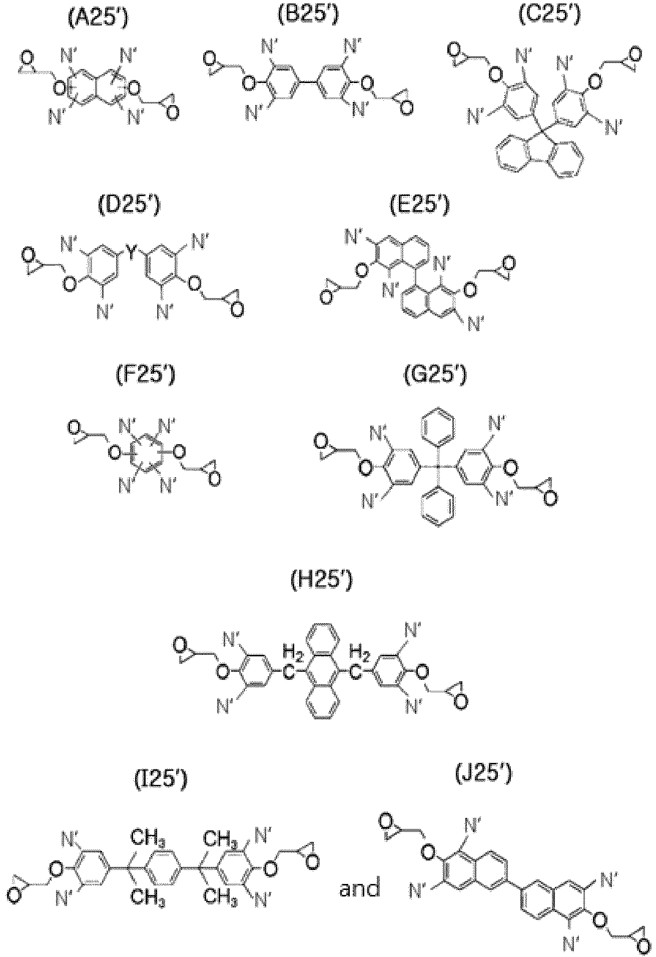 in the above Formulae A25' to J25', one to three of a plurality of N' are -CR_{b}R_{c}-CRₐ=CH₂, where Rₐ, R_{b}, and R_{c} are independently H or an alkyl group having 1 to 6 carbon atoms, and the alkyl group may be a linear chain or a branched chain alkyl group, one to three thereof are the form of the following Formula S3, and the remainder thereof are hydrogen atoms. In the above Formula D25', Y is -CH₂-, -C(CH₃)₂-, -C(CF₃)₂-, -S-or -SO₂-.

In the fourth step, an alkoxysilane reaction of an intermediate (25) or an intermediate (25') in the case in which the optional epoxidation of the 3-1-st step is carried out, with the alkoxysilane of above Formula B2 is carried out to perform the alkoxysilylation of the allyl group of the above intermediate (25) or (25') to produce an alkoxysilylated epoxy compound. In the fourth step, 1 to 5 equivalents of the alkoxysilane of the above Formula B2 with respect to 1 equivalent of the allyl group of the above intermediate (25) or (25') react in the presence of a metal catalyst and an optional solvent to perform the reaction of the above intermediate (25) or (25') with the alkoxysilane of above Formula B2 to produce one of target materials, i.e., the following Formulae (A26) to (J26) (or the above Formulae AI to JI). The reaction conditions of the fourth step in Method 2 are the same as the fourth step in the above Method 1. Particularly, all reaction conditions including the reaction temperature, the reaction time, the equivalent ratio of reacting materials, and the kind and the amount used of the metal catalyst and the optional solvent are the same as those in the fourth step of the above Method 1. 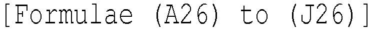 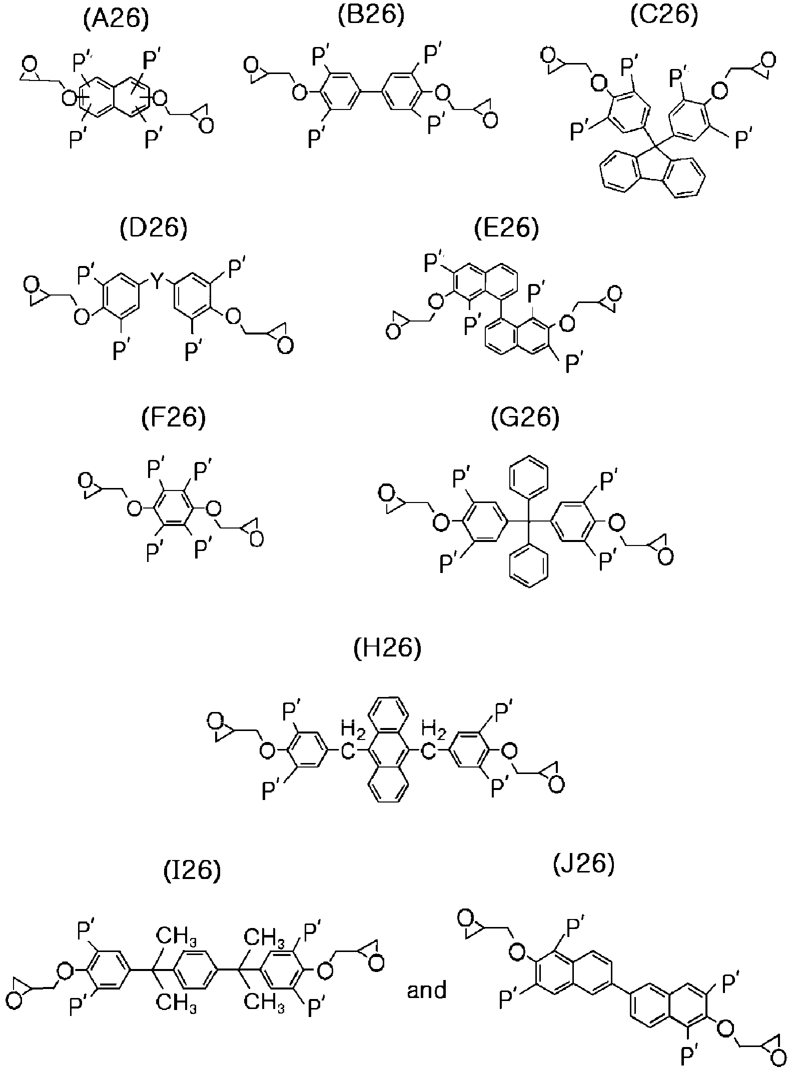 in the above Formulae A26 to J26, at least one of P' , for example, one to four, is the following Formula S1, and the remainder thereof are independently selected from the group consisting of the following Formula S3, hydrogen and -CR_{b}R_{c}-CRₐ=CH₂, where Rₐ, R_{b} and R_{c} are independently H or an alkyl group having 1 to 6 carbon atoms, and the alkyl group may be a linear chain or a branched chain alkyl group. For example, P' may be Formula S3, and Formula S3 may be one to three. In the above Formula D26, Y is -CH₂-, -C(CH₃)₂-, -C(CF₃)₂-, -S- or -SO₂-. In the case in which S1 is one in Formula FI, a compound in which Rₐ, R_{b} and R_{c} are hydrogen, and R₁ to R₃ are an alkoxy group having 1 to 6 carbon atoms in S1 may be excluded.

Reaction Schemes (IV) to (X) according to the above Method 2 are illustrated in the following. A bisphenol A compound where Y is -C(CH₃)₂- in Formula D1 is illustrated. In Reaction Scheme (IV), two hydroxyl groups are allylated during the allylation of the first step and one hydroxyl group is allylated in the 2-1-st step, and three of P' in Formula D26 are - (CH₂)₃SiR₁R₂R₃ (R₁ to R₃ are as defined above), and one thereof is hydrogen. In Reaction Scheme (V), two hydroxyl groups are allylated during the allylation of the first step, one hydroxyl group is allylated in the 2-1-st step, and one allyl group is epoxidized in the optional 3-1-st step. Two of P' in Formula D26 are -(CH₂)₃SiR₁R₂R₃ (R₁ to R₃ are as defined above), one thereof is an epoxy group of Formula S3, and one thereof is hydrogen. In Reaction Scheme (VI), two hydroxyl groups are allylated during the allylation of the first step, one hydroxyl group is allylated in the 2-1-st step, and two allyl groups are epoxidized in the optional 3-1-st step. One of P' in Formula D26 is -(CH₂)₃SiR₁R₂R₃ (R₁ to R₃ are as defined above), two thereof are a substituent of Formula S3, and one thereof is hydrogen. In Reaction Scheme (VII), two hydroxyl groups are allylated in the first step and the 2-1-st step, respectively, and four of P' in Formula D26 are -(CH₂)₃SiR₁R₂R₃ (R₁ to R₃ are as defined above). In Reaction Scheme (VIII), two hydroxyl groups are allylated during the first step and the 2-1-st step, respectively, and one allyl group is epoxidized in the 3-1-st step. Three of P' in Formula D26 are -(CH₂)₃SiR₁R₂R₃ (R₁ to R₃ are as defined above), and one thereof is a substituent of Formula S3. In Reaction Scheme (IX), two hydroxyl groups are allylated in the first step and the 2-1-st step, respectively, and two allyl groups are epoxidized in the optional 3-1-st step. Two of P' in Formula D26 are -(CH₂)₃SiR₁R₂R₃ (R₁ to R₃ are as defined above), and two thereof are substituents of Formula S23. In Reaction Scheme (X), two hydroxyl groups are allylated in the first step and the 2-1-st step, respectively, and three allyl groups are epoxidized in the optional 3-1-st step. One of P' in Formula D26 is -(CH₂)₃SiR₁R₂R₃ (R₁ to R₃ are as defined above), and three thereof are substituents of Formula S3.

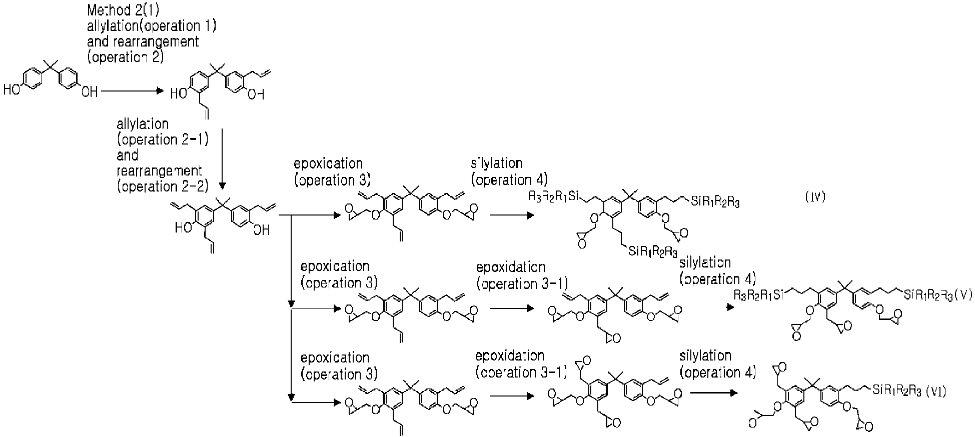

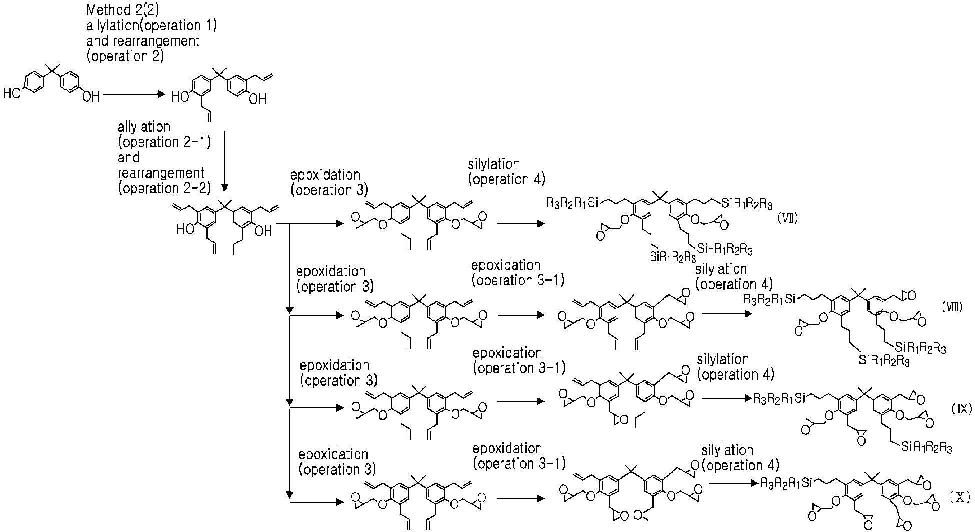

Hereinafter, the present disclosure will be described in detail referring to exemplary embodiments. The following exemplary embodiments are explained for illustration, however the present disclosure is not limited thereto.

### Synthetic Example AI-1(1): Synthesis of mono-alkoxysilylated epoxy compound using dihydroxynaphthalene

### (1) First step: Synthesis of 5-(allyloxy)naphthalene-1-ol

In a 1,000 ml two-necked flask equipped with a refluxing condenser, 20.0 g of 1,5-dihydroxynaphthalene (Sigma-Aldrich), 51.81 g of K₂CO₃, and 500 ml of acetone were added and stirred at room temperature. Then, the temperature of a refluxing apparatus was set to 80°C, and a homogeneously well mixed solution was refluxed (Hereinafter, the temperature described in synthetic examples means the set temperature of the refluxing apparatus). While refluxing the homogeneously well mixed solution, 13.5 ml of allyl bromide (Sigma-Aldrich) was added drop by drop, followed by performing a reaction overnight. After completing the reaction, the reactant was cooled to room temperature and filtered using celite filtration. Organic solvents were evaporated to produce a crude product. A target material in the crude product was extracted with ethyl acetate, washed with water three times, and dried with MgSO₄. MgSO₄ was removed using a filter, and solvents were removed using an evaporator to obtain 5-(allyloxy)naphthalene-1-ol as an intermediate (11). The Reaction Scheme of the first step and NMR data of the intermediate (11) thus obtained are as follows. 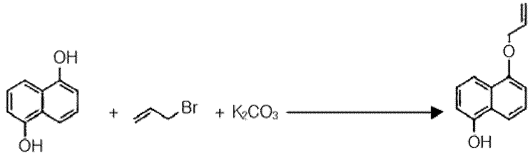 ¹H NMR (400MHz, CDCl₃) : δ=4.70 (dt, J=5.2Hz, 1.6Hz, 2H), 5.33-5.34 (m, 1H), 5.49-5.53 (m, 2H), 6.12-6.20 (m, 1H), 6.82-6.91 (m, 2H), 7.32-7.43 (m, 2H), 7.72 (d, J=8.8Hz, 1H), 7.89 (d, J=8.8Hz, 1H).

### (2) Second step: Synthesis of 2-allylnaphthalene-1,5-diol

In a 100 ml flask, 20.0 g of the intermediate (11) obtained in the first step and 50 ml of 1,2-dichlorobenzene (Sigma-Aldrich) were added, and the flask was inserted in a microwave oven of which power and temperature were set to 300 W and 160°C, followed by reacting for 20 minutes. After completing the reaction, the reactant was cooled to room temperature, and solvents were removed in a vacuum oven to produce 2-allylnaphthalene-1,5-diol as an intermediate (12). The Reaction Scheme of the second step and NMR data of the intermediate (12) thus obtained are as follows. 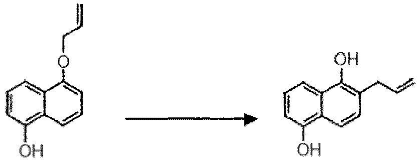 ¹H NMR (400MHz, CDCl₃) : δ=3.57 (d, J=5.8Hz, 2H), 5.09-5.25 (m, 2H), 5.50 (s, 2H), 6.02-6.12 (m, 1H), 6.84 (d, J=8.2Hz, 1H), 7.19 (d, J=8.5Hz, 1H), 7.68-7.72 (m, 2H), 7.89 (d, J=8.8Hz, 1H).

### (3) Third step: Synthesis of 2,2'-(2-allylnaphthalene-1,5-diyl)bis(oxy)bis(methylene)dio xirane

In a 1,000 ml two-necked flask equipped with a refluxing condenser, 7.0 g of the intermediate (12) obtained in the above second step, 22.75 ml of epichlorohydrin (Sigma-Aldrich), 25.95 g of K₂CO₃, and 200 ml of acetonitrile were added and mixed at room temperature. Then, the reaction temperature was elevated to 80°C, and the reaction was performed overnight. After completing the reaction, the reactant was cooled to room temperature and was filtered using celite, and organic solvents were evaporated to obtain an intermediate (13). The Reaction Scheme of the third step and NMR data of the intermediate (13) thus obtained are as follows. 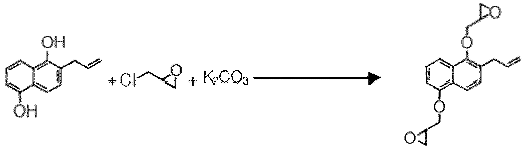 ¹H NMR (400MHz, CDCl₃) : δ=2.76 (dd, J=2. 6Hz, 2H), 2.88 (dd, J=4.2Hz, 2H), 3.10-3.35 (m, 4H), 3.96 (dd, J=5.4Hz, 2H), 4.13 (dd, J=3.2Hz, 2H), 4.96-5.03 (m, 2H), 5.91-6.03 (m, 1H), 6.84 (d, J=8.2Hz, 1H), 7.19 (d, J=8.5Hz, 1H), 7.28-7.38 (m, 2H), 7.90 (d, J=8.8Hz, 1H).

### (4) Fourth step: Synthesis of 3-(1,5-bis(oxirane-2-ylmethoxy)naphthalene-2-yl)propyl)trie thoxysilane

In a 250 ml flask, 10.0 g of the intermediate (13) obtained in the third step, 6.62 ml of triethoxysilane (Sigma-Aldrich), 58 mg of platinum oxide, and 100 ml of toluene were added and well mixed, followed by stirring in an argon charged atmosphere at 85°C for 24 hours. After completing the reaction, the crude product thus obtained was filtered using celite filtration, and solvents were removed using an evaporator to produce a target material of a naphthalene epoxy compound containing an alkoxysilyl group. The Reaction Scheme of the fourth step and NMR data of the target material thus obtained are as follows. 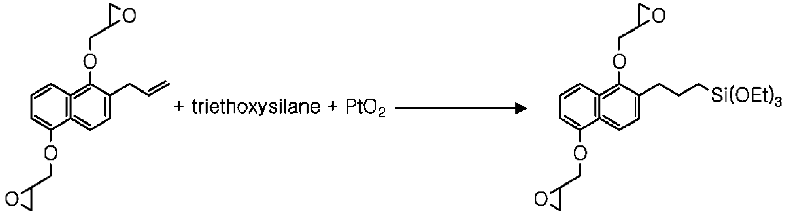 ¹H NMR (400MHz, CDCl₃) : δ=0.65-0.68 (m, 2H), 1.22 (t, J=7.0Hz, 9H), 1.61-1.72 (m, 2H), 2.60 (t, J=7.6Hz, 2H), 2.74 (dd, J=2.6Hz, 2H), 2.86 (dd, J=4.2Hz, 2H), 3.30-3.34 (m, 2H), 3.79 (q, J=1.6Hz, 6H), 3.97 (dd, J=5.2Hz, 2H), 4.14 (dd, J=3.2Hz, 2H), 6.85 (d, J=8.2Hz, 1H), 7.19 (d, J=8.5Hz, 1H), 7.67-7.72 (m, 2H), 7.88 (d, J=8.8Hz, 1H).

### Synthetic Example AI-1(2) : Synthesis of mono-alkoxysilylated epoxy compound using dihydroxynaphthalene

The same produced described in the above Synthetic Example AI-1 (1) was conducted except for conducting the Claisen rearrangement reaction of the second step in the above Synthetic Example AI-1(1) as follows to produce (3-(1,5-bis(oxirane-2-ylmethoxy)naphthalene-2-yl)propyl)tri ethoxysilane.

In the second step, in a 1,000 ml two-necked flask equipped with a refluxing condenser, 10.0 g of the intermediate (11) obtained in the first step of the above Synthetic Example AI-1 (1), and 50 ml of 1, 2-dichlorobenzene (Sigma-Aldrich) were added and well mixed at room temperature. Then, the homogeneous solution thus obtained was refluxed for 8 hours at the set temperature of a refluxing apparatus of 190°C. After completing the reaction, the reactant was cooled to room temperature, and solvents were removed by a vacuum oven to produce 2-allylnaphthalene-1,5-diol as an intermediate (12). The Reaction Scheme and NMR data of the intermediate (12) are the same as those in the second step of the above Synthetic Example AI-1 (1).

### Synthetic Example AI-2(1) : Synthesis of di-alkoxysilylated epoxy compound using dihydroxynaphthalene

### (1) First step: Synthesis of 1,5-bis(allyloxy)naphthalene

In a 1,000 ml two-necked flask equipped with a refluxing condenser, 20.0 g of 1,5-dihydroxynaphthalene (Sigma-Aldrich), 27.0 ml of allyl bromide (Sigma-Aldrich), 103.61 g of K₂CO₃, and 500 ml of acetone were added and stirred at room temperature. Then, the temperature of a refluxing apparatus was set to 80°C, and a homogeneously well mixed solution was refluxed for performing reaction overnight. After completing the reaction, the reactant was cooled to room temperature, filtered using celite filtration and evaporated to produce a crude product. A target material in the crude product was extracted with ethyl acetate, washed with water three times, and dried with MgSO₄. MgSO₄ was removed using a filter, and solvents were removed using an evaporator to obtain 1,5-bis(allyloxy)naphthalene as an intermediate (11). The Reaction Scheme of the first step and NMR data of the intermediate (11) thus obtained are as follows. 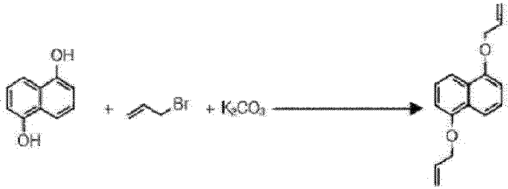 ¹H NMR (400MHz, CDCl₃) : δ=4.70 (dt, J=5.2Hz, 1.6Hz, 4H), 5.32-5.34 (m, 2H), 5.49-5.54 (m, 2H), 6.12-6.21 (m, 2H), 6.84 (d, J=8.0Hz, 2H), 7.35 (dd, J=7. 6, 0.8Hz, 2H), 7.89 (d, J=8. 8Hz, 2H).

### (2) Second step: Synthesis of 2,6-diallylnaphthalene-1,5-diol

In a 100 ml flask, 20.0 g of the intermediate (11) obtained in the first step was added, and the flask was inserted in a microwave oven of which power and temperature was set to 300 W and 160°C, followed by performing reaction for 20 minutes. After completing the reaction, the reactant was cooled to room temperature to produce 2,6-diallylnaphthalene-1,5-diol as an intermediate (12). The Reaction Scheme of the second step and NMR data of the intermediate (12) thus obtained are as follows. 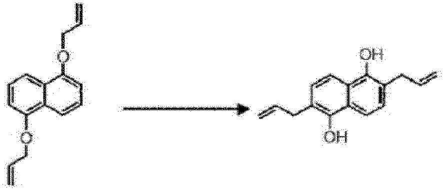 ¹H NMR (400MHz, CDCl₃) : δ=3.57 (dt, J=6.4Hz, 1.6Hz, 4H), 5.21-5.27 (m, 4H), 5.50 (s, 2H), 6.02-6.12 (m, 2H), 7.21 (d, J=8.4Hz, 2H), 7.70 (d, J=8.4Hz, 2H).

### (3) Third step: Synthesis of 2,2'-(2,6-diallylnaphthalene-1,5-diyl)bis(oxy)bis(methylene )dioxirane

In a 1,000 ml two-necked flask equipped with a refluxing condenser, 20.0 g of the intermediate (12) obtained in the second step, 65. 07 ml of epichlorohydrin (Sigma-Aldrich), 74. 15 g of K₂CO₃, and 300 ml of acetonitrile were added and mixed at room temperature. Then, the reaction temperature was elevated to 80°C, and the reaction was performed overnight. After completing the reaction, the reactant was cooled to room temperature and was filtered using celite, and organic solvents were evaporated to obtain an intermediate (13). The Reaction Scheme of the third step and NMR data of the intermediate (13) thus obtained are as follows. 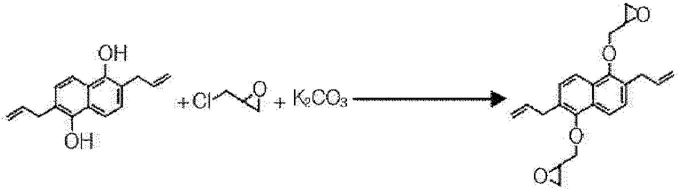 ¹H NMR (400MHz, CDCl₃) : δ=2.77 (dd, J=2. 6Hz, 2H), 2.93 (dd, J=4.4Hz, 2H), 3.44-3.48 (m, 2H), 3.61 (d, J=6.4Hz, 4H), 3.91 (dd, J=6.0Hz, 2H), 4.24 (dd, J=2.8Hz, 2H), 5.07-5.12 (m, 4H), 5.98-6.08 (m, 2H), 7.34 (d, J=8.4Hz, 2H), 7.88 (d, J=8.4Hz, 2H) .

### (4) Fourth step: Synthesis of (3,3'-(1,5-bis(oxirane-2-ylmethoxy)naphthalene-2,6-diyl)bis (propane-3,1-diyl))bis(triethoxysilane)

In a 500 ml flask, 20.0 g of the intermediate (13) obtained in the third step, 23.50 ml of triethoxysilane (Sigma-Aldrich), 200 mg of platinum oxide, and 200 ml of toluene were added and well mixed, followed by stirring in an argon charged atmosphere at 85°C for 24 hours. After completing the reaction, the crude product thus obtained was filtered using celite filtration, and solvents were removed using an evaporator to produce a target material of a naphthalene epoxy compound containing an alkoxysilyl group. The Reaction Scheme of the fourth step and NMR data of the target material thus obtained are as follows. 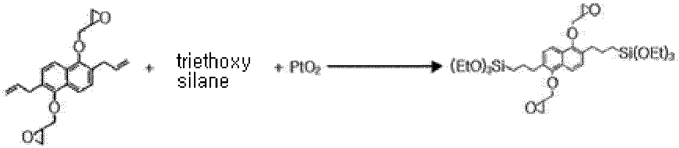 ¹H NMR (400MHz, CDCl₃) : δ=0.64-0.69 (m, 4H), 1.20 (t, J=7.0Hz, 18H), 1.62-1.72 (m, 4H), 2.61 (t, J=7.6Hz, 4H), 2.74 (dd, J=2.6Hz, 2H), 2.86 (dd, J=4.2Hz, 2H), 3.30-3.34 (m, 2H), 3.79 (q, J=1.6Hz, 12H), 3.97 (dd, J=5.2Hz, 2H), 4.14 (dd, J=3.2Hz, 2H), 7.28 (d, J=8.5Hz, 2H), 7.75 (d, J=8.5Hz,2H).

### Synthetic Example AI-2(2) : Synthesis of di-alkoxysilylated epoxy compound using dihydroxynaphthalene

The same procedure described in the above Synthetic Example AI-2 (1) was conducted except for conducting the Claisen rearrangement reaction of the second step in the above Synthetic Example AI-2(1) as follows to produce (3,3'-(1,5-bis(oxirane-2-ylmethoxy)naphthalene-2,6-diyl)bis (propane-3,1-diyl))bis(triethoxysilane).

In the second step, in a 1,000 ml two-necked flask equipped with a refluxing condenser, 20.0 g of the intermediate (11) obtained in the first step of the above Synthetic Example AI-2 (1), and 100 ml of 1, 2-dichlorobenzene (Sigma-Aldrich) were added and stirred at room temperature. Then, the homogeneous solution thus obtained was refluxed for 8 hours at the set temperature of a refluxing apparatus of 190°C. After completing the reaction, the reactant was cooled to room temperature, and solvents were removed by a vacuum oven to produce 2,6-diallylnaphthalene-1,5-diol as an intermediate (12). The Reaction Scheme of the second step and NMR data of the intermediate (12) are the same as those in the second step of the above Synthetic Example AI-2(1).

### Expected Synthetic Example AI-3(1) : Synthesis of tri-alkoxysilylated epoxy compound using dihydroxynaphthalene

### (1) First step: Synthesis of 2,6-bis(allyloxy)naphthalene

In a 1,000 ml two-necked flask equipped with a refluxing condenser, 20.0 g of 2,6-dihydroxynaphthalene (Sigma-Aldrich), 27.0 ml of allyl bromide (Sigma-Aldrich), 103.61 g of K₂CO₃, and 500 ml of acetone are added and stirred at room temperature. Then, the temperature of a refluxing apparatus is set to 80°C, and a homogeneously well mixed solution is refluxed for reaction overnight. After completing the reaction, the reactant is cooled to room temperature, filtered using celite filtration and evaporated to produce a crude product. A target material in the crude product is extracted with ethyl acetate, washed with water three times, and dried with MgSO₄. MgSO₄ is removed using a filter, and solvents are removed using an evaporator to obtain 2,6-bis(allyloxy)naphthalene as an intermediate (11). The Reaction Scheme of the first step is as follows.

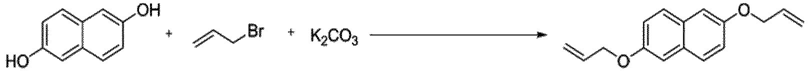

### (2) Second step: Synthesis of 1,5-diallylnaphthalene-2,6-diol

In a 100 ml flask, 20.0 g of the intermediate (11) obtained in the first step is added, and the flask is inserted in a microwave oven of which power and temperature are set to 300 W and 160°C, followed by performing reaction for 20 minutes. After completing the reaction, the reactant is cooled to room temperature to produce 2,6-diallylnaphthalene-1,5-diol as an intermediate (12). The Reaction Scheme of the second step is as follows.

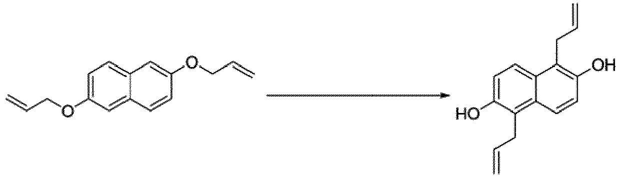

### (3) 2-1-st step: Synthesis of 1,5-dially-6-(allyloxy)naphthalene-2-ol

In a 1,000 ml two-necked flask equipped with a refluxing condenser, 20.0 g of the intermediate (12) obtained in the second step, 29.60 g of K₂CO₃, and 500 ml of acetone are added and mixed at room temperature. Then, the reaction temperature is elevated to the set temperature of a refluxing apparatus of 80°C. While refluxing, 6.78 ml of allyl bromide (Sigma-Aldrich) is added thereto dropwisely, and the reaction is performed overnight. After completing the reaction, the reactant is cooled to room temperature and is filtered using celite, and organic solvents are evaporated to obtain a crude product. A target material in the crude product is extracted with ethyl acetate, washed with water three times, and dried with MgSO₄. MgSO₄ is removed using a filter, solvents are removed using an evaporator, and the product thus obtained is separated by silica column chromatography to obtain 1,5-diallyl-6-(allyloxy)naphthalene-2-ol as an intermediate (23). The Reaction Scheme of the 2-1-st step is as follows.

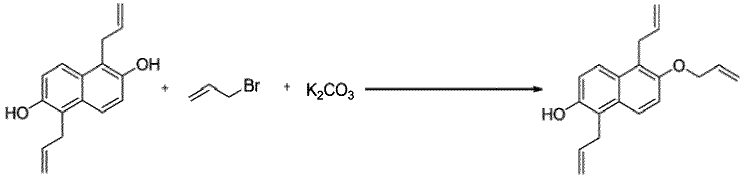

### (4) 2-2-nd step: Synthesis of 1,3,5-triallylnaphthalene-2,6-diol

In a 100 ml flask, 20.0 g of the intermediate (23) obtained in the 2-1-st step is added, and the flask is inserted in an oven of which power and temperature are set to 300 W and 160°C for performing reaction for 20 minutes. After completing the reaction, the reactant is cooled to room temperature to obtain 1,3,5-triallylnaphthalene-2,6-diol as an intermediate (24). The Reaction Scheme of the 2-2-nd step is as follows.

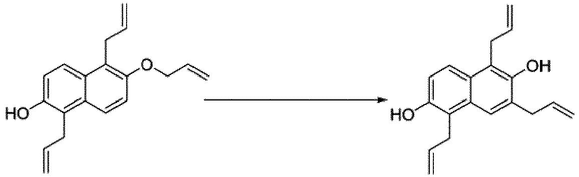

### (5) Third step: Synthesis of 2,2'-(1,3,5-triallylnaphthalene-2,6-diyl)bis(oxy)bis(methyl ene)dioxirane

In a 1,000 ml two-necked flask equipped with a refluxing condenser, 20.0 g of the intermediate (24) obtained in the 2-2-nd step, 55.76 ml of epichlorohydrin (Sigma-Aldrich), 64.49 g of K₂CO₃, and 300 ml of acetonitrile are added and mixed at room temperature. Then, the reaction temperature is elevated to the set temperature of a refluxing apparatus of 80°C, and the reaction is performed overnight. After completing the reaction, the reactant is cooled to room temperature and is filtered using celite, and organic solvents are evaporated to obtain 2,2'-(1,3,5-triallylnaphthalene-2,6-diyl)bis(oxy)bis(methyl ene)dioxirane as an intermediate (25). The Reaction Scheme of the third step is as follows.

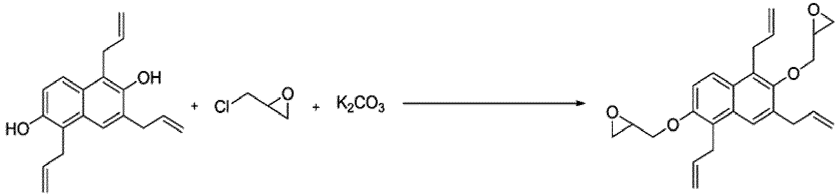

### (6) Fourth step: Synthesis of (3,3',3"-(2,6-bis(oxirane-2-ylmethoxy)naphthalene-1,3,5-tri yl)tris(propane-3,1-diyl))tris(triethoxysilane)

In a 500 ml flask, 20.0 g the intermediate of 2,2'-(1,3,5-triallylnaphthalene-2,6-diyl)bis(oxy)bis(methyl ene)dioxirane obtained in the third step, 31.06 ml of triethoxysilane (Sigma-Aldrich), 348 mg of platinum oxide, and 200 ml of toluene are added and mixed, followed by stirring in an argon charged atmosphere at 85°C for 24 hours. After completing the reaction, the crude product thus obtained is filtered using celite filtration, and solvents are removed using an evaporator to produce a target material of a naphthalene epoxy compound containing an alkoxysilyl group. The Reaction Scheme of the fourth step and NMR data of the target material thus obtained are as follows. 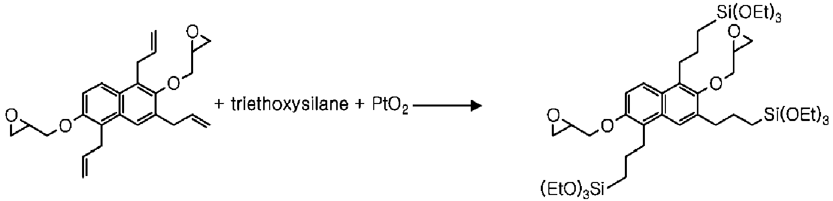 ¹H NMR (400MHz, CDCl₃) : δ=0.65-0.70 (m, 6H), 1.20-1.25 (t, 27H), 1.60-1.70 (m, 6H), 2.60-2.65 (t, 6H), 2.80-2.85 (m, 2H), 2.90-2.95(m, 2H), 3.40-3.45(m, 2H), 3.75-3.80(q, 18H), 4.00-4.05 (m, 2H), 4.30-4.35 (m, 2H), 6.80-7.20(d, 1H), 7.60-7.65(s, 1H), 7.65-7.70(s, 1H).

### Synthetic Example AI-3 (2) : Synthesis of tri-alkoxysilylated epoxy compound using dihydroxynaphthalene

The same procedure described in the above Synthetic Example AI-3 (1) was conducted except for conducting the Claisen rearrangement reaction of the second step and the 2-2-nd step in the above Synthetic Example AI-3(1) as follows to produce (3,3',3"-(2,6-bis(oxirane-2-ylmethoxy)naphthalene-1,3,5-tri yl)tris(propane-3,1-diyl))tris(triethoxysilane).

In the second step, in a 1,000 ml two-necked flask equipped with a refluxing condenser, 20.0 g of the intermediate (11) obtained in the first step of the above Synthetic Example AI-3 (1) and 100 ml of 1, 2-dichlorobenzene (Sigma-Aldrich) were added and well mixed at room temperature. Then, the homogeneous solution thus obtained was refluxed for 8 hours at the set temperature of a refluxing apparatus of 190°C. After completing the reaction, the reactant was cooled to room temperature, and solvents were removed by a vacuum oven to produce 1,5-diallylnaphthalene-2,6-diol as an intermediate (12). The Reaction Scheme of the second step is the same as that of the second step of the above Synthetic Example AI-3(1).

In the 2-1-st step, the same procedure in the 2-1-st step of the above Synthetic Example AI-3 (1) was conducted using the above intermediate (12). Then, in a 1,000 ml two-necked flask equipped with a refluxing condenser, 20.0 g of the intermediate (23) obtained in the 2-1-st step and 100 ml of 1,2-dichlorobenzene (Sigma-Aldrich) were added and well mixed at room temperature. Then, the homogeneous solution thus obtained was refluxed for 8 hours at the set temperature of a refluxing apparatus of 190°C. After completing the reaction, the reactant was cooled to room temperature, and solvents were removed by a vacuum oven to produce 1,3,5-triallylnaphthalene-2,6-diol as an intermediate (24). The Reaction Scheme of the 2-2-nd step is the same as that of the 2-2-nd step of the above Synthetic Example AI-3(1).

### Expected Synthetic Example AI-4(1) : Synthesis of tetra-alkoxysilylated epoxy compound using dihydroxynaphthalene

### (1) First step: Synthesis of 2,6-bis(allyloxy)naphthalene

In a 1,000 ml two-necked flask equipped with a refluxing condenser, 20.0 g of 2,6-dihydroxynaphthalene (Sigma-Aldrich), 27.0 ml of allyl bromide (Sigma-Aldrich), 103.61 g of K₂CO₃, and 500 ml of acetone are added and stirred at room temperature. Then, the temperature of a refluxing apparatus is set to 80°C, and a homogeneously well mixed solution is refluxed for reaction overnight. After completing the reaction, the reactant is cooled to room temperature, filtered using celite filtration and evaporated to produce a crude product. A target material in the crude product is extracted with ethyl acetate, washed with water three times, and dried with MgSO₄. MgSO₄ is removed using a filter, and solvents are removed using an evaporator to obtain 2,6-bis(allyloxy)naphthalene as an intermediate (11). The Reaction Scheme of the first step is as follows.

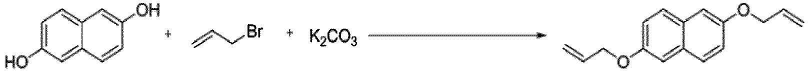

### (2) Second step: Synthesis of 1,5-diallylnaphthalene-2,6-diol

In a 100 ml flask, 20.0 g of the intermediate (11) obtained in the first step is added, and the flask is inserted in a microwave oven of which power and temperature are set to 300 W and 160°C, followed by performing reaction for 20 minutes. Thus, 1,5-diallylnaphthalene-2,6-diol is obtained as an intermediate (12). The Reaction Scheme of the second step is as follows.

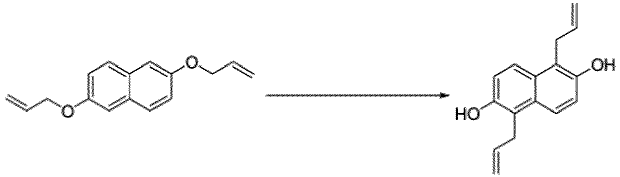

### (3) 2-1-st step: Synthesis of 1,5-dially-2,6-bis(allyloxy)naphthalene

In a 1,000 ml two-necked flask equipped with a refluxing condenser, 20.0 g of the intermediate (12) obtained in the second step, 27.0 ml of allyl bromide (Sigma-Aldrich), 103.61 g of K₂CO₃, and 500 ml of acetone are added and mixed at room temperature. Then, a homogeneously well mixed solution is refluxed at the set temperature of a refluxing apparatus of 80°C to performed reaction overnight. After completing the reaction, the reactant is cooled to room temperature and is filtered using celite, and organic solvents are evaporated to obtain a crude product. A target material in the crude product is extracted with ethyl acetate, washed with water three times, and dried with MgSO₄. MgSO₄ is removed using a filter, and solvents are removed using an evaporator to obtain 1,5-diallyl-2,6-bis(allyloxy)naphthalene as an intermediate (23). The Reaction Scheme of the 2-1-st step is as follows.

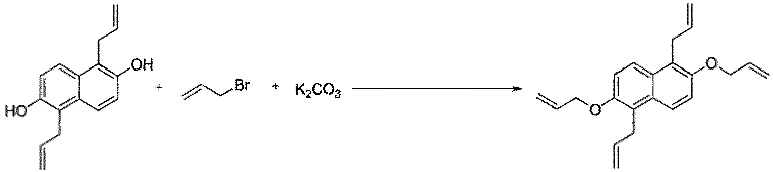

### (4) 2-2-nd step: Synthesis of 1,3,5,7-tetraallylnaphthalene-2,6-diol

In a 100 ml flask, 20.0 g of the intermediate (23) obtained in the 2-1-st step is added, and the flask is inserted in an oven of which power and temperature are set to 300 W and 160°C for performing reaction for 20 minutes. Thus, 1,3,5,7-tetraallylanphthalene-2,6-diol is obtained as an intermediate (24). The Reaction Scheme of the 2-2-nd step is as follows.

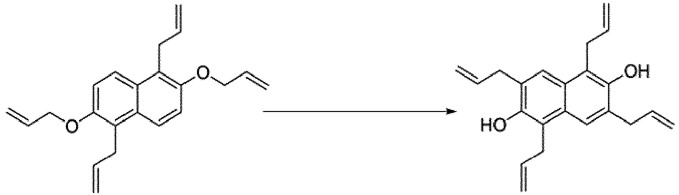

### (5) Third step: Synthesis of 2,2'-(1,3,5,7-tetraallylnaphthalene-2,6-diyl)bis(oxy)bis(me thylene)dioxirane

In a 1,000 ml two-necked flask equipped with a refluxing condenser, 20.0 g of the intermediate (24) obtained in the 2-2-nd step, 55.76 ml of epichlorohydrin (Sigma-Aldrich), 64.49 g of K₂CO₃, and 300 ml of acetonitrile are added and mixed at room temperature. Then, the reaction temperature is elevated to 80°C, and reaction is performed overnight at 80°C. After completing the reaction, the reactant is cooled to room temperature and is filtered using celite, and organic solvents are evaporated to obtain 2,2'-(1,3,5,7-tetraallylnaphthalene-2,6-diyl)bis(oxy)bis(me thylene)dioxirane as an intermediate (25). The Reaction Scheme of the third step is as follows.

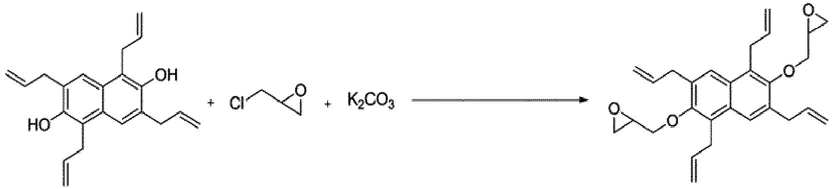

### (6) Fourth step: Synthesis of 3,3',3",3"'-(2,6-bis(oxirane-2-ylmethoxy)naphthalene-1,3,5 ,7-tetrayl)tetrakis(propane-3,1-diyl))tetrakis(triethoxysil ane)

In a 500 ml flask, 20.0 g of the intermediate (25) obtained in the third step, 31.06 ml of triethoxysilane (Sigma-Aldrich), 348 mg of platinum oxide, and 200 ml of toluene are inserted and well mixed, followed by stirring in an argon charged atmosphere at 85°C for 24 hours. After completing the reaction, the crude product thus obtained is filtered using celite filtration, and solvents are removed using an evaporator to produce a target material of a naphthalene epoxy compound containing an alkoxysilyl group. The Reaction Scheme of the fourth step and NMR data of the target material thus obtained are as follows. 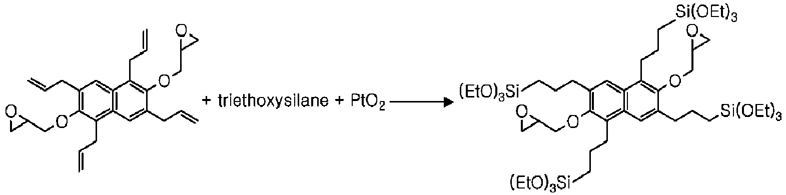 ¹H NMR (400MHz, CDCl₃) : δ=0.65-0.70 (m, 8H), 1.20-1.25 (t, 36H), 1.60-1.70 (m, 8H), 2.60-2.65 (t, 8H), 2.80-2.85 (m, 2H), 2.90-2.95(m, 2H), 3.40-3.45(m, 2H), 3.75-3.80(q, 24H), 4.00-4.05 (m, 2H), 4.30-4.35 (m, 2H), 7.60-7.65(s, 1H), 7.65-7.70(s, 1H).

### Expected Synthetic Example AI-4(2) : Synthesis of tetra-alkoxysilylated epoxy compound using dihydroxynaphthalene

The same procedure described in the above Synthetic Example AI-4 (1) is conducted except for conducting the Claisen rearrangement reaction of the second step and the 2-2-nd step in the above Synthetic Example AI-4(1) as follows to produce (3,3',3",3"'-(2,6-bis(oxirane-2-ylmethoxy)naphthalene-1,3,5 ,7-tetrayl)tetrakis(propane-3,1-diyl))tetrakis(triethoxysil ane).

In the second step, in a 1,000 ml two-necked flask equipped with a refluxing condenser, 20.0 g of the intermediate (11) obtained in the first step of the above Synthetic Example AI-4(1) and 100 ml of 1,2-dichlorobenzene (Sigma-Aldrich) are added and stirred at room temperature. Then, the homogeneous solution thus obtained is refluxed for 8 hours at the set temperature of a refluxing apparatus of 190°C. After completing the reaction, the reactant is cooled to room temperature, and solvents are removed by a vacuum oven to produce 1,5-diallylnaphthalene-2,6-diol as an intermediate (12). The Reaction Scheme of the second step is the same as that of the second step of the above Synthetic Example AI-4 (1).

In the 2-1-st step, the same procedure in the 2-1-st step of the above Synthetic Example AI-4 (1) is conducted using the above intermediate (12). Then, in the 2-2-nd step, in a 1,000 ml two-necked flask equipped with a refluxing condenser, 20.0 g of the intermediate (23) obtained in the 2-1-st step and 100 ml of 1,2-dichlorobenzene (Sigma-Aldrich) are added and well mixed at room temperature. Then, the homogeneous solution thus obtained is refluxed for 8 hours at the temperature of a refluxing apparatus of 190°C. After completing the reaction, the reactant is cooled to room temperature, and solvents are removed by a vacuum oven to produce 1,3,5,7-tetraallylnaphthalene-2,6-diol as an intermediate (24). The Reaction Scheme of the 2-2-nd step is the same as that of the 2-2-nd step of the above Synthetic Example AI-4 (1) .

### Synthetic Example BI-1 (1) : Synthesis of mono-alkoxysilylated epoxy compound using dihydroxybiphenyl

### (1) First step: Synthesis of 4-(allyloxy)biphenyl-4-ol

In a 1,000 ml two-necked flask equipped with a refluxing condenser, 10.0 g of biphenyl-4,4'-diol (Sigma-Aldrich), 22.28 g of K₂CO₃, and 500 ml of acetone were added and stirred at room temperature. Then, the temperature of a refluxing apparatus was set to 80°C, and a homogeneously well mixed solution was refluxed. While refluxing the homogeneous solution, 5.81 ml of allyl bromide (Sigma-Aldrich) was added dropwisely, followed by performing reaction overnight. After completing the reaction, the reactant was cooled to room temperature and filtered using celite filtration. Organic solvents were evaporated to produce a crude product. A target material in the crude product was extracted with ethyl acetate, washed with water three times, and dried with MgSO₄. MgSO₄ was removed using a filter, and solvents were removed using an evaporator to obtain 4-(allyloxy)biphenyl-4-ol as an intermediate (11). The Reaction Scheme of the first step and NMR data of the intermediate (11) thus obtained are as follows. 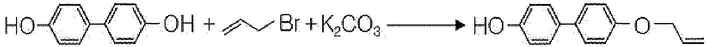 ¹H NMR (400MHz, CDCl₃) : δ=4.56 (dt, J=5.2Hz, 1.6Hz, 2H), 5.30 (m, 2H), 5.41-5.45 (m, 1H), 6.03-6.12 (m, 1H), 6.86(d, J=8.2Hz, 2H), 7.02 (d, J=8.4Hz, 2H), 7.46 (td, J=3.0, 2.2, 8.8Hz, 4H).

### (2) Second step: Synthesis of 3-allylbiphenyl-4,4'-diol

In a 100 ml flask, 20.0 g of the intermediate (11) obtained in the first step and 50 ml of 1,2-dichlorobenzene (Sigma-Aldrich) were added, and the flask was inserted in a microwave oven of which power and temperature were set to 300 W and 160°C, followed by performing reaction for 20 minutes. After completing the reaction, the reactant was cooled to room temperature, and solvents were removed by a vacuum oven to produce 3-diallylbiphenyl-4,4'-diol as an intermediate (12). 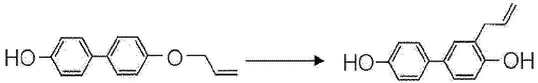 ¹H NMR (400MHz, CDCl₃) : δ=3.35 (d, J=6.4Hz, 2H), 5.08-5.12 (m, 4H), 5.99-6.07 (m, 1H), 6.85-6.90 (m, 3H), 7.30-7.39 (m, 4H).

### (3) Third step: Synthesis of 2,2'-(3-allylbiphenyl-4,4'-diyl)bis(oxy)bis(methylene)dioxi rane

In a 1,000 ml two-necked flask equipped with a refluxing condenser, 10.0 g of the intermediate (12) obtained in the second step, 30.38 ml of epichlorohydrin (Sigma-Aldrich), 35. 13 g of K₂CO₃, and 300 ml of acetonitrile were added and mixed at room temperature. Then, the reaction temperature was elevated to 80°C, and the reaction was performed overnight. After completing the reaction, the reactant was cooled to room temperature and was filtered using celite, and organic solvents were evaporated to obtain an intermediate (13). The Reaction Scheme of the third step and NMR data of the intermediate (13) thus obtained are as follows. 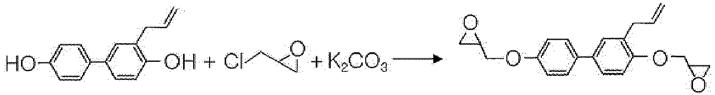 ¹H NMR (400MHz, CDCl₃) : δ=2.75 (dd, J=2. 6Hz, 2H), 2.87 (dd, J=4.2Hz, 2H), 3.10-3.35 (m, 4H), 3.96 (dd, J=5.4Hz, 2H), 4.24 (dd, J=3.2Hz, 2H), 4.97-5.03 (m, 2H), 5.93-6.03 (m, 1H), 6.86-6.95 (m, 3H), 7.31-7.40 (m, 4H).

### (4) Fourth step: Synthesis of (3-(4,4'-bis(oxirane-2-ylmethoxy)biphenyl-3-yl)propyl)triet hoxysilane

In a 250 ml flask, 10.0 g of the intermediate (13) obtained in the third step, 4.84 ml of triethoxysilane (Sigma-Aldrich), 55 mg of platinum oxide (PtO₂), and 100 ml of toluene were added and well mixed, followed by stirring in an argon charged atmosphere at 85°C for 24 hours. After completing the reaction, the crude product thus obtained was filtered using a celite filter, and solvents were removed using an evaporator to produce a target material of a biphenyl epoxy compound containing an alkoxysilyl group. The Reaction Scheme of the fourth step and NMR data of the target material thus obtained are as follows. 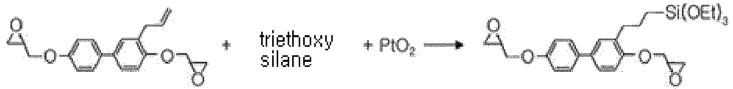 ¹H NMR (400MHz, CDCl₃) : δ=0.64-0.69 (m, 2H), 1.20 (t, J=7.0Hz, 9H), 1.62-1.72 (m, 2H), 2.61 (t, J=7.6Hz, 2H), 2.74 (dd, J=2.6Hz, 2H), 2.86 (dd, J=4.2Hz, 2H), 3.30-3.34 (m, 2H), 3.79 (q, J=1.6Hz, 6H), 3.97 (dd, J=5.2Hz, 2H), 4.14 (dd, J=3.2Hz, 2H), 6.88-6.97 (m, 3H), 7.30-7.43 (m, 4H).

### Synthetic Example BI-1 (2) : Synthesis of mono-alkoxysilylated epoxy compound using dihydroxybiphenyl

The same procedure described in the above Synthetic Example BI-1 (1) was conducted except for conducting the Claisen rearrangement reaction of the second step in the above Synthetic Example BI-1 (1) as follows to produce (3-(4,4'-bis(oxirane-2-ylmethoxy)biphenyl-3-yl)propyl)triet hoxysilane.

In the second step, in a 1,000 ml two-necked flask equipped with a refluxing condenser, 10.0 g of the intermediate (11) obtained in the first step of the above Synthetic Example BI-1 (1), and 50 ml of 1, 2-dichlorobenzene (Sigma-Aldrich) were added and well mixed at room temperature. Then, the homogeneous solution thus obtained was refluxed for 8 hours at the set temperature of a refluxing apparatus of 190°C. After completing the reaction, the reactant was cooled to room temperature, and solvents were removed by a vacuum oven to produce 3-allylbiphenyl-4,4'-diol as an intermediate (12). The Reaction Scheme and NMR data of the intermediate (12) are the same as those in the second step of the above Synthetic Example BI-1 (1).

### Synthetic Example BI-2 (1) : Synthesis of di-alkoxysilylated epoxy compound using dihydroxybiphenyl

### (1) First step: Synthesis of 4,4'-bis(allyloxy)biphenyl

In a 1,000 ml two-necked flask equipped with a refluxing condenser, 10.0 g of biphenyl-4,4'-diol (Sigma-Aldrich), 11.61 ml of allyl bromide (Sigma-Aldrich), 44.56 g of K₂CO₃, and 500 ml of acetone were added and stirred at room temperature. Then, the temperature of a refluxing apparatus was set to 80°C, and a homogeneously well mixed solution was refluxed for reaction overnight. After completing the reaction, the reactant was cooled to room temperature, filtered using celite filtration and evaporated to produce a crude product. A target material in the crude product was extracted with ethyl acetate, washed with water three times, and dried with MgSO₄. MgSO₄ was removed using a filter, and solvents were removed using an evaporator to obtain 4,4'-bis(allyloxy)biphenyl as an intermediate (11). The Reaction Scheme of the first step and NMR data of the intermediate (11) thus obtained are as follows. 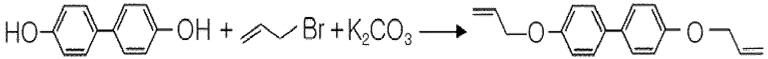 ¹H NMR (400MHz, CDCl₃) : δ=4.56 (dt, J=5.2Hz, 1.6Hz, 4H), 5.30-5.33 (m, 2H), 5.41-5.44 (m, 2H), 6.03-6.12 (m, 2H), 6.96 (td, J=3.0, 2.2, 8.8Hz, 4H), 7.46 (td, J=3.0, 2.2, 8.8Hz, 4H).

### (2) Second step: Synthesis of 3,3'-diallylbiphenyl-4,4'-diol

In a 100 ml flask, 20.0 g of the intermediate (11) obtained in the first step was added, and the flask was inserted in a microwave oven of which power and temperature were set to 300 W and 160°C, followed by performing reaction for 20 minutes. Then, the reactant was cooled to room temperature to produce 3,3'-diallylbiphenyl-4,4'-diol as an intermediate (12). The Reaction Scheme of the second step and NMR data of the intermediate (12) thus obtained are as follows. 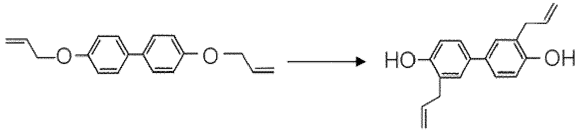 ¹H NMR (400MHz, CDCl₃) : δ=3.35 (d, J=6.4Hz, 4H), 5.14-5.25 (m, 6H), 6.00-6.10 (m, 2H), 6.84 (dd, J=2.0Hz, 7.2Hz, 2H), 7.29 (dd, J=10.6Hz, 4H).

### (3) Third step: Synthesis of 2,2'-(3,3'-diallylbiphenyl-4,4'-diyl)bis(oxy)bis(methylene) dioxirane

In a 1,000 ml two-necked flask equipped with a refluxing condenser, 10.0 g of the intermediate (12) obtained in the second step, 30.38 ml of epichlorohydrin (Sigma-Aldrich), 35. 13 g of K₂CO₃, and 300 ml of acetonitrile were added and mixed at room temperature. Then, the reaction temperature was elevated to 80°C, and reaction was performed overnight. After completing the reaction, the reactant was cooled to room temperature and was filtered using celite, and organic solvents were evaporated to obtain an intermediate (13). The Reaction Scheme of the third step and NMR data of the intermediate (13) thus obtained are as follows. 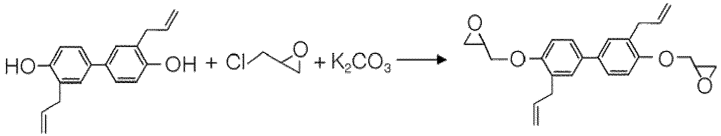 ¹H NMR (400MHz, CDCl₃) : δ=2.75 (dd, J=2.6Hz, 2H), 2.87 (dd, J=4.2Hz, 2H), 3.11-3.35 (m, 6H), 3.96 (dd, J=5.4Hz, 2H), 4.25 (dd, J=3.2Hz, 2H), 5.03-5.13 (m, 4H), 5.93-6.03 (m, 2H), 6.81 (d, J= 7.2Hz, 2H), 7.34-7.42 (m, 4H).

### (4) Fourth step: Synthesis of 3,3'-(4,4'-bis(oxirane-2-ylmethoxy)biphenyl-3,3'-diyl)bis(p ropane-3,1-diyl)bis(triethoxysilane)

In a 500 ml flask, 10.0 g of the intermediate (13) obtained in the third step, 9.67 ml of triethoxysilane (Sigma-Aldrich), 109 mg of platinum oxide, and 200 ml of toluene were added and well mixed, followed by stirring in an argon charged atmosphere at 85°C for 24 hours. After completing the reaction, the crude product thus obtained was filtered using celite filtration, and solvents were removed using an evaporator to produce a target material of a biphenyl epoxy compound containing an alkoxysilyl group. The Reaction Scheme of the fourth step and NMR data of the target material thus obtained are as follows. 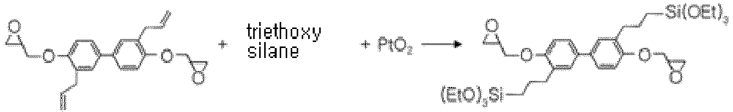 ¹H NMR (400MHz, CDCl₃) : δ=0.64-0.69 (m, 4H), 1.20 (t, J=7.0Hz, 18H), 1.62-1.72 (m, 4H), 2.61 (t, J=7.6Hz, 4H), 2.74 (dd, J=2.6Hz, 2H), 2.86 (dd, J=4.2Hz, 2H), 3.30-3.34 (m, 2H), 3.79 (q, J=1.6Hz, 12H), 3.97 (dd, J=5.2Hz, 2H), 4.14 (dd, J=3.2Hz, 2H), 6.85 (d, J=7.2Hz, 2H), 7.32-7.42 (m, 4H).

### Synthetic Example BI-2 (2) : Synthesis of di-alkoxysilylated epoxy compound using dihydroxybiphenyl

The same procedure described in the above Synthetic Example BI-2 (1) was conducted except for conducting the Claisen rearrangement reaction of the second step in the above Synthetic Example BI-2(1) as follows to produce (3,3'-(4,4'-bis(oxirane-2-ylmethoxy)biphenyl-3,3'-diyl)bis( propane-3,1-diyl))bis(triethoxysilane).

In the second step, in a 1,000 ml two-necked flask equipped with a refluxing condenser, 10.0 g of the intermediate (11) obtained in the first step of the above Synthetic Example BI-2 (1), and 100 ml of 1, 2-dichlorobenzene (Sigma-Aldrich) were added and well mixed at room temperature. Then, the homogeneous solution thus obtained was refluxed for 72 hours at the set temperature of a refluxing apparatus of 190°C. After completing the reaction, the reactant was cooled to room temperature, and solvents were removed by a vacuum oven to produce 3,3'-diallylbiphenyl-4,4'-diol as an intermediate (12). The Reaction Scheme of the second step and NMR data of the intermediate (12) are the same as those in the second step of the above Synthetic Example AI-2(1).

### Expected Synthetic Example BI-3 (1) : Synthesis of tri-alkoxysilylated epoxy compound using dihydroxybiphenyl

### (1) First step: Synthesis of 4,4'-bis(allyloxy)biphenyl

In a 1,000 ml two-necked flask equipped with a refluxing condenser, 10.0 g of biphenyl-4,4'-diol (Sigma-Aldrich), 11.61 ml of allyl bromide (Sigma-Aldrich), 44.56 g of K₂CO₃, and 500 ml of acetone were added and stirred at room temperature. Then, the temperature of a refluxing apparatus was set to 80°C, and a homogeneously well mixed solution was refluxed for performing reaction overnight. After completing the reaction, the reactant was cooled to room temperature, filtered using celite filtration and evaporated to produce a crude product. A target material in the crude product was extracted with ethyl acetate, washed with water three times, and dried with MgSO₄. MgSO₄ was removed using a filter, and solvents were removed using an evaporator to obtain 4,4'-bis(allyloxy)biphenyl as an intermediate (11). The Reaction Scheme of the first step and NMR data of the intermediate (11) are as follows. 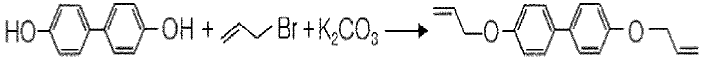 ¹H NMR (400MHz, CDCl₃) : δ=4.56 (dt, J=5.2Hz, 1.6Hz, 4H), 5.30-5.33 (m, 2H), 5.41-5.44 (m, 2H), 6.03-6.12 (m, 2H), 6.96 (td, J=3.0, 2.2, 8.8Hz, 4H), 7.46 (td, J=3.0, 2.2, 8.8Hz, 4H).

### (2) Second step: Synthesis of 3,3'-diallylbiphenyl-4,4'-diol

In a 100 ml flask, 20.0 g of the intermediate (11) obtained in the first step was inserted, and the flask was inserted in a microwave oven of which power and temperature were set to 300 W and 160°C, followed by performing reaction for 20 minutes. After completing the reaction, the reactant was cooled to room temperature to produce 3,3'-diallylbiphenyl-4,4'-diol as an intermediate (12). The Reaction Scheme of the second step and NMR data of the intermediate (12) are as follows. 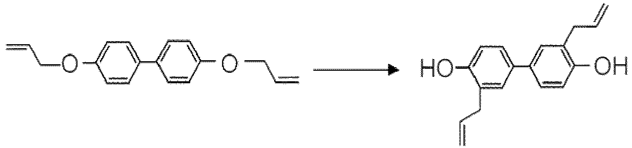 ¹H NMR (400MHz, CDCl₃) : δ=3.35 (d, J=6.4Hz, 4H), 5.14-5.25 (m, 6H), 6.00-6.10 (m, 2H), 6.84 (dd, J=2.0Hz, 7.2Hz, 2H), 7.29 (dd, J=10.6Hz, 4H).

### (3) 2-1-st step: Synthesis of 3,3'-dially-4'-(allyloxy)biphenyl-4-ol

In a 1,000 ml two-necked flask equipped with a refluxing condenser, 20.0 g of the intermediate (12) obtained in the second step, 26.71 g of K₂CO₃, and 500 ml of acetone are added and mixed at room temperature. Then, the reaction temperature is elevated to the set temperature of a refluxing apparatus of 80°C. While refluxing, 6.12 ml of allyl bromide (Sigma-Aldrich) is added thereto dropwisely, and the reaction is performed overnight. After completing the reaction, the reactant is cooled to room temperature and is filtered using celite filtration, and organic solvents are evaporated to obtain a crude product. A target material in the crude product is extracted with ethyl acetate, washed with water three times, and dried with MgSO₄. MgSO₄ is removed using a filter, solvents are removed using an evaporator, and the product thus obtained is separated by silica column chromatography to obtain 3,3'-diallyl-4'-(allyloxy)biphenyl-4-ol as an intermediate (23). The Reaction Scheme of the 2-1-st step is as follows.

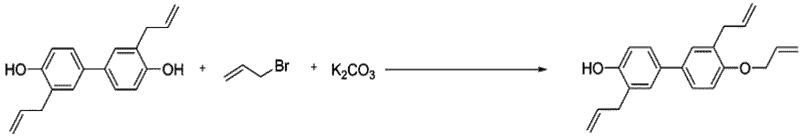

### (4) 2-2-nd step: Synthesis of 3,3',5-triallylbiphenyl-4,4'-diol

In a 100 ml flask, 20.0 g of the intermediate (23) obtained in the first step is added, and the flask is inserted in an oven of which power and temperature are set to 300 W and 160°C for performing reaction for 20 minutes. After completing the reaction, the reactant is cooled to room temperature to obtain 3,3',5-triallylbiphenyl-4,4'-diol as an intermediate (24). The Reaction Scheme of the 2-2-nd step is as follows.

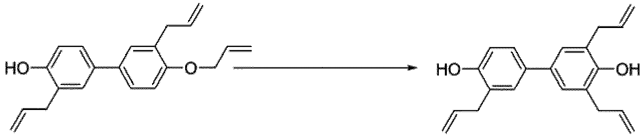

### (5) Third step: Synthesis of 2,2'-(3,3',5-triallylbiphenyl-4,4'-diyl)bis(oxy)bis(methyle ne)dioxirane

In a 1,000 ml two-necked flask equipped with a refluxing condenser, 20.0 g of the intermediate (24) obtained in the 2-2-nd step, 50.31 ml of epichlorohydrin (Sigma-Aldrich), 58.18 g of K₂CO₃, and 300 ml of acetonitrile are added and mixed at room temperature. Then, the reaction temperature is elevated to 80°C, and the reaction is performed overnight. After completing the reaction, the reactant is cooled to room temperature and is filtered using celite, and organic solvents are evaporated to obtain 2,2'-(3,3',5-triallylbiphenyl-4,4'-diyl)bis(oxy)bis(methyle ne)dioxirane as an intermediate (25). The Reaction Scheme of the third step is as follows.

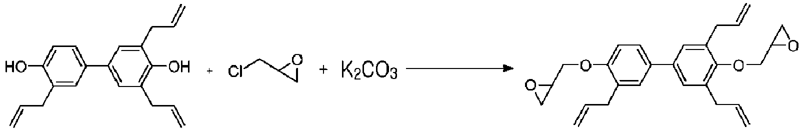

### (6) Fourth step: Synthesis of (3,3',3"-(2,6-bis(oxirane-2-ylmethoxy)biphenyl-1,3,5-triyl) triyl)tris(propane-3,1-diyl))tris(triethoxysilane)

In a 500 ml flask, 20.0 g of the intermediate (23) obtained in the third step, 29.13 ml of triethoxysilane (Sigma-Aldrich), 326 mg of platinum oxide, and 200 ml of toluene are added and well mixed, followed by stirring in an argon charged atmosphere at 85°C for 24 hours. After completing the reaction, the crude product thus obtained is filtered using celite filtration, and solvents are removed using an evaporator to produce a target material of a biphenyl epoxy compound containing an alkoxysilyl group. The Reaction Scheme of the fourth step and NMR data of the target material thus obtained are as follows. 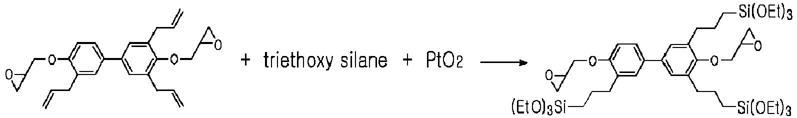 ¹H NMR (400MHz, CDCl₃) : δ=0.60-0.70 (m, 6H), 1.20-1.25 (t, 27H), 1.60-1.70 (m, 6H), 2.50-2.70 (t, 6H), 2.70-2.80 (m, 2H), 2.80-2.90 (m, 2H), 3.30-3.40 (m, 2H), 3.70-4.00 (m, 20H), 4.10-4.20 (m, 2H), 6.90-7.50 (m, 5H).

### Expected Synthetic Example BI-3 (2) : Synthesis of tri-alkoxysilylated epoxy compound using dihydroxybiphenyl

The same procedure described in the above Synthetic Example BI-3 (1) is conducted except for conducting the Claisen rearrangement reaction of the second step and the 2-2-nd step in the above Synthetic Example BI-3(1) as follows to produce (3,3,3"'-(2,6-bis(oxirane-2-ylmethoxy)biphenyl-1,3,5-triyl) tris(propane-3,1-diyl))tris(triethoxysilane).

In the second step, in a 1,000 ml two-necked flask equipped with a refluxing condenser, 10.0 g of the intermediate (11) obtained in the first step of the above Synthetic Example AI-3 (1), and 100 ml of 1, 2-dichlorobenzene (Sigma-Aldrich) are added and well mixed at room temperature. Then, the homogeneous solution thus obtained is refluxed for 72 hours at the set temperature of a refluxing apparatus of 190°C. After completing the reaction, the reactant is cooled to room temperature, and solvents are removed by a vacuum oven to produce 3,3'-diallylbiphenyl-4,4'-diol as an intermediate (12). The Reaction Scheme of the second step and NMR data of the intermediate (12) are the same as those of the second step of the above Synthetic Example BI-3(1).

In the 2-1-st step, the same procedure in the 2-1-st step of the above Synthetic Example BI-3 (1) is conducted using the above intermediate (12). Then, in a 1,000 ml two-necked flask equipped with a refluxing condenser, 20.0 g of the intermediate (23) obtained in the 2-1-st step and 100 ml of 1,2-dichlorobenzene (Sigma-Aldrich) are added and well mixed at room temperature. Then, the homogeneous solution thus obtained is refluxed for 8 hours at the set temperature of a refluxing apparatus of 190°C. After completing the reaction, the reactant is cooled to room temperature, and solvents are removed by a vacuum oven to produce 3,3',5-triallylbiphenyl-4,4'-diol as an intermediate (24). The Reaction Scheme of the 2-2-nd step is the same as that of the 2-2-nd step of the above Synthetic Example BI-3(1).

### Expected Synthetic Example BI-4 (1) : Synthesis of tetra-alkoxysilylated epoxy compound using dihydroxybiphenyl

### (1) First step: Synthesis of 4,4'-bis(allyloxy)biphenyl

In a 1,000 ml two-necked flask equipped with a refluxing condenser, 10.0 g of biphenyl-4,4'-diol (Sigma-Aldrich), 11.61 ml of allyl bromide (Sigma-Aldrich), 44.56 g of K₂CO₃, and 500 ml of acetone were added and mixed at room temperature. Then, the temperature of a refluxing apparatus was set to 80°C, and a homogeneously well mixed solution was refluxed for performing reaction overnight. After completing the reaction, the reactant was cooled to room temperature, filtered using celite filtration and evaporated to produce a crude product. A target material in the crude product was extracted with ethyl acetate, washed with water three times, and dried with MgSO₄. MgSO₄ was removed using a filter, and solvents were removed using an evaporator to obtain 4,4'-bis(allyloxy)biphenyl as an intermediate (11). The Reaction Scheme of the first step and NMR data of the intermediate (11) are as follows. 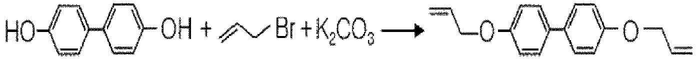 ¹H NMR (400MHz, CDCl₃) : δ=4.56 (dt, J=5.2Hz, 1.6Hz, 4H), 5.30-5.33 (m, 2H), 5.41-5.44 (m, 2H), 6.03-6.12 (m, 2H), 6.96 (td, J=3.0, 2.2, 8.8Hz, 4H), 7.46 (td, J=3.0, 2.2, 8.8Hz, 4H).

### (2) Second step: Synthesis of 3,3'-diallylbiphenyl-4,4'-diol

In a 100 ml flask, 20.0 g of the intermediate (11) obtained in the first step was added, and the flask was inserted in a microwave oven of which power and temperature were set to 300 W and 160°C, followed by performing reaction for 20 minutes. After completing the reaction, the reactant was cooled to room temperature to produce 3,3'-diallylbiphenyl-4,4'-diol as an intermediate (12). The Reaction Scheme of the second step and NMR data of the intermediate (12) are as follows. 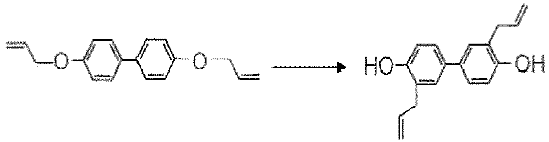 ¹H NMR (400MHz, CDCl₃) : δ=3.35 (d, J=6.4Hz, 4H), 5.14-5.25 (m, 6H), 6.00-6.10 (m, 2H), 6.84 (dd, J=2.0Hz, 7.2Hz, 2H), 7.29 (dd, J=10.6Hz, 4H)

### (3) 2-1-st step: Synthesis of 3,3'-dially-4,4'-bis(allyloxy)biphenyl

In a 1,000 ml two-necked flask equipped with a refluxing condenser, 20.0 g of an intermediate (12) obtained in the second step, 24.40 ml of allyl bromide (Sigma-Aldrich), 93.47 g of K₂CO₃, and 500 ml of acetone are added and mixed at room temperature. Then, a well mixed solution is refluxed at the set temperature of a refluxing apparatus of 80°C to perform the reaction overnight. After completing the reaction, the reactant is cooled to room temperature and is filtered using celite, and organic solvents are evaporated to obtain a crude product. A target material in the crude product is extracted with ethyl acetate, washed with water three times, and dried with MgSO₄. MgSO₄ is removed using a filter, and solvents are removed using an evaporator to obtain 3,3'-diallyl-4,4'-bis(allyloxy)biphenyl as an intermediate (23). The Reaction Scheme of the 2-1-st step is as follows.

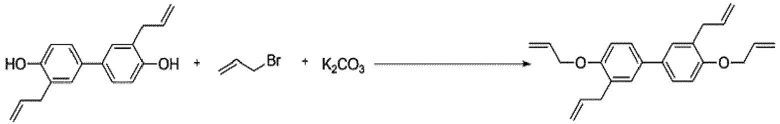

### (4) 2-2-nd step: Synthesis of 3,3',5,5'-tetraallylbiphenyl-4,4'-diol

In a 100 ml flask, 20.0 g of the intermediate (23) obtained in the 2-1-st step is added, and the flask is inserted in an oven of which power and temperature are set to 300 W and 160°C for performing reaction for 20 minutes. After completing the reaction, the reactant is cooled to room temperature to produce 3,3',5,5'-tetraallylbiphenyl-4,4'-diol as an intermediate (24). The Reaction Scheme of the 2-2-nd step is as follows.

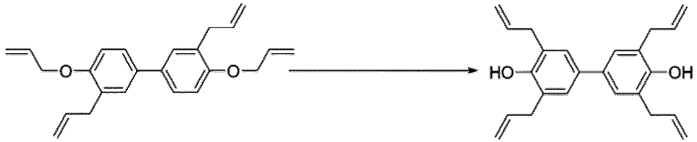

### (5) Third step: Synthesis of 2,2'-(3,3',5,5'-tetraallylbiphenyl-4,4'-diyl)bis(oxy)bis(me thylene)dioxirane

In a 1,000 ml two-necked flask equipped with a refluxing condenser, 20.0 g of the intermediate (24) obtained in the 2-2-nd step, 55.76 ml of epichlorohydrin (Sigma-Aldrich), 64.49 g of K₂CO₃, and 300 ml of acetonitrile are added and mixed at room temperature. Then, the reaction temperature is elevated to 80°C, and the reaction is performed overnight. After completing the reaction, the reactant is cooled to room temperature and is filtered using celite, and organic solvents are evaporated to obtain 2,2'-(3,3',5,5'-tetraallylbiphenyl-4,4'-diyl)bis(oxy)bis(me thylene)dioxirane as an intermediate (25). The Reaction Scheme of the third step is as follows.

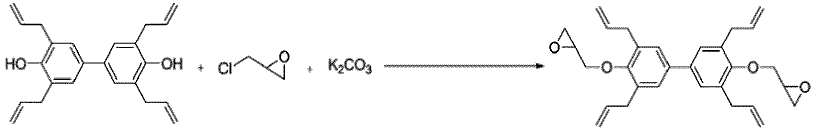

### (6) Fourth step: Synthesis of (3,3',3",3"'-(4,4'-bis(oxirane-2-ylmethoxy)biphenyl-3,3',5 ,5'-tetrayl)tetrakis(propane-3,1-diyl))tetrakis(triethoxysi lane)

In a 500 ml flask, 20.0 g of the intermediate (25) obtained in the third step, 29.29 ml of triethoxysilane (Sigma-Aldrich), 328 mg of platinum oxide, and 200 ml of toluene are added and mixed, followed by stirring in an argon charged atmosphere at 85°C for 24 hours. After completing the reaction, the crude product thus obtained is filtered using celite filtration, and solvents are removed using an evaporator to produce a target material of a biphenyl epoxy compound containing an alkoxysilyl group. The Reaction Scheme of the fourth step and NMR data of the target material thus obtained are as follows. 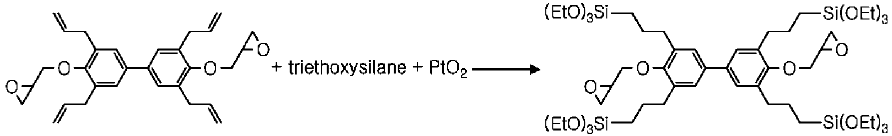 ¹H NMR (400MHz, CDCl₃) : δ=0.60-0.70 (m, 8H), 1.20-1.25 (t, 36H), 1.60-1.70 (m, 8H), 2.50-2.70 (t, 8H), 2.70-2.80 (m, 2H), 2.80-2.90 (m, 2H), 3.30-3.40 (m, 2H), 3.70-4.00 (m, 26H), 4.10-4.20 (m, 2H), 7.30-7.50 (s, 4H).

### Expected Synthetic Example BI-4(2): Synthesis of tetra-alkoxysilylated epoxy compound using dihydroxybiphenyl

The same procedure described in the above Synthetic Example BI-4 (1) is conducted except for conducting the Claisen rearrangement reaction of the second step and the 2-2-nd step in the above Synthetic Example BI-4(1) as follows to produce (3,3',3",3"'-(4,4'-bis(oxirane-2-ylmethoxy)biphenyl-3,3',5, 5'-tetrayl)tetrakis(propane-3,1-diyl))tetrakis(triethoxysil ane).

In the second step, in a 1,000 ml two-necked flask equipped with a refluxing condenser, 10.0 g of the intermediate (11) obtained in the first step of the above Synthetic Example BI-4(1), and 100 ml of 1, 2-dichlorobenzene (Sigma-Aldrich) are added and well mixed at room temperature. Then, the homogeneous solution thus obtained is refluxed for 72 hours at the set temperature of a refluxing apparatus of 190°C. After completing the reaction, the reactant is cooled to room temperature, and solvents are removed by a vacuum oven to produce 3,3'-diallylbiphenyl-4,4'-diol as an intermediate (12). The Reaction Scheme of the second step and NMR data of the intermediate (12) are the same as those of the second step of the above Synthetic Example BI-4(1).

In the 2-1-st step, the same procedure in the 2-1-st step of the above Synthetic Example BI-4 (1) is conducted using the above intermediate (12). Then, in a 1,000 ml two-necked flask equipped with a refluxing condenser, 20.0 g of the intermediate (23) obtained in the 2-1-st step and 100 ml of 1,2-dichlorobenzene (Sigma-Aldrich) are added and well mixed at room temperature. Then, the homogeneous solution thus obtained is refluxed for 8 hours at the set temperature of a refluxing apparatus of 190°C. After completing the reaction, the reactant is cooled to room temperature, and solvents are removed by a vacuum oven to produce 3,3',5,5'-tetraallylbiphenyl-4,4'-diol as an intermediate (24). The Reaction Scheme of the 2-2-nd step is the same as that of the 2-2-nd step of the above Synthetic Example BI-4 (1) .

### Synthetic Example CI-1(1): Synthesis of mono-alkoxysilylated epoxy compound using fluorene

### (1) First step: Synthesis of 4-(9-(4-(allyloxy)phenyl)-9H-fluorene-9-yl)phenol

In a 1,000 ml two-necked flask equipped with a refluxing condenser, 10.0 g of 4,4'-(9H-fluorene-9,9-diyl)diphenol (Sigma-Aldrich), 11.84 g of K₂CO₃, and 500 ml of acetone were added and mixed at room temperature. Then, the temperature of a refluxing apparatus was set to 80°C, and a homogeneously well mixed solution was refluxed. While refluxing the homogeneously well mixed solution, 3.08 ml of allyl bromide (Sigma-Aldrich) was added dropwisely, followed by performing reaction overnight. After completing the reaction, the reactant was cooled to room temperature and filtered using celite filtration. Organic solvents were evaporated to produce a crude product. A target material in the crude product was extracted with ethyl acetate, washed with water three times, and dried with MgSO₄. MgSO₄ was removed using a filter, and solvents were removed using an evaporator to obtain an intermediate (11). The Reaction Scheme of the first step and NMR data of the intermediate (11) thus obtained are as follows. 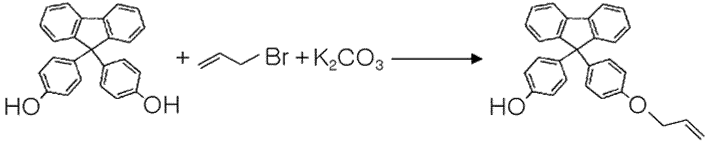 ¹H NMR (400MHz, CDCl₃): δ=4.46 (dt, J=5.2, 1.6Hz, 2H), 5.20-5.25 (m, 2H), 5.35-5.38 (m, 1H), 5.98-6.06 (m, 1H), 6.72-6.76 (m, 4H), 7.06-7.11 (m, 4H), 7.24-7.39 (m, 6H), 7.70-7.79 (m, 2H).

### (2) Second step: Synthesis of 2-allyl-4-(9-(4-hydroxyphenyl)-9H-fluorene-9-yl)phenol

In a 100 ml flask, 20.0 g of the intermediate (11) obtained in the first step and 50 ml of 1,2-dichlorobenzene (Sigma-Aldrich) were added, and the flask was inserted in a microwave oven of which power and temperature were set to 300 W and 160°C, followed by performing reaction for 20 minutes. After completing the reaction, the reactant was cooled to room temperature, and solvents were removed by a vacuum oven to produce 2-allyl-4-(9-(4-hydroxyphenyl)-9H-fluorene-9-yl)phenol as an intermediate (12). The Reaction Scheme of the second step and NMR data of the intermediate (12) thus obtained are as follows. 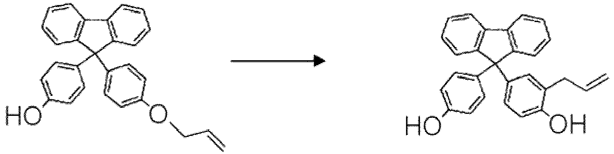 ¹H NMR (400MHz, CDCl₃) : δ=3.28 (d, J=6.0Hz, 2H), 5.04-5.10 (m, 2H), 5.21 (br.s, 2H), 5.87-5.97 (m, 1H), 6.71-6.75 (m, 3H), 7.05-7.11 (m, 4H), 7.24-7.39 (m, 6H), 7.70-7.78 (m, 2H).

### (3) Third step: Synthesis of 2-((2-allyl-4-(9-(4-(oxirane-2-ylmethoxy)phenyl)-9H-fluoren e-9-yl)phenoxy)methyl)oxirane

In a 1,000 ml two-necked flask equipped with a refluxing condenser, 10.0 g of the intermediate (12) obtained in the second step, 18.16 ml of epichlorohydrin (Sigma-Aldrich), 21.00 g of K₂CO₃, and 200 ml of acetonitrile were added and mixed at room temperature. Then, the reaction temperature was elevated to 80°C, and the reaction was performed overnight. After completing the reaction, the reactant was cooled to room temperature and was filtered using celite, and organic solvents were evaporated to obtain an intermediate (13). The Reaction Scheme of the third step and NMR data of the intermediate (13) thus obtained are as follows. 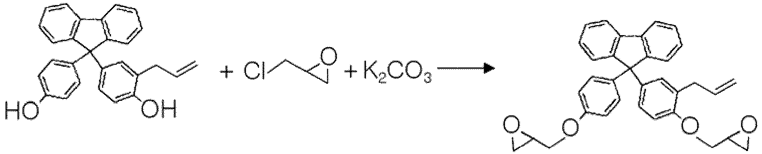 ¹H NMR (400MHz, CDCl₃) : δ=2.77 (dd, J=2.6Hz, 2H), 2.87 (dd, J=4.2Hz, 2H), 3.10-3.36 (m, 4H), 3.98 (dd, J=5.4Hz, 2H), 4.14 (dd, J=3.2Hz, 2H), 4.97-5.04 (m, 2H), 5.92-6.03 (m, 1H), 6.75-6.85 (m, 3H), 7.01-7.12 (m, 4H), 7.24-7.39 (m, 6H), 7.70-7.78 (m, 2H).

### (4) Fourth step: Synthesis of triethoxy(3-(2-(oxirane-2-ylmethoxy)-5-(9-(4-(oxirane-2-ylm ethoxy)phenyl)-9H-fluorene-9-yl)phenyl)propyl)silane

In a 250 ml flask, 10.0 g of the intermediate (13) obtained in the third step, 3.74 ml of triethoxysilane (Sigma-Aldrich), 41 mg of platinum oxide, and 100 ml of toluene were added and well mixed, followed by stirring in an argon charged atmosphere at 85°C for 72 hours. After completing the reaction, the crude product thus obtained was filtered using celite filtration, and solvents were removed using an evaporator to produce a target material of a fluorene epoxy compound containing an alkoxysilyl group. The Reaction Scheme of the fourth step and NMR data of the target material thus obtained are as follows. 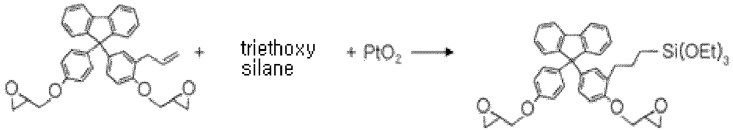 ¹H NMR (400MHz, CDCl₃) : δ=0.64-0.69 (m, 2H), 1.21 (t, J=7.0Hz, 9H), 1.62-1.74 (m, 2H), 2.64 (t, J=7.6Hz, 2H), 2.74 (dd, J=2.6Hz, 2H), 2.87 (dd, J=4.2Hz, 2H), 3.29-3.34 (m, 2H), 3.79 (q, J=1.6Hz, 6H), 3.97 (dd, J=5.2Hz, 2H), 4.14 (dd, J=3.2Hz, 2H), 6.81-6.87 (m, 3H), 6.96-7.07 (m, 4H), 7.24-7.39 (m, 6H), 7.70-7.78 (m, 2H).

### Synthetic Example CI-1(2): Synthesis of mono-alkoxysilylated epoxy compound using fluorene

The same procedure described in the above Synthetic Example CI-1 (1) was conducted except for conducting the Claisen rearrangement reaction of the second step in the above Synthetic Example CI-1(1) as follows to produce triethoxy(3-(2-(oxirane-2-ylmethoxy)-5-(9-(4-(oxirane-2-ylm ethoxy)phenyl)-9H-fluorene-9-yl)phenyl)propyl)silane.

In the second step, in a 1,000 ml two-necked flask equipped with a refluxing condenser, 10.0 g of the intermediate (11) obtained in the first step of the above Synthetic Example CI-1(1), and 50 ml of 1, 2-dichlorobenzene (Sigma-Aldrich) were added and well mixed at room temperature. Then, the homogeneous solution thus obtained was refluxed for 96 hours at the set temperature of a refluxing apparatus of 190°C. After completing the reaction, the reactant was cooled to room temperature, and solvents were removed by a vacuum oven to produce 2-allyl-4-(9-(4-hydroxyphenyl)-9H-fluorene-9-yl)phenol as an intermediate (12). The Reaction Scheme and NMR data of the intermediate (12) are the same as those in the second step of the above Synthetic Example CI-1(1).

### Synthetic Example CI-2(1): Synthesis of di-alkoxysilylated epoxy compound using fluorene

### (1) First step: Synthesis of 9,9-bis(4-(allyloxy)phenyl)-9H-fluorene

In a 1,000 ml two-necked flask equipped with a refluxing condenser, 10.0 g of 4,4'-(9H-fluorene-9,9-diyl)diphenol (Sigma-Aldrich), 6.17 ml of allyl bromide (Sigma-Aldrich), 23.68 g of K₂CO₃, and 500 ml of acetone were added and mixed at room temperature. Then, the temperature of a refluxing apparatus was set to 80°C, and a homogeneously well mixed solution was refluxed for reaction overnight. After completing the reaction, the reactant was cooled to room temperature, filtered using celite filtration and evaporated to produce a crude product. A target material in the crude product was extracted with ethyl acetate, washed with water three times, and dried with MgSO₄. MgSO₄ was removed using a filter, and solvents were removed using an evaporator to obtain 9,9-bis(4-allyloxy)phenyl-9H-fluorene as an intermediate (11). The Reaction Scheme of the first step and NMR data of the intermediate (11) thus obtained are as follows. 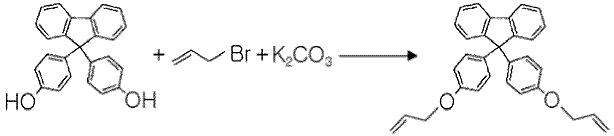 ¹H NMR (400MHz, CDCl₃) : δ=4.46 (td, J=1.4, 2.4Hz, 4H), 5.25 (qd, J=1.6, 1.2, 10.4Hz, 2H), 5.35-5.38 (m, 2H), 5.97-6.06 (m, 2H), 6.75 (td, J=3.2, 2.0, 8.8Hz, 4H), 7.10 (td, J=3.2, 2.0, 8.8Hz, 4H), 7.23-7.39 (m, 6H), 7.70-7.79 (m, 2H).

### (2) Second step: Synthesis of 4,4'-(9H-fluorene-9,9-diyl)bis(2-alllylphenol)

In a 100 ml flask, 20.0 g of the intermediate (11) obtained in the first step was added, and the flask was inserted in a microwave oven of which power and temperature were set to 300 W and 160°C, followed by performing reaction for 20 minutes. After completing the reaction, the reactant was cooled to room temperature to produce 4,4'-(9H-fluorene-9,9-diyl)bis(2-allylphenol) as an intermediate (12). The Reaction Scheme of the second step and NMR data of the intermediate (12) thus obtained are as follows. 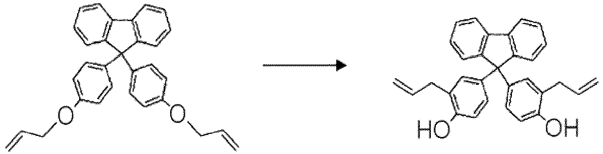 ¹H NMR (400MHz, CDCl₃) : δ=3.28 (d, J=6.0Hz, 4H), 5.04-5.09 (m, 4H), 5.21 (s, 2H), 5.87-5.97 (m, 2H), 6.62 (d, J=8.4Hz, 2H), 6.88 (dd, J=2.4, 6.0Hz, 2H), 6.96 (d, J=2.4Hz, 2H), 7.22-7.36 (m, 6H), 7.74 (d, J=7.2Hz, 2H).

### (3) Third step: Synthesis of 2,2'-(4,4'-9H-fluorene-9,9-diyl)bis(2-allyl-4,1-phenylene)) bis(oxy)bis(methylene)dioxirane

In a 1,000 ml two-necked flask equipped with a refluxing condenser, 10.0 g of the intermediate (12) obtained in the second step, 18.16 ml of epichlorohydrin (Sigma-Aldrich), 21.00 g of K₂CO₃, and 300 ml of acetonitrile were added and mixed at room temperature. Then, the reaction temperature was elevated to 80°C, and the reaction was performed overnight. After completing the reaction, the reactant was cooled to room temperature and was filtered using celite, and organic solvents were evaporated to obtain an intermediate (13). The Reaction Scheme of the third step and NMR data of the intermediate (13) thus obtained are as follows. 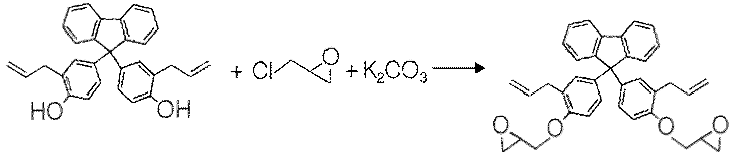 ¹H NMR (400MHz, CDCl₃) : δ=2.75 (dd, J=2.6Hz, 2H), 2.87 (dd, J=4.2Hz, 2H), 3.11-3.35 (m, 6H), 3.96 (dd, J=5.4Hz, 2H), 4.12 (dd, J=3.2Hz, 2H), 4.97-5.03 (m, 4H), 5.93-6.03 (m, 2H), 6.69 (d, J=8.4Hz, 2H), 6.80-6.83(m, 2H), 7.05 (s, 2H), 7.22-7.36 (m, 6H), 7.74 (d, J=7.2Hz, 2H).

### (4) Fourth step: Synthesis of 3,3'-(5,5'-(9H-fluorene-9,9-diyl)bis(2-oxirane-2-ylmethoxy) -5,1-phenylene))bis(propane-3,1-diyl)-bis(triethoxysilane)

In a 500 ml flask, 10.0 g of the intermediate (13) obtained in the third step, 7.48 ml of triethoxysilane (Sigma-Aldrich), 84 mg of platinum oxide, and 200 ml of toluene were added and mixed, followed by stirring in an argon charged atmosphere at 85°C for 24 hours. After completing the reaction, the crude product thus obtained was filtered using celite filtration, and solvents were removed using an evaporator to produce a target material. The Reaction Scheme of the fourth step and NMR data of the target material thus obtained are as follows. 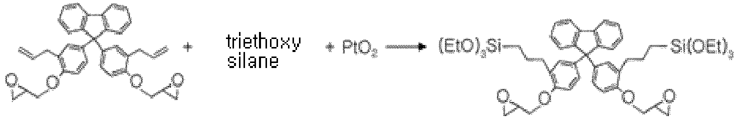 ¹H NMR (400MHz, CDCl₃) : δ=0.66-0.70 (m, 4H), 1.20 (t, J=7.0Hz, 18H), 1.63-1.71 (m, 4H), 2.61 (t, J=7.6Hz, 4H), 2.75 (dd, J=2.6Hz, 2H), 2.86 (dd, J=4.2Hz, 2H), 3.30-3.35 (m, 2H), 3.79 (q, J=1.6Hz, 12H), 3.96 (dd, J=5.2Hz, 2H), 4.14 (dd, J=3.2Hz, 2H), 6.69 (d, J=8.4Hz, 2H), 6.80-6.83(m, 2H), 7.03 (s, 2H), 7.21-7.36 (m, 6H), 7.73 (d, J=7.2Hz, 2H).

### Synthetic Example CI-2(2): Synthesis of di-alkoxysilylated epoxy compound using fluorene

The same procedure described in the above Synthetic Example CI-2 (1) was conducted except for conducting the Claisen rearrangement reaction of the second step in the above Synthetic Example CI-2(1) as follows to produce (3,3'-(5,5'-(9H-fluorene-9,9-diyl)bis(2-(oxirane-2-ylmethox y)-5,1-phenylene))bis(propane-3,1-diyl))-bis(triethoxysilan e).

In the second step, in a 1,000 ml two-necked flask equipped with a refluxing condenser, 10.0 g of the intermediate (11) obtained in the first step of the above Synthetic Example CI-2(1), and 100 ml of 1, 2-dichlorobenzene (Sigma-Aldrich) were added and well mixed at room temperature. Then, the homogeneous solution thus obtained was refluxed for 96 hours at the set temperature of a refluxing apparatus of 190°C. After completing the reaction, the reactant was cooled to room temperature, and solvents were removed by a vacuum oven to produce 4,4'-(9H-fluorene-9,9-diyl)bis(2-allylphenol) as an intermediate (12). The Reaction Scheme of the second step and NMR data of the intermediate (12) are the same as those in the second step of the above Synthetic Example CI-2(1).

### Expected Synthetic Example CI-3(1): Synthesis of tri-alkoxysilylated epoxy compound using dihydroxyfluorene

### (1) First step: Synthesis of 9,9-bis(4-allyloxy)phenyl)-9H-fluorene

In a 1,000 ml two-necked flask equipped with a refluxing condenser, 10.0 g of 4,4'-(9H-fluorene-9,9-diyl)diphenol (Sigma-Aldrich), 6.17 ml of allyl bromide (Sigma-Aldrich), 23.68 g of K₂CO₃, and 500 ml of acetone were added and mixed at room temperature. Then, the temperature of a refluxing apparatus was set to 80°C, and a homogeneously well mixed solution was refluxed for reaction overnight. After completing the reaction, the reactant was cooled to room temperature, filtered using celite filtration and evaporated to produce a crude product. A target material in the crude product was extracted with ethyl acetate, washed with water three times, and dried with MgSO₄. MgSO₄ was removed using a filter, and solvents were removed using an evaporator to obtain 9,9-bis(4-allyloxy)phenyl)-9H-fluorene as an intermediate (11). The Reaction Scheme of the first step and NMR data of the intermediate (11) are as follows. 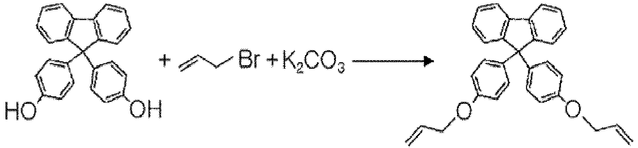 ¹H NMR (400MHz, CDCl₃) : δ=4.46 (td, J=1.4, 2.4Hz, 4H), 5.25 (qd, J=1.6, 1.2, 10.4Hz, 2H), 5.35-5.38 (m, 2H), 5.97-6.06 (m, 2H), 6.75 (td, J=3.2, 2.0, 8.8Hz, 4H), 7.10 (td, J=3.2, 2.0, 8.8Hz, 4H), 7.23-7.39 (m, 6H), 7.70-7.79 (m, 2H).

### (2) Second step: Synthesis of 4,4'-(9H-fluorene-9,9-diyl)bis(2-allylphenol)

In a 100 ml flask, 20.0 g of the intermediate (11) obtained in the first step was added, and the flask was inserted in a microwave oven of which power and temperature were set to 300 W and 160°C, followed by performing reaction for 20 minutes. After completing the reaction, the reactant was cooled to room temperature to produce 4,4'-(9H-fluorene-9,9-diyl)bis(2-allylphenol) as an intermediate (12). The Reaction Scheme of the second step and NMR data of the intermediate (12) are as follows. 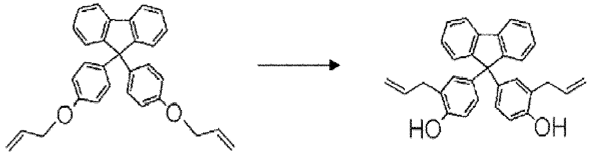 ¹H NMR (400MHz, CDCl₃) : δ=3.28 (d, J=6.0Hz, 4H), 5.04-5.09 (m, 4H), 5.21 (s, 2H), 5.87-5.97 (m, 2H), 6.62 (d, J=8.4Hz, 2H), 6.88 (dd, J=2.4, 6.0Hz, 2H), 6.96 (d, J=2.4Hz, 2H), 7.22-7.36 (m, 6H), 7.74 (d, J=7.2Hz, 2H).

### (3) 2-1-st step: Synthesis of 2-ally-4-(9-(3-allyl-4-(allyloxy)phenyl)-9H-fluorene-9-yl)p henol

In a 1,000 ml two-necked flask equipped with a refluxing condenser, 20.0 g of the intermediate (12) obtained in the second step, 16.52 g of K₂CO₃, and 500 ml of acetone are added and mixed at room temperature. Then, the reaction temperature is elevated to the set temperature of a refluxing apparatus of 190°C. While refluxing, 3.79 ml of allyl bromide (Sigma-Aldrich) is added thereto dropwisely, and the reaction is performed overnight. After completing the reaction, the reactant is cooled to room temperature and is filtered using celite, and organic solvents are evaporated to obtain a crude product. A target material in the crude product is extracted with ethyl acetate, washed with water three times, and dried with MgSO₄. MgSO₄ is removed using a filter, solvents are removed using an evaporator, and the product thus obtained is separated by silica column chromatography to obtain 2-allyl-4-(9-(3-allyl-4-(allyloxy)phenyl)-9H-fluorene-9-yl) phenol as an intermediate (23). The Reaction Scheme of the 2-1-st step is as follows.

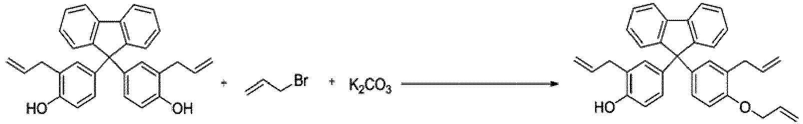

### (4) 2-2-nd step: Synthesis of 2,6-diallyl-4-(9-(3-allyl-4-hydroxyphenyl)-9H-fluorene-9-yl )phenol

In a 100 ml flask, 20.0 g of the intermediate (23) obtained in the first step is added, and the flask is inserted in an oven of which power and temperature are set to 300 W and 160°C for performing reaction for 20 minutes. After completing the reaction, the reactant is cooled to room temperature to obtain an intermediate (24). The Reaction Scheme of the 2-2-nd step is as follows.

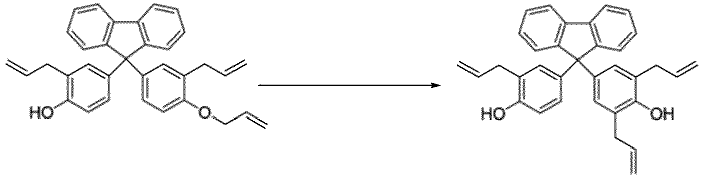

### (5) Third step: Synthesis of 2-((2-allyl-4-(9-(3,5-diallyl-4-(oxirane-2-ylmethoxy)phenyl )-9H-fluorene-9-yl)phenoxy)methyl)oxirane

In a 1,000 ml two-necked flask equipped with a refluxing condenser, 20.0 g of the intermediate (24) obtained in the 2-2-nd step, 32.76 ml of epichlorohydrin (Sigma-Aldrich), 37.88 g of K₂CO₃, and 300 ml of acetonitrile are added and mixed at room temperature. Then, the reaction temperature is elevated to 80°C, and the reaction is performed overnight. After completing the reaction, the reactant is cooled to room temperature and is filtered using celite, and organic solvents are evaporated to obtain 2-((2-allyl-4-(9-(3,5-diallyl-4-(oxirane-2-ylmethoxy)phenyl )-9H-fluorene-9-yl)phenoxy)methyl)oxirane as an intermediate (25). The Reaction Scheme of the third step is as follows.

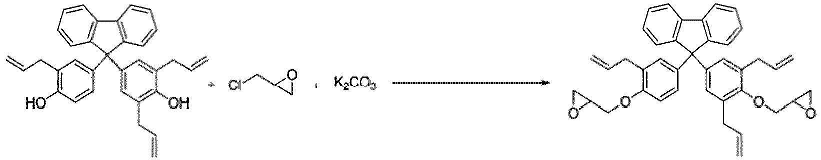

### (6) Fourth step: Synthesis of 2,2'-(2-(oxirane-2-ylmethoxy)-5-(9-(4-(oxirane-2-ylmethoxy) -3-(2-(triethoxysilyl)ethyl)phenyl)-9H-fluorene-9-yl)-1,3-p henylene)bis(ethane-2,1-diyl))bis(triethoxysilane)

In a 500 ml flask, 20.0 g the intermediate (25) obtained in the third step, 29.13 ml of triethoxysilane (Sigma-Aldrich), 326 mg of platinum oxide, and 200 ml of toluene are added and well mixed, followed by stirring in an argon charged atmosphere at 85°C for 24 hours. After completing the reaction, the crude product thus obtained is filtered using celite filtration, and solvents are removed using an evaporator to produce a target material of a fluorene epoxy compound containing an alkoxysilyl group. The Reaction Scheme of the fourth step and NMR data of the target material thus obtained are as follows. 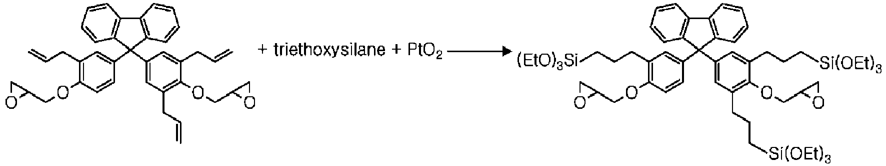 ¹H NMR (400MHz, CDCl₃) : δ=0.60-0.70 (m, 6H), 1.20-1.25 (t, 27H), 1.60-1.70 (m, 6H), 2.50-2.70(t, 6H), 2.70-2.80 (m, 2H), 2.80-2.90 (m, 2H), 3.30-3.40 (m, 2H), 3.70-4.00 (m, 20H), 4.10-4.20 (m, 2H), 6.70-7.00 (m, 5H), 7.20-7.40 (m, 6H), 7.70-7.90 (d, 2H).

### Expected Synthetic Example CI-3(2): Synthesis of tri-alkoxysilylated epoxy compound using dihydroxyfluorene

The same procedure described in the above Synthetic Example CI-3 (1) is conducted except for conducting the Claisen rearrangement reaction of the second step and the 2-2-nd step in the above Synthetic Example CI-3(1) as follows to produce (2,2'-(2-(oxirane-2-ylmethoxy)-5-(9-(4-(oxirane-2-ylmethoxy )-3-(2-triethoxysilyl)ethyl)phenyl)-9H-fluorene-9-yl)-1,3-p henylene)bis(ethane-2,1-diyl))bis(triethoxysilane).

In the second step, in a 1,000 ml two-necked flask equipped with a refluxing condenser, 10.0 g of the intermediate (11) obtained in the first step of the above Synthetic Example CI-3(1), and 100 ml of 1, 2-dichlorobenzene (Sigma-Aldrich) are added and well mixed at room temperature. Then, the homogeneous solution thus obtained is refluxed for 96 hours at the set temperature of a refluxing apparatus of 190°C. After completing the reaction, the reactant is cooled to room temperature, and solvents are removed by a vacuum oven to produce 4,4'-(9H-fluorene-9,9-diyl)bis((2-allylphenol) as an intermediate (12). The Reaction Scheme of the second step and NMR data of the intermediate (12) are the same as those of the second step of the above Synthetic Example CI-3(1).

In the 2-1-st step, the same procedure in the 2-1-st step of the above Synthetic Example CI-3 (1) is conducted using the above intermediate (12). Then, in a 1,000 ml two-necked flask equipped with a refluxing condenser, 20.0 g of the intermediate (23) obtained in the 2-1-st step and 100 ml of 1,2-dichlorobenzene (Sigma-Aldrich) are added and well mixed at room temperature. Then, the homogeneous solution thus obtained is refluxed for 8 hours at the set temperature of a refluxing apparatus of 190°C. After completing the reaction, the reactant is cooled to room temperature, and solvents are removed by a vacuum oven to produce an intermediate (24). The Reaction Scheme of the 2-2-nd step is the same as that of the 2-2-nd step of the above Synthetic Example CI-3(1).

### Expected Synthetic Example CI-4(1): Synthesis of tetra-alkoxysilylated epoxy compound using dihydroxyfluorene

### (1) First step: Synthesis of 9,9-bis(4-allyloxy)phenyl)-9H-fluorene

In a 1,000 ml two-necked flask equipped with a refluxing condenser, 10.0 g of 4,4'-(9H-fluorene-9,9-diyl)diphenol (Sigma-Aldrich), 6.17 ml of allyl bromide (Sigma-Aldrich), 23.68 g of K₂CO₃, and 500 ml of acetone were added and mixed at room temperature. Then, the temperature of a refluxing apparatus was set to 80°C, and a homogeneously well mixed solution was refluxed for performing reaction overnight. After completing the refluxing reaction, the reactant was cooled to room temperature, filtered using celite filtration and evaporated to produce a crude product. A target material in the crude product was extracted with ethyl acetate, washed with water three times, and dried with MgSO₄. MgSO₄ was removed using a filter, and solvents were removed using an evaporator to obtain 9,9-bis(4-(allyloxy)phenyl)-9H-fluorene as an intermediate (11). The Reaction Scheme of the first step and NMR data of the intermediate (11) are as follows. 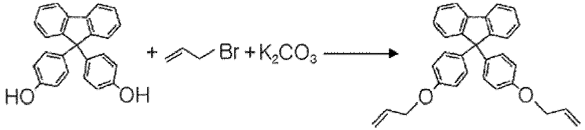 ¹H NMR (400MHz, CDCl₃) : δ=4.46 (td, J=1.4, 2.4Hz, 4H), 5.25 (qd, J=1.6, 1.2, 10.4Hz, 2H), 5.35-5.38 (m, 2H), 5.97-6.06 (m, 2H), 6.75 (td, J=3.2, 2.0, 8.8Hz, 4H), 7.10 (td, J=3.2, 2.0, 8.8Hz, 4H), 7.23-7.39 (m, 6H), 7.70-7.79 (m, 2H).

### (2) Second step: Synthesis of 4,4'-(9H-fluorene-9,9-diyl)bis(2-allylphenol)

In a 100 ml flask, 20.0 g of the intermediate (11) obtained in the first step was added, and the flask was inserted in a microwave oven of which power and temperature were set to 300 W and 160°C, followed by performing reaction for 20 minutes. After completing the reaction, the reactant was cooled to room temperature, and solvents were removed in a vacuum oven to produce 4,4'-(9H-fluorene-9,9-diyl)bis(2-allylphenol) as an intermediate (12). The Reaction Scheme of the second step and NMR data of the intermediate (12) are as follows. 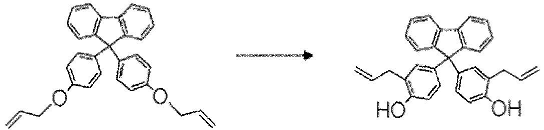 ¹H NMR (400MHz, CDCl₃) : δ=3.28 (d, J=6.0Hz, 4H), 5.04-5.09 (m, 4H), 5.21 (s, 2H), 5.87-5.97 (m, 2H), 6.62 (d, J=8.4Hz, 2H), 6.88 (dd, J=2.4, 6.0Hz, 2H), 6.96 (d, J=2.4Hz, 2H), 7.22-7.36 (m, 6H), 7.74 (d, J=7.2Hz, 2H)

### (3) 2-1-st step: Synthesis of 9,9-bis(3-allyl-4-(allyloxy)phenyl)-9H-fluorene

In a 1,000 ml two-necked flask equipped with a refluxing condenser, 20.0 g of the intermediate (12) obtained in the second step, 15.09 ml of allyl bromide (Sigma-Aldrich), 57.82 g of K₂CO₃, and 500 ml of acetone are added and mixed at room temperature. Then, a well mixed solution is refluxed at the set temperature of a refluxing apparatus of 80°C to perform the reaction overnight. After completing the reaction, the reactant is cooled to room temperature and is filtered using celite, and organic solvents are evaporated to obtain a crude product. A target material in the crude product is extracted with ethyl acetate, washed with water three times, and dried with MgSO₄. MgSO₄ is removed using a filter, and solvents are removed using an evaporator to obtain 9,9-bis(3-allyl-4-(allyloxy)phenyl)-9H-fluorene as an intermediate (23). The Reaction Scheme of the 2-1-st step is as follows.

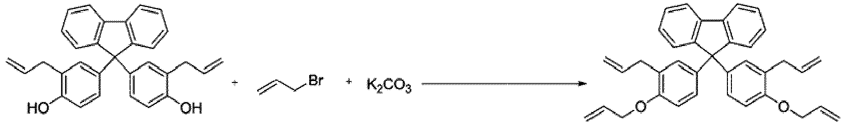

### (4) 2-2-nd step: Synthesis of 4,4'-(9H-fluorene-9,9-diyl)bis(2,6-diallylphenol)

In a 100 ml flask, 20.0 g of the intermediate (23) obtained in the 2-1-st step is added, and the flask is inserted in an oven of which power and temperature are set to 300 W and 160°C for performing reaction for 20 minutes. After completing the reaction, the reactant is cooled to room temperature to produce 4,4'-(9H-fluorene-9,9-diyl)bis(2,6-diallylphenol) as an intermediate (24). The Reaction Scheme of the 2-2-nd step is as follows.

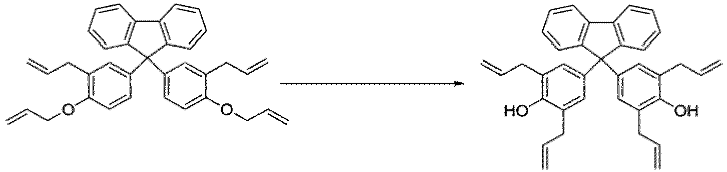

### (5) Third step: Synthesis of 2,2'-(4,4'-(9H-fluorene-9,9-diyl)bis(2,6-diallyl-4,1-phenyl ene))bis(oxy)bis(methylene)dioxirane

In a 1,000 ml two-necked flask equipped with a refluxing condenser, 20.0 g of the intermediate (24) obtained in the 2-2-nd step, 37. 84 ml of epichlorohydrin (Sigma-Aldrich), 43.76 g of K₂CO₃, and 300 ml of acetonitrile are added and mixed at room temperature. Then, the reaction temperature is elevated to 80°C, and the reaction is performed overnight. After completing the reaction, the reactant is cooled to room temperature and is filtered using celite, and organic solvents are evaporated to obtain 2,2'-(4,4'-(9H-fluorene-9,9-diyl)bis(2,6-diallyl-4,1-phenyl ene))bis(oxy)bis(methylene)dioxirane as an intermediate (25). The Reaction Scheme of the third step is as follows.

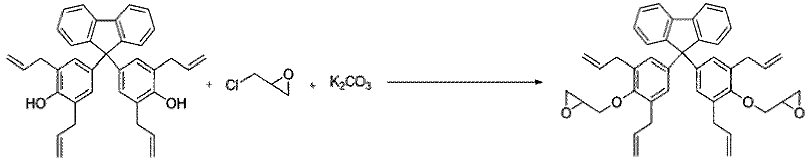

### (6) Fourth step: Synthesis of (3,3',3",3"'-(5,5'-(9H-fluorene-9,9-diyl)bis(2-oxirane-2-y lmethoxy)benzene-5,2,1-triyl)tetrakispropane-3,1-diyl))tetr akis(triethoxysilane)

In a 500 ml flask, 20.0 g the intermediate (25) obtained in the third step, 21.57 ml of triethoxysilane (Sigma-Aldrich), 241 mg of platinum oxide, and 200 ml of toluene are added and mixed, followed by stirring in an argon charged atmosphere at 85°C for 24 hours. After completing the reaction, the crude product thus obtained is filtered using celite filtration, and solvents are removed using an evaporator to produce a target material of a fluorene epoxy compound containing an alkoxysilyl group. The Reaction Scheme of the fourth step and NMR data of the target material thus obtained are as follows. 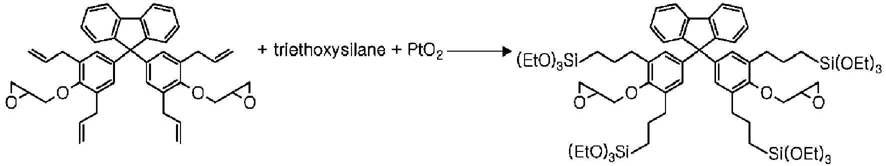 ¹H NMR (400MHz, CDCl₃) : δ=0.60-0.70 (m, 8H), 1.20-1.25 (t, 36H), 1.60-1.70 (m, 8H), 2.50-2.70(t, 8H), 2.70-2.80(m, 2H), 2.80-2.90 (m, 2H), 3.30-3.40 (m, 2H), 3.70-4.00 (m, 26H), 4.10-4.20(m, 2H), 6.70-7.00 (s, 4H), 7.20-7.40 (m, 6H), 7.70-7.90 (d, 2H).

### Expected Synthetic Example CI-4(2): Synthesis of tetra-alkoxysilylated epoxy compound using dihydroxyfluorene

The same procedure described in the above Synthetic Example CI-4 (1) is conducted except for conducting the Claisen rearrangement reaction of the second step and the 2-2-nd step in the above Synthetic Example CI-4(1) as follows to produce (3,3',3",3"'-(5,5'-(9H-fluorene-9,9-diyl)bis(2-oxirane-2-yl methoxy)benzene-5,3,1-triyl))tetrakispropane-3,1-diyl))tetr akis(triethoxysilane).

In the second step, in a 1,000 ml two-necked flask equipped with a refluxing condenser, 10.0 g of the intermediate (11) obtained in the first step of the above Synthetic Example CI-4(1), and 100 ml of 1, 2-dichlorobenzene (Sigma-Aldrich) are added and well mixed at room temperature. Then, the homogeneous solution thus obtained is refluxed for 96 hours at the set temperature of a refluxing apparatus of 190°C. After completing the reaction, the reactant is cooled to room temperature, and solvents are removed by a vacuum oven to produce 4,4'-(9H-fluorene-9,9-diyl)bis(2-allylphenol) as an intermediate (12). The Reaction Scheme of the second step and NMR data of the intermediate (12) are the same as those of the second step of the above Synthetic Example CI-4(1).

In the 2-1-st step, the same procedure in the 2-1-st step of the above Synthetic Example CI-4 (1) is conducted using the above intermediate (12). Then, in a 1,000 ml two-necked flask equipped with a refluxing condenser, 20.0 g of intermediate (23) obtained in the 2-1-st step and 100 ml of 1,2-dichlorobenzene (Sigma-Aldrich) are added and well mixed at room temperature. Then, the homogeneous solution thus obtained is refluxed for 8 hours at the set temperature of a refluxing apparatus of 190°C. After completing the reaction, the reactant is cooled to room temperature, and solvents are removed by a vacuum oven to produce 4,4'-(9H-fluorene-9,9-diyl)bis(2,6-diallylphenol) as an intermediate (24). The Reaction Scheme of the 2-2-nd step is the same as that of the 2-2-nd step of the above Synthetic Example CI-4(1).

### Synthetic Example DI-1(1): Synthesis of mono-alkoxysilylated epoxy compound using bisphenol A

### (1) First step: Synthesis of 4-(2-(4-(allyloxy)phenyl)propane-2-yl)phenol

In a 1,000 ml two-necked flask equipped with a refluxing condenser, 20.0 g of bisphenol A (Sigma-Aldrich), 36.35 g of K₂CO₃, and 500 ml of acetone were added and mixed at room temperature. Then, the temperature of a refluxing apparatus was set to 80°C, and a homogeneously well mixed solution was refluxed. While refluxing the homogeneously well mixed solution, 8.33 ml of allyl bromide (Sigma-Aldrich) was added dropwisely, followed by performing reaction overnight. After completing the reaction, the reactant was cooled to room temperature and filtered using celite filtration. Organic solvents were evaporated to produce a crude product. A target material in the crude product was extracted with ethyl acetate, washed with water three times, and dried with MgSO₄. MgSO₄ was removed using a filter, and solvents were removed using an evaporator to obtain 4-(2-(4-(allyloxy)phenyl)propane-2-yl)phenol as an intermediate (11). The Reaction Scheme of the first step and NMR data of the intermediate (11) thus obtained are as follows. 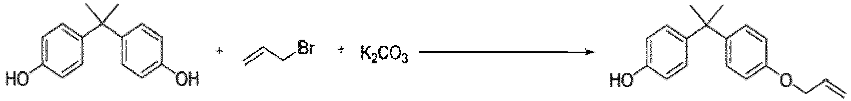 ¹H NMR (400MHz, CDCl₃) : δ=1.60 (s, 6H), 4.87 (s, 1H), 4.60 (d, J=5.2Hz, 2H), 5.33 (dd, J=1.4Hz, 1H), 5.44 (dd, J=1.6Hz, 1H), 6.05-6.15 (m, 1H), 6.47(d, J=8.2Hz, 2H), 6.70 (d, J=8.4Hz, 2H), 7.28 (d, J=10.8Hz, 4H).

### (2) Second step: Synthesis of 2-allyl-4-(2-(4-hydroxyphenyl)propane-2-yl)phenol

In a 100 ml flask, 20.0 g of the intermediate (11) obtained in the first step and 50 ml of 1,2-dichlorobenzene (Sigma-Aldrich) were added, and the flask was inserted in a microwave oven of which power and temperature were set to 300 W and 160°C, followed by performing reaction for 20 minutes. After completing the reaction, the reactant was cooled to room temperature, and solvents were removed by a vacuum oven to produce 2-allyl-4-(2-(4-hydroxyphenyl)propane-2-yl)phenol as an intermediate (12). The Reaction Scheme of the second step and NMR data of the intermediate (12) thus obtained are as follows. 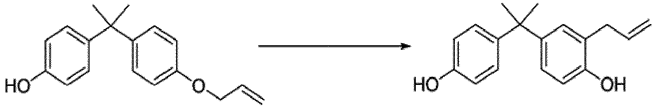 ¹H NMR (400MHz, CDCl₃) : δ=1.60 (s, 6H), 3.36 (d, J=6.4Hz, 2H), 4.86 (br.s, 2H), 5.08-5.12 (m, 2H), 5.92-6.03 (m, 1H), 6.76 (m, 3H), 6.94 (m, 4H).

### (3) Third step: Synthesis of 2-((2-allyl-4-(2-(4-(oxirane-2-ylmethoxy)phenyl)propane-2-y l)phenoxy)methyl)oxirane

In a 500 ml two-necked flask equipped with a refluxing condenser, 7.0 g of the intermediate (12) obtained in the second step, 19.35 ml of epichlorohydrin (Sigma-Aldrich), 21. 64 g of K₂CO₃, and 200 ml of acetonitrile were added and mixed at room temperature. Then, the reaction temperature was elevated to 80°C, and the reaction was performed overnight. After completing the reaction, the reactant was cooled to room temperature and was filtered using celite, and organic solvents were evaporated to obtain 2-((2-allyl-4-(2-(4-(oxirane-2-ylmethoxy)phenyl)propane-2-y l)phenoxy)methyl)oxirane as an intermediate (13). The Reaction Scheme of the third step and NMR data of the intermediate (13) thus obtained are as follows. 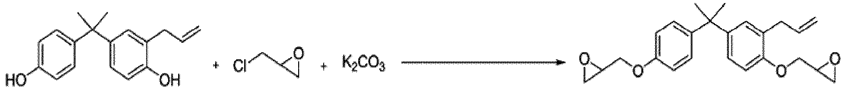 ¹H NMR (400MHz, CDCl₃) : δ=1.60 (s, 6H), 2.76 (dd, J=2. 6Hz, 2H), 2.87 (dd, J=4.2Hz, 2H), 3.30-3.36 (m, 4H), 3.95-3.98 (m, 2H), 4.17-4.20 (m, 2H), 4.97-5.03 (m, 2H), 5.93-5.98 (m, 1H), 6.72 (m, 3H), 6.96-7.01 (m, 4H).

### (4) Fourth step: Synthesis of triethoxy(3-(2-(oxirane-2-ylmethoxy)-5-(2-(4-(oxirane-2-ylm ethoxy)phenyl)propane-2-yl)phenyl)propyl)silane

In a 250 ml flask, 10.0 g of the intermediate (13) obtained in the third step, 5.95 ml of triethoxysilane (Sigma-Aldrich), 100 mg of platinum oxide, and 100 ml of toluene were added and well mixed, followed by stirring in an argon charged atmosphere at 85°C for 24 hours. After completing the reaction, the crude product thus obtained was filtered using celite filtration, and solvents were removed using an evaporator to produce a target material of a bisphenol A epoxy compound containing an alkoxysilyl group. The Reaction Scheme of the fourth step and NMR data of the target material thus obtained are as follows. 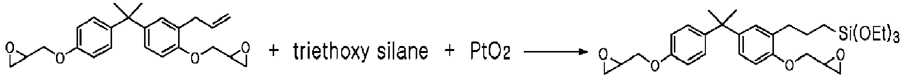 ¹H NMR (400MHz, CDCl₃) : δ=0.65-0.70 (m, 2H), 1.23 (t, J=7.0Hz, 9H), 1.61 (s, 6H), 1.60-1.71 (m, 2H), 2.62 (t, J=7. 6Hz, 2H), 2.74 (dd, J=2.6Hz, 2H), 2.86 (dd, J=4.2Hz, 2H), 3.30-3.34 (m, 2H), 3.80 (q, 1.6Hz, 6H), 3.98 (dd, J=5.2Hz, 2H), 4.13 (dd, J=3.2Hz, 2H), 6.72 (m, 3H), 6.96-7.03 (m, 4H).

### Synthetic Example DI-1(2): Synthesis of mono-alkoxysilylated epoxy compound using bisphenol A

The same procedure described in the above Synthetic Example DI-1 (1) was conducted except for conducting the Claisen rearrangement reaction of the second step in the above Synthetic Example DI-1(1) as follows to produce triethoxy(3-(2-(oxirane-2-ylmethoxy)-5-(2-(4-(oxirane-2-ylm ethoxy)phenyl)propane-2-yl)phenyl)propyl)silane.

In the second step, in a 1,000 ml two-necked flask equipped with a refluxing condenser, 8.0 g of the intermediate (11) obtained in the first step of the above Synthetic Example DI-1(1), and 250 ml of 1, 2-dichlorobenzene (Sigma-Aldrich) were added and well mixed at room temperature. Then, the homogeneous solution thus obtained was refluxed for 8 hours at the set temperature of a refluxing apparatus of 190°C. After completing the reaction, the reactant was cooled to room temperature, and solvents were removed by a vacuum oven to produce 2-allyl-4-(2-(4-hydroxyphenyl)propane-2-yl)phenol as an intermediate (12). The Reaction Scheme and NMR data of the intermediate (12) are the same as those in the second step of the above Synthetic Example DI-2(1).

### Synthetic Example DI-2(1): Synthesis of di-alkoxysilylated epoxy compound using bisphenol A

### (1) First step: Synthesis of 4,4'-(propane-2,2-diyl)bis(allyloxybenzene)

In a 1,000 ml two-necked flask equipped with a refluxing condenser, 20.0 g of bisphenol A (Sigma-Aldrich), 18.94 ml of allyl bromide (Sigma-Aldrich), 72.69 g of K₂CO₃, and 500 ml of acetone were added and mixed at room temperature. Then, the temperature of a refluxing apparatus was set to 80°C, and a homogeneously well mixed solution was refluxed for performing reaction overnight. After completing the reaction, the reactant was cooled to room temperature, filtered using celite filtration and evaporated to produce a crude product. A target material in the crude product was extracted with ethyl acetate, washed with water three times, and dried with MgSO₄. MgSO₄ was removed using a filter, and solvents were removed using an evaporator to obtain 4,4'-(propane-2,2-diyl)bis(allyloxybenzene) as an intermediate (11). The Reaction Scheme of the first step and NMR data of the intermediate (11) thus obtained are as follows. 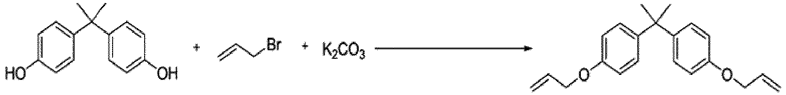 ¹H NMR (400MHz, CDCl₃): δ=1.60 (s, 6H), 4.61 (d, J=5.2Hz, 4H), 5.31 (dd, J=1.4Hz, 2H), 5.45 (dd, J=1.6Hz, 2H), 6.06-6.15 (m, 2H), 6.69 (d, J=8.4Hz, 4H), 7.28 (d, J=10.8Hz, 4H).

### (2) Second step: Synthesis of 4,4'-(propane-2,2-diyl)bis(2-alllylphenol)

In a 100 ml flask, 20.0 g of the intermediate (11) obtained in the first step was added, and the flask was inserted in a microwave oven of which power and temperature were set to 300 W and 160°C, followed by performing reaction for 20 minutes. After completing the reaction, the reactant was cooled to room temperature to produce 2,2'-diallylbisphenol A as an intermediate (12). The Reaction Scheme of the second step and NMR data of the intermediate (12) thus obtained are as follows. 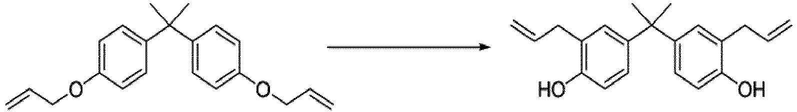 ¹H NMR (400MHz, CDCl₃) : δ=1.60 (s, 6H), 3.35 (d, J=6.4Hz, 4H), 4.86 (s, 2H), 5.08-5.12 (m, 4H), 5.93-6.03 (m, 2H), 6.75 (d, J=8.4Hz, 2H), 6.94 (dd, J=10.6Hz, 4H).

### (3) Third step: Synthesis of 2,2'-(4,4'-(propane-2,2-diyl)bis(2-allyl-4,1-phenylene))bis (oxy)bis(methylene)dioxirane

In a 1,000 ml two-necked flask equipped with a refluxing condenser, 29.0 g of the intermediate (12) obtained in the second step, 73.54 ml of epichlorohydrin (Sigma-Aldrich), 85. 67 g of K₂CO₃, and 300 ml of acetonitrile were added and mixed at room temperature. Then, the reaction temperature was elevated to 80°C, and the reaction was performed overnight. After completing the reaction, the reactant was cooled to room temperature and was filtered using celite, and organic solvents were evaporated to obtain an intermediate (13). The Reaction Scheme of the third step and NMR data of the intermediate (13) thus obtained are as follows. 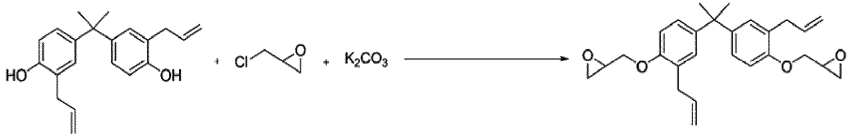 ¹H NMR (400MHz, CDCl₃) : δ=1.61 (s, 6H), 2.75 (dd, J=2. 6Hz, 2H), 2.87 (dd, J=4.2Hz, 2H), 3.32-3.36 (m, 6H), 3.94-3.98 (m, 2H), 4.16-4.20 (m, 2H), 4.97-5.03 (m, 4H), 5.93-5.98 (m, 2H), 6.71 (d, J=8.4Hz, 2H), 6.97-7.00 (m, 4H).

### (4) Fourth step: Synthesis of 3,3'-(5,5'-(propane-2,2-diyl)bis(2-oxirane-2-ylmethoxy)-5,1 -phenylene))bis(propane-3,1-diyl)bis(triethoxysilane)

In a 500 ml flask, 26.25 g of the intermediate (13) obtained in the fourth step, 25.35 ml of triethoxysilane (Sigma-Aldrich), 250 mg of platinum oxide, and 200 ml of toluene were added and mixed, followed by stirring in an argon charged atmosphere at 85°C for 24 hours. After completing the reaction, the crude product thus obtained was filtered using celite filtration, and solvents were removed using an evaporator to produce a target material of a bisphenol A epoxy compound containing an alkoxysilyl group. The Reaction Scheme of the fourth step and NMR data of the target material thus obtained are as follows. 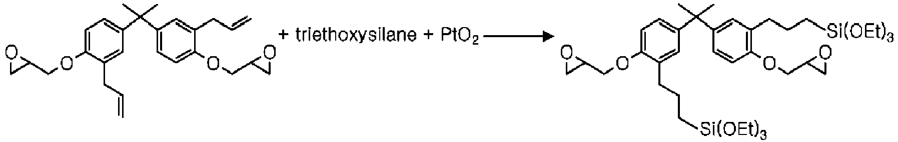 ¹H NMR (400MHz, CDCl₃) : δ=0.64-0.69 (m, 4H), 1.22 (t, J=7.0Hz, 18H), 1.60 (s, 6H), 1.62-1.72 (m, 4H), 2.61 (t, J=7. 6Hz, 4H), 2.74 (dd, J=2.6Hz, 2H), 2.86 (dd, J=4.2Hz, 2H), 3.30-3.34 (m, 2H), 3.79 (q, 1.6Hz, 12H), 3.97 (dd, J=5.2Hz, 2H), 4.14 (dd, J=3.2Hz, 2H), 6.70 (d, J=7.6Hz, 2H), 6.94 (dd, J=2.8Hz, 2H), 6.99 (d, J=7.6Hz, 2H).

### Synthetic Example DI-2(2): Synthesis of di-alkoxysilylated epoxy compound using bisphenol A

The same procedure described in the above Synthetic Example DI-2 (1) was conducted except for conducting the Claisen rearrangement reaction of the second step in the above Synthetic Example DI-2(1) as follows to produce (3,3'-(5,5'-(propane-2,2-diyl)bis(2-(oxirane-2-ylmethoxy)-5 ,1-phenylene))bis(propane-3,1-diyl))bis(triethoxysilane).

In the second step, in a 1,000 ml two-necked flask equipped with a refluxing condenser, 20.0 g of the intermediate (11) obtained in the first step of the above Synthetic Example DI-2(1), and 250 ml of 1, 2-dichlorobenzene (Sigma-Aldrich) were added and well mixed at room temperature. Then, the homogeneous solution thus obtained was refluxed for 8 hours at the set temperature of a refluxing apparatus of 190°C. After completing the reaction, the reactant was cooled to room temperature, and solvents were removed by a vacuum oven to produce 2,2'-diallylbisphenol A as an intermediate (12). The Reaction Scheme of the second step and NMR data of the intermediate (12) are the same as those in the second step of the above Synthetic Example DI-2(1).

### Synthetic Example DI-2-1: Synthesis of mono-alkoxysilylated epoxy compound using bisphenol A

### (1) 3-1th step: Synthesis of 2-((2-allyl-4-(2-(4-(oxirane-2-ylmethoxy)-3-(oxirane-2-ylme thoxy)phenyl)propane-2-yl)phenoxy)methyl)oxirane

In a 500 ml, 15.0 g of 2,2'-(4,4'-(propane-2,2-diyl)bis(2-allyl-4,1-phenylene))bis (oxy) bis (methylene) dioxirane obtained in the third step of the above Synthetic Example DI-2(1), 10.39 g of 77 mol% 3-chloroperoxybenzoic acid, and 300 ml of methylene chloride were added and stirred at room temperature for 18 hours. Then, the reactant was worked-up with an aqueous sodium thiosulfate pentahydrate solution and extracted with ethyl acetate. Then, the product thus obtained was washed with an 1 N aqueous sodium hydroxide solution and brine, dried with MgSO₄ and filtered using a filter. After removing solvents by evaporation, the product thus obtained was separated by silica column chromatography to produce 2-((2-allyl-4-(2-(4-(oxirane-2-ylmethoxy)-3-(oxirane-2-ylme thoxy)phenyl)propane-2-yl)phenoxy)methyl)oxirane as an intermediate (13'). The Reaction Scheme of the 3-1th step and NMR data of the final product are as follows. 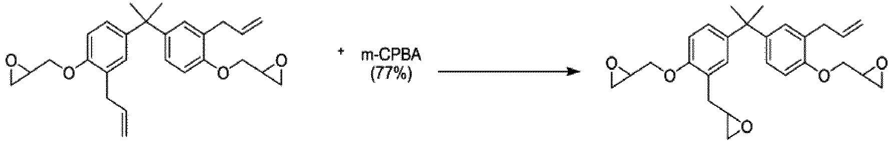 ¹H NMR (400MHz, CDCl₃) : δ=1.60 (s, 6H), 2.53-2.57 (m, 1H), 2.73-2.81 (m, 5H), 2.89-2.92 (m, 3H), 3.16-3.18 (m, 1H), 3.31-3.35 (m, 3H), 3.90-3.97 (m, 2H), 4.22-4.25 (m, 2H), 4.97-5.04 (m, 2H), 5.93-5.97 (m, 1H), 6.66-6.82 (m, 2H), 6.73-6.75 (m, 2H), 7.03-7.05 (m, 2H).

### (2) Fourth step: Synthesis of triethoxy(3-(2-oxirane-2-ylmethoxy)-5-(2-(4-(oxirane-2-ylme thoxy)-3-(oxirane-2-ylmethoxy)phenyl)-propane-2-yl)phenyl)p ropyl)silane

In a 250 ml flask, 10.0 g the intermediate (13') obtained in the 3-1-st step, 5.01 ml of triethoxysilane (Sigma-Aldrich), 100 mg of platinum oxide, and 100 ml of toluene were added and mixed, followed by stirring in an argon charged atmosphere at 85°C for 24 hours. After completing the reaction, the crude product thus obtained was filtered using celite filtration, and solvents were removed using an evaporator to produce a target material of a bisphenol A epoxy compound containing an alkoxysilyl group. The Reaction Scheme of the fourth step and NMR data of the target material thus obtained are as follows. 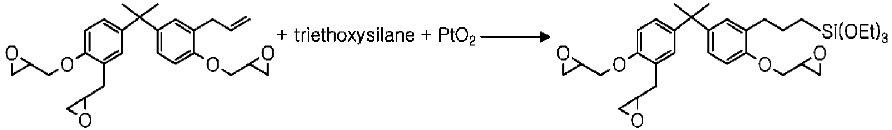 ¹H NMR (400MHz, CDCl₃) : δ=0.64-0.69 (m, 2H), 1.20 (t, J=7.0Hz, 9H), 1.60 (s, 6H), 1.62-1.72 (m, 2H), 2.53-2.57 (m, 1H), 2.61 (t, J=7.6Hz, 2H), 2.73-2.81 (m, 5H), 2.89-2.92 (m, 3H), 3.16-3.18 (m, 1H), 3.35-3.37 (m, 1H), 3.79 (q, 1. 6Hz, 6H), 3.90-3.97 (m, 2H), 4.22-4.25 (m, 2H), 6.66-6.82 (m, 2H), 6.73-6.75 (m, 2H), 7.03-7.05 (m, 2H).

### Expected Synthetic Example DI-3(1): Synthesis of tri-alkoxysilylated epoxy compound using bisphenol A

### (1) First step: Synthesis of 4,4'-(propane-2,2-diyl)bis(allyloxybenzene)

In a 1,000 ml two-necked flask equipped with a refluxing condenser, 20.0 g of bisphenol A (Sigma-Aldrich), 18.94 ml of allyl bromide (Sigma-Aldrich), 72.69 g of K₂CO₃, and 500 ml of acetone were added and mixed at room temperature. Then, the temperature of a refluxing apparatus was set to 80°C, and a homogeneously well mixed solution was refluxed for performing reaction overnight. After completing the reaction, the reactant was cooled to room temperature, filtered using celite filtration and evaporated to produce a crude product. A target material in the crude product was extracted with ethyl acetate, washed with water three times, and dried with MgSO₄. MgSO₄ was removed using a filter, and solvents were removed using an evaporator to obtain 4,4'-(propane-2,2-diyl)bis(allyloxybenzene) as an intermediate (11). The Reaction Scheme of the first step and NMR data of the intermediate (11) thus obtained are as follows. 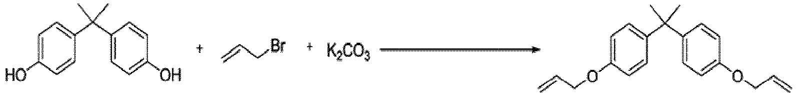 ¹H NMR (400MHz, CDCl₃): δ=1.60 (s, 6H), 4.61 (d, J=5.2Hz, 4H), 5.31 (dd, J=1.4Hz, 2H), 5.45 (dd, J=1.6Hz, 2H), 6.06-6.15 (m, 2H), 6.69 (d, J=8.4Hz, 4H), 7.28 (d, J=10.8Hz, 4H).

### (2) Second step: Synthesis of 4,4'-(propane-2,2-diyl)bis(2-allylphenol)

In a 100 ml flask, 20.0 g of the intermediate (11) obtained in the first step was added, and the flask was inserted in a microwave oven of which power and temperature were set to 300 W and 160°C, followed by performing reaction for 20 minutes. After completing the reaction, the reactant was cooled to room temperature to produce 4,4'-(propane-2,2-diyl)bis(2-allylphenol) as an intermediate (12). The Reaction Scheme of the second step and NMR data of the intermediate (12) are as follows. 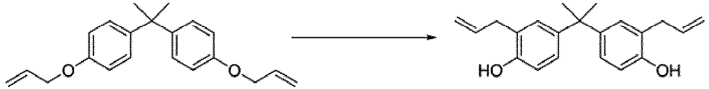 ¹H NMR (400MHz, CDCl₃) : δ=1.60 (s, 6H), 3.35 (d, J=6.4Hz, 4H), 4.86 (s, 2H), 5.08-5.12 (m, 4H), 5.93-6.03 (m, 2H), 6.75 (d, J=8.4Hz, 2H), 6.94 (dd, J=10.6Hz, 4H).

### (3) 2-1-st step: Synthesis of 2-ally-4-(2-(3-allyl-4-(allyloxy)phenyl-propane-2-yl)phenol

In a 1,000 ml two-necked flask equipped with a refluxing condenser, 20.0 g of the intermediate (12) obtained in the second step, 23.07 g of K₂CO₃, and 500 ml of acetone are added and mixed at room temperature. Then, a homogeneously mixed solution is refluxed at the set temperature of a refluxing apparatus of 80°C. While refluxing the homogeneously mixed solution, 5.29 ml of allyl bromide (Sigma-Aldrich) is added thereto dropwisely, and the reaction is performed overnight. After completing the reaction, the reactant is cooled to room temperature and is filtered using celite, and organic solvents are evaporated to obtain a crude product. A target material in the crude product is extracted with ethyl acetate, washed with water three times, and dried with MgSO₄. MgSO₄ is removed using a filter, solvents are removed using an evaporator, and the product thus obtained is separated by silica column chromatography to obtain 2-allyl-4-(2-(3-allyl-4-(allyloxy)phenyl)propane-2-yl)pheno 1 as an intermediate (23). The Reaction Scheme of the 2-1-st step is as follows.

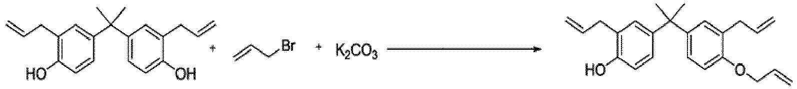

### (4) 2-2-nd step: Synthesis of 2,6-diallyl-4-(2-(3-allyl-4-hydroxyphenyl)propane-2-yl)phen ol

In a 100 ml flask, 20.0 g of the intermediate (23) obtained in the 2-1-st step is added, and the flask is inserted in an oven of which power and temperature are set to 300 W and 160°C for performing reaction for 20 minutes. After completing the reaction, the reactant is cooled to room temperature to obtain 2,6-diallyl-4-(2-(3-allyl-4-hydroxyphenyl)propane-2-yl)phen ol as an intermediate (24). The Reaction Scheme of the 2-2-nd step is as follows.

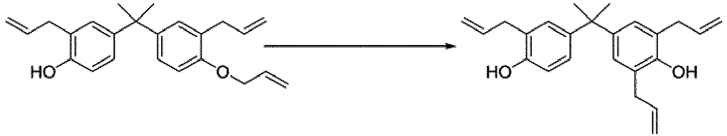

### (5) Third step: Synthesis of 2-((2-allyl-4-(2-(3,5-diallyl-4-(oxirane-2-ylmethoxy)phenyl )propane-2-yl)phenoxy)methyl)oxirane

In a 1,000 ml two-necked flask equipped with a refluxing condenser, 20.0 g of the intermediate (24) obtained in the 2-2-nd step, 44.24 ml of epichlorohydrin (Sigma-Aldrich), 51.16 g of K₂CO₃, and 300 ml of acetonitrile are added and mixed at room temperature. Then, the reaction temperature was elevated to 80°C, and the reaction was performed overnight. After completing the reaction, the reactant is cooled to room temperature and is filtered using celite, and organic solvents are evaporated to obtain 2-((2-allyl-4-(2-(3,5-diallyl-4-(oxirane-2-ylmethoxy)phenyl )propane-2-yl)phenoxy)methyl)oxirane as an intermediate (25). The Reaction Scheme of the third step is as follows.

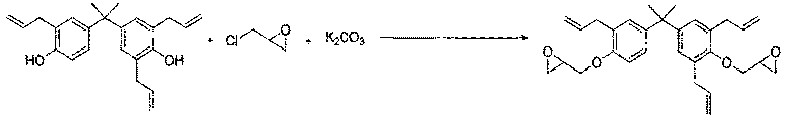

### (6) Fourth step: Synthesis of 3,3'-(2-oxirane-2-ylmethoxy)-5-(2-(4-(oxirane-2-ylmethoxy)-3-(3-(triethoxysilyl)propyl)phenyl)propane-2-yl)-1,3-phenyl ene)bis(propane-3,1-diyl))bis(triethoxysilane)

In a 500 ml flask, 20.0 g the intermediate (23) obtained in the third step, 26.47 ml of triethoxysilane (Sigma-Aldrich), 296 mg of platinum oxide, and 200 ml of toluene are added and mixed, followed by stirring in an argon charged atmosphere at 85°C for 24 hours. After completing the reaction, the crude product thus obtained is filtered using celite filtration, and solvents are removed using an evaporator to produce a target material of a bisphenol A epoxy compound containing an alkoxysilyl group. The Reaction Scheme of the fourth step and NMR data of the target material thus obtained are as follows. 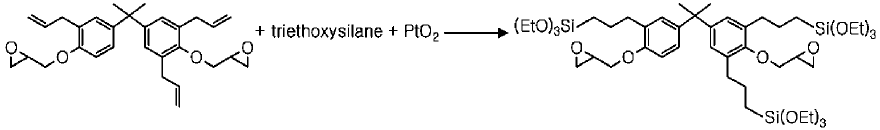 ¹H NMR (400MHz, CDCl₃) : δ=0.60-0.70 (m, 6H), 1.20-1.25 (t, 27H), 1.60-1.80 (m, 12H), 2.50-2.70 (t, 6H), 2.70-2.80 (m, 2H), 2.80-2.90 (m, 2H), 3.30-3.40 (m, 2H), 3.70-4.00 (m, 20H), 4.10-4.20 (m, 2H), 6.80-7.10 (m, 5H).

### Expected Synthetic Example DI-3(2): Synthesis of tri-alkoxysilylated epoxy compound using bisphenol A

The same procedure described in the above Synthetic Example DI-3 (1) is conducted except for conducting the Claisen rearrangement reaction of the second step and the 2-2-nd step in the above Synthetic Example DI-3(1) as follows to produce (3,3'-(2-oxirane-2-ylmethoxy)-5-(2-(4-(oxirane-2-ylmethoxy) -3-(3-triethoxysilyl)propyl)phenyl)propane-2-yl)-1,3-phenyl ene)bis(propane-3,1-diyl))bis(triethoxysilane).

In the second step, in a 1,000 ml two-necked flask equipped with a refluxing condenser, 20.0 g of the intermediate (11) obtained in the first step of the above Synthetic Example DI-3(1), and 250 ml of 1, 2-dichlorobenzene (Sigma-Aldrich) are added and well mixed at room temperature. Then, the homogeneous solution thus obtained is refluxed for 8 hours at the set temperature of a refluxing apparatus of 190°C. After completing the reaction, the reactant is cooled to room temperature, and solvents are removed by a vacuum oven to produce 4,4'-(propane-2,2-diyl)bis(2-allylphenol) as an intermediate (12). The Reaction Scheme of the second step and NMR data of the intermediate (12) are the same as those of the second step of the above Synthetic Example DI-3(1).

In the 2-1-st step, the same procedure in the 2-1-st step of the above Synthetic Example DI-3 (1) is conducted using the above intermediate (12). Then, in a 1,000 ml two-necked flask equipped with a refluxing condenser, 20.0 g of the intermediate (23) obtained in the 2-1-st step and 100 ml of 1,2-dichlorobenzene (Sigma-Aldrich) are added and well mixed at room temperature. Then, the homogeneous solution thus obtained is refluxed for 8 hours at the set temperature of a refluxing apparatus of 190°C. After completing the reaction, the reactant is cooled to room temperature, and solvents are removed by a vacuum oven to produce 2,6-diallyl-4-(2-(3-allyl-4-hydroxyphenyl)propane-2-yl)phen ol as an intermediate (24). The Reaction Scheme of the 2-2-nd step is the same as that of the 2-2-nd step of the above Synthetic Example DI-3(1).

### Expected Synthetic Example DI-4(1): Synthesis of tetra-alkoxysilylated epoxy compound using bisphenol A

### (1) First step: Synthesis of 4,4'-(propane-2,2-diyl)bis(allyloxybenzene)

In a 1,000 ml two-necked flask equipped with a refluxing condenser, 20.0 g of bisphenol A (Sigma-Aldrich), 18.94 ml of allyl bromide (Sigma-Aldrich), 72.69 g of K₂CO₃, and 500 ml of acetone were added and mixed at room temperature. Then, the temperature of a refluxing apparatus was set to 80°C, and a homogeneously well mixed solution was refluxed for performing reaction overnight. After completing the reaction, the reactant was cooled to room temperature, filtered using celite filtration and evaporated to produce a crude product. A target material in the crude product was extracted with ethyl acetate, washed with water three times, and dried with MgSO₄. MgSO₄ was removed using a filter, and solvents were removed using an evaporator to obtain 4,4'-(propane-2,2-diyl)bis(allyloxybenzene) as an intermediate (11). The Reaction Scheme of the first step and NMR data of the intermediate (11) are as follows. 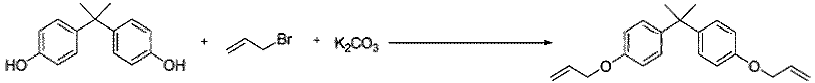 ¹H NMR (400MHz, CDCl₃) : δ=1.60 (s, 6H), 4.61 (d, J=5.2Hz, 4H), 5.31 (dd, J=1.4Hz, 2H), 5.45 (dd, J=1.6Hz, 2H), 6.06-6.15 (m, 2H), 6.69 (d, J=8.4Hz, 4H), 7.28 (d, J=10.8Hz, 4H).

### (2) Second step: Synthesis of 4,4'-(propane-2,2-diyl)bis(2-allylphenol)

In a 100 ml flask, 20.0 g of the intermediate (11) obtained in the first step was added, and the flask was inserted in a microwave oven of which power and temperature were set to 300 W and 160°C, followed by performing reaction for 20 minutes. After completing the reaction, the reactant was cooled to room temperature to produce 4,4'-(propane-2,2-diyl)bis(2-allylphenol). The Reaction Scheme of the second step and NMR data of an intermediate (12) are as follows. 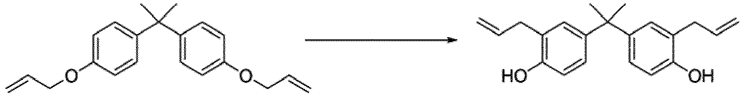 ¹H NMR (400MHz, CDCl₃) : δ=1.60 (s, 6H), 3.35 (d, J=6.4Hz, 4H), 4.86 (s, 2H), 5.08-5.12 (m, 4H), 5.93-6.03 (m, 2H), 6.75 (d, J=8.4Hz, 2H), 6.94 (dd, J=10.6Hz, 4H).

### (3) 2-1-st step: Synthesis of 4,4'-(propane-2,2-diyl)bis(2-allyl-1-(allyloxy)benzene)

In a 1,000 ml two-necked flask equipped with a refluxing condenser, 20.0 g of the intermediate (12) obtained in the second step, 21.07 ml of allyl bromide (Sigma-Aldrich), 83.31 g of K₂CO₃, and 500 ml of acetone are added and mixed at room temperature. Then, a well mixed solution is refluxed at the set temperature of a refluxing apparatus of 80°C to perform the reaction overnight. After completing the reaction, the reactant is cooled to room temperature and is filtered using celite, and organic solvents are evaporated to obtain a crude product. A target material in the crude product is extracted with ethyl acetate, washed with water three times, and dried with MgSO₄. MgSO₄ is removed using a filter, and solvents are removed using an evaporator, to obtain 4,4'-(propane-2,2-diyl)bis(2-allyl-l-(allyloxy)benzene) as an intermediate (23). The Reaction Scheme of the 2-1-st step is as follows.

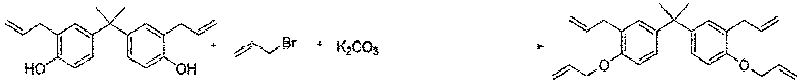

### (4) 2-2-nd step: Synthesis of 4,4'-(propane-2,2-diyl)bis(2,6-diallylphenol)

In a 100 ml flask, 20.0 g of the intermediate (23) obtained in the 2-1-st step is added, and the flask is inserted in an oven of which power and temperature are set to 300 W and 160°C for performing reaction for 20 minutes. After completing the reaction, the reactant is cooled to room temperature to produce 4,4'-(propane-2,2-diyl)bis(2,6-diallylphenol) as an intermediate (24). The Reaction Scheme of the second step is as follows.

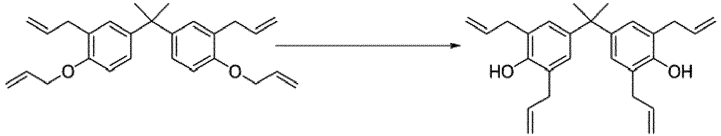

### (5) Third step: Synthesis of 2,2'-(4,4'-(propane-2,2-diyl)bis(2,6-diallyl-4,1-phenylene) )bis(oxy)bis(methylene)dioxirane

In a 1,000 ml two-necked flask equipped with a refluxing condenser, 20.0 g of the intermediate (24) obtained in the 2-2-nd step, 49.71 ml of epichlorohydrin (Sigma-Aldrich), 57.68 g of K₂CO₃, and 300 ml of acetonitrile are added and mixed at room temperature. Then, the reaction temperature is elevated to 80°C, and the reaction is performed overnight. After completing the reaction, the reactant is cooled to room temperature and is filtered using celite, and organic solvents are evaporated to obtain 2,2'-(4,4'-(propane-2,2-diyl)bis(2,6-diallyl-4,1-phenylene) ) bis (oxy) bis (methylene) dioxirane as an intermediate (25). The Reaction Scheme of the third step is as follows.

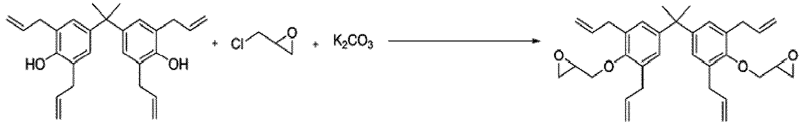

### (6) Fourth step: Synthesis of (3,3',3",3"'-(5,5'-(propane-2,2-diyl)bis(2-oxirane-2-ylmet hoxy)benzene-5,3,1-triyl)tetrakis(propane-3,1-diyl))tetraki s(triethoxysilane)

In a 500 ml flask, 20.0 g of the intermediate (25) obtained in the third step, 26.83 ml of triethoxysilane (Sigma-Aldrich), 300 mg of platinum oxide, and 200 ml of toluene are added and mixed, followed by stirring in an argon charged atmosphere at 85°C for 24 hours. After completing the reaction, the crude product thus obtained is filtered using celite filtration, and solvents are removed using an evaporator to produce a target material of a bisphenol A epoxy compound containing an alkoxysilyl group. The Reaction Scheme of the fourth step and NMR data of the target material thus obtained are as follows. 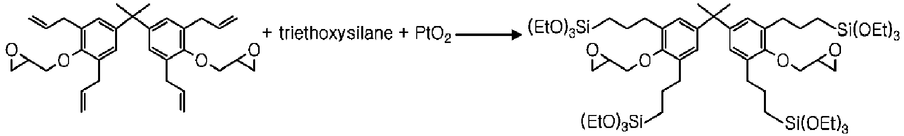 ¹H NMR (400MHz, CDCl₃) : δ=0.60-0.70 (m, 8H), 1.20-1.25 (t, 36H), 1.60-1.80 (m, 14H), 2.50-2.70 (t, 8H), 2.70-2.80 (m, 2H), 2.80-2.90 (m, 2H), 3.30-3.40 (m, 2H), 3.70-4.00 (m, 26H), 4.10-4.20 (m, 2H), 6.80-7.10 (s, 4H).

### Expected Synthetic Example DI-4(2): Synthesis of tetra-alkoxysilylated epoxy compound using bisphenol A

The same procedure described in the above Synthetic Example DI-4 (1) is conducted except for conducting the Claisen rearrangement reaction of the second step and the 2-2-nd step in the above Synthetic Example DI-4(1) as follows to produce (3,3',3",3"'-(5,5'-(propane-2,2-diyl)bis(2-(oxirane-2-ylmet hoxy)benzene-5,3,1-triyl))tetrakis(propane-3,1-diyl))tetrak is(triethoxysilane).

In the second step, in a 1,000 ml two-necked flask equipped with a refluxing condenser, 20.0 g of the intermediate (11) obtained in the first step of the above Synthetic Example DI-4(1), and 250 ml of 1, 2-dichlorobenzene (Sigma-Aldrich) are added and well mixed at room temperature. Then, the homogeneous solution thus obtained is refluxed for 8 hours at the set temperature of a refluxing apparatus of 190°C. After completing the reaction, the reactant is cooled to room temperature, and solvents are removed by a vacuum oven to produce 4,4'-(propane-2,2-diyl)bis(2-allylphenol) as an intermediate (12). The Reaction Scheme of the second step and NMR data of the intermediate (12) are the same as those of the second step of the above Synthetic Example DI-4(1).

In the 2-1-st step, the same procedure in the 2-1-st step of the above Synthetic Example DI-4 (1) is conducted using the above intermediate (12). Then, in the 2-2-nd step, in a 1,000 ml two-necked flask equipped with a refluxing condenser, 20.0 g of the intermediate (23) obtained in the 2-1-st step and 100 ml of 1,2-dichlorobenzene (Sigma-Aldrich) are added and well mixed at room temperature. Then, the homogeneous solution thus obtained is refluxed for 8 hours at the set temperature of a refluxing apparatus of 190°C. After completing the reaction, the reactant is cooled to room temperature, and solvents are removed by a vacuum oven to produce 4,4'-(propane-2,2-diyl)bis(2,6-diallylphenol) as an intermediate (24). The Reaction Scheme of the 2-2-nd step is the same as that of the 2-2-nd step of the above Synthetic Example DI-4(1).

### Evaluation of physical properties: Manufacturing of cured product and evaluation of heat resistance

### 1. Manufacturing of epoxy cured product

A mixture solution was prepared by dissolving an epoxy compound, a phenol-based curing agent (HF-1M™ Meiwa Plastic Industries, Ltd., equivalent 107), and triphenylphosphine curing catalyst (Aldrich) in methyl ethyl ketone according to the components illustrated in the following Table 1 so that a solid content became 40 wt%, and mixing until a homogeneous solution was obtained (The solid content means the amount of a solid phase material in the mixture). Then, the mixture solution was inserted in a vacuum oven heated to 100 °C to remove solvents and then, cured in a preheated hot press to obtain cured products according to Examples 1 to 14 and Comparative Examples 1 to 5.

### 2. Manufacturing of composite (cured product) including epoxy compound and inorganic particles

An epoxy compound, and a silica slurry (70 wt% of solid content, a 2-methoxyethanol solvent, average silica particle size distribution of 450 nm to 3 µm) were dissolved in methyl ethyl ketone according to the components illustrated in the following Table 2 so that a solid content became 40 wt%. The mixture thus obtained was mixed in a rate of 1,500 rpm for 1 hour, and a phenol-based curing agent (HF-1M™ Meiwa Plastic Industries, Ltd., equivalent 107) was added, followed by further mixing for 50 minutes. Then, a triphenylphosphine (Aldrich) curing catalyst was added and mixed for 10 minutes to obtain an epoxy mixture. The mixture was inserted in a heated vacuum oven heated to 100°C to remove solvents, and was cured in a preheated hot press to manufacture epoxy filler composites (5 mm x 5 mm x 3 mm) according to Examples 15 to 30 and Comparative Examples 6 to 13.

### 3. Evaluation of physical properties

### A. Evaluation of heat resistance

The dimensional changes with respect to the temperature of the cured products according to the examples and comparative examples illustrated in the following Tables 1 and 2 were evaluated by using a Thermo-mechanical analyzer (expansion mode, Force 0.03 N) and are illustrated in the following Tables 1 and 2. The specimens of the epoxy cured products and the silica filler composites were manufactured into a size of 5 mm x 5 mm x 3 mm and evaluation thereof were conducted.

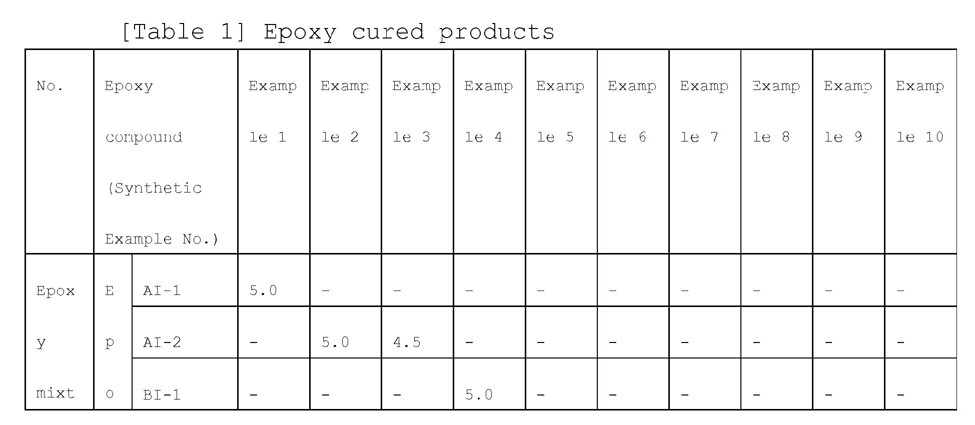

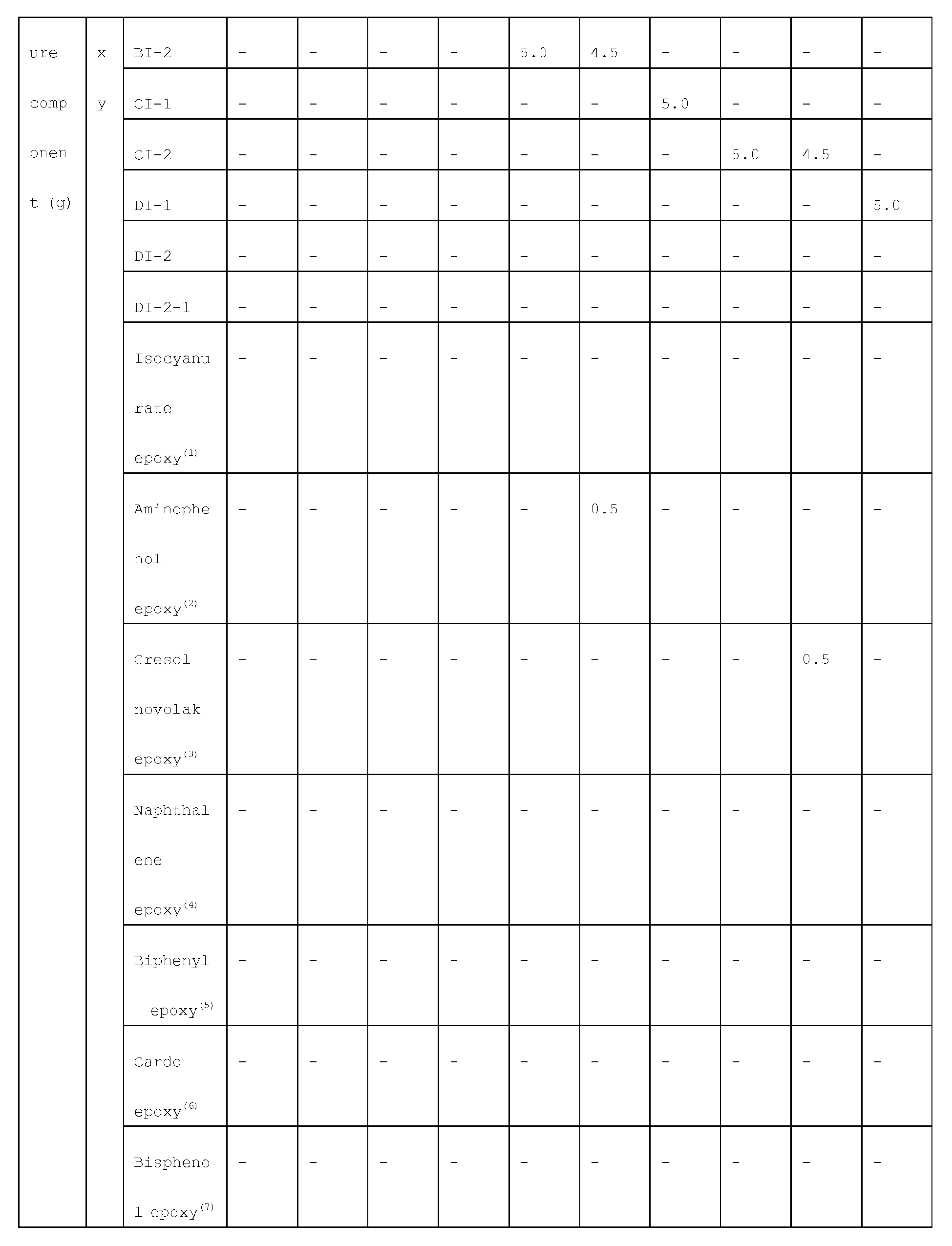

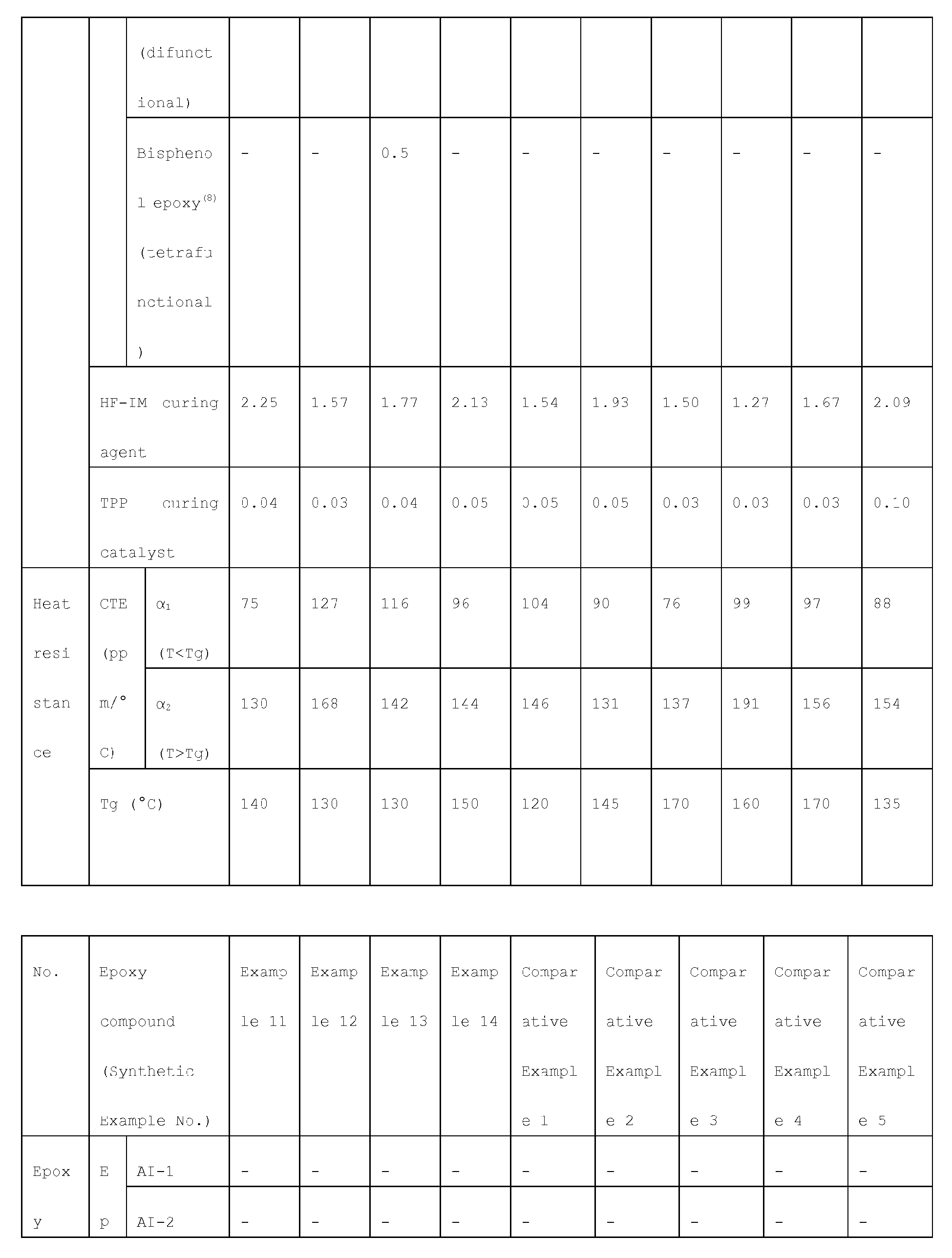

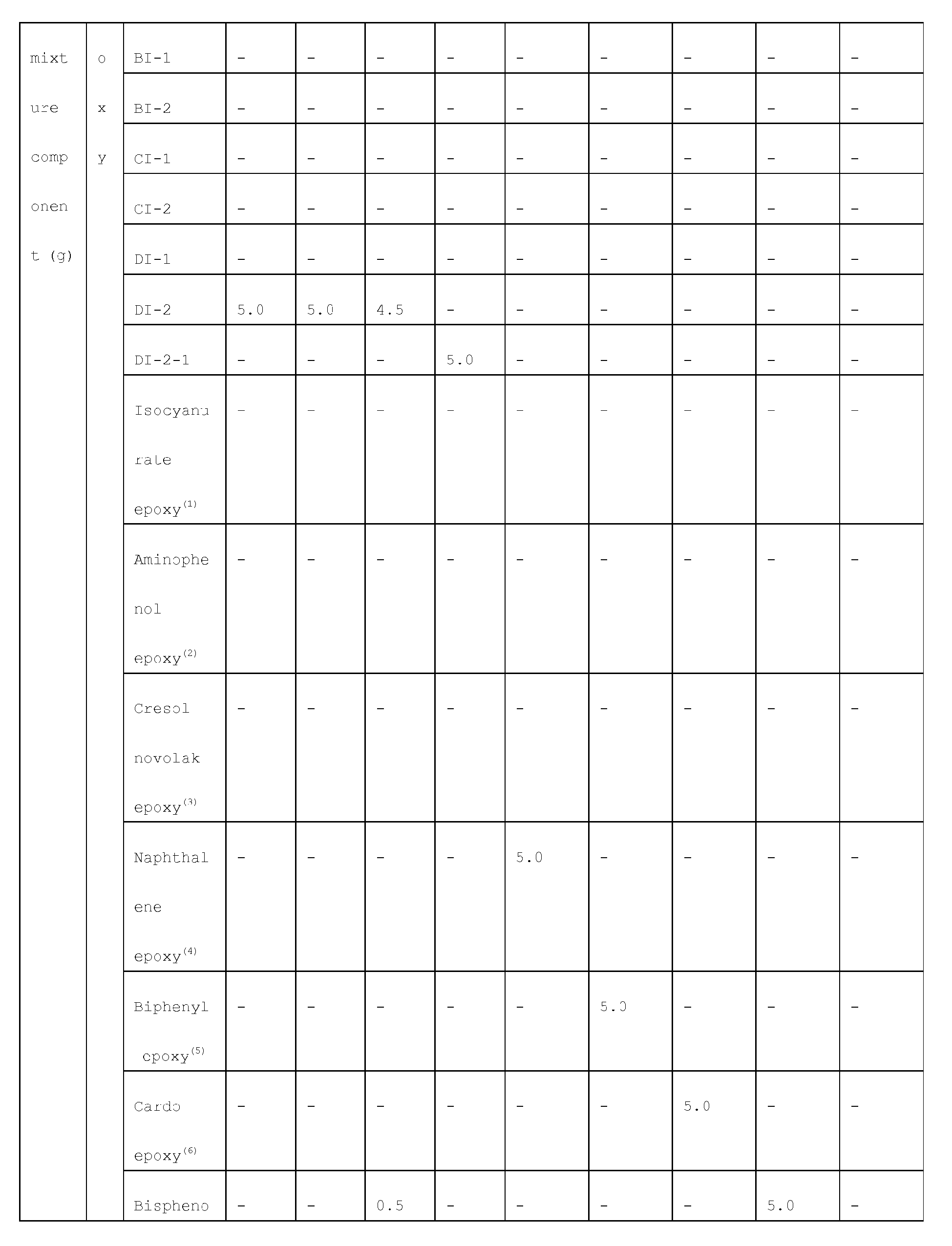

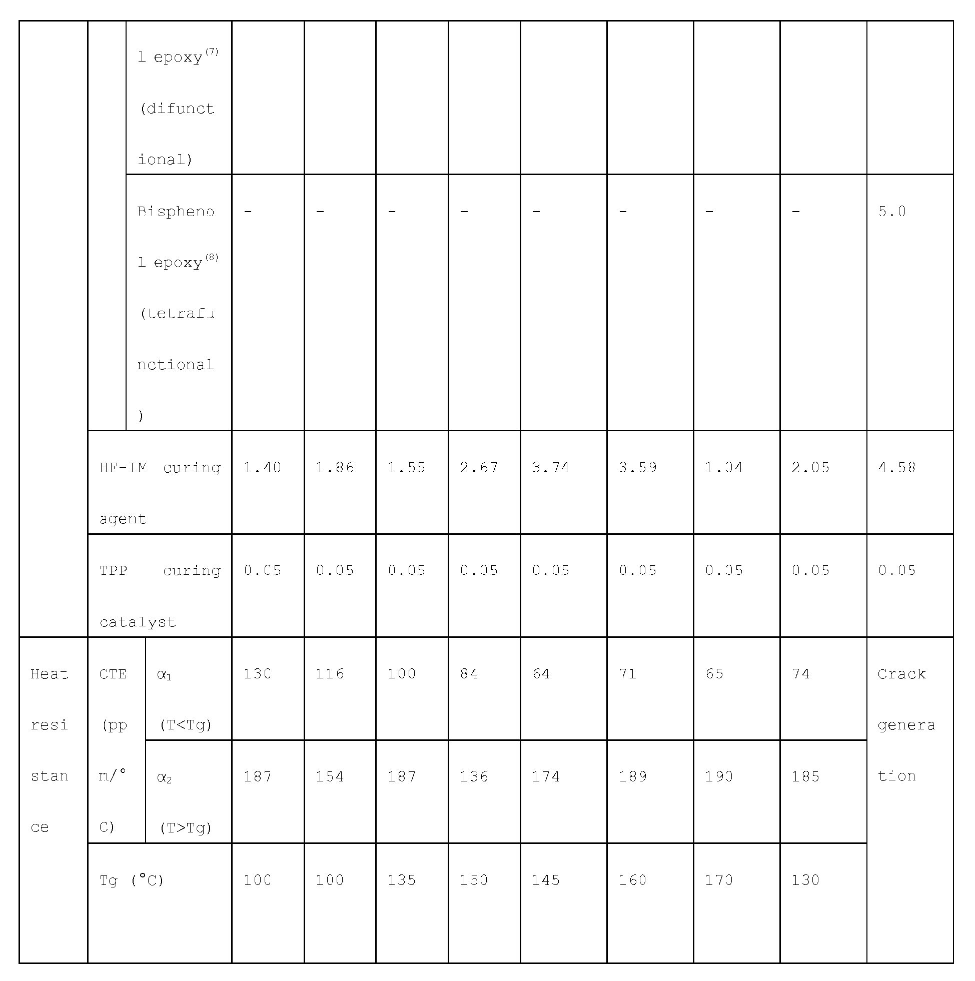

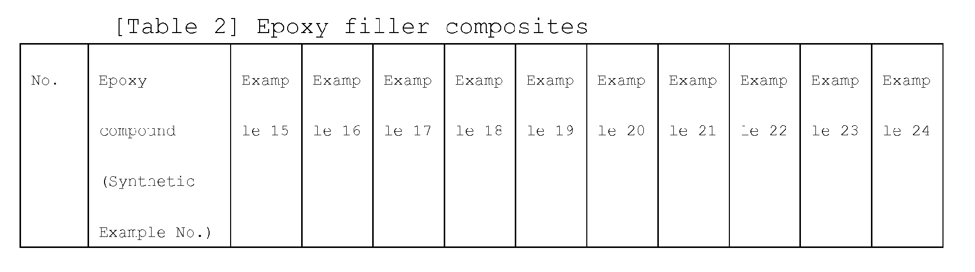

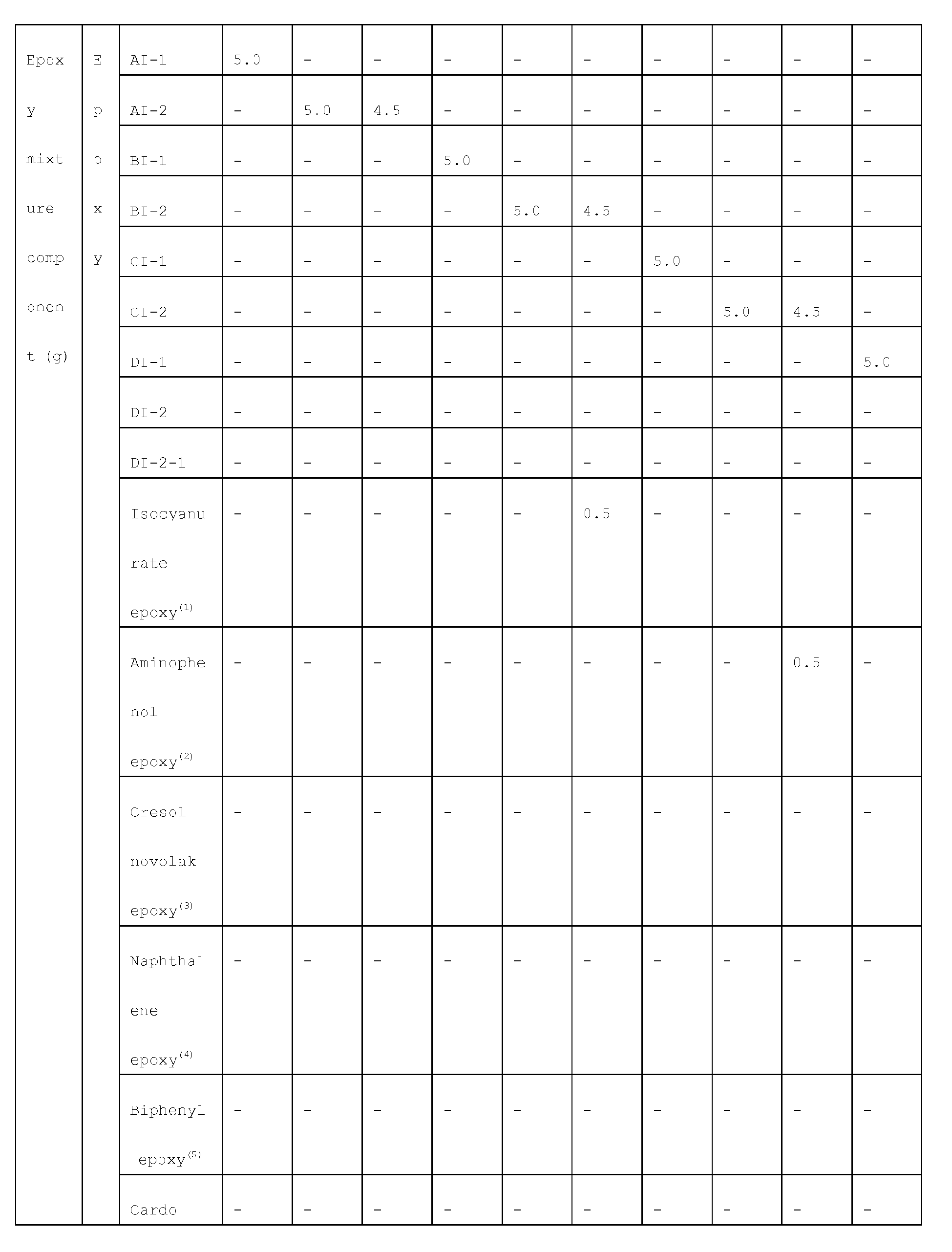

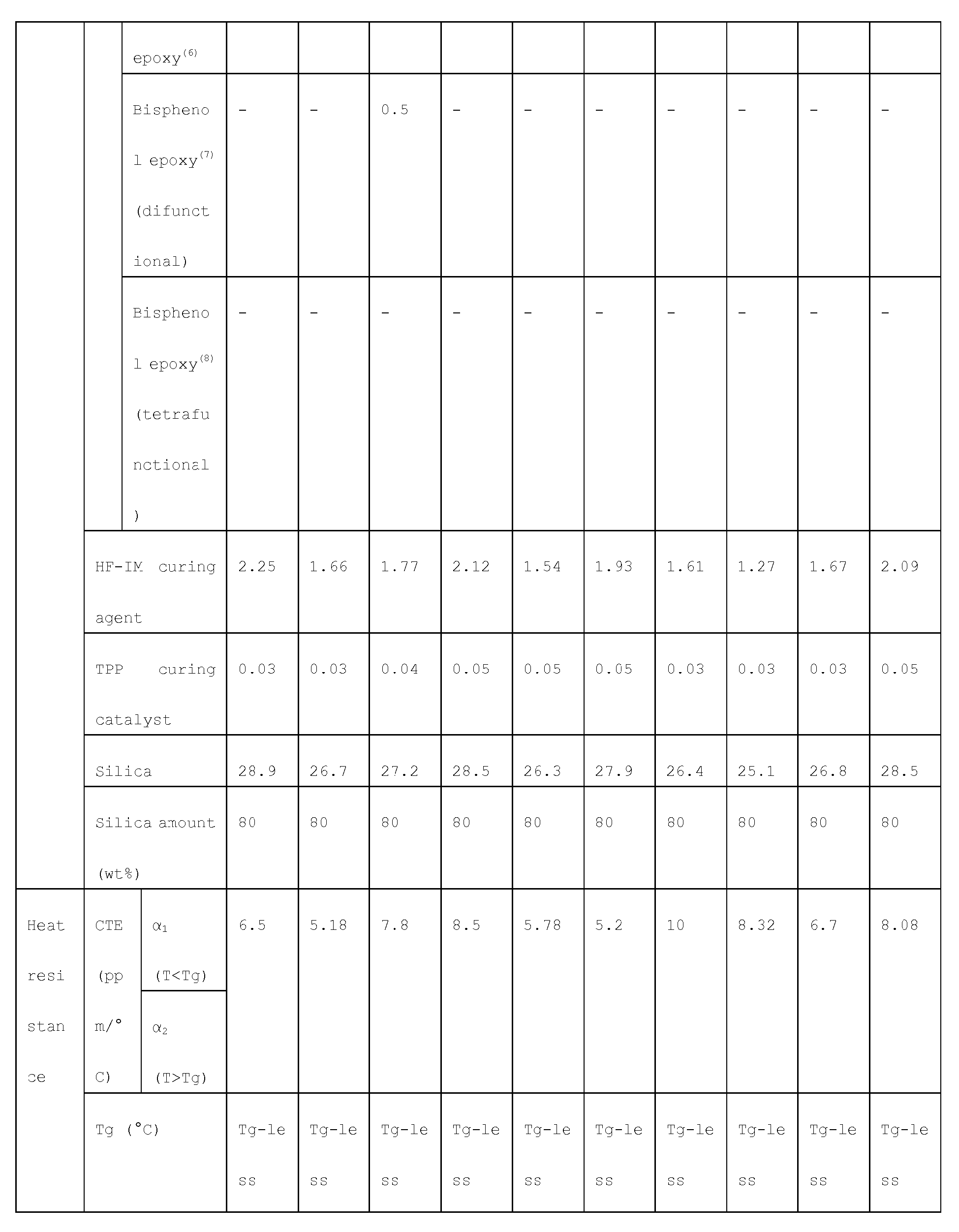

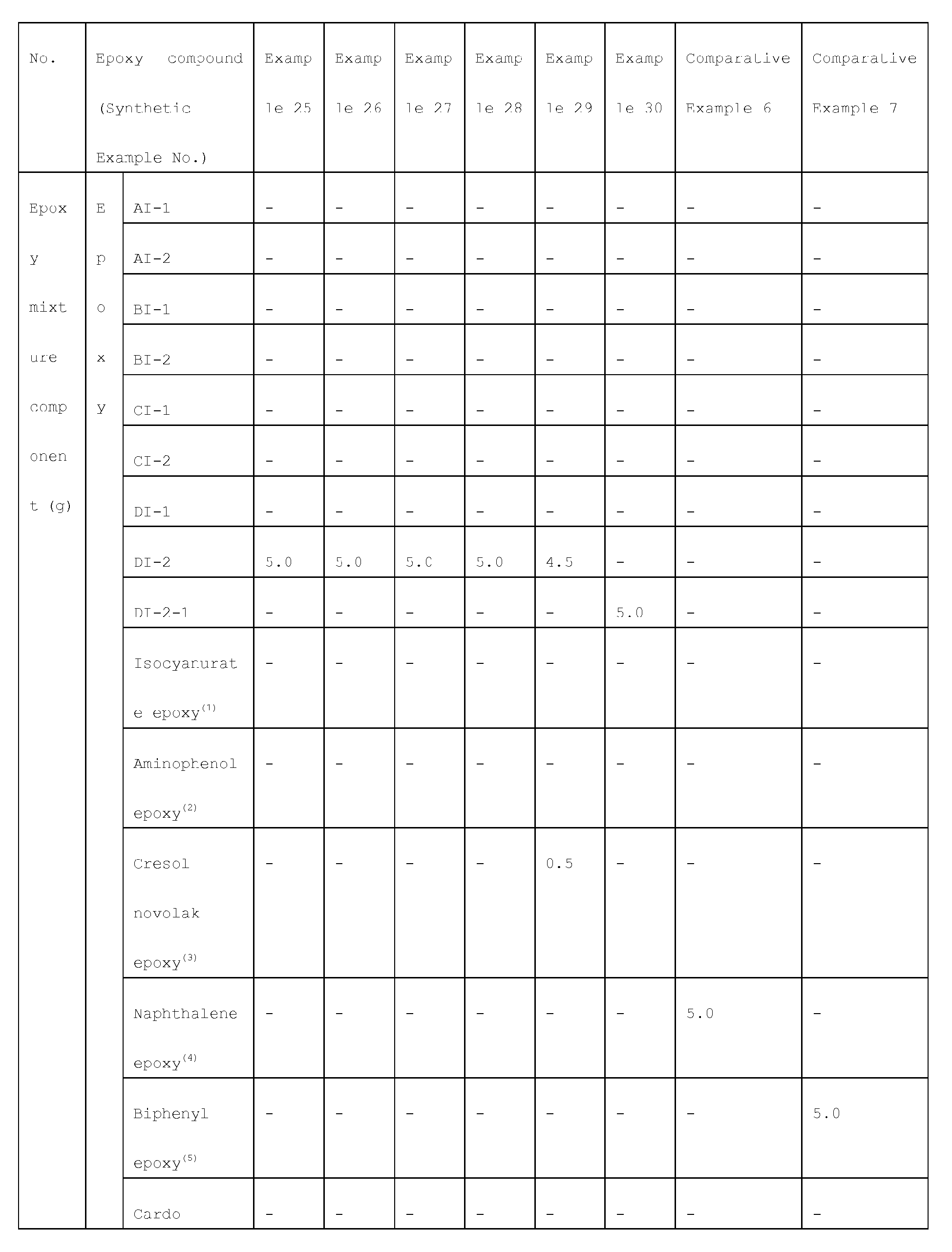

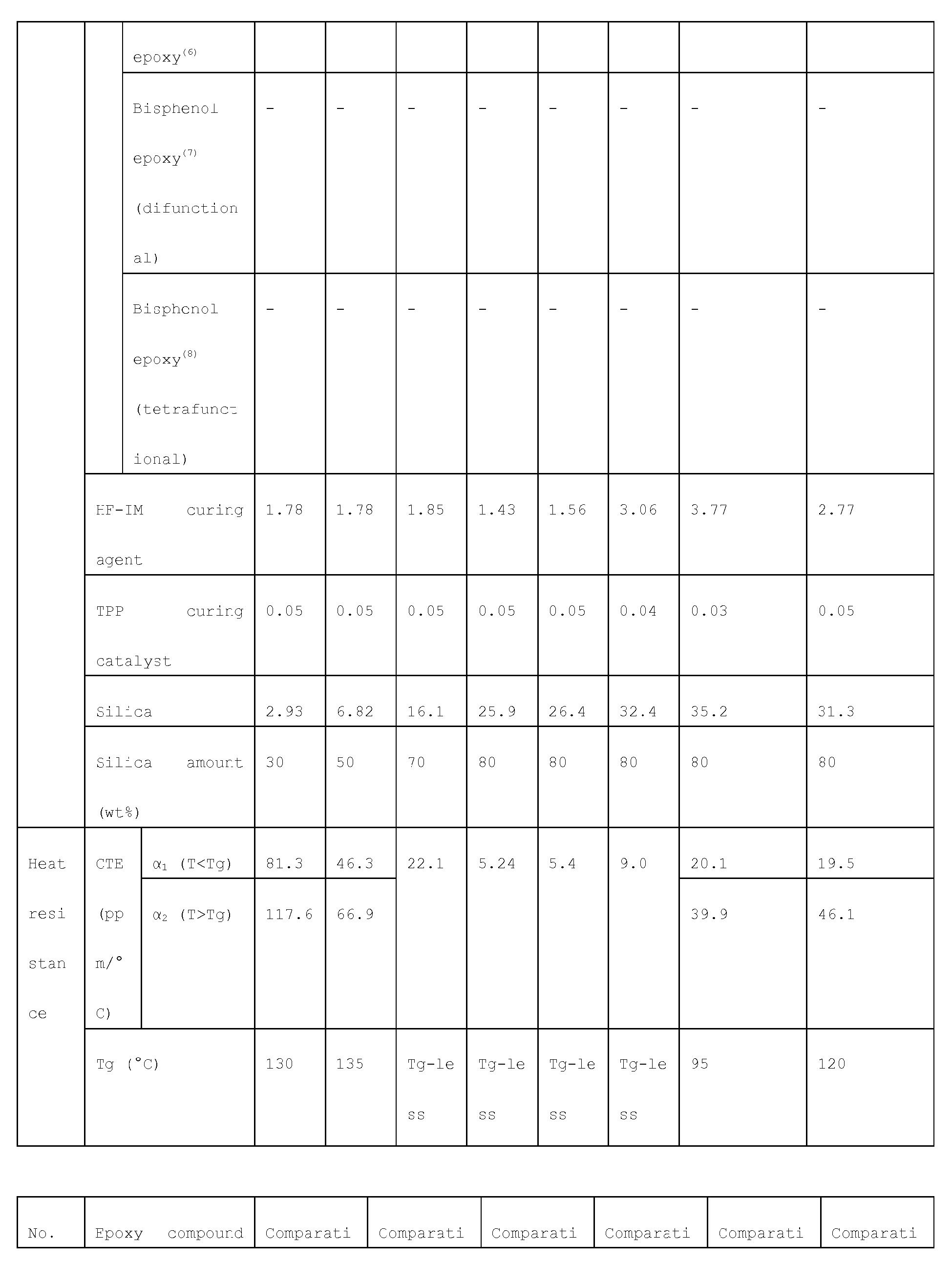

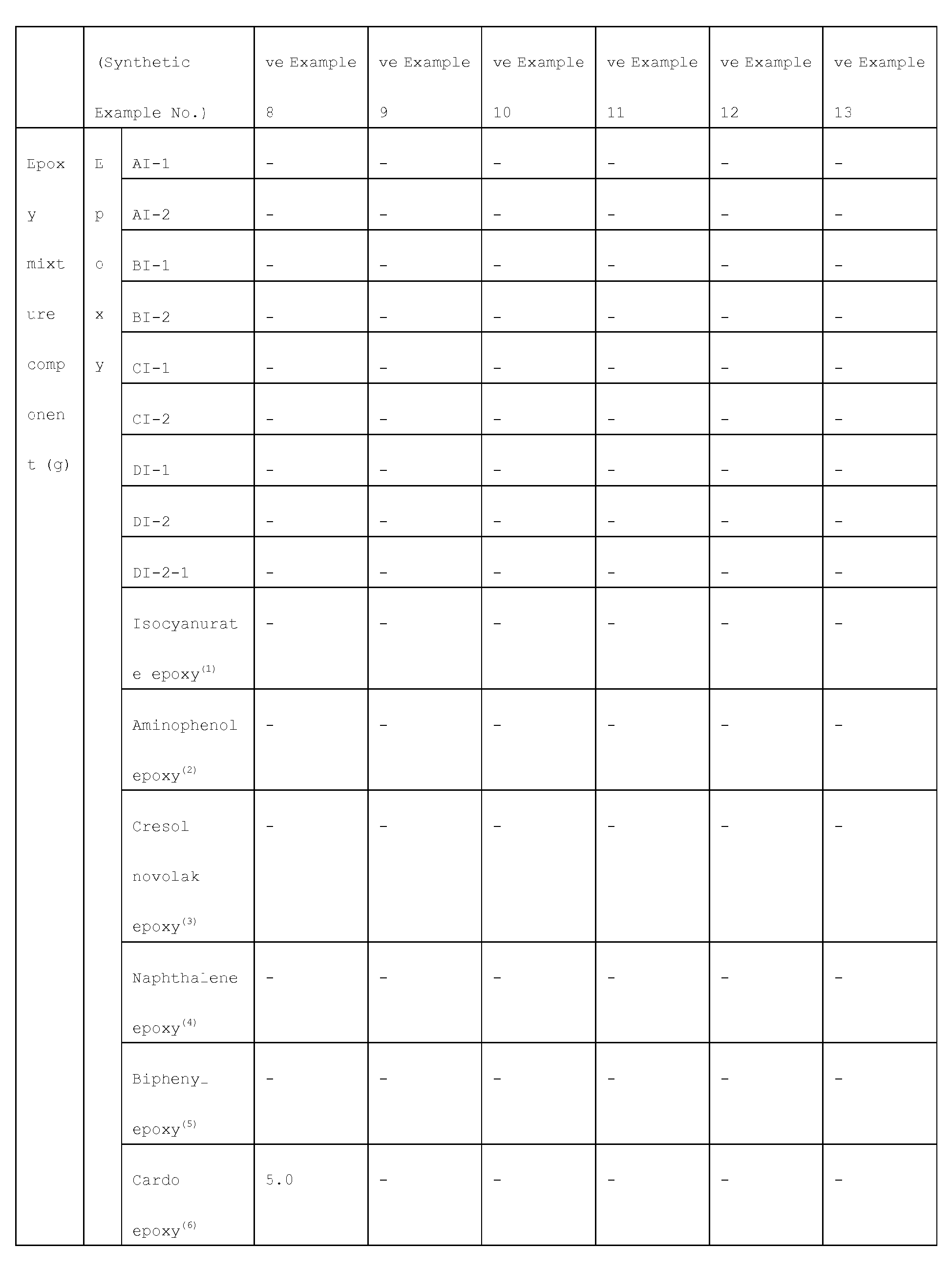

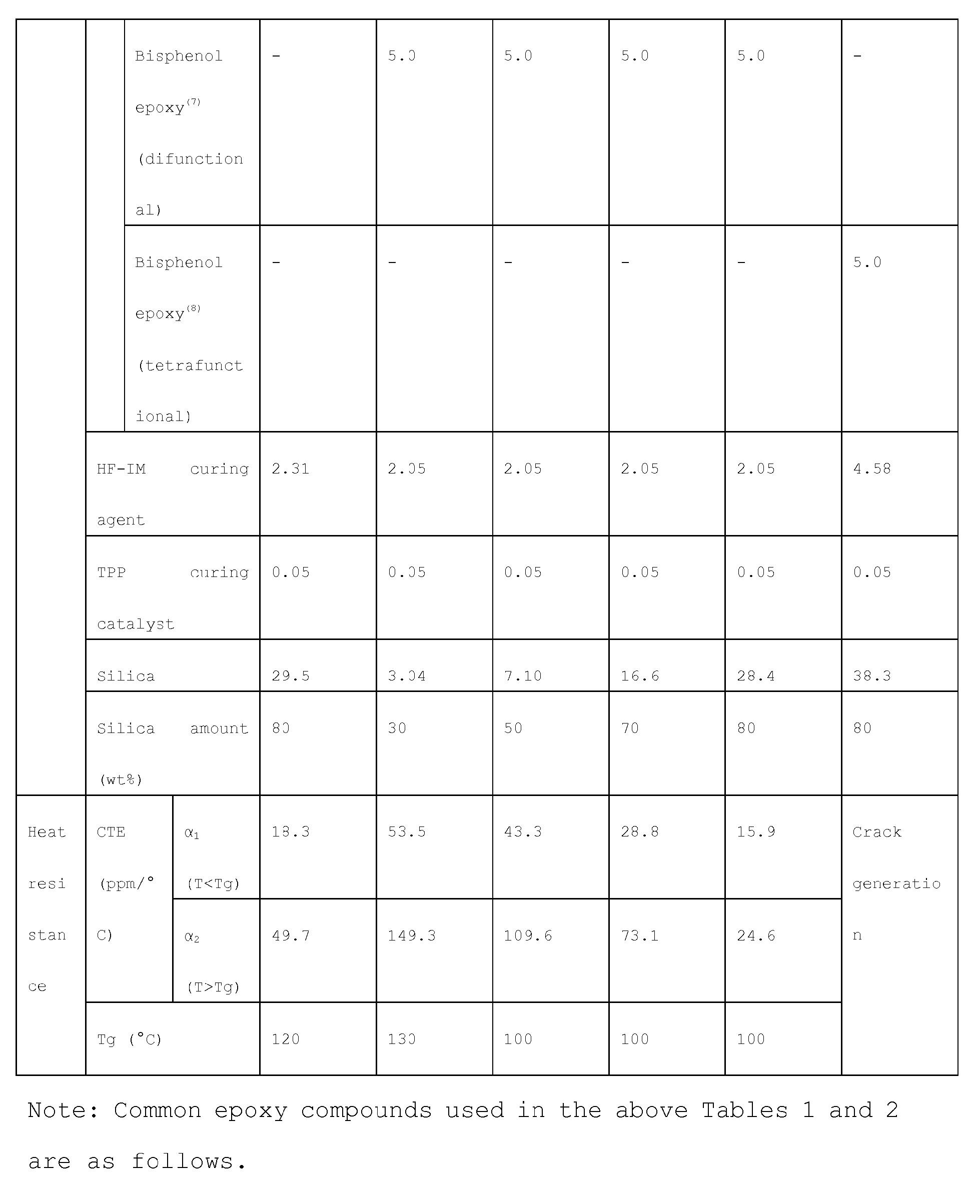

(1) Isocyanurate epoxy 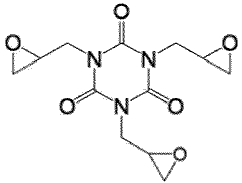 (2) Aminophenol epoxy 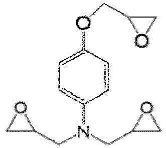 (3) Cresol novolak epoxy (softening point: 54) 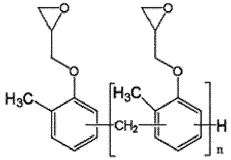 (4) Naphthalene epoxy 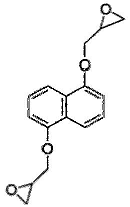 (5) Biphenyl epoxy 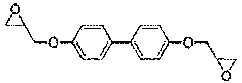 (6) Cardo (fluorene) epoxy 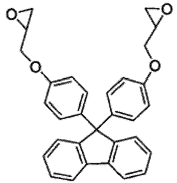 (7) Bisphenol epoxy (difunctional) 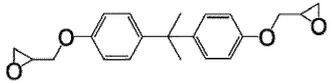 (8) Bisphenol epoxy (tetrafunctional) 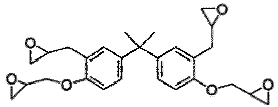 (9) The epoxy compounds prepared by Claisen rearrangement using microwaves or heating may be used in the synthetic examples.

As shown in the above Tables 1 and 2, for the cured product of the alkoxysilylated epoxy compound of the present disclosure, the CTE increased, and Tg decreased when compared to the cured product of an epoxy compound not having an alkoxysilyl group. However, the epoxy composite having an alkoxysilyl group of the present disclosure exhibited improved heat resistant property in view of the CTE and the glass transition property when compared to those of the epoxy composite not having an alkoxysilyl group.

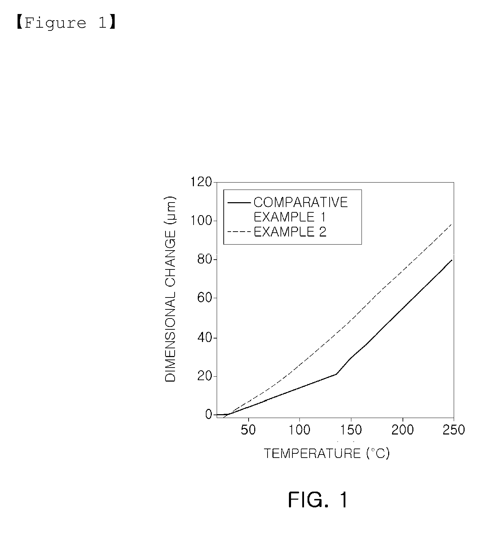

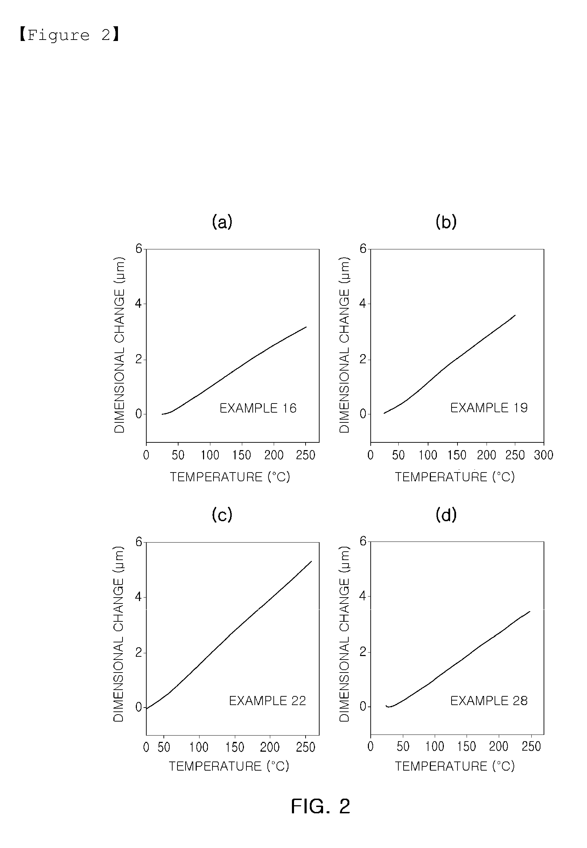

Particularly, as shown in the above Table 1 and FIG. 1, the CTE increased by about 63 ppm/°C, and the glass transition temperature decreased by about 15°C for the cured product of Example 2 when compared to those of the cured product of Comparative Example 1. However, as shown in the above Table 2 and FIGS. 2A to 2D, good heat resistance property of very low CTE of 5 to 8 ppm/°C and Tg-less were observed for the alkoxysilylated epoxy composite in the case when filled with 80 wt% of silica.

More particularly, as shown in Table 2, the CTE of the composites of Examples 15 to 17 (silica filling rate of 80 wt%) was 5 to 7 ppm/°C and was very low when compared to the CTE of the composite of Comparative Example 6 (silica filling rate of 80 wt%) of 20 ppm/°C. The CTE of the composites of Examples 18 to 20 (silica filling rate of 80 wt%) was 5 to 8 ppm/°C and was decreased when compared to the CTE of the composite of Comparative Example 7 (silica filling rate of 80 wt%) of 20 ppm/°C. The CTE of the composites of Examples 21 to 23 (silica filling rate of 80 wt%) was 6 to 10 ppm/°C and was decreased when compared to the CTE of the composite of Comparative Example 8 (silica filling rate of 80 wt%) of 18 ppm/°C. The CTE of the composites of Examples 28 to 30 (silica filling rate of 80 wt%) was 5 to 9 ppm/°C and was decreased when compared to the CTE of the composite of Comparative Example 12 (silica filling rate of 80 wt%) of 16 ppm/°C.

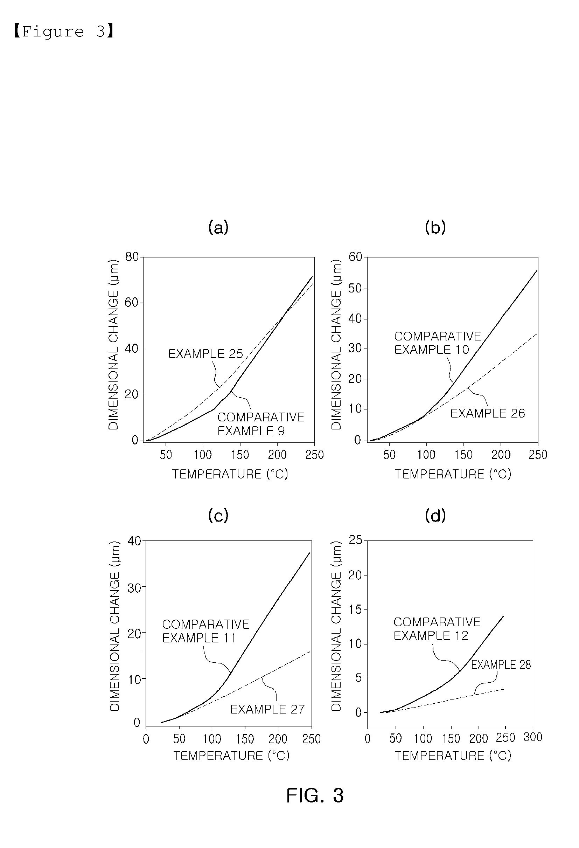

Meanwhile, in the alkoxysilylated epoxy composite according to the present disclosure having the silica filling rate of 30 to 80 wt%, the CTE of α2 (the CTE at greater than or equal to Tg) was markedly lower than the CTE of α2 of an epoxy composite having the same core structure and not having an alkoxysilyl group. Thus, as shown in FIGS. 3A to 3D, the CTE at the temperature range of room temperature to 250°C was decreased when compared to an epoxy composite having the same core structure and not having an alkoxysilyl group.

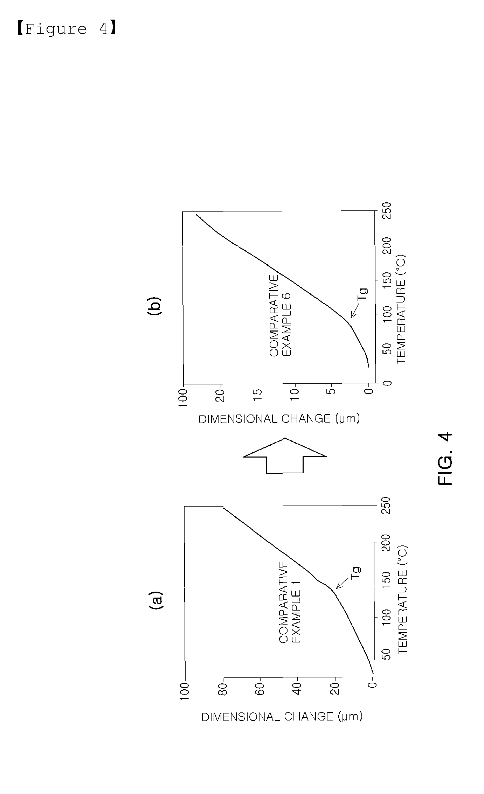

In addition, the glass transition temperature of a common epoxy compound not having an alkoxysilyl group was decreased by the formation of a composite with inorganic particles. For example, in the naphthalene epoxy compound, Tg of the composite of Comparative Example 6 was decreased to 95°C when compared to Tg of 145°C of the cured product of Comparative Example 1 as shown in FIGS. 4A and 4B. As shown in the above Tables 1 and 2, similar results were obtained for the biphenyl-based and the cardo-based epoxy compounds.

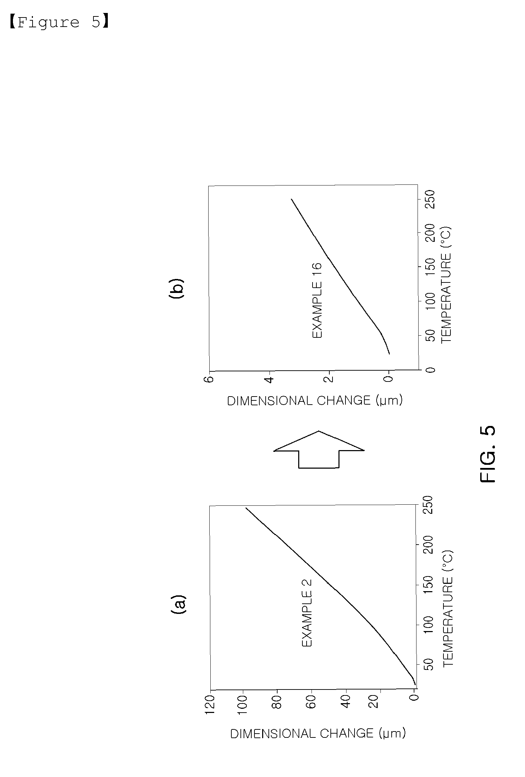

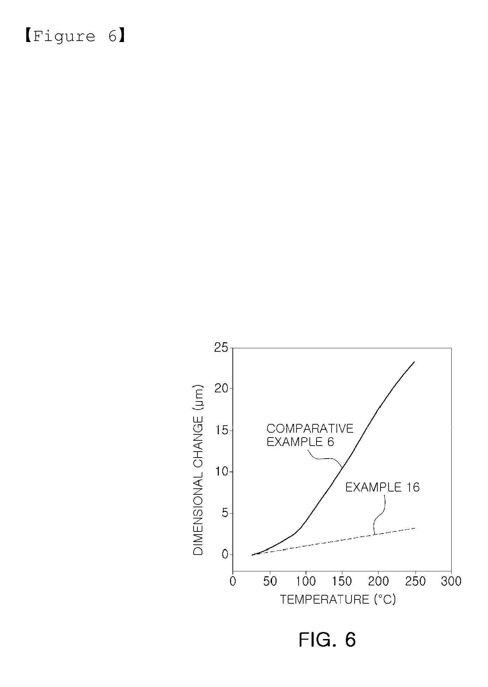

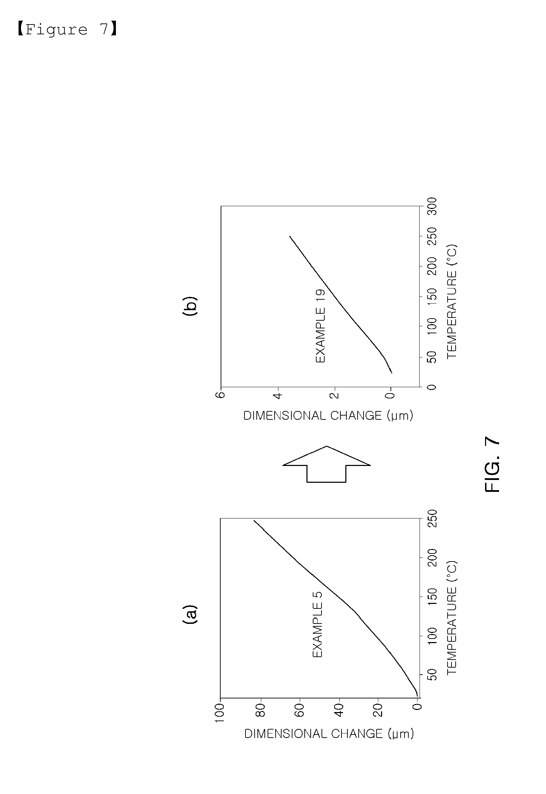

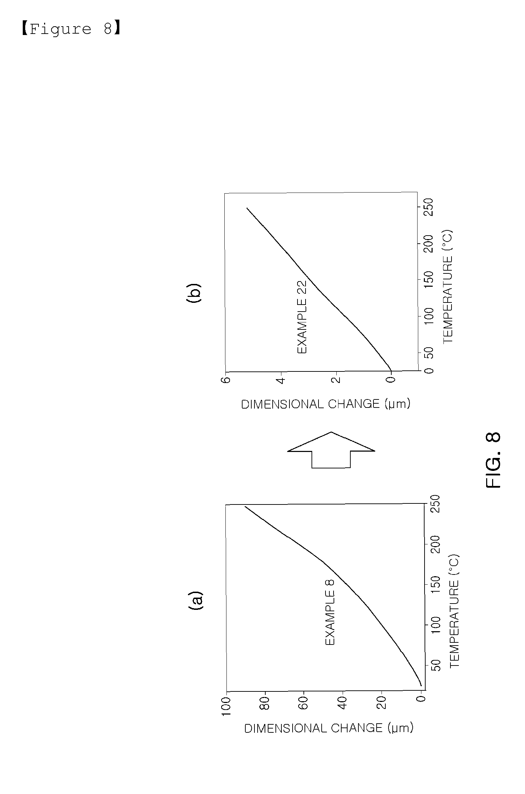

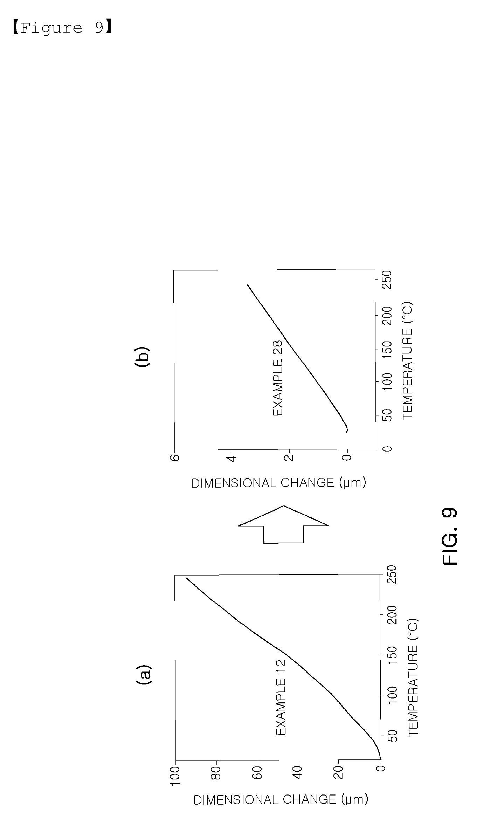

However, Tg was increased for the composite of the alkoxysilylated epoxy compound according to the present disclosure when compared to the cured product of the epoxy compound, and excellent Tg property may be attained for the composite of the alkoxysilylated epoxy compound according to the present disclosure when compared to the composite of the epoxy compound not having an alkoxysilyl group. As shown in Tables 1 and 2 and FIGS. 5A, 5B and 6, the composite of the epoxy compound containing a naphthalene core structure according to Example 16 exhibited Tg-less property of not exhibiting glass transition property in the temperature range of room temperature to 250°C and good heat resistance property (glass transition temperature) when compared to the cured product of Example 2 (Tg = 130°C) and the composite of Comparative Example 6 (Tg = 95°C). As shown in Tables 1 and 2 and FIGS. 7A and 7B, the composite of the epoxy compound containing a biphenyl core structure according to Example 19 exhibited Tg-less property of not exhibiting glass transition property in the temperature range of room temperature to 250°C and good heat resistant property (glass transition temperature) when compared to the cured product of Example 5 (Tg = 120°C) and the composite of Comparative Example 7 (Tg = 120°C). As shown in Tables 1 and 2 and FIGS. 8A and 8B, the composite of the epoxy compound containing a cardo (fluorene) core structure according to Example 22 exhibited Tg-less property of not exhibiting glass transition property in the temperature range of room temperature to 250°C and good heat resistant property (glass transition temperature) when compared to the cured product of Example 8 (Tg = 160°C) and the composite of Comparative Example 8 (Tg = 120°C). As shown in Tables 1 and 2 and FIGS. 9A and 9B, the composite of the epoxy compound containing a bisphenol A core structure according to Example 28 exhibited Tg-less property of not exhibiting glass transition property in the temperature range of room temperature to 250°C and good heat resistant property (glass transition temperature) when compared to the cured product of Example 12 (Tg = 100°C) and the composite of Comparative Example 12 (Tg = 100°C).

As described above, the alkoxysilylated epoxy composite of the present disclosure exhibits decreased CTE and good heat resistance property of high Tg (or Tg-less) when compared to an epoxy composite not having an alkoxysilyl group. In addition, the decrease of the CTE and the increase of Tg or Tg-less property in the alkoxysilylated epoxy compound are due to the improvement of bonding property of an epoxy compound and inorganic particles in a composite. From the above-described properties, it would be confirmed that the bonding property of the epoxy compound and the inorganic particles in the composite are improved.
While exemplary embodiments have been shown and described above, it will be apparent to those skilled in the art that modifications and variations could be made without departing from the scope of the present disclosure as defined by the appended claims.

## Claims

1. An epoxy composition comprising at least one epoxy compound containing an alkoxysilyl group selected from the group consisting of the following Formulae AI to KI and inorganic particles: 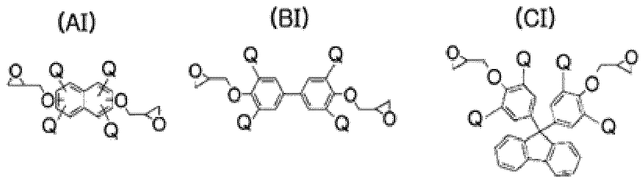 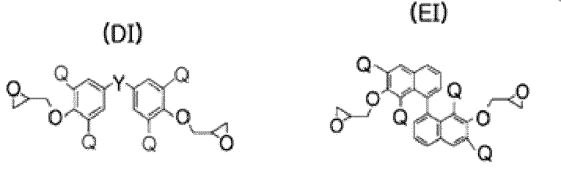 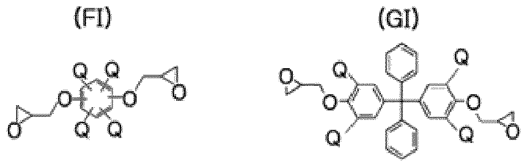 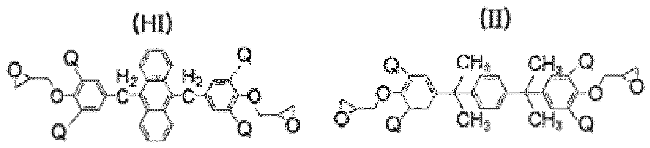 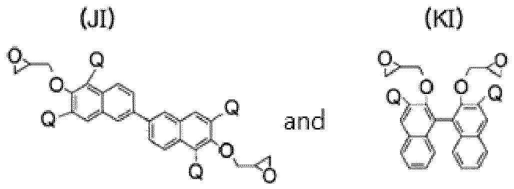 in the above Formulae AI to KI, at least one of a plurality of Q has the form of the following Formula S1, and the remainder thereof are independently selected from the group consisting of the following Formula S3, hydrogen, and -CR_{b}R_{c}-CRₐ=CH₂, where Rₐ, R_{b} and R_{c} are independently H, or an alkyl group having 1 to 6 carbon atoms, and the alkyl group may be a linear chain or a branched chain alkyl group,
in the above DI, Y is -CH₂, -C(CH₃)₂-, -C(CF₃)₂-, -S-, or -SO₂-,
[Formula S1] -CR_{b}R_{c}-CHRₐ-CH₂-SiR₁R₂R₃
in Formula S1, Rₐ, R_{b} and R_{c} are independently H, or an alkyl group having 1 to 6 carbon atoms, at least one of R₁ to R₃ is an alkoxy group having 1 to 6 carbon atoms, and the remainder thereof are alkyl groups having 1 to 10 carbon atoms, while the alkyl group and the alkoxy group may be a linear chain or a branched chain alkyl group or alkoxy group,
in the case in which Formula FI includes one instance of Formula S1, a compound in which all of Rₐ, R_{b} and R_{c} in the above Formula S1 are hydrogen, and R₁ to R₃ are alkoxy groups having 1 to 6 carbon atoms is excluded, 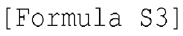 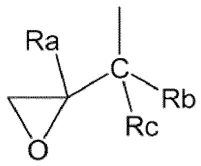 in Formula S3, Rₐ, R_{b} and R_{c} are independently H, or an alkyl group having 1 to 6 carbon atoms, and the alkyl group may be a linear chain or a branched chain alkyl group.

2. An epoxy composition comprising at least one epoxy compound containing an alkoxysilyl group selected from the group consisting of the following Formulae AI to KI, inorganic particles, and a curing agent: 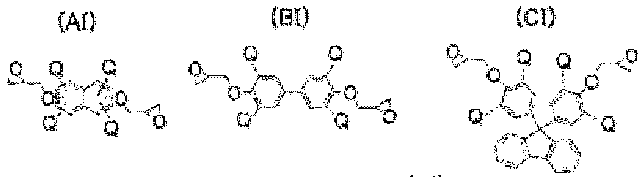 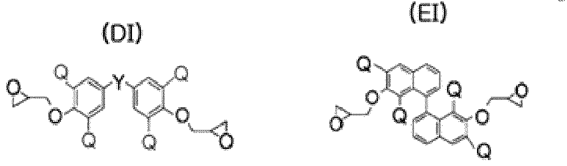 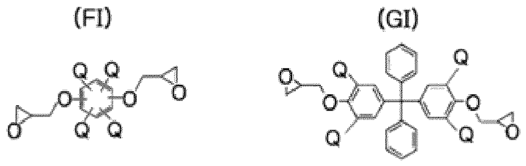 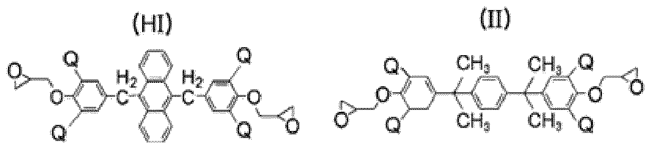 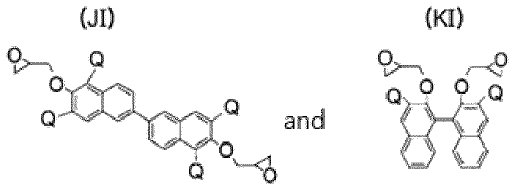 in the above Formulae AI to KI, at least one of a plurality of Q has the form of the following Formula S1, and the remainder thereof are independently selected from the group consisting of the following Formula S3, hydrogen, and -CR_{b}R_{c}-CRₐ=CH₂, where Rₐ, R_{b} and R_{c} are independently H, or an alkyl group having 1 to 6 carbon atoms, and the alkyl group may be a linear chain or a branched chain alkyl group,
in the above DI, Y is -CH₂, -C(CH₃)₂-, -C(CF₃)₂-, -S-, or -SO₂-,
[Formula S1] -CR_{b}R_{c}-CHRₐ-CH₂-SiR₁R₂R₃
in Formula S1, Rₐ, R_{b} and R_{c} are independently H, or an alkyl group having 1 to 6 carbon atoms, at least one of R₁ to R₃ is an alkoxy group having 1 to 6 carbon atoms, and the remainder thereof are alkyl groups having 1 to 10 carbon atoms, while the alkyl group and the alkoxy group may be a linear chain or a branched chain alkyl group or alkoxy group,
in the case in which Formula FI includes one instance of Formula S1, a compound in which all of Rₐ, R_{b} and R_{c} in the above Formula S1 are hydrogen, and R₁ to R₃ are alkoxy groups having 1 to 6 carbon atoms is excluded, 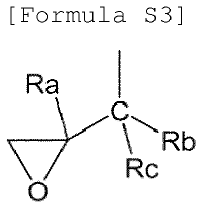 in Formula S3, Rₐ, R_{b} and R_{c} are independently H, or an alkyl group having 1 to 6 carbon atoms, and the alkyl group is a linear chain or a branched chain alkyl group.

3. The epoxy composition of claim 1 or 2, wherein R₁ to R₃ are an ethoxy group.

4. The epoxy composition of claim 1 or 2, wherein the epoxy compound containing an alkoxysilyl group is selected from the group consisting of the above Formulae AI to DI.

5. The epoxy composition of claim 4, wherein the epoxy compound containing an alkoxysilyl group is the above Formula DI.

6. The epoxy composition of claim 5, wherein Y in the above Formula DI is -C(CH₃)₂-.

7. The epoxy composition of claim 4, wherein the epoxy compound containing an alkoxysilyl group is one of compounds in the following Formula M:

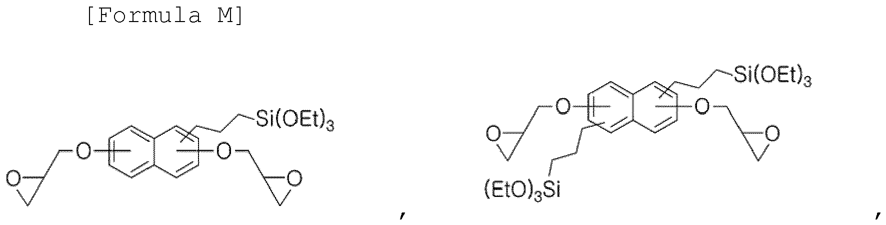

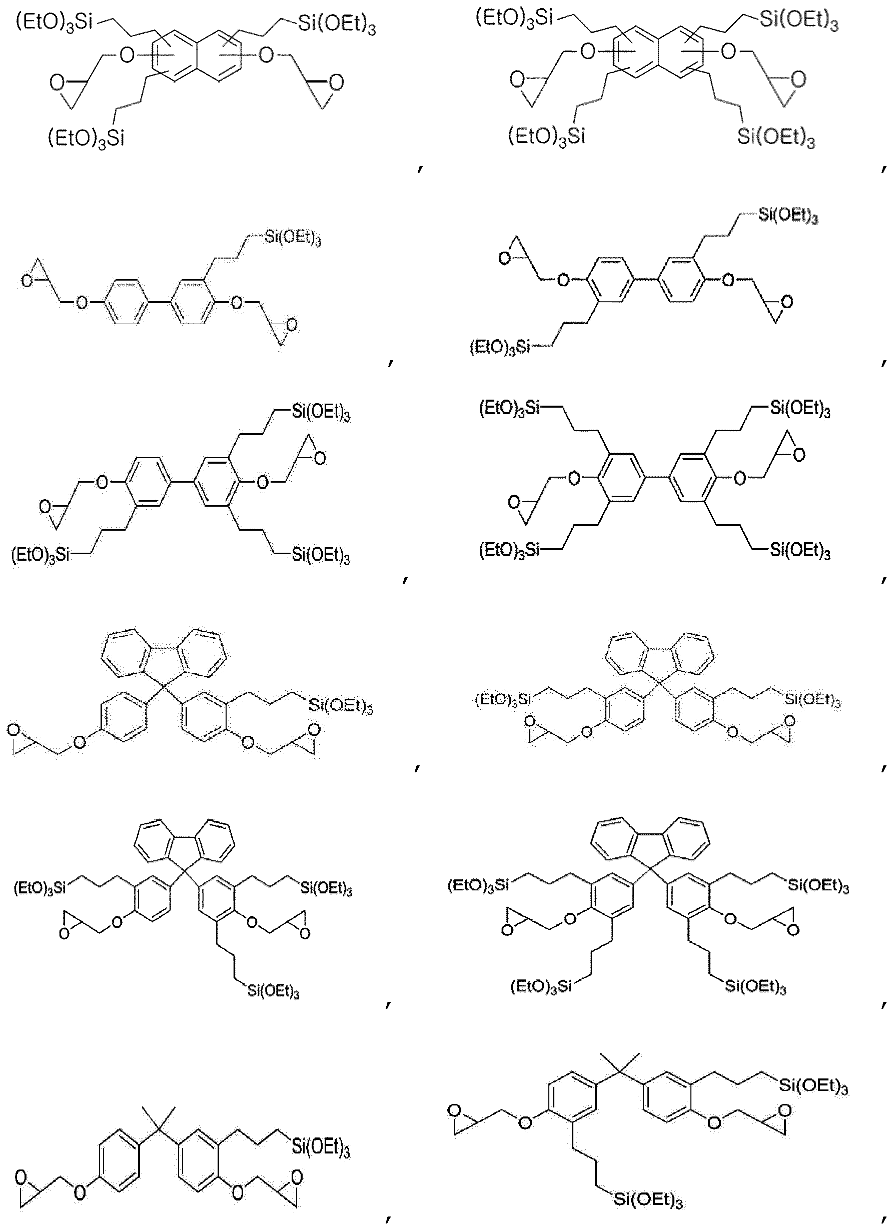

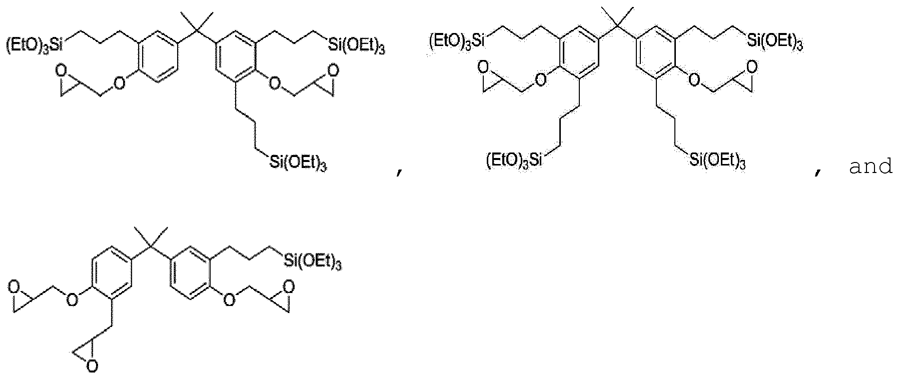

8. The epoxy composition of claim 1 or 2, wherein the epoxy compound containing an alkoxysilyl group is an epoxy polymer selected from the group consisting of the following Formulae AP to KP: 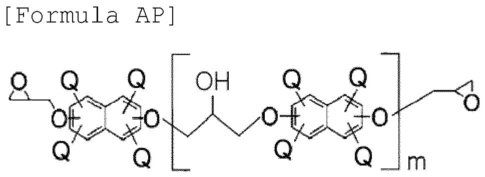 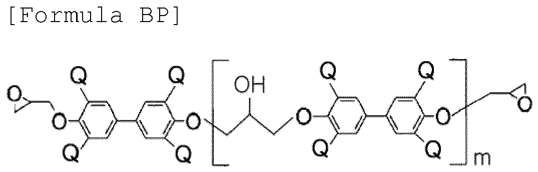 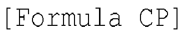 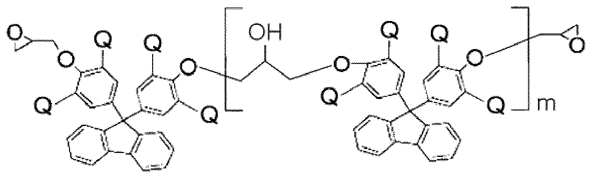 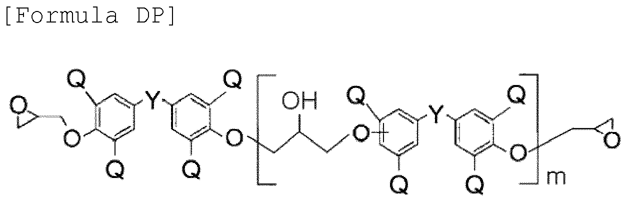 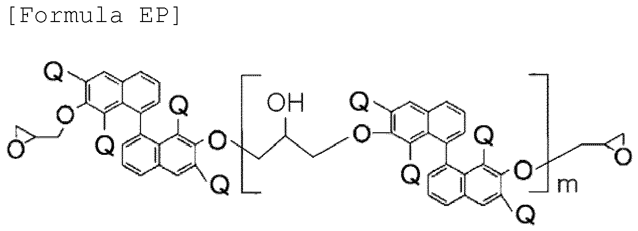 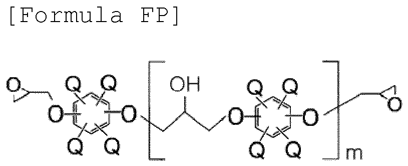 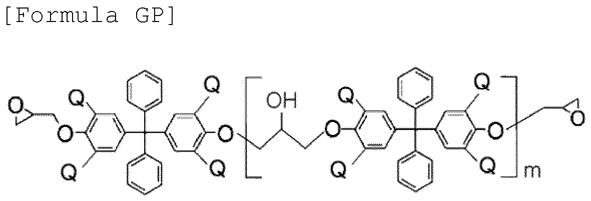 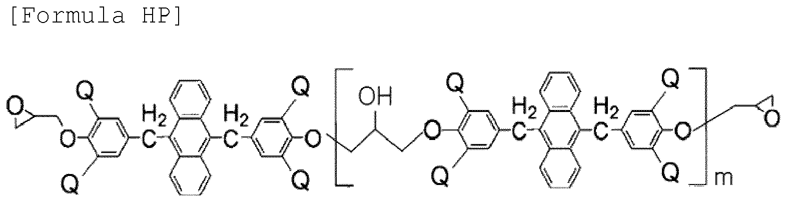 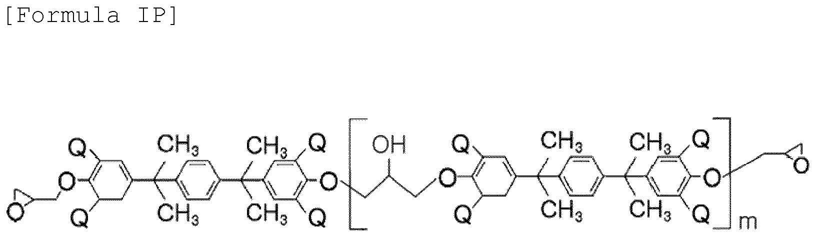 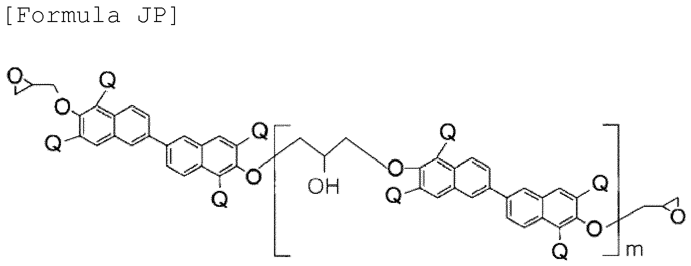 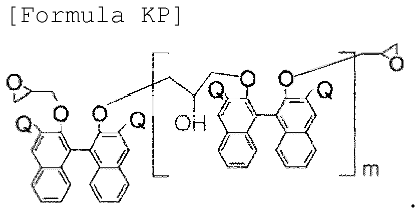 in the above Formulae AP to KP, at least one of a plurality of Q has the form of the following Formula S1, and the remainder thereof are independently selected from the group consisting of the following Formula S3, hydrogen, and -CR_{b}R_{c}-CRₐ=CH₂, where Rₐ, R_{b} and R_{c} are independently H, or an alkyl group having 1 to 6 carbon atoms, and the alkyl group may be a linear chain or a branched chain alkyl group,
m is an integer from 1 to 100,
in the above DP, Y is -CH₂, -C(CH₃)₂-, -C(CF₃)₂-, -S-, or -SO₂-,
[Formula S1] -CR_{b}R_{c}-CHRₐ-CH₂-SiR₁R₂R₃
in Formula S1, Rₐ, R_{b} and R_{c} are independently H, or an alkyl group having 1 to 6 carbon atoms, at least one of R₁ to R₃ is an alkoxy group having 1 to 6 carbon atoms, and the remainder thereof are alkyl groups having 1 to 10 carbon atoms, while the alkyl group and the alkoxy group may be a linear chain or a branched chain alkyl group or alkoxy group, 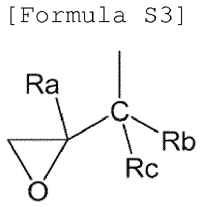 in Formula S3, Rₐ, R_{b} and R_{c} are independently H, or an alkyl group having 1 to 6 carbon atoms, and the alkyl group may be a linear chain or a branched chain alkyl group.

9. The epoxy composition of claim 1 or 2, further comprising at least one epoxy compound selected from the group consisting of a glycidyl ether-based epoxy compound, a glycidyl-based epoxy compound, a glycidyl amine-based epoxy compound, a glycidyl ester-based epoxy compound, a rubber modified epoxy compound, an aliphatic polyglycidyl-based epoxy compound and an aliphatic glycidyl amine-based epoxy compound.

10. The epoxy composition of claim 9, wherein the epoxy compound comprises bisphenol A, bisphenol F, bisphenol S, biphenyl, naphthalene, benzene, thiodiphenol, fluorene, anthracene, isocyanurate, triphenylmethane, 1,1,2,2-tetraphenylethane, tetraphenylmethane, 4,4'-diaminodiphenylmethane, aminophenol, a cyclo aliphatic compound, or a novolak unit, as a core structure.

11. The epoxy composition of claim 10, wherein the epoxy compound comprises the bisphenol A, the biphenyl, the naphthalene, or the fluorene as the core structure.

12. The epoxy composition of claim 9, wherein the epoxy composition comprises 10 wt% to 100 wt% of the epoxy compound containing an alkoxysilyl group and 0 wt% to 90 wt% of at least one epoxy compound selected from the group consisting of the glycidyl ether-based epoxy compound, the glycidyl-based epoxy compound, the glycidyl amine-based epoxy compound, the glycidyl ester-based epoxy compound, the rubber modified epoxy compound, the aliphatic polyglycidyl-based epoxy compound and the aliphatic glycidyl amine-based epoxy compound based on the total amount of the epoxy compound.

13. The epoxy composition of claim 12, wherein the epoxy composition comprises 30 wt% to 100 wt% of the epoxy compound containing an alkoxysilyl group and 0 wt% to 70 wt% of at least one epoxy compound selected from the group consisting of the glycidyl ether-based epoxy compound, the glycidyl-based epoxy compound, the glycidyl amine-based epoxy compound, the glycidyl ester-based epoxy compound, the rubber modified epoxy compound, the aliphatic polyglycidyl-based epoxy compound and the aliphatic glycidyl amine-based epoxy compound based on the total amount of the epoxy compound.

14. The epoxy composition of claim 1 or 2, wherein the inorganic particle is at least one selected from the group consisting of a metal oxide selected from the group consisting of silica, zirconia, titania, alumina, silicon nitride and aluminum nitride, T-10 type silsesquioxane, ladder type silsesquioxane and cage type silsesquioxane.

15. The epoxy composition of claim 1 or 2, wherein an content of the inorganic particles is 5 wt% to 95 wt% based on a total solid content of the epoxy composition.

16. The epoxy composition of claim 15, wherein an content of the inorganic particles is 30 wt% to 95 wt% based on a total solid content of the epoxy composition.

17. The epoxy composition of claim 15, wherein, an content of the inorganic particles is 5 wt% to 60 wt% based on a total solid content of the epoxy composition.

18. The epoxy composition of claim 1 or 2, further comprising a curing accelerator.

19. An electronic material comprising the epoxy composition according to claim 1 or 2.

20. An electronic material comprising the epoxy composition according to claim 9.

21. A substrate comprising the epoxy composition according to claim 1 or 2.

22. A substrate comprising the epoxy composition according to claim 9.

23. A film comprising the epoxy composition according to claim 1 or 2.

24. A film comprising the epoxy composition according to claim 9.

25. A laminate comprising a metal layer placed on a base layer formed by using the epoxy composition according to claim 1 or 2.

26. A printed circuit board comprising the laminate according to claim 25.

27. A semiconductor device comprising the printed circuit board according to claim 26.

28. A laminate comprising a metal layer placed on a base layer formed by using the epoxy composition according to claim 9.

29. A printed circuit board comprising the laminate according to claim 28.

30. A semiconductor device comprising the printed circuit board according to claim 29.

31. A semiconductor packaging material comprising the epoxy composition according to claim 1 or 2.

32. A semiconductor device comprising the semiconductor packaging material according to claim 31.

33. A semiconductor packaging material comprising the epoxy composition according to claim 9.

34. A semiconductor device comprising the semiconductor packaging material according to claim 33.

35. An adhesive comprising the epoxy composition according to claim 1 or 2.

36. An adhesive comprising the epoxy composition according to claim 9.

37. A paint composition comprising the epoxy composition according to claim 1 or 2.

38. A paint composition comprising the epoxy composition according to claim 9.

39. A composite material comprising the epoxy composition according to claim 1 or 2.

40. A composite material comprising the epoxy composition according to claim 9.

41. A cured product of the epoxy composition according to claim 1 or 2.

42. A cured product of the epoxy composition according to claim 9.

43. The cured product of claim 41, wherein the cured product has a coefficient of thermal expansion of less than or equal to 60 ppm/°C.

44. The cured product of claim 42, wherein the cured product has a coefficient of thermal expansion of less than or equal to 60 ppm/°C.

45. The cured product of claim 41, wherein the cured product has a glass transition temperature of 100°C or above, or does not exhibit the glass transition temperature.

46. The cured product of claim 42, wherein the cured product has a glass transition temperature of 100°C or above, or does not exhibit the glass transition temperature.

47. A method of preparing an epoxy compound containing an alkoxysilyl group of Formulae (A14) to (K14) comprising:
a first step of preparing an intermediate (11) of the following Formulae (A11) to (K11) by reacting one starting material of the following Formulae (AS) to (KS) and an allyl compound of the following Formula B1 in the presence of a base and an optional solvent;
a second step of preparing an intermediate (12) of the following Formulae (A12) to (K12) by irradiating electromagnetic waves onto one of the above intermediate (11) in the presence of an optional solvent;
a third step of preparing an intermediate (13) of the following Formulae (A13) to (K13) by reacting one of the above intermediate (12) with epichlorohydrin in the presence of a base and an optional solvent;
an optional 3-1-st step of preparing an intermediate (13') of the following Formulae (A13') to (K13') by reacting one of the above intermediate (13) with a peroxide in the presence of an optional base and an optional solvent; and
a fourth step of reacting one of the above intermediate (13) or one of the above intermediate (13') with alkoxysilane of the following Formula B2 in the presence of a metal catalyst and an optional solvent; 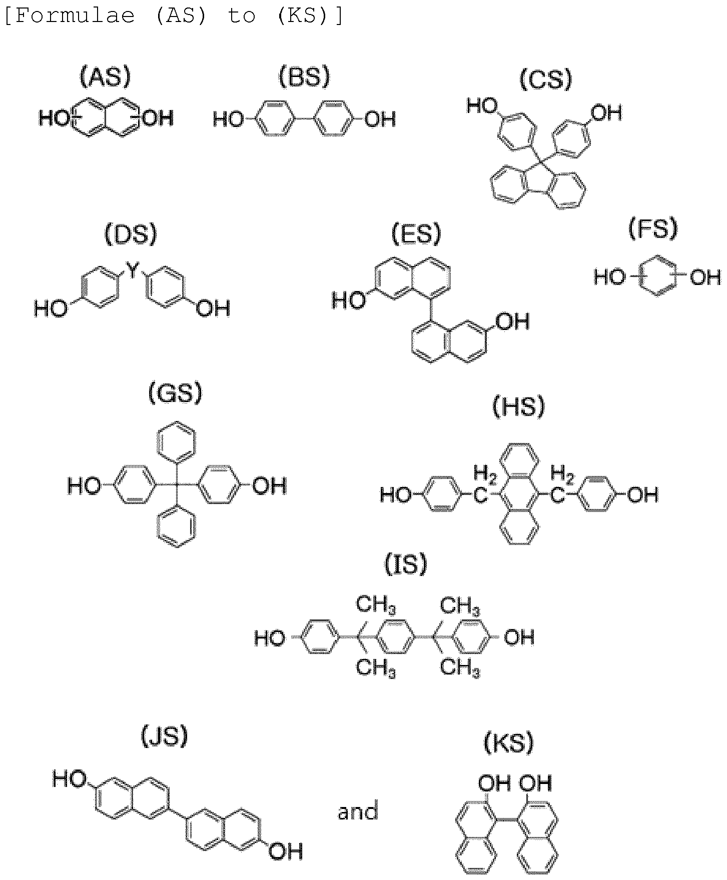 in the above Formula DS, Y is -CH₂-, -C(CH₃)₂-, -C(CF₃)₂-, -S- or -SO₂-, 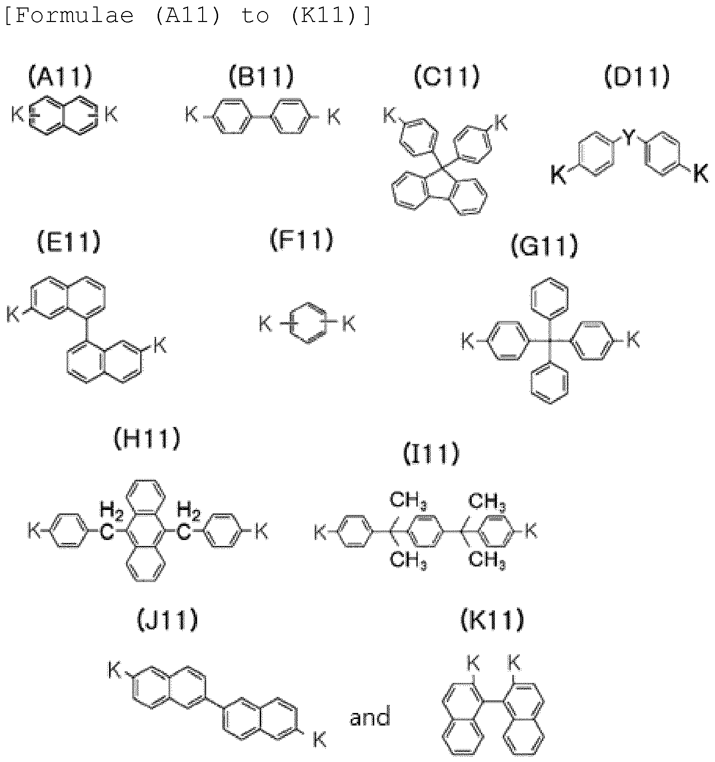 in the above Formulae A11 to K11, at least one of K is -O-CH₂-CRₐ=CR_{b}R_{c}, where Rₐ, R_{b} and R_{C} are independently H or an alkyl group having 1 to 6 carbon atoms, and the alkyl group may be a linear chain or a branched chain alkyl group, and the remainder thereof are hydroxyl groups,
in the above Formula D11, Y is -CH₂-, -C(CH₃)₂-, -C(CF₃)₂-, -S- or -SO₂-, 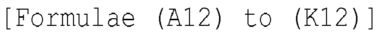 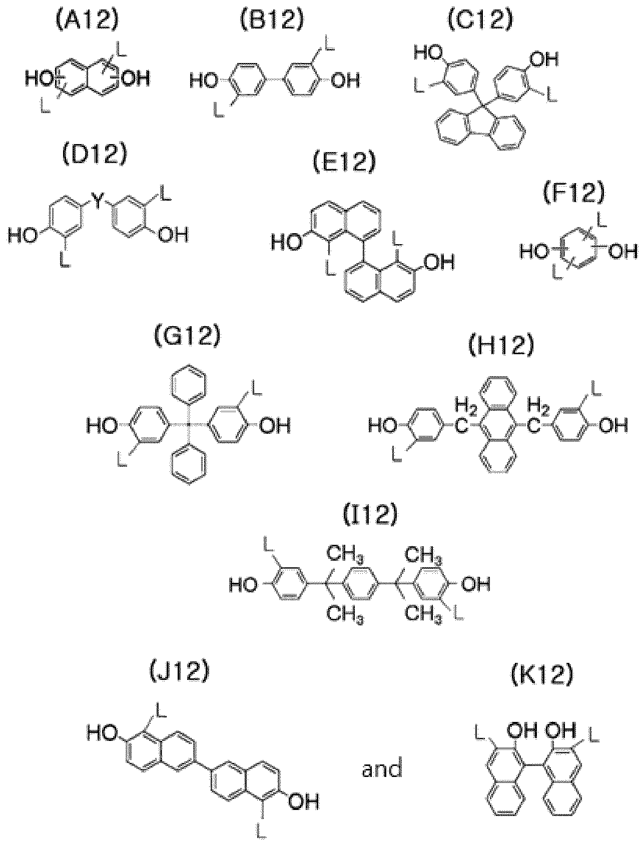 in the above Formulae A12 to K12, at least one of L is -CR_{b}R_{c}-CRₐ=CH₂, where Rₐ, R_{b} and R_{C} are independently H or an alkyl group having 1 to 6 carbon atoms, and the alkyl group may be a linear chain or a branched chain alkyl group, and the remainder thereof are hydrogen,
in the above Formula D12, Y is -CH₂-, -C(CH₃)₂-, -C(CF₃)₂-, -S- or -SO₂-, 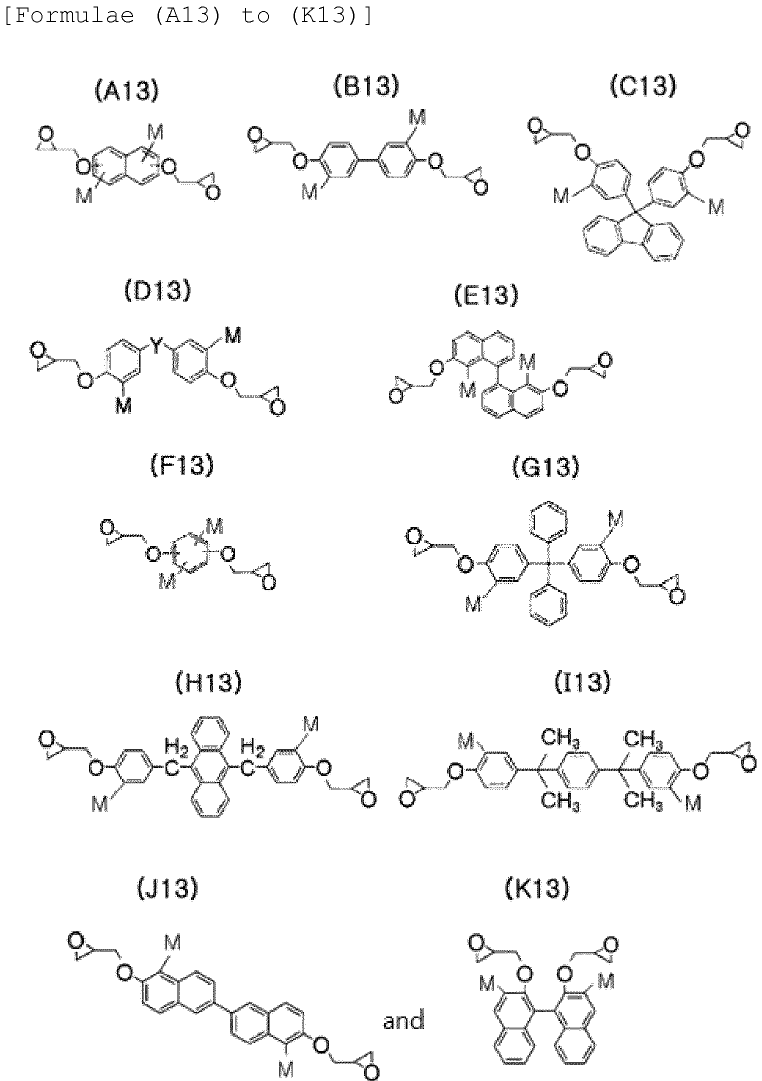 in the above Formulae A13 to K13, at least one of M is -CR_{b}R_{c}-CRₐ=CH₂, where Rₐ, R_{b} and R_{C} are independently H or an alkyl group having 1 to 6 carbon atoms, and the alkyl group may be a linear chain or a branched chain alkyl group, and the remainder thereof are hydrogen atoms,
in the above Formula D13, Y is -CH₂-, -C(CH₃)₂-, -C(CF₃)₂-, -S- or -SO₂-, 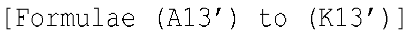 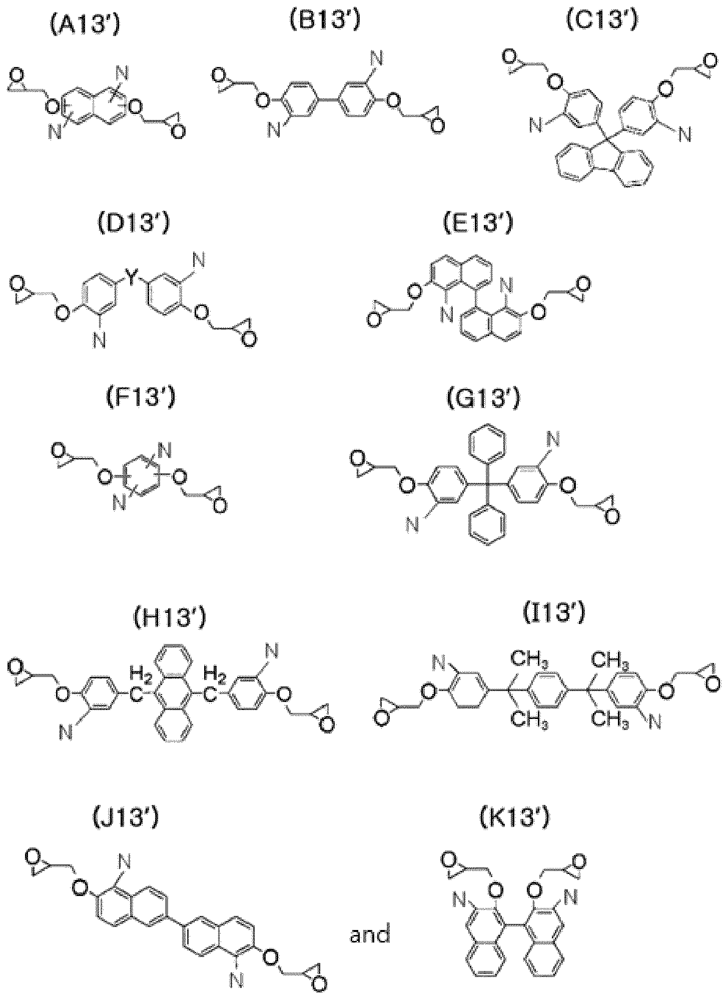 in the above Formulae A13' to K13', one of N is -CR_{b}R_{c}-CRₐ=CH₂, where Rₐ, R_{b} and R_{C} are independently H or an alkyl group having 1 to 6 carbon atoms, and the alkyl group may be a linear chain or a branched chain alkyl group, and the remainder thereof is the following Formula S3,
in the above Formula D13' , Y is -CH₂-, -C (CH₃)₂-, -C(CF₃)₂-, -S- or -SO₂-, 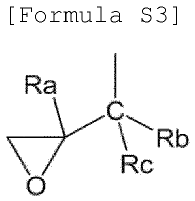 in the above Formula S3, Rₐ, R_{b} and R_{C} are independently H or an alkyl group having 1 to 6 carbon atoms, and the alkyl group may be a linear chain or a branched chain alkyl group, 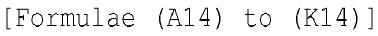 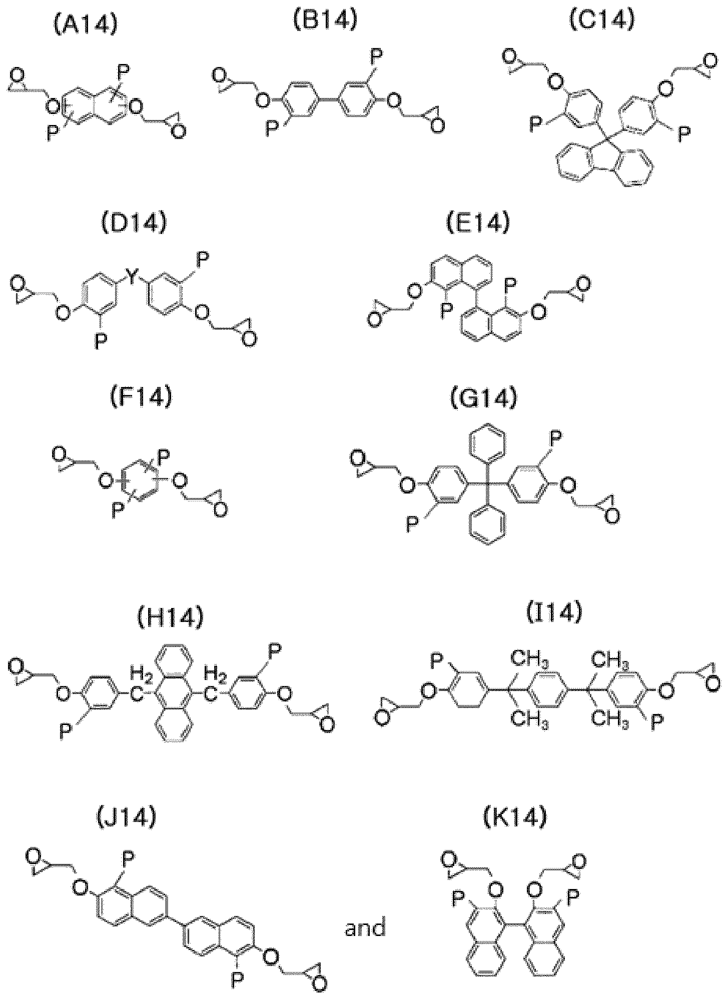 in the above Formulae A14 to K14, at least one of P is the following Formula S1, and the remainder thereof are the form of the following Formula S3, hydrogen or -CR_{b}R_{c}-CRₐ=CH₂, where Rₐ, R_{b} and R_{C} are independently H or an alkyl group having 1 to 6 carbon atoms, and the alkyl group may be a linear chain or a branched chain alkyl group,
in the above Formula D14, Y is -CH₂-, -C(CH₃)₂-, -C(CF₃)₂-, -S- or -SO₂-,
[Formula S1] -CR_{b}R_{c}-CHRₐ-CH₂-SiR₁R₂R₃
in Formula S1, Rₐ, R_{b} and R_{c} are independently H, or an alkyl group having 1 to 6 carbon atoms, at least one of R₁ to R₃ is an alkoxy group having 1 to 6 carbon atoms, and the remainder thereof are alkyl groups having 1 to 10 carbon atoms, while the alkyl group and the alkoxy group may be a linear chain or a branched chain alkyl group or alkoxy group.
in the case in which Formula F14 includes one instance of Formula S1, a compound in which all of Rₐ, R_{b} and R_{c} in the above Formula S1 are hydrogen, and R₁ to R₃ are alkoxy groups having 1 to 6 carbon atoms is excluded, 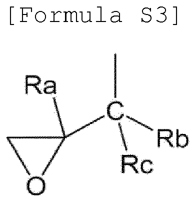 in the above Formula S3, Rₐ, R_{b} and R_{C} are independently H or an alkyl group having 1 to 6 carbon atoms, and the alkyl group may be a linear chain or a branched chain alkyl group, 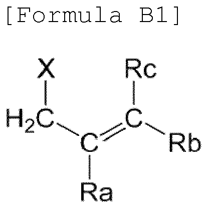 in the above Formula B1, X is Cl, Br, I, -O-SO₂-CH₃, -O-SO₂-CF₃, or -O-SO₂-C₆H₄-CH₃, Rₐ, R_{b} and R_{C} are independently H or an alkyl group having 1 to 6 carbon atoms, and the alkyl group may be a linear chain or a branched chain alkyl group,
[Formula B2] HSiR₁R₂R₃
in the above Formula B2, at least one of R₁ to R₃ is an alkoxy group having 1 to 6 carbon atoms, and the remainder thereof are alkyl groups having 1 to 10 carbon atoms, while the alkyl group and the alkoxy group may be a linear chain or a branched chain alkyl group or alkoxy group.

48. A method of preparing an epoxy compound containing an alkoxysilyl group of the following Formulae (A26) to (J26) comprising:
a first step of preparing an intermediate (11) of the following Formulae (A11) to (J11) by reacting one starting material of the following Formulae (AS) to (JS) with an allyl compound of the following Formula B1 in the presence of a base and an optional solvent;
a second step of preparing an intermediate (12) of the following Formulae (A12) to (J12) by irradiating electromagnetic waves onto one of the above intermediate (11) in the presence of an optional solvent;
a 2-1-st step of preparing an intermediate (23) of the following Formulae (A23) to (J23) by reacting one of the above intermediate (12) with an allyl compound of the following Formula B1 in the presence of a base and an optional solvent;
a 2-2-nd step of preparing an intermediate (24) of the following Formulae (A24) to (J24) by irradiating electromagnetic waves onto the above intermediate (23) in the presence of an optional solvent;
a third step of preparing an intermediate (25) of the following Formulae (A25) to (J25) by reacting one of the above intermediate (24) with epichlorohydrin in the presence of a base and an optional solvent;
an optional 3-1-st step of preparing an intermediate (25') of the following Formulae (A25') to (K25') by reacting one of the above intermediate (25) with a peroxide in the presence of an optional base and an optional solvent; and
a fourth step of reacting one of the above intermediate (25) or one of the above intermediate (25') with an alkoxysilane of the following Formula B2 in the presence of a metal catalyst and an optional solvent; 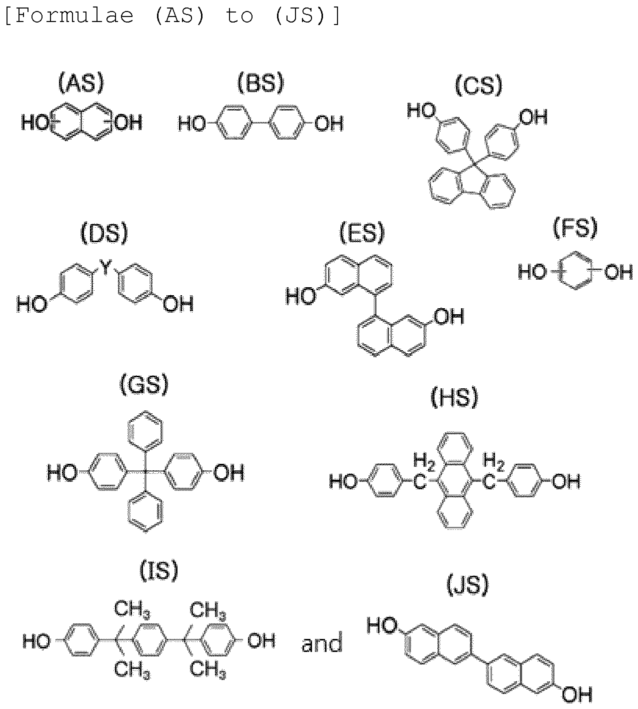 in the above Formula DS, Y is -CH₂-, -C(CH₃)₂-, -C(CF₃)₂-, -S- or -SO₂-, 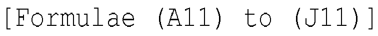 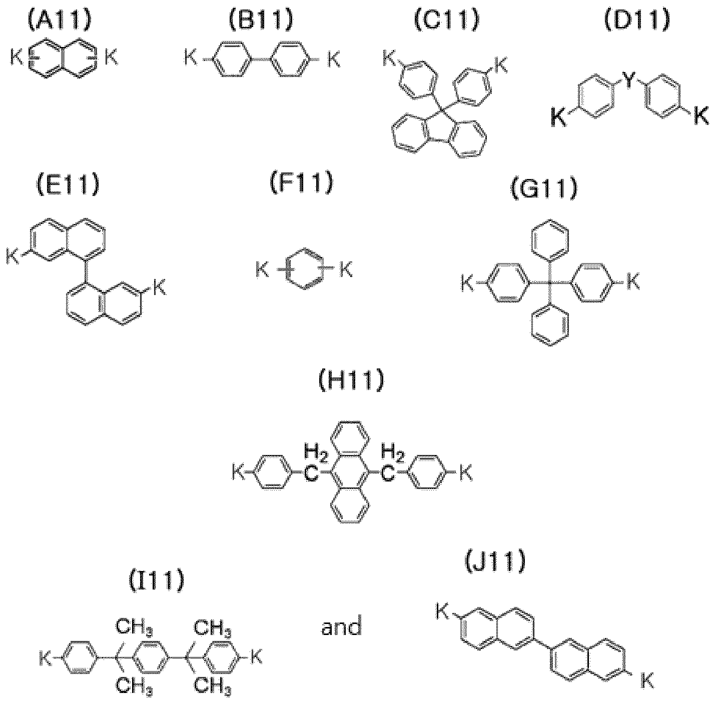 in the above Formulae A11 to J11, at least one of K is -O-CH₂-CRₐ=CR_{b}R_{c}, where Rₐ, R_{b} and R_{C} are independently H or an alkyl group having 1 to 6 carbon atoms, and the alkyl group may be a linear chain or a branched chain alkyl group, and the remainder thereof are hydroxyl groups,
in the above Formula D11, Y is -CH₂-, -C(CH₃)₂-, -C(CF₃)₂-, -S- or -SO₂-, 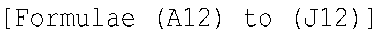 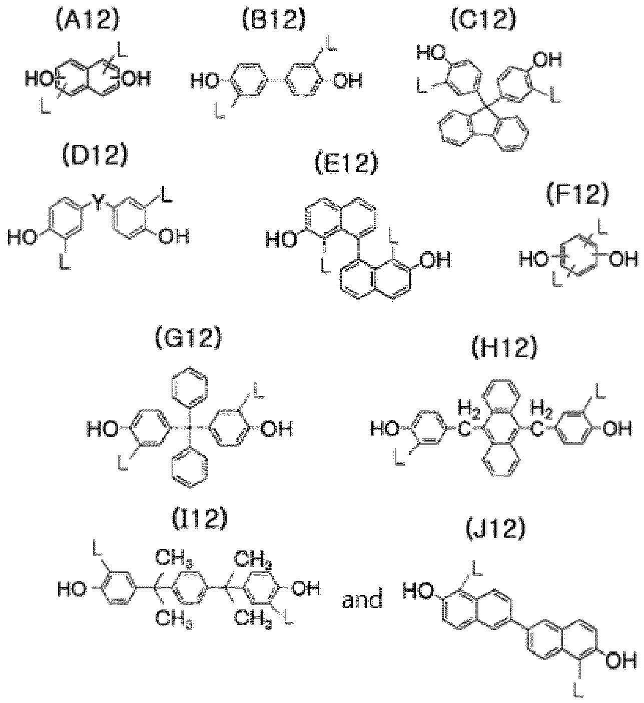 in the above Formulae A12 to J12, at least one of L is -CR_{b}R_{c}-CRₐ=CH₂, where Rₐ, R_{b} and R_{C} are independently H or an alkyl group having 1 to 6 carbon atoms, and the alkyl group may be a linear chain or a branched chain alkyl group, and the remainder thereof are hydrogen atoms,
in the above Formula D12, Y is -CH₂-, -C(CH₃)₂-, -C(CF₃)₂-, -S- or -SO₂-, 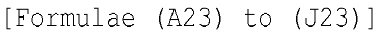 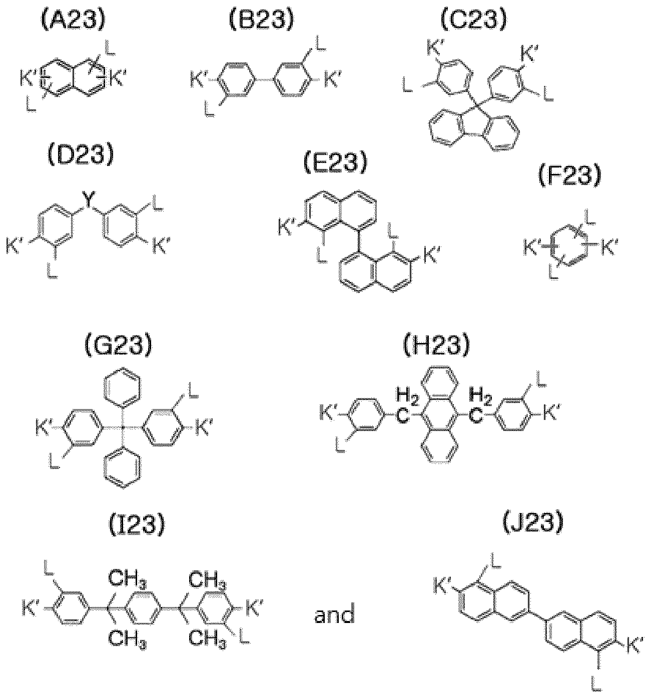 in the above Formulae A23 to J23, at least one of K' is -O-CH₂-CRₐ=CR_{b}R_{c}, where Rₐ, R_{b} and R_{C} are independently H or an alkyl group having 1 to 6 carbon atoms, and the alkyl group may be a linear chain or a branched chain alkyl group, and the remainder thereof are hydroxyl groups, and at least one of L is -CR_{b}R_{c}-CRₐ=CH₂, where Rₐ, R_{b} and R_{C} are independently H or an alkyl group having 1 to 6 carbon atoms, and the alkyl group may be a linear chain or a branched chain alkyl group, and the remainder thereof are hydrogen atoms,
in the above Formula D23, Y is -CH₂-, -C(CH₃)₂-, -C(CF₃)₂-, -S- or -SO₂-, 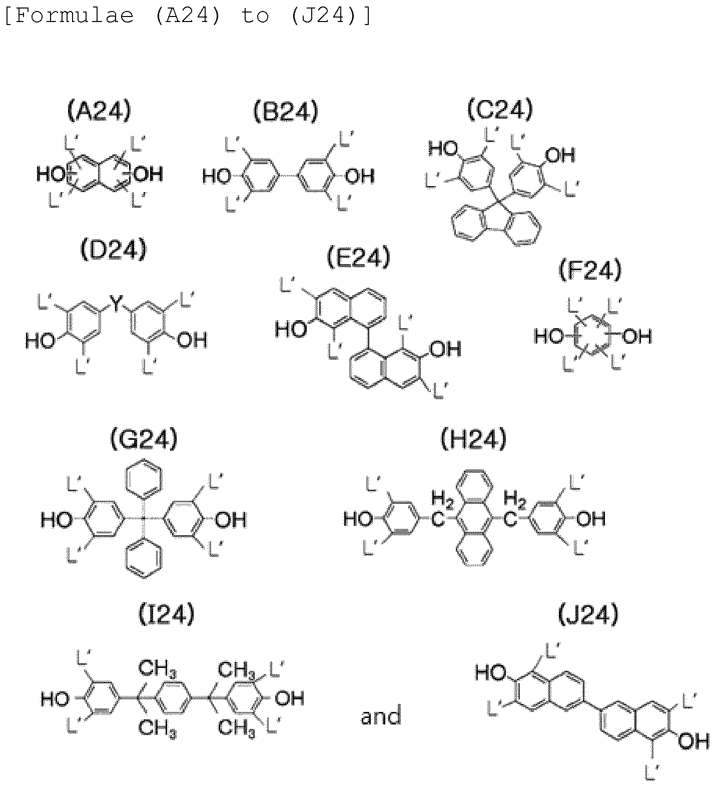 in the above Formulae A24 to J24, at least two of a plurality of L' are -CR_{b}R_{c}-CRₐ=CH₂, where Rₐ, R_{b} and R_{c} are independently H or an alkyl group having 1 to 6 carbon atoms, and the alkyl group may be a linear chain or a branched chain alkyl group, and the remainder thereof are hydrogen atoms,
in the above Formula D24, Y is -CH₂-, -C(CH₃)₂-, -C(CF₃)₂-, -S- or -SO₂-, 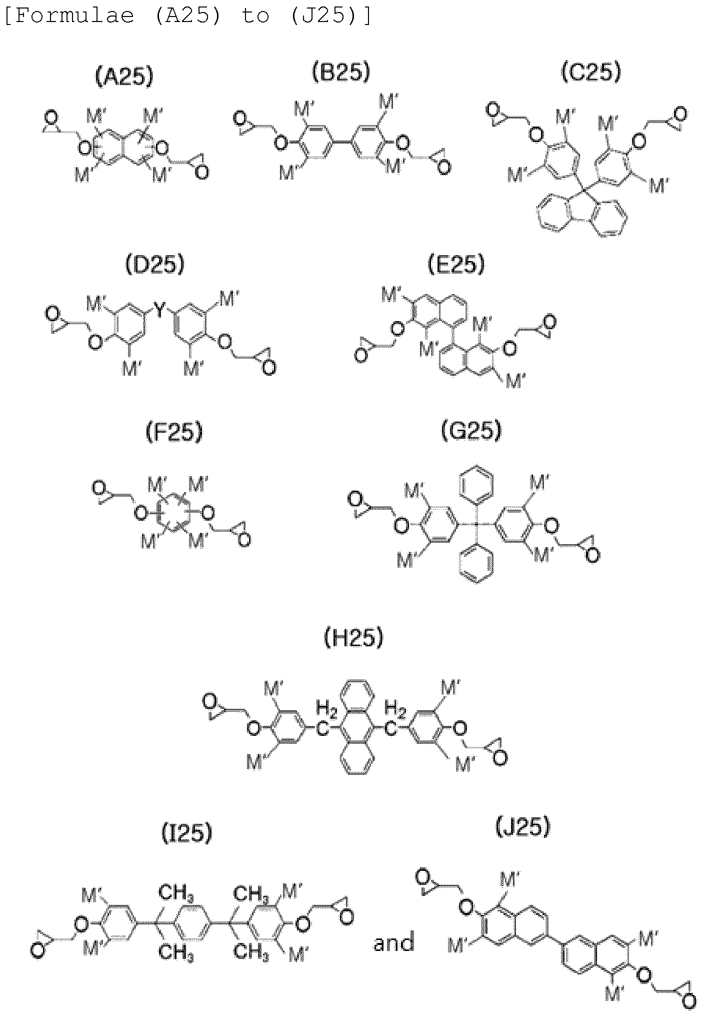 in the above Formulae A25 to J25, at least two of a plurality of M' are -CR_{b}R_{c}-CRₐ=CH₂, where Rₐ, R_{b} and R_{c} are independently H or an alkyl group having 1 to 6 carbon atoms, and the alkyl group may be a linear chain or a branched chain alkyl group, and the remainder thereof are hydrogen atoms,
in the above Formula D25, Y is -CH₂-, -C(CH₃)₂-, -C(CF₃)₂-, -S- or -SO₂-, 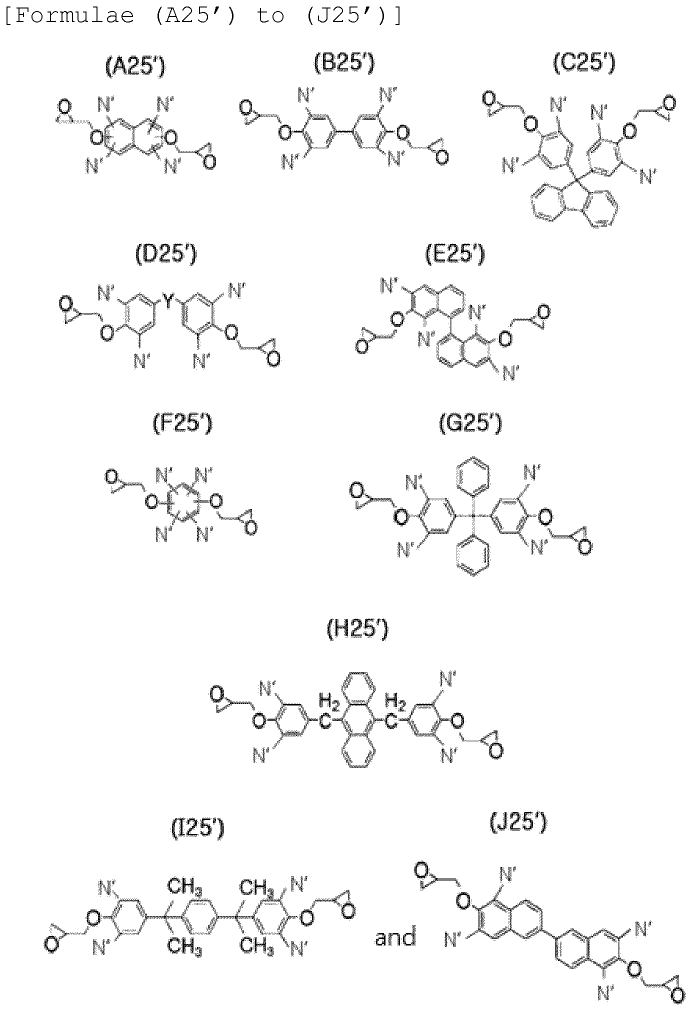 in the above Formulae A25' to J25', one to three of a plurality of N' are -CR_{b}R_{c}-CRₐ=CH₂, where Rₐ, R_{b} and R_{c} are independently H or an alkyl group having 1 to 6 carbon atoms, and the alkyl group may be a linear chain or a branched chain alkyl group, one to three thereof are the form of the following Formula S3, and the remainder thereof are hydrogen atoms,
in the above Formula D25' , Y is -CH₂-, -C (CH₃)₂-, -C (CF₃)₂-, -S- or -SO₂-, 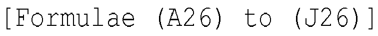 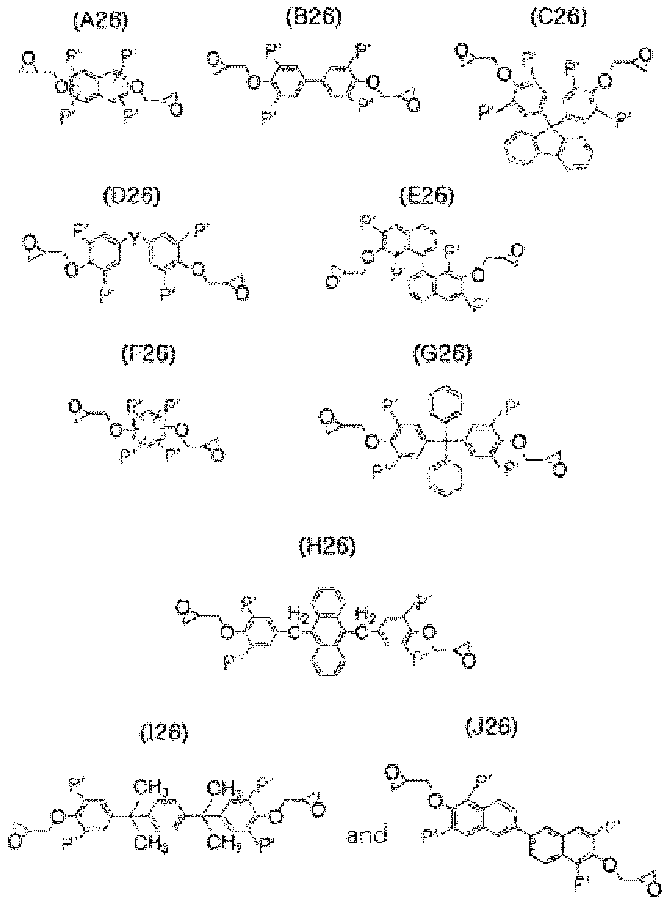 in the above Formulae A26 to J26, at least one of P' is the following Formula S1, and the remainder thereof are independently selected from the group consisting of the following Formula S3, hydrogen and -CR_{b}R_{c}-CRₐ=CH₂, where Rₐ, R_{b} and R_{c} are independently H or an alkyl group having 1 to 6 carbon atoms, and the alkyl group may be a linear chain or a branched chain alkyl group,
in the above Formula D26, Y is -CH₂-, -C(CH₃)₂-, -C(CF₃)₂-, -S- or -SO₂-,
[Formula S1] -CR_{b}R_{c}-CHRₐ-CH₂-SiR₁R₂R₃
in Formula S1, Rₐ, R_{b} and R_{c} are independently H, or an alkyl group having 1 to 6 carbon atoms, at least one of R₁ to R₃ is an alkoxy group having 1 to 6 carbon atoms, and the remainder thereof are alkyl groups having 1 to 10 carbon atoms, while the alkyl group and the alkoxy group may be a linear chain or a branched chain alkyl group or alkoxy group,
in the case in which Formula F26 includes one instance of Formula S1, a compound in which all of Rₐ, R_{b} and R_{c} in the above Formula S1 are hydrogen, and R₁ to R₃ are alkoxy groups having 1 to 6 carbon atoms is excluded, 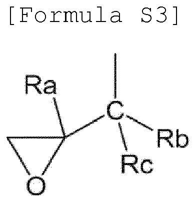 in the above Formula S3, Rₐ, R_{b} and R_{c} are independently H or an alkyl group having 1 to 6 carbon atoms, and the alkyl group may be a linear chain or a branched chain alkyl group, 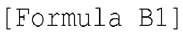 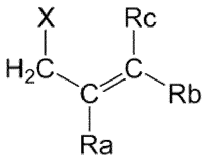 in the above Formula B1, X is Cl, Br, I, -O-SO₂-CH₃, -O-SO₂-CF₃, or -O-SO₂-C₆H₄-CH₃, Rₐ, R_{b} and R_{c} are independently H or an alkyl group having 1 to 6 carbon atoms, and the alkyl group may be a linear chain or a branched chain alkyl group,
[Formula B2] HSiR₁R₂R₃
in the above Formula B2, at least one of R₁ to R₃ is an alkoxy group having 1 to 6 carbon atoms, and the remainder thereof are alkyl groups having 1 to 10 carbon atoms, while the alkyl group and the alkoxy group may be a linear chain or a branched chain alkyl group or alkoxy group.

49. The method of preparing an epoxy compound containing an alkoxysilyl group of claim 47 or 48, wherein 0.5 to 10 equivalents of an allyl group of the allyl compound of the above Formula B1 react with respect to 1 equivalent of a hydroxyl group of the starting material in the first step.

50. The method of preparing an epoxy compound containing an alkoxysilyl group of claim 47 or 48, wherein the first step is performed at a temperature of from room temperature to 100°C for 1 to 120 hours.

51. The method of preparing an epoxy compound containing an alkoxysilyl group of claim 47 or 48, wherein the base in the first step is at least one selected from the group consisting of KOH, NaOH, K₂CO₃, Na₂CO₃, KHCO₃, NaHCO₃, NaH, triethylamine and diisopropylamine.

52. The method of preparing an epoxy compound containing an alkoxysilyl group of claim 47 or 48, wherein the solvent in the first step is at least one selected from the group consisting of acetonitrile, tetrahydrofuran, methyl ethyl ketone, dimethylformamide, dimethyl sulfoxide and methylene chloride.

53. The method of preparing an epoxy compound containing an alkoxysilyl group of claim 47 or 48, wherein the second step is performed at a temperature from 120°C to 250°C for 1 to 1,000 minutes.

54. The method of preparing an epoxy compound containing an alkoxysilyl group of claim 47 or 48, wherein the solvent in the second step is at least one selected from the group consisting of xylene, 1,2-dichlorobenzene and N,N-diethylaniline.

55. The method of preparing an epoxy compound containing an alkoxysilyl group of claim 47 or 48, wherein the electromagnetic waves in the second step is microwaves.

56. The method of preparing an epoxy compound containing an alkoxysilyl group of claim 48, wherein 0.5 to 10 equivalents of an allyl group of the allyl compound of the above Formula B1 react with respect to 1 equivalent of a hydroxyl group of the intermediate (12) in the 2-1-st step.

57. The method of preparing an epoxy compound containing an alkoxysilyl group of claim 48, wherein the 2-1-st step is performed at a temperature of from room temperature to 100°C for 1 to 120 hours.

58. The method of preparing an epoxy compound containing an alkoxysilyl group of claim 48, wherein the base in the 2-1-st step is at least one selected from the group consisting of KOH, NaOH, K₂CO₃, Na₂CO₃, KHCO₃, NaHCO₃, NaH, triethylamine and diisopropylamine.

59. The method of preparing an epoxy compound containing an alkoxysilyl group of claim 48, wherein the solvent in the 2-1-st step is at least one selected from the group consisting of acetonitrile, tetrahydrofuran, methyl ethyl ketone, dimethylformamide, dimethyl sulfoxide and methylene chloride.

60. The method of preparing an epoxy compound containing an alkoxysilyl group of claim 48, wherein the 2-2-nd step is performed at a temperature from 120°C to 250°C for 1 to 1,000 minutes.

61. The method of preparing an epoxy compound containing an alkoxysilyl group of claim 48, wherein the solvent in the 2-2-nd step is at least one selected from the group consisting of xylene, 1,2-dichlorobenzene and N,N-diethylaniline.

62. The method of preparing an epoxy compound containing an alkoxysilyl group of claim 48, wherein the electromagnetic waves in the 2-2-nd step are microwaves.

63. The method of preparing an epoxy compound containing an alkoxysilyl group of claim 47 or 48, wherein 1 to 10 equivalents of a glycidyl group of the epichlorohydrin react with respect to 1 equivalent of a hydroxyl group of the intermediate (12) or intermediate (24) in the third step.

64. The method of preparing an epoxy compound containing an alkoxysilyl group of claim 47 or 48, wherein the third step is performed at a temperature of from room temperature to 100°C for 1 to 120 hours.

65. The method of preparing an epoxy compound containing an alkoxysilyl group of claim 47 or 48, wherein the base in the third step is at least one selected from the group consisting of KOH, NaOH, K₂CO₃, Na₂CO₃, KHCO₃, NaHCO₃, NaH, triethylamine and diisopropylamine.

66. The method of preparing an epoxy compound containing an alkoxysilyl group of claim 47 or 48, wherein the solvent in the third step is at least one selected from the group consisting of acetonitrile, tetrahydrofuran, methyl ethyl ketone, dimethylformamide, dimethyl sulfoxide, methylene chloride, and H₂O.

67. The method of preparing an epoxy compound containing an alkoxysilyl group of claim 47 or 48, wherein 1 to 10 equivalents of a peroxide group of the peroxide react with respect to 1 equivalent of an allyl group of the intermediate (13) or intermediate (25) in the 3-1-st step.

68. The method of preparing an epoxy compound containing an alkoxysilyl group of claim 47 or 48, wherein the peroxide in the 3-1-st step is at least one selected from the group consisting of meta-chloroperoxybenzoic acid (m-CPBA), H₂O₂ and dimethyldioxirane (DMDO).

69. The method of preparing an epoxy compound containing an alkoxysilyl group of claim 47 or 48, wherein the 3-1-st step is performed at a temperature of from room temperature to 100°C for 1 to 120 hours.

70. The method of preparing an epoxy compound containing an alkoxysilyl group of claim 47 or 48, wherein the solvent in the 3-1-st step is at least one selected from the group consisting of acetonitrile, tetrahydrofuran, methyl ethyl ketone, dimethylformamide, dimethyl sulfoxide, methylene chloride, and H₂O.

71. The method of preparing an epoxy compound containing an alkoxysilyl group of claim 47 or 48, wherein the base in the 3-1-st step is selected from the group consisting of KOH, NaOH, K₂CO₃, KHCO₃, triethylamine and diisopropylamine.

72. The method of preparing an epoxy compound having an alkoxysilyl group of claim 47 or 48, wherein 1 to 5 equivalents of an alkoxysilane of the above Formula B2 react with respect to 1 equivalent of an allyl group of the intermediate (15'), intermediate (25) or intermediate (25') in the fourth step.

73. The method of preparing an epoxy compound having an alkoxysilyl group of claim 47 or 48, wherein the fourth step is performed at a temperature of from room temperature to 120°C for 1 to 72 hours.

74. The method of preparing an epoxy compound having an alkoxysilyl group of claim 47 or 48, wherein the metal catalyst in the fourth step includes PtO₂ or H₂PtCl₆.

75. The method of preparing an epoxy compound having an alkoxysilyl group of claim 47 or 48, wherein , the solvent in the fourth step is at least one selected from the group consisting of toluene, acetonitrile, tetrahydrofuran, methyl ethyl ketone, dimethylformamide, dimethyl sulfoxide, and methylene chloride.
